# EUROPEAN PATENT APPLICATION

(11) **EP 3 816 163 A1**
(43) Date of publication of application: **05.05.2021**
(21) Application number: 19826858.3
(22) Date of filing: 25.06.2019
(51) Int. Cl.: C07D 403/12, C07D 413/12, C07D 471/04, C07D 487/04, C07D 491/04, C07D 495/04, C07D 498/04, A61K 31/55, A61P 1/00, A61P 11/00

(54) **CELL NECROSIS INHIBITOR, PREPARATION METHOD THEREFOR AND USE THEREOF**

(30) Priority: 26.06.2018 CN 201810676651; 14.06.2019 CN 201910517015
(71) Applicant: Shanghai Institute of Organic Chemistry, Chinese Academy of Sciences, Shanghai 200032 (CN)
(72) Inventor: MA, Dawei, Shanghai 200032 (CN); JI, Yuhua, Shanghai 201210 (CN); HE, Weiming, Shanghai 200032 (CN); FANG, Chao, Shanghai 200032 (CN); ZHAO, Jinlong, Shanghai 200032 (CN); WANG, Kailiang, Shanghai 200032 (CN); XIA, Shanghua, Shanghai 200032 (CN); LI, Zheng, Shanghai 200032 (CN); LI, Ying, Shanghai 200032 (CN)
(74) Representative: Lahrtz, Fritz
(86) International application number: PCT/CN2019/092705
(87) International publication number: WO 2020/001420

(57) **Abstract**

Provided by the present application are a cell necrosis inhibitor, a preparation method therefor and a use thereof; in particular, provided by the present application is an inhibitor of cell necrosis and/or human receptor-interacting protein 1 kinase (RIP1). The inhibitor has a structure as shown in the following formula I. The compound and a composition comprising same may be used to prevent and/or treat diseases involving cell death and/or inflammation.

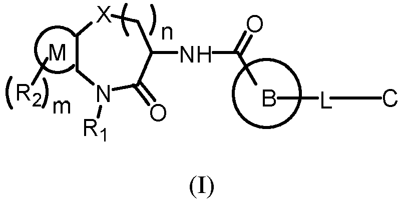

## Description

### FIELD OF THE APPLICATION

This application relates to a compound that inhibits cell necrosis and/or receptor interacting protein 1 (RIP1) kinase, and preparation method and use thereof. The compound of the present application and the composition comprising the same can be used in methods for preventing and/or treating diseases involving cell death and/or inflammation.

### BACKGROUND

Programmed necrotic cell death, also known as programmed necrosis, is a new way of cell death discovered in recent years. Programmed necrosis is a highly inflammatory form of cell death and is regarded as an important pathological factor in many degenerative and inflammatory diseases, which include neurodegenerative diseases, stroke, coronary heart disease, myocardial infarction, retinal degenerative diseases, inflammatory bowel disease, kidney disease, liver disease, and many other related diseases.

The activation of nuclear factor kappa B (NF-κB) induced by tumor necrosis factor alpha (TNF-α) plays a critical role in the immune system and inflammatory response. RIP1 is a multifunctional signal transducer involved in mediating NF-κB activation, apoptosis and cell necrosis, and is a crossover point that determines the cell death, thereby playing an important role in processes such as cell survival and apoptosis or programmed necrosis and the like. The activity of RIP1 kinase critically participates in mediating programmed cell necrosis, a necrotic cell death pathway independent of caspase.

Studies show that Necrostatin-1 (Nec-1), a RIP1 kinase inhibitor known in the art, exhibits effective therapeutic effects in a variety of inflammatory diseases. Later, some RIP1 kinase inhibitors with different structures were discovered in the art. However, the existing RIP1 kinase inhibitors have defects in different aspects, such as unsatisfactory activity, poor pharmacokinetic properties, or low oral bioavailability etc., and some cannot pass through the blood-brain barrier to enter into the central nervous system. All these shortcomings impede the further research and clinical application for them.

Therefore, there is still a need in the art to provide highly effective and selective small molecule RIP1 kinase inhibitors with clinical value to block RIP1-dependent programmed cell necrosis, and further to prevent and treat diseases or disorders mediated by RIP1 kinase or diseases or disorders caused by programmed cell necrosis.

### SUMMARY

The present application provides a new RIP1 kinase inhibitor, which can be used to prevent and treat diseases or disorders mediated by RIP1 kinase or diseases or disorders caused by programmed cell necrosis.

In one aspect, the present application provides a compound of Formula (I): 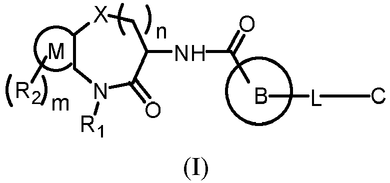 or a pharmaceutically acceptable salt thereof, wherein:
X is O, S or CH₂;
ring M has a structure of 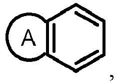 wherein ring A is selected from the group consisting of substituted or unsubstituted 5- to 6-membered heteroaryl and substituted or unsubstituted 5-to 6-membered heterocyclyl;
ring B is selected from the group consisting of substituted or unsubstituted 5- to 12-membered aryl, substituted or unsubstituted 5- to 12-membered heteroaryl, and substituted or unsubstituted 5- to 12-membered heterocyclyl;
C is selected from the group consisting of substituted or unsubstituted (C₃-C₁₂) cycloalkyl, substituted or unsubstituted 5- to 12-membered aryl, substituted or unsubstituted 5- to 12-membered heteroaryl, and substituted or unsubstituted 5- to 12-membered heterocyclyl;
L is selected from the group consisting of O, S, NH, N(CH₃), substituted or unsubstituted C₁-C₆ alkylene-O-, substituted or unsubstituted C₁-C₆ alkylene-NH-, (substituted or unsubstituted C₁-C₆ alkylene)₂-N-, substituted or unsubstituted C₁-C₆ alkylene, substituted or unsubstituted C₃-C₆ alkenylene, and substituted or unsubstituted C₃-C₆ alkenylene-O-;
R₁ is selected from the group consisting of H and substituted or unsubstituted C₁-C₆ alkyl; R₂ is selected from the group consisting of H, halo, hydroxyl, oxy, benzyl, substituted or unsubstituted C₁-C₆ alkyl, substituted or unsubstituted C₁-C₆ alkoxy, and C₁-C₆ acyl;
m is 0, 1, 2 or 3;
n is 1, 2 or 3;
wherein "substituted" refers to the substitution of one or more hydrogen atoms on the group with a substituent selected from the group consisting of halo, cyano, alkyl, acyl, sulfonyl, hydroxyl, amino, benzyl, oxy, (C₁-C₄) alkyl, halo(C₁-C₄) alkyl, (C₁-C₄)alkoxy, halo(C₁-C₄)alkoxy, nitro, and (C₁-C₄)alkylC(O)-.

In another aspect, the present application provides a method for preparing a compound of Formula (I): 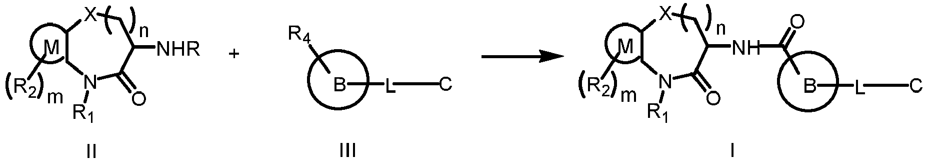 wherein R₄ is -COOH or -COO⁻G⁺, in which G⁺ is an alkali metal ion;
when R is H, the method comprises: reacting a compound of Formula (II) with a compound of Formula (III) in an inert solvent in the presence of a condensation reagent and a base, to obtain the compound of Formula (I); and
when R is an amino protecting group, the method comprises: removing R from the compound of Formula (II) under an acidic condition, and then reacting the compound of Formula (II) from which R is removed with the compound of Formula (III) in an inert solvent in the presence of a condensation reagent and a base, to obtain the compound of Formula (I).

In yet another aspect, the present application provides a pharmaceutical composition comprising a therapeutically effective amount of a compound of Formula (I) or a pharmaceutically acceptable salt thereof and a pharmaceutically acceptable carrier.

In another aspect, the present application provides use of the compound of Formula (I) or a pharmaceutically acceptable salt thereof, or a pharmaceutical composition comprising the same in the manufacture of medicaments for treating or preventing diseases or disorders mediated by RIP1 kinase or diseases or disorders caused by programmed cell necrosis.

In another aspect, the present application provides a method for inhibiting RIP1 kinase in a subject, comprising administering to the subject an effective amount of a compound of Formula (I) or a pharmaceutically acceptable salt thereof, or pharmaceutical composition comprising the same.

In yet another aspect, the present application provides a combination of drugs comprising (a) a compound of Formula (I) or a pharmaceutically acceptable salt thereof; and (b) at least one additional active agent.

In a further aspect, the present application provides an intermediate compound of Formula (II): 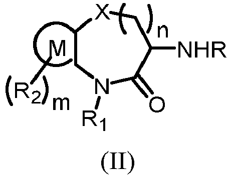 wherein:
R is H or an amino protecting group;
X is O, S or CH₂;
R₁ is selected from the group consisting of H and substituted or unsubstituted C₁-C₆ alkyl; R₂ is selected from the group consisting of H, halo, hydroxyl, oxy, benzyl, substituted or unsubstituted C₁-C₆ alkyl, substituted or unsubstituted C₁-C₆alkoxy, and C₁-C₆ acyl;
m is 0, 1, 2 or 3; and
n is 1, 2 or 3;
wherein "substituted" refers to the substitution of one or more hydrogen atoms on the group with a substituent selected from the group consisting of halo, cyano, alkyl, acyl, sulfonyl, hydroxyl, amino, benzyl, oxy, (C₁-C₄) alkyl, halo(C₁-C₄) alkyl, (C₁- C₄)alkoxy, halo(C₁-C₄)alkoxy, nitro, and (C₁-C₄)alkylC(O)-.

### BRIEF DESCRIPTION OF THE DRAWINGS

Fig. 1 shows the body temperature of mice as a function of time after administration of different doses (10 mg/kg, 20 mg/kg, and 30 mg/kg) of RIP1-034 to a mouse model of TNF-α-induced lethal shock.
Fig. 2 shows the plasma concentration of each mouse as a function of time after a single oral administration (10 mg/kg) of Compound RIP1-034 of the present application.
Fig. 3 shows the average plasma concentration of mice as a function of time after a single oral administration (10 mg/kg) of the compound RIP1-034 of the present application.

### DETAILED DESCRIPTION

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the claims. One skilled in the art will recognize many methods and materials similar or equivalent to those described herein, which could be used in the practice of the present invention. The present invention is in no way limited to the methods and materials described. In the event that one or more of the incorporated literature and similar materials differs from or contradicts this application, including but not limited to defined terms, term usage, described techniques, or the like, this application controls.

It is appreciated that certain features of the present disclosure, which are, for clarity, described in the context of separate embodiments, can also be provided in combination in a single embodiment. Conversely, various features of the present disclosure, which are, for brevity, described in the context of a single embodiment, can also be provided separately or in any suitable sub-combination.

### Definitions

As used herein, unless otherwise stated, the following definitions are used. For the purposes of this application, the chemical elements are identified in accordance with the Periodic Table of the Elements, CAS version, Handbook of Chemistry and Physics, 75th Ed. In addition, general principles of organic chemistry and specific functional moieties and reactivity are described in "Organic Chemistry", Thomas Sorrell, University Science Books, Sausalito, 1999 and "March's Advanced Organic Chemistry", 5th Edition, Smith, M.B. and March, J., John Wiley & Sons, New York: 2001, which are incorporated herein by reference in their entireties.

Linking substituents are described herein. Where the structure clearly requires a linking group, the Markush variables listed for that group are understood to be linking groups. For example, if the structure requires a linking group and the Markush group definition for that variable lists "alkyl", then it is understood that the "alkyl" represents a linking alkylene group.

As used herein, the term "substituted", whether it is preceded by the term "optionally", means that the chemical group has one or more hydrogen atoms that is/are removed and replaced by suitable substituents. Unless otherwise specified, an "optionally substituted" group may have a suitable substituent at each substitutable position of the group, and when more than one position in any given structure can be substituted with more than one substituent selected from particular groups, the substituent may be the same or different at each position. The combinations of substituents contemplated by the present application are preferably those resulting in the formation of stable or chemically feasible compounds. The term "stable" as used herein refers to a compound that remains substantially unchanged when it is subjected to conditions that allow its production, detection, and in certain embodiments recovery and purification, and when it is used for one or more of the purposes disclosed herein. Unless specifically indicated as "unsubstituted", the chemical moiety described herein should be understood to include a substituent. For example, when referring to "aryl", it includes substituted aryl and unsubstituted aryl.

When a bond to a substituent is shown to cross a bond linking two atoms in a ring, then such substituent may be bonded to any atom in the ring. When a substituent is listed without indicating the atom via which such substituent is bonded to the rest of the compound of a given formula, then such substituent may be bonded via any atom in such formula. Combinations of substituents and/or variables are permissible, but only if such combinations result in stable compounds.

When any variable (such as Rⁱ) occurs more than one time in any constituent or formula of a compound, its definition at each occurrence is independent of each other. Thus, for example, if a group is shown to be substituted with 0-2 Rⁱ moieties, then the group may optionally be substituted with up to two Rⁱ moieties, and Rⁱ at each occurrence is independently selected from the definition of Rⁱ.

The term "Cᵢ₋ⱼ" as used herein indicates a range of carbon atom numbers, wherein i and j are integers and j is greater than i, and the range of the carbon atom numbers includes the endpoints (i.e., i and j) and each integer between the endpoints. For example, C₁₋₆ indicates a range of 1 to 6 carbon atoms, including 1 carbon atom, 2 carbon atoms, 3 carbon atoms, 4 carbon atoms, 5 carbon atoms and 6 carbon atoms. In some embodiments, the term "C₁₋₁₂" indicates 1 to 12, particularly 1 to 10, particularly 1 to 8, particularly 1 to 6, particularly 1 to 5, particularly 1 to 4, particularly 1 to 3, or particularly 1 to 2 carbon atoms.

As used herein, the term "hydrocarbon" refers to a group linked via a carbon atom having no =O or =S substituent, which generally has at least one carbon-hydrogen bond and a main carbon skeleton, and may optionally contain heteroatom(s). Therefore, the hydrocarbon group may include, but is not limited to, alkyl, alkenyl, alkynyl, aryl, heteroaryl, cycloalkyl, heterocyclyl, and the like.

As used herein, the term "alkyl", whether as part of another term or used independently, refers to a saturated linear or branched-chain hydrocarbon group. The term "Cᵢ₋ⱼ alkyl" refers to an alkyl having i to j carbon atoms. In some embodiments, the alkyl group contains 1 to 12 carbon atoms. In some embodiments, the alkyl group contains 1 to 11 carbon atoms, 1 to 10 carbon atoms, 1 to 9 carbon atoms, 1 to 8 carbon atoms, 1 to 7 carbon atoms, 1 to 6 carbon atoms, 1 to 5 carbon atoms, 1 to 4 carbon atoms, 1 to 3 carbon atoms, or 1 to 2 carbon atoms. Examples of alkyl include, but are not limited to, methyl, ethyl, 1-propyl (n-propyl), 2-propyl (isopropyl), 1-butyl (n-butyl), 2-methyl-1-propyl (isobutyl), 2-butyl (neobutyl), 2-methyl-2-propyl (tert-butyl), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, and the like. Examples of "C₁₋₁₂ alkyl" include, but are not limited to, methyl, ethyl, propyl, butyl, pentyl, hexyl, heptyl, octyl, nonyl, decyl, undecyl, and dodecyl. Examples of "C₁₋₆ alkyl" include, but are not limited to, methyl, ethyl, n-propyl, isopropyl, n-butyl, isobutyl, neobutyl, tert-butyl, n-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, and the like.

The term "halo" as used herein refers to F, Cl, Br or I.

The term "cyano" as used herein refers to -CN.

The term "hydroxyl" as used herein refers to -OH.

The term "amino" as used herein refers to -NH₂.

The term "nitro" as used herein refers to -NO₂.

The term "oxy" as used herein refers to an oxygen atom with a double bond to another atom (such as carbon or sulfur). For example, if it is directly linked to a carbon atom, a carbonyl group (C=O) is formed.

The term "acyl" as used herein refers to a functional group containing a carbonyl group, such as -C(=O)R', wherein R' is hydrogen or a hydrocarbon group. In some embodiments, acyl is a group represented by the formula alkylC(O)-.

The term "sulfonyl" as used herein refers to the -S(O)₂-R' group, wherein R' is a hydrocarbon group.

The term "haloalkyl" as used herein refers to an alkyl group substituted with one or more halogen atoms, wherein the one or more halogen atoms independently replace one or more hydrogen atoms on one or more carbon atoms of the alkyl group. For example, the term "C₁₋₆ haloalkyl" includes C₁₋₆ alkyl having 1 to 7, 1 to 6, 1 to 5, 1 to 4, 1 to 3, or 1 to 2 halogen atoms, and examples include, but are not limited to, chloromethyl, difluoromethyl, trichloromethyl, trifluoromethyl, 2-bromoethyl, 2,2,2-trifluoroethyl, tetrafluoroethyl, pentafluoroethyl, 2,2-difluoropropyl, 2,2,2-trifluoropropyl, 4,4,4-trifluorobutyl, 5,5,5-trifluoropentyl, and 6,6,6-trifluorohexyl, etc.

The term "alkenyl" as used herein, whether used as part of another term or used independently, refers to a linear or branched-chain hydrocarbon group having at least one carbon-carbon double bond, which may optionally be substituted independently with one or more substituents described herein, and includes radicals having "cis" and "trans" orientations, or "E" and "Z" orientations. In some embodiments, the alkenyl group contains 2 to 12 carbon atoms. In some embodiments, the alkenyl group contains 2 to 11 carbon atoms. In some embodiments, the alkenyl group contains 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, or 2 to 3 carbon atoms. In some embodiments, the alkenyl group contains 2 carbon atoms. Examples of alkenyl groups include, but are not limited to, ethenyl, propenyl, butenyl, pentenyl, 1-methyl-2-buten-1-yl, 5-hexenyl, and the like.

The term "alkynyl" as used herein, whether used as part of another term or used independently, refers to a linear or branched-chain hydrocarbon group having at least one carbon-carbon triple bond, which may optionally be substituted independently with one or more substituents described herein. In some embodiments, the alkynyl group contains 2 to 12 carbon atoms. In some embodiments, the alkynyl group contains 2 to 11 carbon atoms. In some embodiments, the alkynyl group contains 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, or 2 to 3 carbon atoms. In some embodiments, the alkynyl group contains 2 carbon atoms. Examples of alkynyl groups include, but are not limited to, ethynyl, 1-propynyl, 2-propynyl, and the like.

The term "alkylene" as used herein refers to a divalent alkyl group, the term "alkenylene" as used herein refers to a divalent alkenyl group, and the term "alkynylene" as used herein refers to a divalent alkynyl group.

As used herein, the term "alkoxy", whether used as part of another term or used independently, refers to an alkyl group as defined above attached to a parent molecule via an oxygen atom. The term "Cᵢ₋ⱼ alkoxy" refers to the alkyl moiety of an alkoxy group having i to j carbon atoms. In some embodiments, the alkoxy group contains 1 to 12 carbon atoms. In some embodiments, the alkoxy group contains 1 to 11 carbon atoms. In some embodiments, the alkoxy group contains 1 to 10 carbon atoms, 1 to 9 carbon atoms, 1 to 8 carbon atoms, 1 to 7 carbon atoms, 1 to 6 carbon atoms, 1 to 5 carbon atoms, 1 to 4 carbon atoms, 1 to 3 carbon atoms, or 1 to 2 carbon atoms. Examples of "C₁₋₁₂ alkoxy group" include, but are not limited to, methoxy, ethoxy, propoxy (e.g. n-propoxy and isopropoxy), tert-butoxy, neopentyloxy, and n-hexyloxy, and the like.

The term "haloalkoxy" as used herein refers to an alkoxy group substituted with one or more halogen atoms, wherein the one or more halogen atoms independently replace one or more hydrogen atoms on one or more carbon atoms of the alkoxy group. For example, the term "C₁₋₆ haloalkoxy" includes C₁₋₆ alkoxy groups having 1 to 7, 1 to 6, 1 to 5, 1 to 4, 1 to 3, or 1 to 2 halogen atoms.

As used herein, the term "aryl", whether used as part of another term or used independently, refers to a monocyclic or polycyclic ring system having a total of 5 to 20 ring members, wherein at least one ring in the ring system is aromatic, and each ring in the ring system contains 3 to 12 ring members. Examples of "aryl" include, but are not limited to, phenyl, biphenyl, naphthyl, anthracyl, and the like, which may bear one or more substituents. Also included within the scope of the term "aryl", as it is used herein, is a group in which an aromatic ring is fused to one or more additional rings. In the case of a polycyclic ring system, only one of the rings needs to be aromatic (for example, 2,3- dihydroindole), although all of the rings can be aromatic (for example, quinoline). The second ring may be fused or bridged. Examples of polycyclic aryl groups include, but are not limited to, benzofuranyl, indanyl, phthalimidyl, naphthimidyl, phenanthridinyl, tetrahydronaphthyl, and the like. Aryl group may be optionally substituted at one or more ring positions with one or more substituents described herein.

The term "benzyl" as used herein refers to -CH₂-phenyl.

As used herein, the terms "cycloalkyl", "carbocyclyl" and "carbocyclic ring" are interchangeable and whether used as part of another term or independently, refer to saturated, partially unsaturated or fully unsaturated (that is, aromatic) monocyclic and polycyclic ring systems, wherein all ring atoms are carbon, and which contain at least 3 ring-forming carbon atoms. In some embodiments, the cycloalkyl group may contain 3 to 12 ring-forming carbon atoms, 3 to 11 ring-forming carbon atoms, 3 to 10 ring-forming carbon atoms, 3 to 9 ring-forming carbon atoms, 3 to 8 Ring carbon atoms, 3 to 7 ring carbon atoms, 3 to 6 ring carbon atoms, 3 to 5 ring carbon atoms, 4 to 12 ring carbon atoms, 4 to 11 ring carbon atoms, 4 to 10 ring-forming carbon atoms, 4 to 9 ring-forming carbon atoms, 4 to 8 ring-forming carbon atoms, 4 to 7 ring-forming carbon atoms, 4 to 6 ring-forming carbon atoms, or 4 to 5 ring-forming carbon atoms carbon atom. The cycloalkyl group may be optionally substituted at one or more ring positions with one or more substituents described herein. The cycloalkyl can be saturated, partially unsaturated or fully unsaturated. In some embodiments, the cycloalkyl may be a saturated cyclic alkyl group. In some embodiments, the cycloalkyl may be an unsaturated cyclic alkyl group containing at least one double bond or triple bond in the ring system.

In some embodiments, the cycloalkyl may be a saturated or unsaturated monocyclic carbocyclic ring system, examples of which include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl, and the like.

In some embodiments, the cycloalkyl may be a saturated or unsaturated polycyclic (e.g., bicyclic and tricyclic) carbocyclic ring system, which may be a fused, spiro or bridged ring system. As used herein, the term "fused ring" refers to a ring system having two rings sharing two adjacent atoms, the term "spiro ring" refers to a ring system having two rings connected through one single common atom, and the term "bridged ring" refers to a ring system having two rings sharing three or more atoms. Examples of fused carbocyclic groups include, but are not limited to, naphthyl, benzopyrenyl, anthracenyl, acenaphthenyl, fluorenyl, and the like. Examples of spiro carbocyclyl include, but are not limited to, spiro[5.5]undecyl, spiro-pentadienyl, spiro[3.6]-decyl, and the like. Examples of bridged carbocyclyl include, but are not limited to, bicyclo[1,1,1]pentenyl, bicyclo[2,2,1]heptenyl, bicyclo[2.2.1]heptanyl, bicyclo[2.2.2]octanyl, bicyclo[3.3.1]nonanyl, bicyclo[3.3.3]undecanyl, and the like.

The term "heteroatom" as used herein refers to nitrogen, oxygen, sulfur or phosphorus, and includes any oxidized form of nitrogen or sulfur, and any quaternized form of basic nitrogen.

The term "heteroaryl" as used herein, whether used as part of another term or used independently, refers to an aryl group having one or more heteroatoms in addition to carbon atoms, which may optionally independently be substituted with one or more substituents described herein. Examples of heteroaryl groups include, but are not limited to, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, benzofuranyl, and pteridinyl, etc. The heteroaryl group also include groups in which a heteroaromatic ring is fused to one or more aryl, cycloalkyl or heterocyclyl rings. Non-limiting examples include indolyl, isoindolyl, benzothienyl, benzofuranyl, dibenzofuranyl, indazolyl, benzimidazolyl, benzothiazolyl, quinolinyl, isoquinolinyl, cinnolinyl, phthalazinyl, quinazolinyl, quinoxalinyl, 4H-quinazinyl, carbazolyl, acridinyl, phenazinyl, phenothiazinyl, phenoxazinyl, tetrahydroquinolinyl, tetrahydroisoquinolinyl, pyrido[2,3-b]-1,4-oxazin-3(4H)-one. In some embodiments, the term "5 to 10-membered heteroaryl" refers to a 5 to 6-membered heteroaryl ring having 1 to 3 heteroatoms independently selected from nitrogen, oxygen, sulfur or phosphorus, or an 8- to 10-membered bicyclic heteroaryl group having 1 to 4 heteroatoms independently selected from nitrogen, oxygen, sulfur or phosphorus.

The term "heterocycle" or "heterocyclyl" as used herein refers to a saturated, partially unsaturated or fully unsaturated carbocyclic group in which one or more ring atoms are heteroatoms independently selected from nitrogen, oxygen, sulfur or phosphorus, and the remaining ring atoms are carbon, wherein one or more ring atoms may be optionally substituted independently with one or more substituents. In some embodiments, the heterocyclyl is a saturated heterocyclyl. In some embodiments, the heterocyclyl is an unsaturated heterocyclyl having one or more double bonds in the ring system. In some embodiments, the heterocyclyl may comprise carbon, nitrogen, sulfur or phosphorus in any oxidized form and basic nitrogen in any quaternized form. "Heterocyclyl" also includes radicals wherein the heterocyclyl radicals are fused with a saturated, partially unsaturated, or fully unsaturated (i.e., aromatic) carbocyclic or heterocyclic ring. The heterocyclyl may be carbon or nitrogen linked. In some embodiments, the heterocyclyl is carbon linked. In some embodiments, the heterocyclyl is nitrogen linked. For example, a group derived from pyrrole may be pyrrol-1-yl (nitrogen-linked) or pyrrol-3-yl (carbon-linked). Further, a group derived from imidazole may be imidazol-1-yl (nitrogen-linked) or imidazol-3-yl (carbon-linked).

In some embodiments, the term "3 to 12-membered heterocyclyl" refers to a 3 to 12 membered saturated or partially unsaturated monocyclic or polycyclic heterocyclic ring system having 1 to 3 heteroatoms independently selected from nitrogen, oxygen, sulfur or phosphorus. Fused, spiro and bridged ring systems are also included in the above definition. Examples of monocyclic heterocyclic groups include, but are not limited to, oxetanyl, 1,1-dioxidothietanyl, pyrrolidinyl, tetrahydrofuryl, tetrahydrothienyl, pyrrolyl, furyl, thienyl, pyrazolyl, imidazolyl, thiazolyl, oxazolyl, piperidinyl, piperazinyl, morpholinyl, pyridinyl, pyrazinyl, pyrimidinyl, pyridazinyl, triazinyl, pyridonyl, pyrimidinonyl, pyrazinonyl, pyridazinonyl, pyrrolidinyl, and triazinonyl etc. Examples of fused heterocyclic groups include, but are not limited to, phenyl fused ring or pyridyl fused ring, such as quinolyl, isoquinolyl, quinoxalinyl, quinazinyl, quinazolinyl, azaindolizinyl, pterridinyl, benzopyranyl, isobenzopyranyl, indolyl, isoindolyl, indazinyl, indazolyl, purinyl, benzofuryl, isobenzofuryl, benzimidazolyl, benzothienyl, benzothiazolyl, carbazolyl, phenazinyl, phenothiazinyl, phenanthridinyl, imidazo[1,2-a]pyridyl, [1,2,4]triazolo[4,3-a]pyridyl, and [1,2,3]triazolo[4,3-a]pyridyl, etc. Examples of spiro heterocyclic group include, but are not limited to, spiropyranyl, and spirooxazinyl, etc. Examples of bridged heterocyclic groups include, but are not limited to, morpholinyl, hexamethylenetetramine, 3-aza-bicyclo[3.1.0]hexane, 8-aza-bicyclo[3.2.1]octane, 1-aza-bicyclo[2.2.2]octane, and 1,4-diazabicyclo[2.2.2]octane (DABCO), etc.

Suitable monovalent substituents on substitutable carbon atoms of "optionally substituted" groups are independently halo; -(CH₂)₀₋₄R^{∘}; -(CH₂)₀₋₄OR^{∘}; -O(CH₂)₀₋₄R^{∘}; -O-(CH₂)₀₋₄C(O)OR^{∘}; -(CH₂)₀₋₄CH(OR^{∘})₂; -(CH₂)₀₋₄SR^{∘}; -(CH₂)₀₋₄Ph, which can be substituted with R^{∘}; -(CH₂)₀₋₄O(CH₂)₀₋₁Ph, which can be substituted with R^{∘}; -CH=CHPh, which can be substituted with R^{∘}; -(CH₂)₀₋₄O(CH₂)₀₋₁-pyridyl, which can be substituted with R^{∘}; -NO₂; - CN; -N₃; -(CH₂)₀₋₄N(R^{∘})₂; -(CH₂)₀₋₄N(R^{∘})C(O)R^{∘}; -N(R^{∘})C(S)R^{∘}; -(CH₂)₀₋₄N(R^{∘})C(O)NR^{∘}₂; -N(R^{∘})C(S)NR^{∘}₂; -(CH₂)₀₋₄N(R^{∘})C(O)OR^{∘}; - N(R^{∘})N(R^{∘})C(O)R^{∘}; -N(R^{∘})N(R^{∘})C(O)NR^{∘}₂; -N(R^{∘})N(R^{∘})C(O)OR^{∘}; -(CH₂)₀₋₄C(O)R^{∘}; - C(S)R^{∘}; -(CH₂)₀₋₄C(O)OR^{∘}; -(CH₂)₀₋₄C(O)SR^{∘}; -(CH₂)₀₋₄C(O)OSiR^{∘}₃; -(CH₂)₀₋₄OC(O)R^{∘}; - OC(O)(CH₂)₀₋₄SR^{∘}-; -(CH₂)₀₋₄SC(O)R^{∘}; -(CH₂)₀₋₄C(O)NR^{∘}₂; -C(S)NR^{∘}₂; -C(S)SR^{∘}; - SC(S)SR^{∘}; -(CH₂)₀-₄OC(O)NR^{∘}₂; -C(O)N(OR^{∘})R^{∘}; -C(O)C(O)R^{∘}; -C(O)CH₂C(O)R^{∘}; - C(NOR^{∘})R^{∘}; -(CH₂)₀₋₄SSR^{∘}; -(CH₂)₀₋₄S(O)₂R^{∘}; -(CH₂)₀₋₄S(O)₂OR^{∘}; -(CH₂)₀₋₄OS(O)₂R^{∘}; - S(O)₂NR^{∘}₂; -(CH₂)₀₋₄S(O)R^{∘}; -N(R^{∘})S(O)₂NR^{∘}₂; -N(R^{∘})S(O)₂R^{∘}; -N(OR^{∘})R^{∘}; -C(NH)NR^{∘}₂; -P(O)₂R^{∘}; -P(O)R^{∘}₂; -OP(O)R^{∘}₂; -OP(O)(OR^{∘})₂; SiR^{∘}₃; -(C₁₋₄ linear or branched alkylene)O-N(R^{∘})₂; or -(C₁₋₄ linear or branched alkylene)C(O)O-N(R^{∘})₂, where each R°may be substituted as defined below and is independently hydrogen, a C₁₋₆ aliphatic group, -CH₂Ph, - O(CH₂)₀₋₁Ph, -CH₂-(5- to 6-membered heteroaryl ring), or a 5-6 membered saturated, partially unsaturated or aryl ring having 0-4 heteroatoms independently selected from nitrogen, oxygen or sulfur; or, despite the above definition, two independently occurring R^{∘} form, together with their intervening atom, a 3-12 membered saturated, partially unsaturated or fully unsaturated monocyclic or bicyclic ring having 0-4 heteroatoms independently selected from nitrogen, oxygen or sulfur, and can be substituted as defined below.

Suitable monovalent substituent on R^{∘} (or on the ring formed by two independently occurring R^{∘} together with their intervening atom) is independently halo, - (CH₂)₀₋₂R^{•}, -(haloR^{•}), -(CH₂)₀₋₂OH, -(CH₂)₀₋₂OR^{•}, -(CH₂)₀₋₂CH(OR^{•})₂, -O(haloR^{•}), -CN, - N₃, -(CH₂)₀₋₂C(O)R^{•}, -(CH₂)₀₋₂C(O)OH, -(CH₂)₀₋₂C(O)OR^{•}, -(CH₂)₀₋₂SR^{•}, -(CH₂)₀₋₂SH, - (CH₂)₀₋₂NH₂, -(CH₂)₀₋₂NHR^{•}, -(CH₂)₀₋₂NR^{•}₂, -NO₂, -SiR^{•}₃, -OSiR^{•}₃, -C(O)SR^{•}, -(C₁₋₄ linear or branched alkylene)C(O)OR^{•}, or -SSR^{•}, where each R^{•} is unsubstituted or is only substituted by one or more halo when preceded with "halo", and is independently selected from a C₁₋₄ aliphatic group, -CH2Ph, -O(CH₂)₀₋₁Ph, or a 5-6 membered saturated, partially unsaturated or fully unsaturated ring having 0-4 heteroatoms independently selected from nitrogen, oxygen or sulfur. Suitable divalent substituent on a saturated carbon atom of R^{∘} includes =O and =S.

Suitable divalent substituent on a saturated carbon atom of an "optionally substituted" group includes: =O, =S, =NNR^{∗}₂, =NNHC(O)R^{∗}, =NNHC(O)OR^{∗}, =NNHS(O)₂R^{∗}, =NR^{∗}, =NOR^{∗}, -O(C(R^{∗}₂))₂₋₃O-, or -S(C(R^{∗}₂))₂₋₃S-, where R^{∗}, in each occurrence, is selected from hydrogen, a C₁₋₆ aliphatic group which may be substituted as defined below, or an unsubstituted 5-6 membered saturated, partially unsaturated or aryl ring having 0-4 heteroatoms independently selected from nitrogen, oxygen or sulfur. Suitable divalent substituent bonded to a substitutable ortho carbon of an "optionally substituted" group includes -O(CR^{∗}₂)₂₋₃O-, where R^{∗}, in each occurrence, is selected from hydrogen, a C₁₋₆ aliphatic group which may be substituted as defined below, or an unsubstituted 5-6 membered saturated, partially unsaturated or fully unsaturated ring having 0-4 heteroatoms independently selected from nitrogen, oxygen or sulfur.

Suitable substituent on the aliphatic group of R^{∗} includes halo, - R^{•}, -(haloR^{•}), -OH, -OR^{•}, -O(haloR^{•}), -CN, -C(O)OH, -C(O)OR^{•}, -NH₂, -NHR^{•}, -NR^{•}₂, or - NO₂, where each R^{•} is unsubstituted or is substituted with only one or more halo when preceded with "halo", and is independently a C₁₋₄ aliphatic group, -CH₂Ph, -O(CH₂)₀₋₁Ph, or a 5-6 membered saturated, partially unsaturated or fully unsaturated ring having 0-4 heteroatoms independently selected from nitrogen, oxygen or sulfur.

Suitable substituent on a substitutable nitrogen atom of an "optionally substituted" group includes -R^{†}, -NR^{†}₂, -C(O)R^{†}, -C(O)OR^{†}, -C(O)C(O)R^{†}, - C(O)CH₂C(O)R^{†}, -S(O)₂R^{†}, -S(O)₂NR^{†}₂, -C(S)NR^{†}₂, -C(NH)NR^{†}₂, or -N(R^{†})S(O)₂R^{†}, where each R^{†} is independently hydrogen, a C₁₋₆ aliphatic group which may be substituted as defined below, unsubstituted -OPh, or an unsubstituted 5-6 membered saturated, partially unsaturated or fully unsaturated ring having 0-4 heteroatoms independently selected from nitrogen, oxygen or sulfur; or despite the above definition, two independently occurring R^{†} form, together with their intervening atom, an unsubstituted 3-12 membered saturated, partially unsaturated or fully unsaturated monocyclic or bicyclic ring having 0-4 heteroatoms independently selected from nitrogen, oxygen or sulfur.

Suitable substituent on the aliphatic group of R^{†} is independently halo, - R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, where each R^{•} is unsubstituted or is only substituted with one or more halo when preceded with "halo", and is independently a C₁₋₄ aliphatic group, -CH₂Ph, -O(CH₂)₀₋₁Ph, or a 5-6 membered saturated, partially unsaturated or aryl ring having 0-4 heteroatoms independently selected from nitrogen, oxygen or sulfur.

The term "protecting group" as used herein refers to a group of atoms that block, reduce or prevent the reactivity of a functional group when linked to a reactive functional group in a molecule. For example, the "amino protecting group" is a substituent attached to an amino group that blocks or protects the amino functional group in a compound. Suitable amino protecting groups include, but are not limited to, acetyl, trifluoroacetyl, triphenylmethyl, allyloxycarbonyl, trimethylsilyl (TMS), tert-butoxycarbonyl (Boc), benzyloxycarbonyl (CBZ) and 9-fluorenylmethyleneoxycarbonyl (Fmoc) etc. Similarly, the "hydroxyl protecting group" refers to a substituent to a hydroxyl group that blocks or protects the hydroxyl functional group. Suitable protecting groups include acetyl and silyl. The "carboxy protecting group" refers to a substituent to a carboxyl group that blocks or protects the carboxyl functional group. Common carboxyl protecting groups include phenylsulfonylethyl, cyanoethyl, 2-(trimethylsilyl)ethyl, 2-(trimethylsilyl)ethoxymethyl, 2-(p-toluenesulfonyl)ethyl, 2-(p-nitrophenylsulfinyl)ethyl, 2-(diphenylphosphino)-ethyl, and nitroethyl, etc. For a general description of the protecting groups and their uses, see P. G. M. Wuts and T. W. Greene, Greene's Protective Groups in Organic Synthesis, 4th Edition, Wiley-Interscience, New York, 2006.

### Compounds

In one aspect, the present application provides a compound of Formula (I): 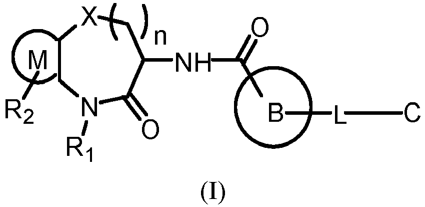 or a pharmaceutically acceptable salt thereof, wherein:
X is O, S or CH₂;
ring M has a structure of 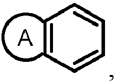 in which ring A is selected from the group consisting of substituted or unsubstituted 5- to 6-membered heteroaryl and substituted or unsubstituted 5-to 6-membered heterocyclyl;
ring B is selected from the group consisting of substituted or unsubstituted 5- to 12-membered aryl, substituted or unsubstituted 5- to 12-membered heteroaryl, and substituted or unsubstituted 5- to 12-membered heterocyclyl;
C is selected from the group consisting of substituted or unsubstituted (C₃-C₁₂) cycloalkyl, substituted or unsubstituted 5- to 12-membered aryl, substituted or unsubstituted 5- to 12-membered heteroaryl, and substituted or unsubstituted 5- to 12-membered heterocyclyl;
L is selected from the group consisting of O, S, NH, N(CH₃), substituted or unsubstituted C₁-C₆ alkylene-O-, substituted or unsubstituted C₁-C₆ alkylene-NH-, (substituted or unsubstituted C₁-C₆ alkylene)₂-N-, substituted or unsubstituted C₁-C₆ alkylene, substituted or unsubstituted C₃-C₆ alkenylene, and substituted or unsubstituted C₃-C₆ alkenylene-O-;
R₁ is selected from the group consisting of H and substituted or unsubstituted C₁-C₆ alkyl; R₂ is selected from the group consisting of H, halo, hydroxyl, cyano, oxy, benzyl, substituted or unsubstituted amino, substituted or unsubstituted C₁-C₆ alkyl, substituted or unsubstituted C₁-C₆ alkoxy and C₁-C₆ acyl; and
n is 1, 2 or 3;
wherein "substituted" refers to the substitution of one or more hydrogen atoms on the group with a substituent selected from the group consisting of halo, cyano, alkyl, acyl, sulfonyl, hydroxyl, amino, benzyl, oxy, (C₁-C₄) alkyl, halo(C₁-C₄) alkyl, (C₁-C₄)alkoxy, halo(C₁-C₄)alkoxy, nitro, and (C₁-C₄)alkylC(O)-.

In some embodiments, X is O or S.

In some embodiments, X is O.

In some embodiments, X is S.

In some embodiments, ring A is substituted or unsubstituted 5-membered heteroaryl or substituted or unsubstituted 5-membered heterocyclyl. In some embodiments, ring A is substituted or unsubstituted 5-membered heteroaryl or substituted or unsubstituted 5-membered heterocyclyl, wherein the 5-membered heteroaryl and 5-membered heterocyclyl contain one or more heteroatoms selected from N or O.

In some embodiments, ring A is substituted or unsubstituted 6-membered heteroaryl or substituted or unsubstituted 6-membered heterocyclyl. In some embodiments, ring A is substituted or unsubstituted 6-membered heteroaryl or substituted or unsubstituted 6-membered heterocyclyl, wherein the 6-membered heteroaryl and 6-membered heterocyclyl contain one or more heteroatoms selected from N or O.

In some embodiments, ring B is substituted or unsubstituted 5- to 12-membered aryl, substituted or unsubstituted 5- to 6-membered heteroaryl, or substituted or unsubstituted 5- to 6-membered heterocyclyl.

In some embodiments, ring B is substituted or unsubstituted 5- to 10-membered aryl. In some embodiments, ring B is substituted or unsubstituted 5- to 6-membered aryl. In some embodiments, ring B is substituted or unsubstituted phenyl.

In some embodiments, ring B is substituted or unsubstituted 5- to 6-membered heteroaryl or substituted or unsubstituted 5- to 6-membered heterocyclyl. In some embodiments, ring B is substituted or unsubstituted 5- to 6-membered heteroaryl, or substituted or unsubstituted 5- to 6-membered heterocyclyl, wherein the 5- to 6-membered heteroaryl and 5- to 6-membered heterocyclyl contain one or more heteroatoms selected from N or O.

In some embodiments, ring B is a group selected from the group consisting of: 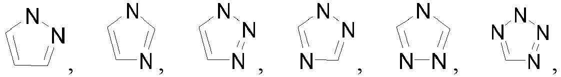 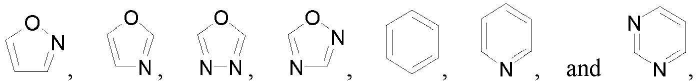 each of which is optionally substituted with one or more substituents described herein.

In some embodiments, C is substituted or unsubstituted 5- to 12-membered aryl. In some embodiments, C is substituted or unsubstituted 5-10-membered aryl. In some embodiments, C is substituted or unsubstituted 5- to 6-membered aryl. In some embodiments, C is substituted or unsubstituted 6-membered aryl. In some embodiments, C is substituted or unsubstituted phenyl. In some embodiments, C is substituted with one or more groups selected from the group consisting of halo, cyano, hydroxyl, amino, nitro, alkyl, haloalkyl, alkoxy, and haloalkoxy. In some embodiments, C is substituted with one or more groups selected from the group consisting of halo, cyano, hydroxyl, amino, nitro and alkyl. In some embodiments, C is substituted with one or more groups selected from the group consisting of halo and alkyl.

In some embodiments, L is O, NH or substituted or unsubstituted C₁-C₆ alkylene. In some embodiments, L is O, NH or unsubstituted C₁-C₆ alkylene. In some embodiments, L is O. In some embodiments, L is NH. In some embodiments, L is methylene.

In some embodiments, R₁ is H. In some embodiments, R₁ is substituted or unsubstituted C₁-C₆ alkyl. In some embodiments, R₁ is unsubstituted C₁-C₆ alkyl. In some embodiments, R₁ is methyl, ethyl or propyl. In some embodiments, R₁ is methyl.

In some embodiments, R₂ is selected from the group consisting of H, halo, hydroxyl, oxy, benzyl, substituted or unsubstituted C₁-C₆ alkyl, substituted or unsubstituted C₁-C₆ alkoxy, and C₁-C₆ acyl. In some embodiments, R₂ is H, halo, hydroxyl, oxy, benzyl, methyl, trifluoromethyl, methoxy or acetyl.

In some embodiments, m is 0 or 1. In some embodiments, m is 0. In some embodiments, m is 1.

In some embodiments, n is 1 or 2. In some embodiments, n is 1.

In some embodiments, the present application provides a compound of Formula (Ia): 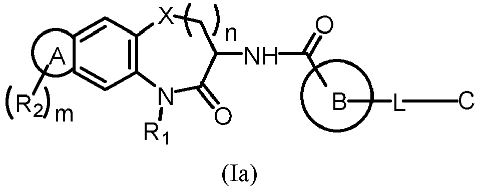 or a pharmaceutically acceptable salt thereof, wherein ring A, ring B, C, L, R₁, R₂, m and n are as defined above.

In some embodiments, the present application provides a compound of Formula (Ib): 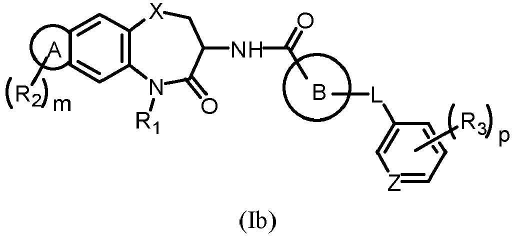 or a pharmaceutically acceptable salt thereof, wherein ring A, ring B, R₁, R₂, and m are as defined above, L is O or CH₂, Z is N or CH, R₃ is selected from halo or substituted or unsubstituted C₁-C₆ alkyl, and p is 0, 1, 2 or 3.

In some embodiments, in the compounds of Formula (Ia) and (Ib), the structural moiety represented by the formula 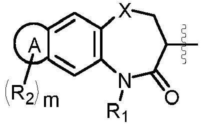 is selected from the group consisting of: 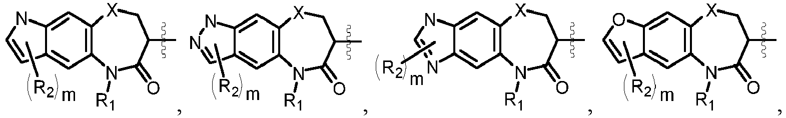 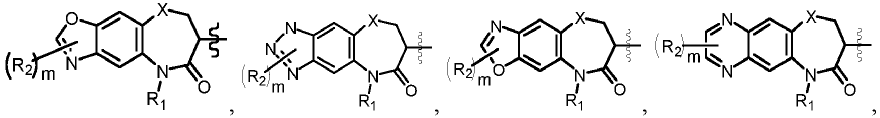 and

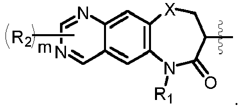

In some embodiments, R₂ is selected from the group consisting of H, halo, hydroxyl, oxy, benzyl, substituted or unsubstituted C₁-C₆ alkyl, substituted or unsubstituted C₁-C₆ alkoxy, and C₁-C₆ acyl. In some embodiments, R₂ is H, Cl, hydroxyl, oxy, benzyl, methyl, trifluoromethyl, methoxy, or acetyl.

In some embodiments, m is 0 or 1. In some embodiments, m is 0. In some embodiments, m is 1.

In some embodiments, the present application provides a compound of Formula (Ic): 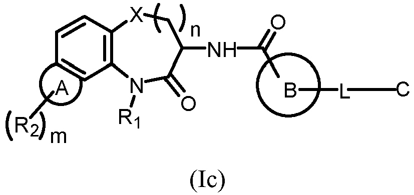 or a pharmaceutically acceptable salt thereof, wherein ring A is substituted or unsubstituted 6-membered heteroaryl or substituted or unsubstituted 6-membered heterocyclyl, and ring B, C, L, R₁, R₂, m and n are as defined above.

In some embodiments, the present application provides a compound of Formula (Id): 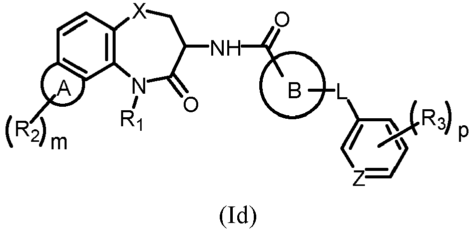 or a pharmaceutically acceptable salt thereof, wherein ring A is substituted or unsubstituted 6-membered heteroaryl or substituted or unsubstituted 6-membered heterocyclyl, L is O or CH₂, Z is N or CH, R₃ is halo and substituted or unsubstituted C₁-C₆ alkyl, p is 0, 1, 2 or 3, and ring B, R₁, R₂ and m are as defined above.

In some embodiments, in the compounds of Formula (Ic) and (Id), the structural moiety represented by the formula 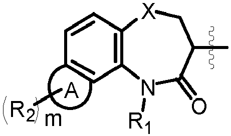 is selected from the group consisting of:

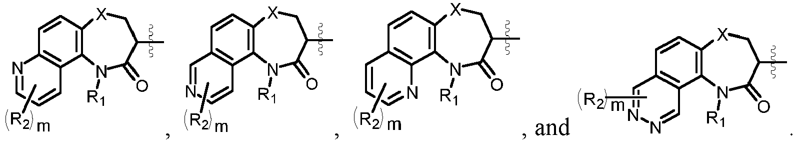

In some embodiments, R₂ is selected from the group consisting of H, halo, hydroxyl, oxy, benzyl, substituted or unsubstituted C₁-C₆ alkyl, substituted or unsubstituted C₁-C₆ alkoxy, and C₁-C₆ acyl. In some embodiments, R₂ is H.

In some embodiments, m is 0 or 1. In some embodiments, m is 0. In some embodiments, m is 1.

In some embodiments, the present application provides a compound of Formula (I) selected from the group consisting of:

| Compound No. | Chemical structure | Compound No. | Chemical structure |
|---|---|---|---|
| RIP1-001 | 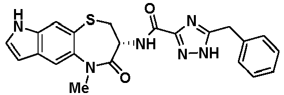 | RIP1-059 | 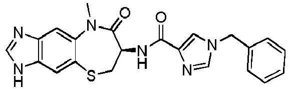 |
| RIP 1-002 | 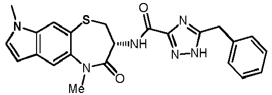 | RIP 1-060 | 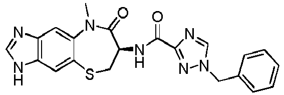 |
| RIP 1-003 | 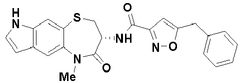 | RIP 1-061 | 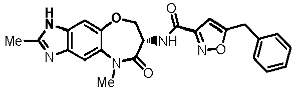 |
| RIP 1-004 | 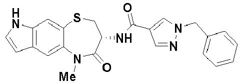 | RIP 1-062 | 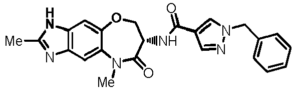 |
| RIP1-005 | 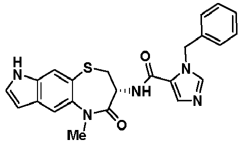 | RIP 1-063 | 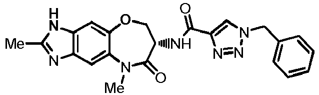 |
| RIP 1-006 | 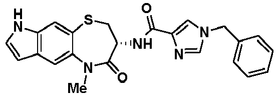 | RIP 1-064 | 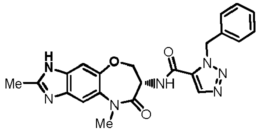 |
| RIP 1-007 | 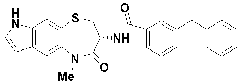 | RIP 1-065 | 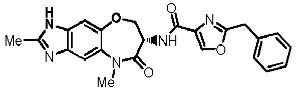 |
| RIP 1-008 | 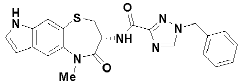 | RIP 1-066 | 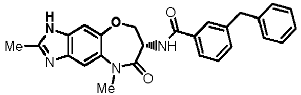 |
| RIP 1-009 | 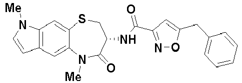 | RIP 1-067 | 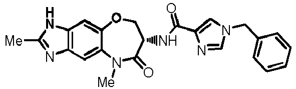 |
| RIP1-010 | 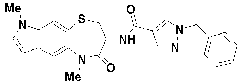 | RIP1-068 | 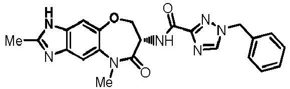 |
| RIP1-011 | 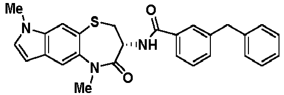 | RIP 1-069 | 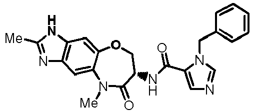 |
| RIP1-012 | 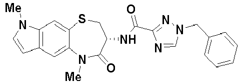 | RIP1-070 | 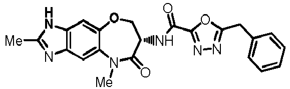 |
| RIP1-013 | 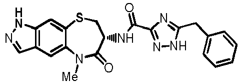 | RIP1-071 | 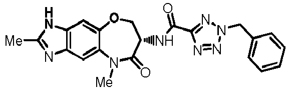 |
| RIP1-014 | 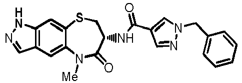 | RIP 1-072 | 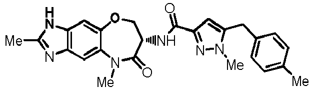 |
| RIP1-015 | 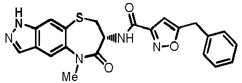 | RIP 1-073 | 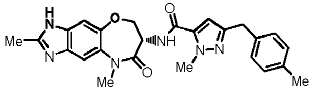 |
| RIP1-016 | 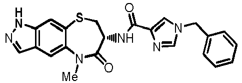 | RIP1-074 | 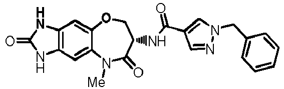 |
| RIP1-017 | 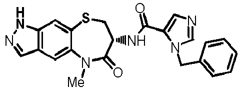 | RIP1-075 | 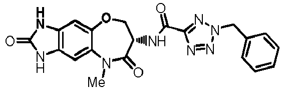 |
| RIP1-018 | 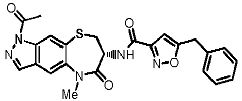 | RIP1-076 | 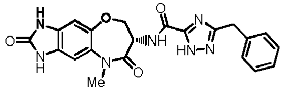 |
| RIP1-019 | 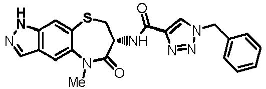 | RIP 1-077 | 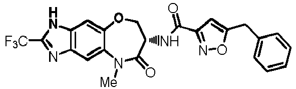 |
| RIP 1-020 | 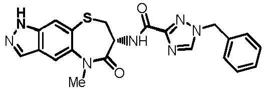 | RIP 1-078 | 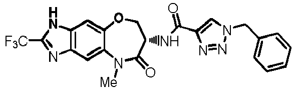 |
| RIP 1-021 | 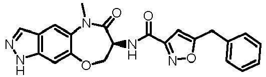 | RIP 1-079 | 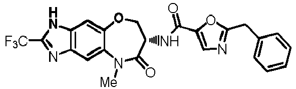 |
| RIP1-022 | 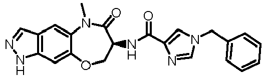 | RIP1-080 | 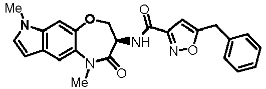 |
| RIP 1-023 | 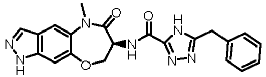 | RIP 1-081 | 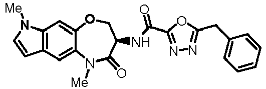 |
| RIP1-024 | 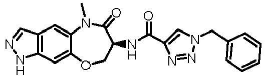 | RIP1-082 | 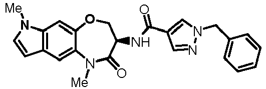 |
| RIP1-025 | 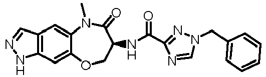 | RIP1-083 | 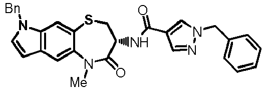 |
| RIP 1-026 | 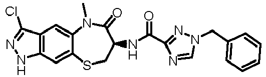 | RIP 1-084 | 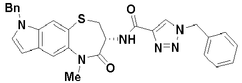 |
| RIP 1-027 | 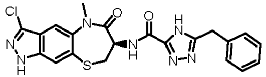 | RIP1-085 | 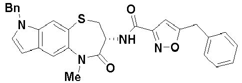 |
| RIP1-028 | 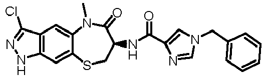 | RIP1-086 | 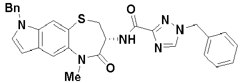 |
| RIP 1-029 | 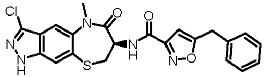 | RIP1-087 | 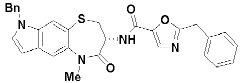 |
| RIPI-030 | 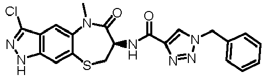 | RIP1-088 | 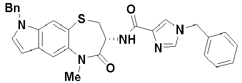 |
| RIPI-031 | 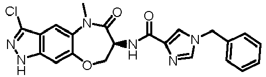 | RIP1-089 | 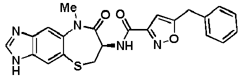 |
| RIP1-032 | 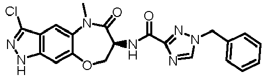 | RIP 1-090 | 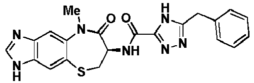 |
| RIP1-033 | 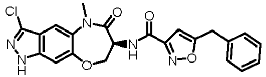 | RIP1-091 | 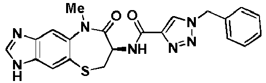 |
| RIP1-034 | 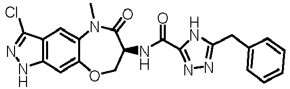 | RIP 1-092 | 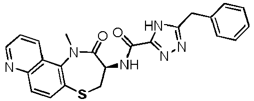 |
| RIP1-035 | 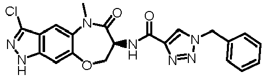 | RIP 1-093 | 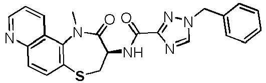 |
| RIP1-036 | 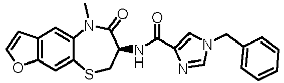 | RIP 1-094 | 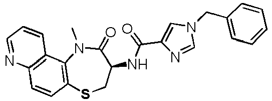 |
| RIP1-037 | 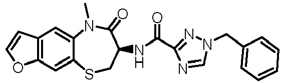 | RIP1-095 | 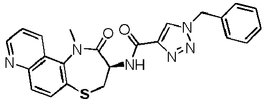 |
| RIP1-038 | 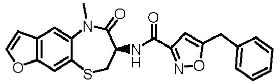 | RIP 1-096 | 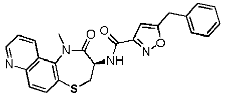 |
| RIP1-039 | 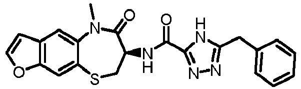 | RIP 1-097 | 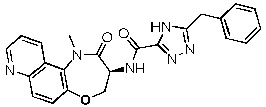 |
| RIP 1-040 | 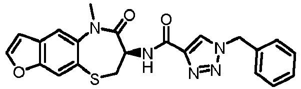 | RIP 1-098 | 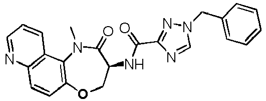 |
| RIP 1-041 | 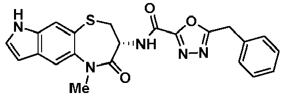 | RIP 1-099 | 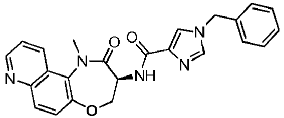 |
| RIP1-042 | 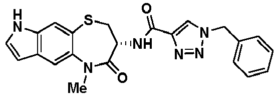 | RIP1-100 | 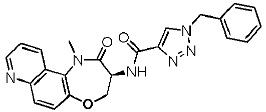 |
| RIP 1-043 | 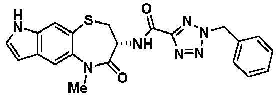 | RIP1-101 | 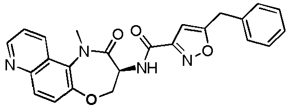 |
| RIP1-044 | 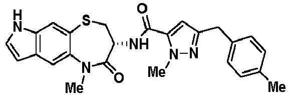 | RIP1-102 | 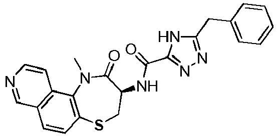 |
| RIP1-045 | 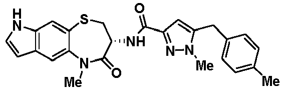 | RIP1-103 | 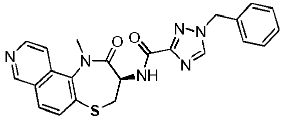 |
| RIP1-046 | 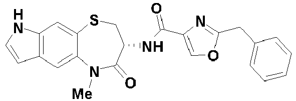 | RIP1-104 | 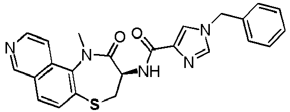 |
| RIP1-047 | 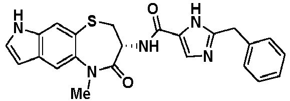 | RIP1-105 | 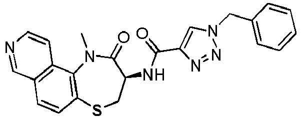 |
| RIP1-048 | 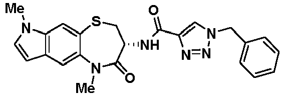 | RIP1-106 | 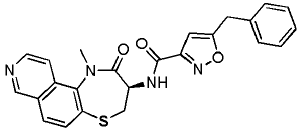 |
| RIP1-049 | 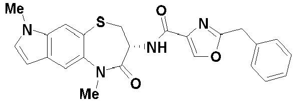 | RIP1-107 | 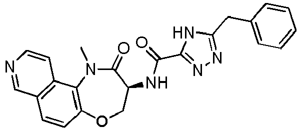 |
| RIP1-050 | 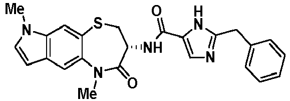 | RIP1-108 | 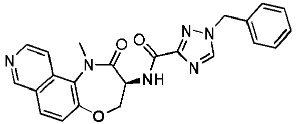 |
| RIP1-051 | 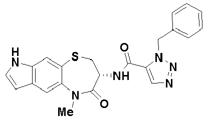 | RIP1-109 | 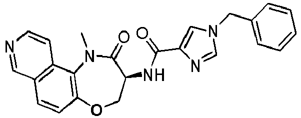 |
| RIP1-052 | 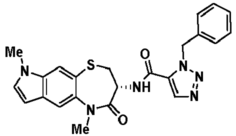 | RIP1-110 | 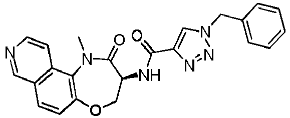 |
| RIP1-054 | 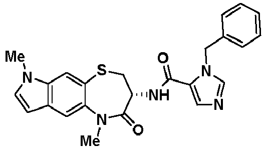 | RIP1-111 | 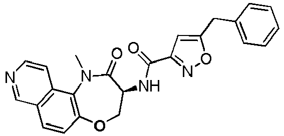 |
| RIP1-055 | 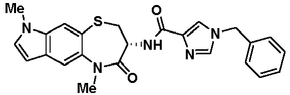 | RIP1-112 | 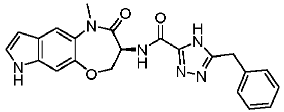 |
| RIP1-056 | 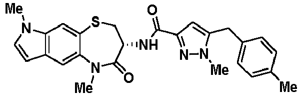 | RIP1-113 | 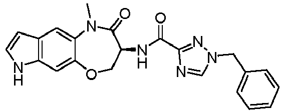 |
| RIP1-057 | 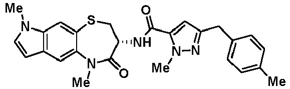 | RIP1-114 | 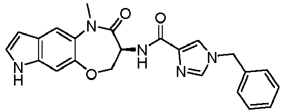 |
| RIP1-058 | 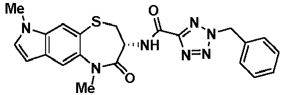 | RIP1-115 | 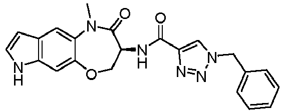 |
| RIP1-119 | 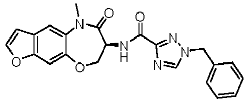 | RIP1-116 | 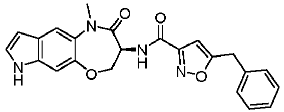 |
| RIP1-120 | 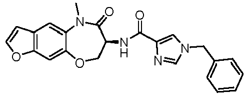 | RIP1-167 | 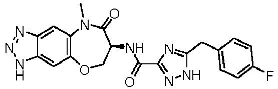 |
| RIP1-121 | 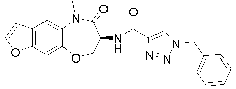 | RIP1-168 | 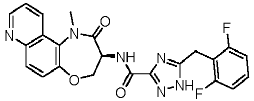 |
| RIP1-122 | 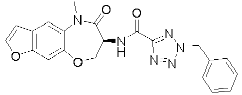 | RIP1-169 | 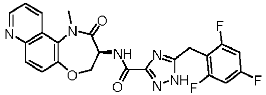 |
| RIP1-123 | 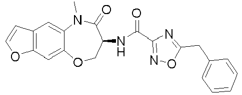 | RIP1-170 | 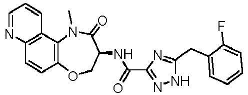 |
| RIP1-124 | 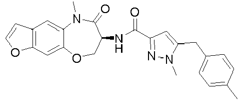 | RIP1-171 | 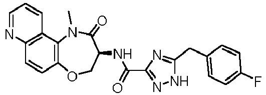 |
| RIP1-125 | 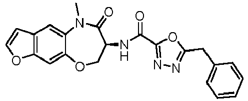 | RIP1-172 | 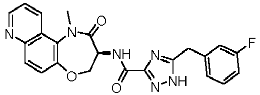 |
| RIP1-126 | 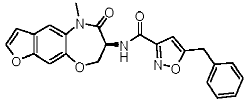 | RIP1-173 | 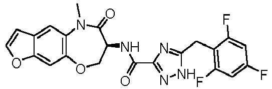 |
| RIP1-127 | 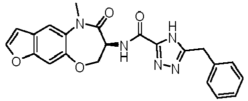 | RIP1-174 | 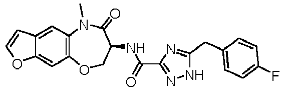 |
| RIP1-128 | 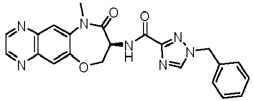 | RIP1-175 | 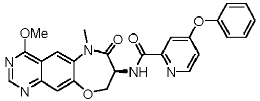 |
| RIP1-129 | 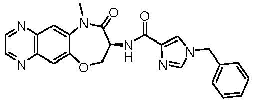 | RIP1-176 | 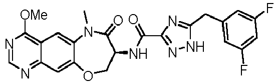 |
| RIP1-130 | 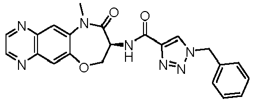 | RIP1-177 | 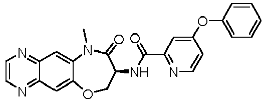 |
| RIP1-131 | 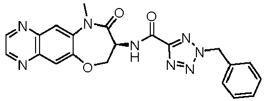 | RIP1-178 | 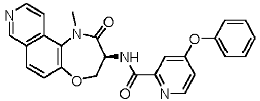 |
| RIP1-132 | 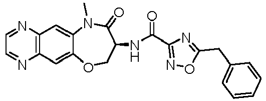 | RIP1-179 | 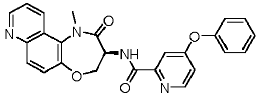 |
| RIP1-133 | 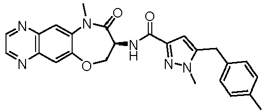 | RIP1-180 | 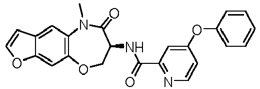 |
| RIP1-134 | 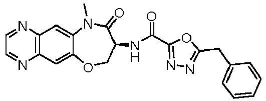 | RIP1-181 | 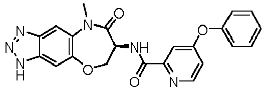 |
| RIP1-135 | 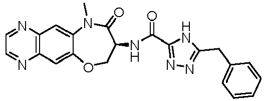 | RIP1-182 | 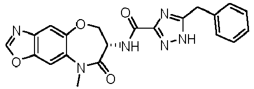 |
| RIP1-136 | 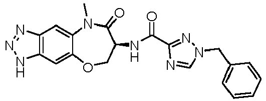 | RIP1-183 | 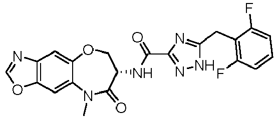 |
| RIP1-137 | 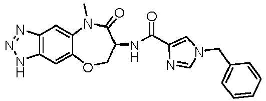 | RIP1-184 | 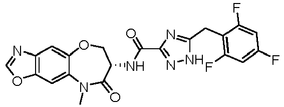 |
| RIP1-138 | 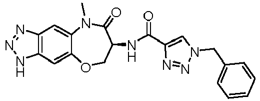 | RIP1-185 | 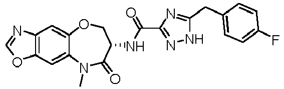 |
| RIP1-139 | 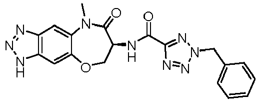 | RIP1-186 | 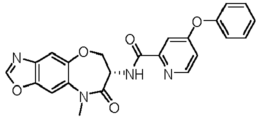 |
| RIP1-140 | 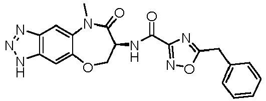 | RIP1-187 | 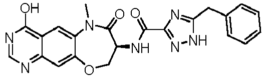 |
| RIP1-141 | 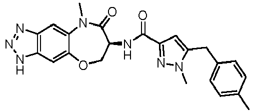 | RIP1-188 | 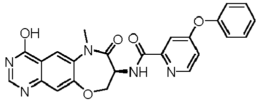 |
| RIP1-142 | 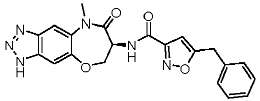 | RIP1-189 | 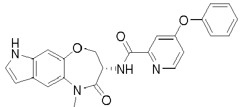 |
| RIP1-143 | 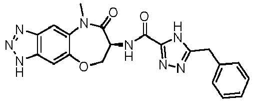 | RIP1-190 | 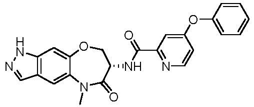 |
| RIP1-145 | 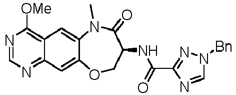 | RIP1-193 | 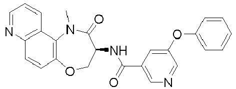 |
| RIP1-146 | 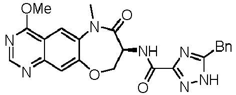 | RIP1-194 | 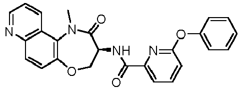 |
| RIP1-147 | 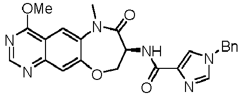 | RIP1-195 | 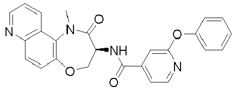 |
| RIP1-148 | 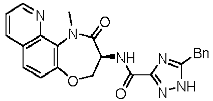 | RIP1-196 | 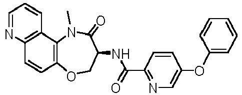 |
| RIP1-149 | 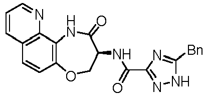 | RIP1-197 | 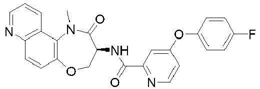 |
| RIP1-150 | 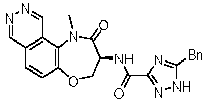 | RIP1-198 | 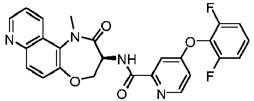 |
| RIP1-153 | 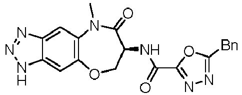 | RIP1-199 | 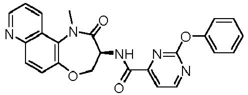 |
| RIP1-156 | 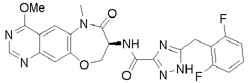 | RIP1-200 | 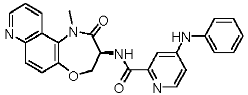 |
| RIP1-157 | 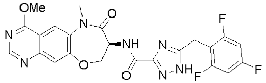 | RIP1-201 | 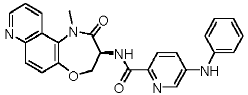 |
| RIP1-158 | 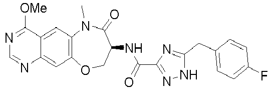 | RIP1-202 | 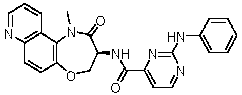 |
| RIP1-159 | 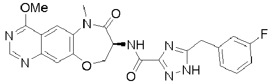 | RIP 1-203 | 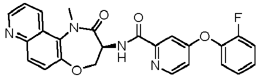 |
| RIP1-160 | 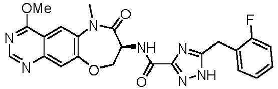 | RIP1-204 | 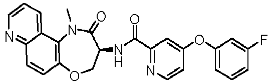 |
| RIP1-161 | 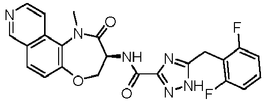 | RIP1-205 | 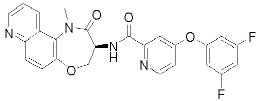 |
| RIP1-162 | 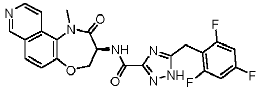 | RIP1-206 | 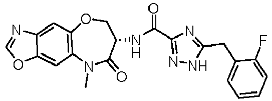 |
| RIP1-163 | 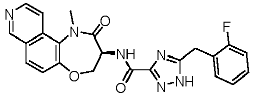 | RIP 1-207 | 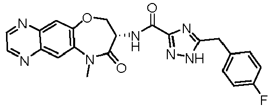 |
| RIP1-164 | 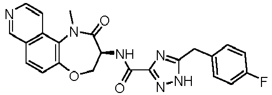 | RIP1-208 | 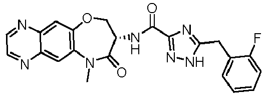 |
| RIP1-165 | 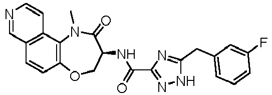 | RIP1-209 | 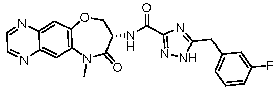 |
| RIP1-166 | 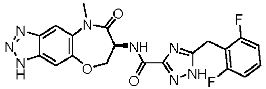 | RIP1-210 | 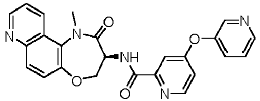 |
| RIP1-211 | 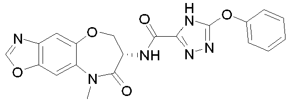 | RIP1-212 | 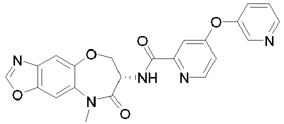 |
| RIP1-213 | 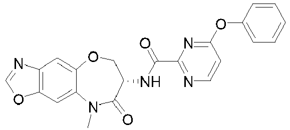 | RIP1-214 | 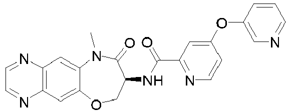 |
| RIP1-215 | 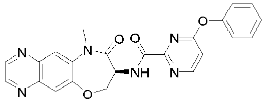 | RIP1-217 | 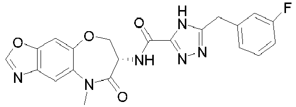 |
| RIP1-218 | 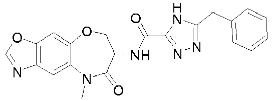 | RIP1-219 | 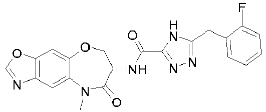 |
| RIP 1-220 | 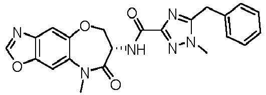 | RIP1-221 | 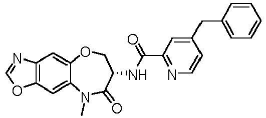 |
| RIP1-222 | 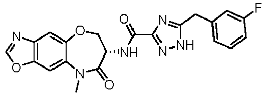 | | |

The compound provided herein can exist in a number of different forms or derivatives, all within the scope of the present application. These forms or derivatives include, for example, tautomers, stereoisomers, racemic mixtures, regioisomers, salts, prodrugs, solvated forms, different crystal forms or polymorphs, and active metabolites.

The compound provided herein may comprise one or more asymmetric centers, and thus can exist in various stereoisomeric forms, such as enantiomers and/or diastereomers. Therefore, the compound and composition thereof provided herein may be in the form of an individual enantiomer, diastereomer or geometric isomer, or may be in the form of a mixture of stereoisomers. In some embodiments, the compounds provided herein are enantiopure compounds. In some embodiments, mixtures of enantiomers or diastereomers are provided.

The term "enantiomer" as used herein refers to two stereoisomers of a compound, which are non-superimposable mirror images of one another. The term "diastereomer" as used herein refers to a pair of optical isomers that are not mirror images of one another. Diastereomers have different physical properties, such as melting points, boiling points, spectral properties and reactivities.

In addition, unless otherwise indicated, certain compounds provided herein may have one or more double bonds that can exist as either the Z or E isomer. The present application additionally encompasses the compounds as individual isomers substantially free of other isomers and alternatively, as mixtures of various isomers, e.g., racemic mixtures of enantiomers. In addition to the above-mentioned compounds per se, the present application also encompasses compositions comprising one or more compounds

The term "isomer" as used herein includes any and all geometric isomers and stereoisomers. For example, "isomers" include cis- and trans- isomers, E- and Z- isomers, Rand S- enantiomers, diastereomers, D-isomers, L-isomers, racemic mixtures thereof, and other mixtures thereof. For example, in some embodiments, a stereoisomer can be provided in a form that is substantially free of one or more corresponding enantiomers, which can be said to be "stereochemically enriched".

When a particular enantiomer is preferred, the compound of the present application can be provided as an enantiomer that is substantially free of the opposite enantiomer, and can be referred to as "optically enriched". As used herein, "optically enriched" means that the compound is made up of a significantly greater proportion of one enantiomer. In some embodiments, the compound is made up of at least about 90 wt% of a preferred enantiomer. In some embodiments, the compound is made up of at least about 95 wt%, 98 wt%, or 99 wt% of a preferred enantiomer. Preferred enantiomers can be isolated from racemic mixtures by any method known in the art, including chiral high pressure liquid chromatography (HPLC), formation and crystallization of chiral salts, or prepared by asymmetric syntheses. See, for example, Jacques et al., Enantiomers, Racemates and Resolutions (Wiley Interscience, New York, 1981); Wilen, S.H. et al., Tetrahedron 33:2725 (1977); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972).

The compound provided herein can also exist in different tautomeric forms, and all such forms are embraced within the scope of this application. The term "tautomer" or "tautomeric form" refers to structural isomers of different energies which are interconvertible via a low energy barrier. For example, proton tautomers (also known as protonic tautomers) include interconversion via migration of a proton, such as keto-enol, amide-imidic acid, lactam-lactim, enamine-imine isomerizations, and annular forms where a proton can occupy two or more positions of a heterocyclic system, such as 1H- and 3H-imidazole, 1H-, 2H- and 4H-1,2,4-triazole, 1H- and 2H-isoindole, and 1H- and 2H-pyrazole. Valence tautomers include interconversions by reorganization of some of the bonding electrons. Tautomeric forms can be in equilibrium or sterically locked into one form by appropriate substitution. Unless otherwise indicated, the compounds of the present application identified by name or structure as one particular tautomeric form are intended to include other tautomeric forms.

In some embodiments, the compound of the present application is an S-enantiomer. In some embodiments, the compound of the present application is an R-enantiomer.

The compounds of the present application also include prodrugs, active metabolic derivatives (active metabolites), active intermediates, and pharmaceutically acceptable salts thereof.

The term "prodrug" as used herein refers to a compound or a pharmaceutically acceptable salt thereof, which, when metabolized under physiological conditions or when converted by solvolysis, yields the desired active compound. Prodrugs include, but are not limited to, esters, amides, carbamates, carbonates, ureides, solvates or hydrates of the active compound. Generally, the prodrug is inactive, or less active than the active compound, but can provide one or more advantageous handling, administration, and/or metabolic properties. For example, some prodrugs are esters of the active compound; during metabolysis, the ester group is cleaved to yield the active drug. Also, some prodrugs are activated enzymatically to yield the active compound, or compounds which, upon further chemical reaction, yield the active compound. Prodrugs can proceed from prodrug form to active form in a single step, or can have one or more intermediate forms that can themselves have activity or may be inactive. Preparation and use of prodrugs are described in T. Higuchi and V. Stella, "Prodrugs as Novel Delivery Systems", Vol. 14 of the A.C.S. Symposium Series, and Bioreversible Carriers in Drug Design, Edward B. edited by Roche, American Pharmaceutical Association and Pergamon Press, 1987.

The term "metabolite" as used herein, such as active metabolite, overlaps with prodrugs as described above. Therefore, such metabolites are pharmacologically active compounds or compounds that further metabolize to pharmacologically active compounds that are derivatives resulting from metabolic process in the body of a subject. For example, such metabolites can result from oxidation, reduction, hydrolysis, amidation, deamidation, esterification, deesterification, enzymatic cleavage, and the like, of the administered compound or salt or prodrug. Of these, active metabolites are such pharmacologically active derivative compounds. For prodrugs, the prodrug compound is generally inactive or of lower activity than the metabolic product. For active metabolites, the parent compound may be either an active compound or may be an inactive prodrug.

Prodrugs and active metabolites can be identified by routine techniques konwn in the art. See, for example, Bertolini et al., 1997, J Med Chem 40: 2011-2016; Shan et al., J Pharm Sci 86: 756-757; and Bagshawe, 1995, DrugDev Res 34: 220-230.

The term "active intermediate" as used herein refers to an intermediate compound in the synthesis process, which shows the same or essentially the same biological activity as the final synthesized compound.

Compounds of the present application can be formulated as or be in the form of pharmaceutically acceptable salts. Unless indicated to the contrary, a compound provided herein includes pharmaceutically acceptable salts of such compound.

The term "pharmaceutically acceptable" as used herein indicates that the substance or composition is compatible chemically and/or toxicologically, with the other ingredients constituting a formulation, and/or the subjects being treated therewith.

Unless otherwise indicated, the term "pharmaceutically acceptable salt" as used herein includes a salt that retains the biological effectiveness of the free acids and free bases of the specified compound, and is not biologically undesirable. Contemplated pharmaceutically acceptable salt forms include, but are not limited to, mono, bis, tris, tetrakis, and so on. Pharmaceutically acceptable salts are non-toxic in the amounts and concentrations at which they are administered. The preparation of such salts can facilitate the pharmacological use by altering the physical characteristics of a compound without preventing it from exerting its physiological effect. Useful alterations in physical properties include lowering the melting point to facilitate the transmucosal administration, and increasing the solubility to facilitate the administration of higher concentrations of drugs.

Pharmaceutically acceptable salts may include acid addition salts, such as those containing sulfate, chloride, hydrochloride, fumarate, maleate, phosphate, sulfamate, acetate, citrate, lactate, tartrate, methanesulfonate, ethanesulfonate, benzenesulfonate, p-toluenesulfonate, cyclohexylsulfonate and quinate. Pharmaceutically acceptable salts can be obtained from acids such as sulfuric acid, hydrochloric acid, fumaric acid, maleic acid, phosphoric acid, sulfamic acid, acetic acid, citric acid, lactic acid, tartaric acid, methanesulfonic acid, ethanesulfonic acid, benzenesulfonic acid, p-toluenesulfonic acid, cyclohexylsulfonic acid and quinic acid.

Pharmaceutically acceptable salts may also include base addition salts, such as those containing benzathine, chloroprocaine, choline, diethanolamine, ethanolamine, tert-butylamine, ethylenediamine, meglumine, procaine, aluminum, calcium, lithium, magnesium, potassium, sodium, ammonium, zinc and alkylamines, when acidic functional groups such as carboxylic acid or phenol are present. See, for example, Remington's Pharmaceutical Sciences, 19th Edition, Mack Publishing Co., Easton, PA, Volume 2, Page 1457, 1995; "Handbook of Pharmaceutical Salts: Properties, Selection, and Use", Stahl and Wermuth, Wiley-VCH, Weinheim, Germany, 2002. Such salts can be prepared using the appropriate corresponding bases.

Pharmaceutically acceptable salts can be prepared by standard techniques. For example, the free base form of a compound can be dissolved in a suitable solvent such as an aqueous or an aqueous-alcohol solution containing an appropriate acid, and then isolated by evaporating the solution. Therefore, if the particular compound is a base, the desired pharmaceutically acceptable salt can be prepared by any suitable method available in the art, for example, treatment of the free base with an inorganic acid (such as hydrochloric acid, hydrobromic acid, sulfuric acid, nitric acid, and phosphoric acid, etc.) or with an organic acid (such as acetic acid, maleic acid, succinic acid, mandelic acid, fumaric acid, malonic acid, pyruvic acid, oxalic acid, glycolic acid, salicylic acid, pyranosyl acids such as glucuronic acid or galacturonic acid, α-hydroxy acids such as citric acid or tartaric acid, amino acids such as aspartic acid or glutamic acid, aromatic acids such as benzoic acid or cinnamic acid, and sulfonic acids such as p-toluenesulfonic acid or ethanesulfonic acid).

Similarly, if the particular compound is an acid, the desired pharmaceutically acceptable salt can be prepared by any suitable method, for example, treatment of the free acid with an inorganic or organic base, such as an amine (primary, secondary or tertiary), an alkali metal hydroxide or alkaline earth metal hydroxide, etc. Illustrative examples of suitable salts include organic salts derived from amino acids (such as L-glycine, L-lysine and L-arginine), ammonia, primary amines, secondary amines, tertiary amines, cyclic amines (such as hydroxyethylpyrrolidine, piperidine, morpholine or piperazine), and inorganic salts derived from sodium, calcium, potassium, magnesium, manganese, iron, copper, zinc, aluminum and lithium.

It should also be understood that the compound of the present application can exist in an unsolvated form, a solvated form (e.g. hydrated form) and a solid form (e.g. crystal or polymorphic form), and the present application is intended to encompass all such forms.

The term "solvate" or "solvated form" as used herein refers to a solvent addition form that contains a stoichiometric or non-stoichiometric amount of solvent. Some compounds tend to trap fixed molar ratios of solvent molecules in the crystalline solid state, thus forming a solvate. If the solvent is water, the solvate formed is a hydrate; and if the solvent is alcohol, the solvate formed is an alcoholate. Examples of solvents that form solvates include, but are not limited to, water, isopropanol, methanol, ethanol, DMSO, ethyl acetate, acetic acid, ethanolamine, acetone, and ether, etc.

The terms "crystal form", "polymorphic form" and and "polymorph" as used herein can be used interchangeably, and refer to a crystal structure in which a compound (or a salt or solvate thereof) is crystallized in different crystal packing arrangements, all of which have the same elemental composition. Different crystal forms usually have different X-ray diffraction patterns, infrared spectra, melting points, density, hardness, crystal shape, optical and electrical properties, stability and solubility, etc. Recrystallization solvent, crystallization rate, storage temperature and other factors may cause one crystal form to dominate. Polymorphs of the compound can be prepared by crystallization under different conditions.

The present application is also intended to include all isotopes of atoms in the compound. Isotopes of an atom include atoms having the same atomic number but different mass numbers. For example, unless otherwise indicated, hydrogen, carbon, nitrogen, oxygen, phosphorus, sulfur, fluorine, chlorine, bromine or iodine in the compound of the present application also include their isotopes, for example, but not limited to, ¹H, ²H, ³H, ¹¹C, ¹²C, ¹³C, ¹⁴C, ¹⁴N, ¹⁵N, ¹⁶O, ¹⁷O, ¹⁸O, ³¹P, ³²P, ³²S, ³³S, ³⁴S, ³⁶S, ¹⁷F, ¹⁹F, ³⁵Cl, ³⁷Cl, ⁷⁹Br, ⁸¹Br, ¹²⁷I and ¹³¹I. In some embodiments, hydrogen includes protium, deuterium, tritium or a combination thereof. In some embodiments, carbon includes ¹²C, ¹³C or a combination thereof. In some embodiments, the abundance of various isotopic atoms of a certain element can be the state that the element naturally occurs in nature, or a state in which a certain isotope is enriched.

### Synthesis of compounds

The synthesis of the compounds (including pharmaceutically acceptable salts thereof) of the present application is illustrated in the synthesis scheme in examples below. The compound of the present application can be prepared by any known organic synthesis techniques, and can be synthesized according to any possible synthetic routes. Therefore, the schemes provided herein are merely exemplary and are not meant to limit other possible methods that can be used to prepare the compound of the present application.

The reactions used to prepare the compounds of the present application may be carried out in suitable solvents. Suitable solvents can be substantially non-reactive with the starting materials (reactants), the intermediates or products at the temperatures at which the reactions are carried out (e.g., temperatures that can range from the solvent's freezing point to the solvent's boiling point). A given reaction can be carried out in one solvent or in a mixture of more than one solvent. Depending on the particular reaction step, suitable solvents for a particular reaction step can be selected by those skilled in the art.

The preparation of the compounds of the prsent application may involve the protection and deprotection of various chemical groups. The needs of protection and deprotection and the choices of suitable protecting groups can be determined by those skilled in the art. The chemistry of protecting groups can be found in, for example, T. W. Greene and P. G. M. Wuts, Protective Groups in Organic Synthesis, 3rd edition, Wiley & Sons, Inc., New York (1999).

Reactions can be monitored by any suitable method known in the art. For example, the formation of product can be monitored by spectroscopic means, such as nuclear magnetic resonance spectroscopy (such as ¹H or ¹³C NMR), infrared spectroscopy, spectrophotometry (such as ultraviolet-visible), mass spectrometry, or by chromatographic method, such as high-performance liquid chromatography (HPLC), liquid chromatography-mass spectroscopy (LCMS) or thin layer chromatography (TLC). Compounds can be purified in a variety of ways, including HPLC and normal phase silica chromatography.

For illustrative purposes, the general synthetic route for preparing the compounds of the present application as well as intermediates are shown below.

In one aspect, the present application provides a method for preparing the compound of Formula (I) of the present application as shown below: 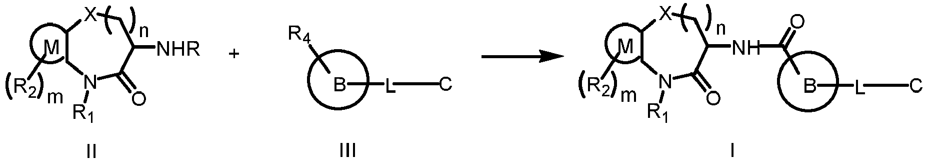 wherein R₄ is -COOH or -COO⁻G⁺, in which G⁺ is an alkali metal ion;
when R is H, the method comprises reacting a compound of Formula (II) with a compound of Formula (III) in an inert solvent in the presence of a condensation reagent and a base, to obtain a compound of Formula (I); and
when R is an amino protecting group, the method comprises removing R from the compound of Formula (II) under an acidic condition, and then reacting the compound of Formula (II) from which R is removed with the compound of Formula (III) in an inert solvent in the presence of a condensation reagent and a base, to obtain a compound of Formula (I).

In some embodiments, G⁺ is Li⁺, Na⁺ or K⁺.

In some embodiments, the compound of Formula (I) of the present application can be prepared through a scheme selected from the group consisting of: 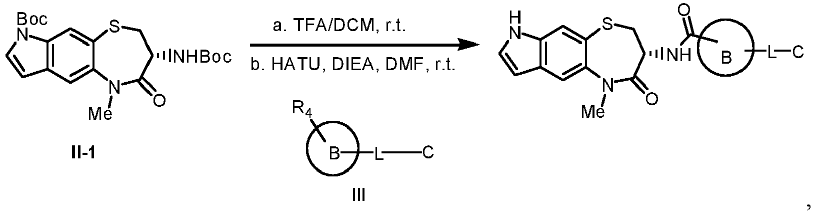 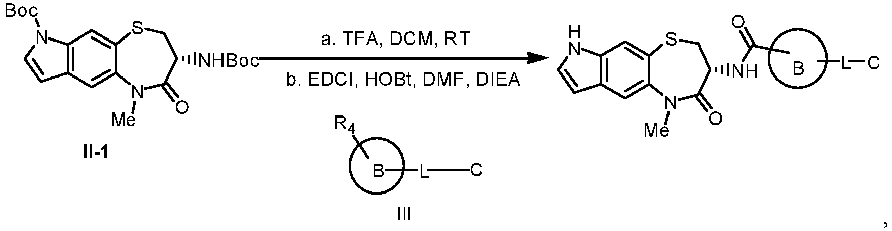 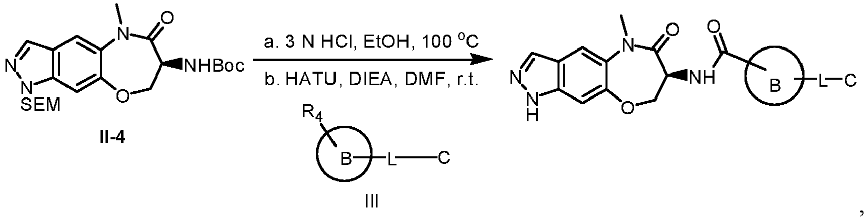 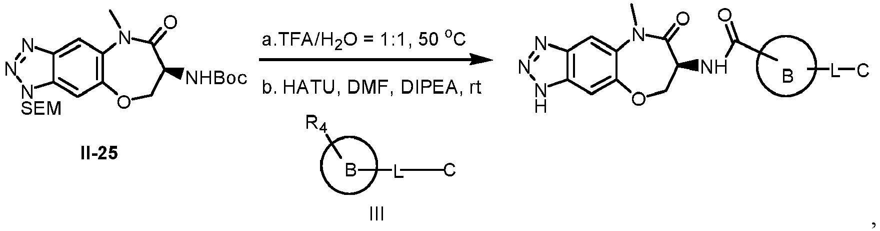 and

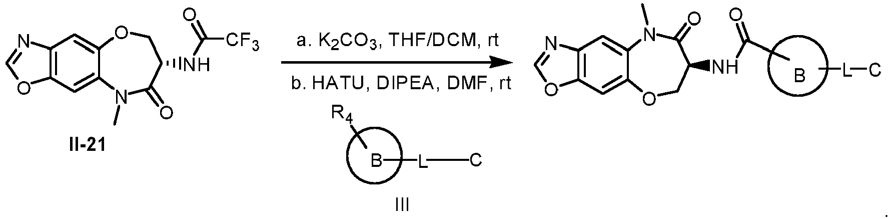

In some embodiments, R is H, and the compound of Formula (II) is reacted with the compound of Formula (III) in an inert solvent in the presence of a condensation reagent and a base, to obtain the compound of Formula (I).

In some embodiments, the inert solvent is selected from DMF, DMSO, acetonitrile, THF, DCM or a combination thereof.

In some embodiments, the condensation reagent is selected from HATU, DCC, HOBt, HBTU, HCTU, TBTU, TSTU, TNTU, EDCI, CDI, PyBOP or a combination thereof.

In some embodiments, the base is selected from DIEA (diisopropylethylamine), triethylamine, DMAP, pyridine or a combination thereof.

In some embodiments, R is an amino protecting group, and R is removed from the compound of Formula (II) under an acidic condition, and then the compound of Formula (II) from which R is removed is reacted with the compound of Formula (III) in an inert solvent in the presence of a condensation reagent and a base, to obtain the compound of Formula (I).

In some embodiments, the acidic condition means that the reaction system contains hydrochloric acid or trifluoromethanesulfonic acid.

In some embodiments, the inert solvent is selected from DMF, DMSO, acetonitrile, THF, DCM or a combination thereof.

In some embodiments, the condensation reagent is selected from HATU, DCC, HOBt, HBTU, HCTU, TBTU, TSTU, TNTU, EDCI, CDI, PyBOP or a combination thereof.

In some embodiments, the base is selected from DIEA, triethylamine, DMAP, pyridine or a combination thereof.

In some embodiments, a functional group including, but not limited to, acyl, and alkyl, etc., can be further introduced into the compound of Formula (I) by a conventional method, for example, as shown in scheme below:

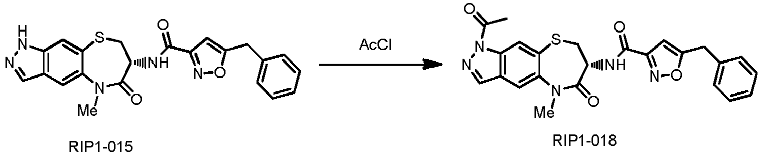

In yet another aspect, the present application provides an intermediate compound of Formula (II) for preparing the compound of Formula (I): 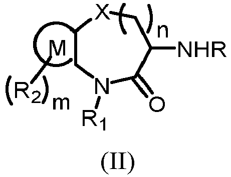 wherein:
R is H or an amino protecting group;
X is O, S or CH₂;
R₁ is selected from the group consisting of H and substituted or unsubstituted C₁-C₆ alkyl; R₂ is selected from the group consisting of H, halo, hydroxyl, oxy, benzyl, substituted or unsubstituted C₁-C₆ alkyl, substituted or unsubstituted C₁-C₆ alkoxy, and C₁-C₆ acyl;
m is 0, 1, 2 or 3; and
n is 1, 2 or 3;
wherein "substituted" refers to the substitution of one or more hydrogen atoms on the group with a substituent selected from the group consisting of halo, cyano, alkyl, acyl, sulfonyl, hydroxyl, amino, benzyl, oxy, (C₁-C₄) alkyl, halo(C₁-C₄) alkyl, (C₁-C₄)alkoxy, halo(C₁-C₄)alkoxy, nitro, and (C₁-C₄)alkylC(O)-.

In some embodiments, the intermediate compound of Formula (II) is selected from the group consisting of: 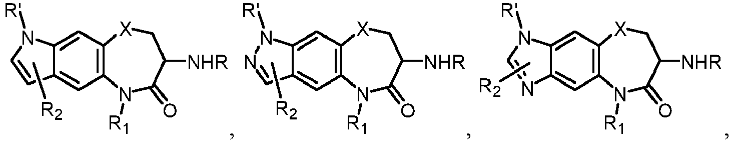 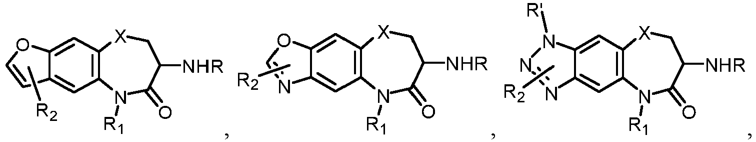 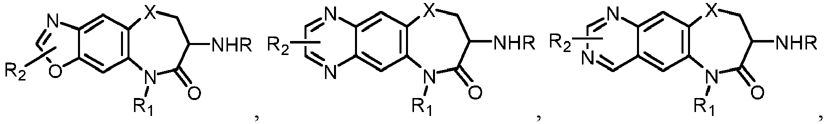 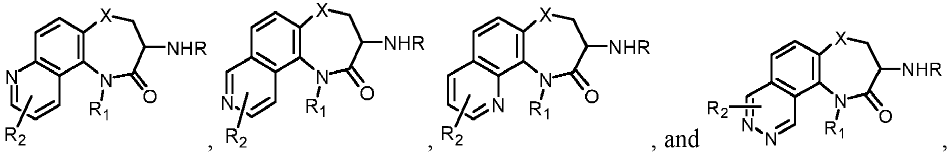 wherein R' is selected from H, Boc, SEM, (C₁-C₄) alkyl and benzyl.

In some embodiments, the intermediate compound of Formula (II) is selected from the group consisting of: 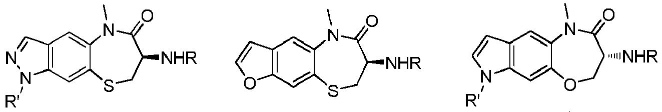 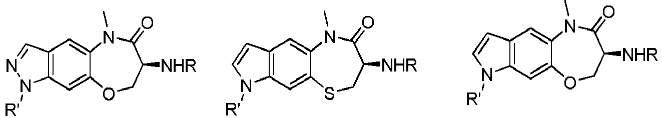 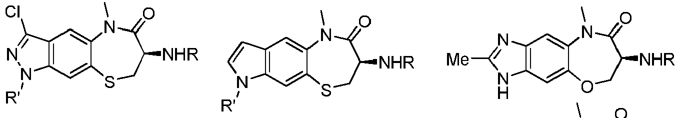 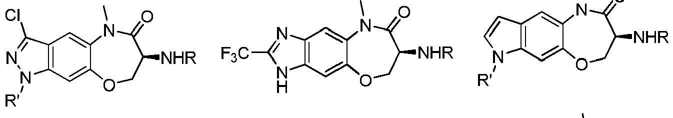 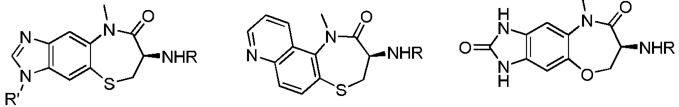 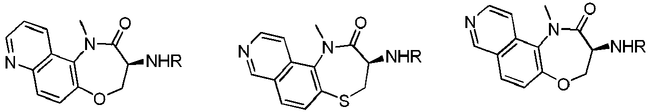 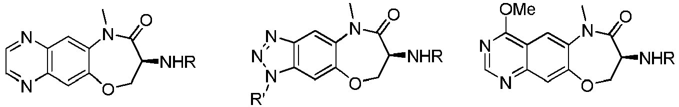 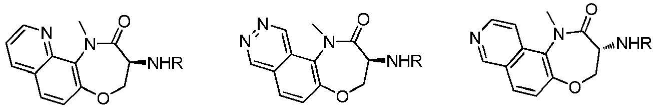 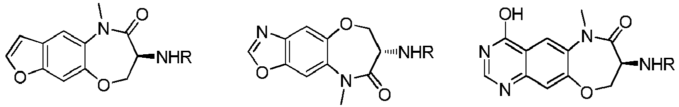 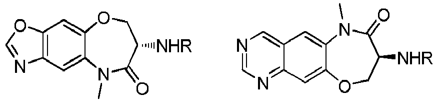 wherein R is H, Boc or TFA, and R' is selected from H, Boc or SEM.

In some embodiments, the compound of Formula (II) of the present application can be prepared through a scheme shown below: 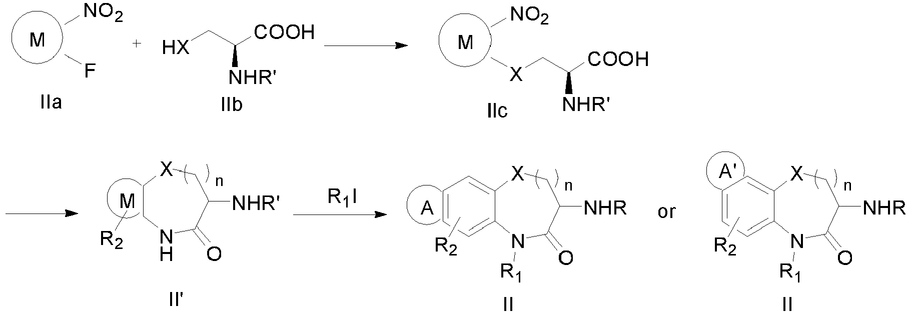 which comprises:
(a) reacting a compound of Formula IIa with a compound of Formula IIb in an inert solvent in the presence of a base, to obtain a compound of Formula IIc;
(b) reducing the nitro group in the compound of Formula IIc in an alcohol solvent under a hydrogen atmosphere and/or in the presence of a metal catalyst, and then cyclizing in an inert solvent in the presence of a condensation reagent and a base, to obtain a compound of Formula II'; and
(c) reacting the compound of Formula II' with R₁I in the presence of a base to obtain the compound of Formula II.

In some embodiments, the base in Step (a) is selected from cesium carbonate, potassium carbonate, NaOH, NaH, n-BuLi, KHMDS, or a combination thereof.

In some embodiments, the inert solvent in Steps (a) and (b) is selected from DMF, DMSO, acetonitrile, THF, or a combination thereof.

In some embodiments, Step (a) is carried out at a temperature of -20°C to 100°C.

In some embodiments, the alcohol solvent in Step (b) is selected from methanol, ethanol, isopropanol, n-propanol, n-butanol, isobutanol, or a combination thereof.

In some embodiments, the metal catalyst in Step (b) is Pd/C.

In some embodiments, the condensation reagent in Step (b) is selected from the group consisting of HATU, DCC, HOBt, HBTU, HCTU, TBTU, TSTU, TNTU, EDCI, CDI, PyBOP, or a combination thereof.

In some embodiments, the base in Step (c) is selected from DIEA, triethylamine, DMAP, pyridine, or a combination thereof.

In some embodiments, the compound of Formula (II) of the present application can be prepared through a scheme shown below: 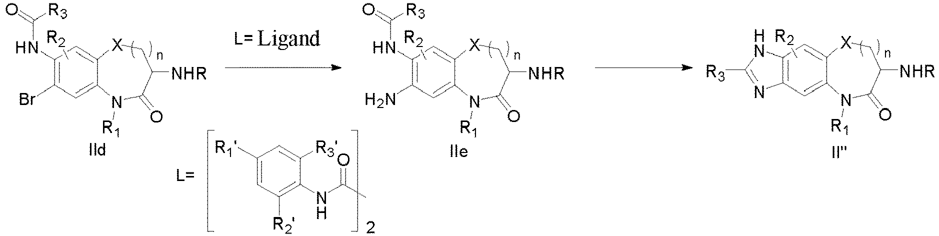 which comprises
(a) subjecting a compound of Formula IId to a coupling reaction with aqueous ammonia in an inert solvent in the presence of a copper catalyst and a ligand L, to obtain a compound of Formula IIe; and
(b) subjecting the compound of Formula IIe to a dehydration and ring-closing reaction under an acidic or basic condition to obtain a compound of Formula II".

In some embodiments, R₃ is methyl or trifluoromethyl, R₁', R_{2'}, and R₃' can be H, methyl, methoxy, phenyl, benzyl, phenoxy, naphthyl etc., and other groups are as defined above.

In some embodiments, the ligand L in Step (a) is selected from the group consisting of:

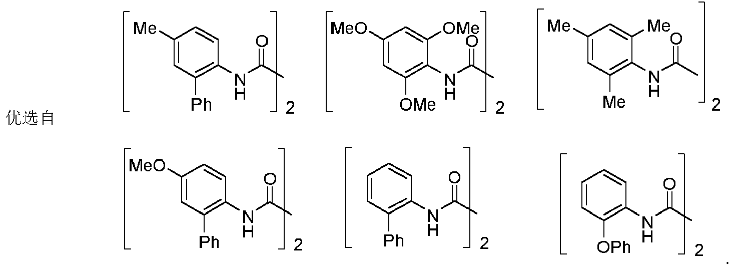

In some embodiments, the inert solvent in Step (a) is selected from DMSO, DMF, 1,4-dioxane, or a combination thereof.

In some embodiments, the copper catalyst in Step (a) is selected from CuI, CuCN, CuBr, CuCl, Cu₂O, or a combination thereof.

In some embodiments, the weight ratio of aqueous ammonia to the inert solvent in Step (a) is 1:10 to 1:1.

In some embodiments, the amount of the copper catalyst in Step (a) is 0.5-20 mol% relative to the compound of Formula IId.

In some embodiments, the amount of the ligand L in Step (a) is 0.5-30 mol% relative to the compound of Formula IId.

In some embodiments, Step (a) is carred out at a temperature of 40°C to 150°C.

In some embodiments, the acidic condition in Step (b) means that the reaction system contains an acid selected from the group consisting of acetic acid, 15% sulfuric acid, or a combination thereof.

In some embodiments, the basic condition in Step (b) means that the reaction system contains a base selected from the group consisting of sodium carbonate, potassium carbonate, cesium carbonate, calcium carbonate, potassium phosphate, or a combination thereof.

In some embodiments, Step (b) is carried out at a temperature of from room temperature (10°C-40°C) to 80°C, for example, 10°C to 80°C, 15°C to 80°C, 20°C to 80°C, 25°C to 80°C, 30°C to 80°C, 35°C to 80°C, or 40°C to 80°C.

An exemplary compound of Formula (II) can be prepared by a scheme shown below: 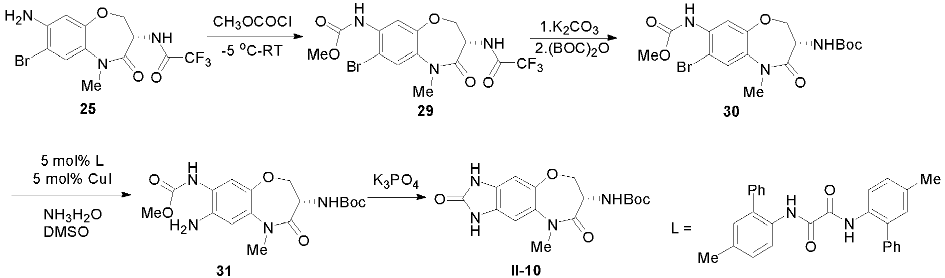 wherein Compound **25** is reacted with methyl chloroformate in dichloromethane under a basic condition at a temperature of from -5°C to room temperature to obtain Intermediate **29.** The trifluoroacetyl protecting group of Intermediate **29** is removed under a basic condition, and then the primary amino group is protected with Boc by reacting with Boc anhydride, thereby producing Compound **30.** Then, in an inert solvent, in the presence of a copper catalyst and a ligand L, Compound **30** is subjected to a coupling reaction with aqueous ammonia to obtain Compound **31.** Subsequently, a dehydration and ring-closure reaction is carried out by heating under a basic condition to obtain a compound of Formula **11-10.**

### Use of the compound

In one aspect, the present application provides a compound of Formula (I) or a pharmaceutically acceptable salt thereof, capable of inhibiting the activity of RIP 1 kinase.

Therefore, in another aspect, the present application provides a method for inhibiting RIP1 kinase in a subject, comprising administering to the subject an effective amount of the compound of the present application or a pharmaceutically acceptable salt thereof.

In some embodiments, the compound of the present application can inhibit the activity of RIP1 kinase with an IC₅₀ value of 0.1 nM-1000 µM, 1 nM-500 µM, 0.1 nM-100 µM, 0.1 nM-80 µM, 0.1 nM-50 µM, 0.1 nM-40 µM, 0.1 nM-30 µM, 0.1 nM-20 µM, 0.1 nM-10 µM, 0.1 nM-5 µM, 0.1 nM-1 µM, 0.1 nM-0.5 µM, 0.1 nM-0.1 µM, 0.1 nM-0.05 µM, 0.1 nM-40 nM, 0.1 nM-30 nM, 0.1 nM-20 nM, 0.1 nM-10 nM, 0.1 nM-5 nM, 0.1 nM-4 nM, 0.1 nM-3 nM, 0.1 nM-2 nM, 0.1 nM-1 nM, and 0.1 nM-0.5 nM.

Therefore, in another aspect, the compound of the present application or a pharmaceutically acceptable salt thereof can be used in the manufacture of a medicament for inhibiting the activity of RIP1 kinase.

In yet another aspect, the compound of the present application or a pharmaceutically acceptable salt thereof can be used in the manufacture of a medicament for preventing or treating RIP1 kinase-related diseases.

In yet another aspect, the compound of the present application or a pharmaceutically acceptable salt thereof can be used in the manufacture of a medicament for preventing or treating diseases or disorders caused by programmed cell necrosis.

### Pharmaceutical composition

In one aspect, the present application provides a pharmaceutical composition comprising a compound of the present application or a pharmaceutically acceptable salt thereof.

In some embodiments, the pharmaceutical composition of the present application comprises more than one compound of the present application or a pharmaceutically acceptable salt thereof.

In some embodiments, the pharmaceutical composition of the present application comprises one or more compounds of the present application or pharmaceutically acceptable salts thereof, and a pharmaceutically acceptable carrier.

Pharmaceutically acceptable carriers are conventional pharmaceutical carriers in the art, and can be prepared by methods known in the pharmaceutical field. In some embodiments, the compounds of the present application or pharmaceutically acceptable salts thereof can be mixed with pharmaceutically acceptable carriers to prepare the pharmaceutical compositions.

The term "pharmaceutically acceptable" as used herein indicates that the compound, material, composition and/or dosage form are suitable for contact with human or animal tissues without causing excessive toxicity, irritation, allergic reactions, other problems or complications, and has a reasonable benefit/risk ratio. In some embodiments, the pharmaceutically acceptable compounds, materials, compositions and/or dosage forms are those approved by regulatory agencies (for example, the U.S. Food and Drug Administration, China National Medical Products Administration, and European Medicines Agency) or listed in recognized pharmacopoeias (e.g. U.S. Pharmacopoeia, Chinese Pharmacopoeia, and European Pharmacopoeia) for use in animals, especially humans.

The term "pharmaceutically acceptable carrier" as used herein refers to a pharmaceutically acceptable material, composition or vehicle, such as a liquid or solid filler, diluent, excipient, solvent or packaging material, involved in carrying or delivering the compound of the present application or a pharmaceutically acceptable salt thereof from one position, body fluid, tissue, organ (internal or external) or body part to another position, body fluid, tissue, organ or body part. The pharmaceutically acceptable carrier can be a vehicle, diluent, excipient or other materials that can be used in contact with animal tissues without excessive toxicity or adverse reactions. Exemplary pharmaceutically acceptable carriers include, but are not limited to, carbohydrates, starch, cellulose, malt, tragacanth, gelatin, Ringer's solution, alginic acid, isotonic saline, and buffers, etc. The pharmaceutically acceptable carriers that can be used in the present application include those known in the art, such as those disclosed in "Remington Pharmaceutical Sciences" Mack Pub. Co., New Jersey (1991).

The pharmaceutical composition may also contain pharmaceutically acceptable aids required for approximating the physiological conditions, including, but not limited to, for example, pharmaceutically acceptable liquid, gel, or solid carriers, aqueous media (e.g., sodium chlorine injection, Ringer's injection, isotonic dextrose injection, sterile water injection, or dextrose and lactated Ringer's injection), non-aqueous medium (e.g., plant-derived fixed oil, cotton seed oil, corn oil, sesame oil, or peanut oil), antimicrobial substances, isotonic substances (such as sodium chloride or glucose), buffers (such as phosphate or citrate buffers), antioxidants (such as sodium bisulfate), anesthetics (e.g. procaine hydrochloride), suspending agents/dispersants (e.g. sodium carboxymethyl cellulose, hydroxypropylmethyl cellulose, or polyvinylpyrrolidone), chelating agents (e.g. EDTA (ethylenediaminetetraacetic acid) or EGTA (ethylene glycol bis(2-aminoethyl ether)tetraacetic acid)), emulsifiers (e.g. polysorbate 80 (Tween-80)), diluents, odorants, flavorants, sweeteners, adjuvants, aids, or non-toxic auxiliary substances, other components known in the art, or various combinations of the foregoing. Suitable components can include, for example, fillers, binders, disintegrants, buffers, preservatives, lubricants, flavoring agents, thickening agents, coloring agents or emulsifiers.

The form of the pharmaceutical composition depends on multiple factors, including, for example, the route of administration, the severity of disease, or the dosage of administration, etc.

In some embodiments, the pharmaceutical composition can be formulated to be administered to a subject via an appropriate route including, but not limited to, an oral route, injection (such as intravenous injection, intramuscular injection, subcutaneous inj ection, intradermal inj ection, intracardiac injection, intrathecal injection, intrapleural injection, and intraperitoneal injection, etc.), mucosal routes (such as intranasal administration, and intraoral administration, etc.), sublingual route, rectal route, transdermal route, intraocular route, and pulmonary route. According to the desired route of administration, the pharmaceutical composition can be formulated into tablets, capsules, pills, dragees, powders, granules, cachets, lozenges, suppositories, suspensions, emulsions, syrups, aerosols (as solid or in a liquid medium), sprays, ointments, pastes, patches, creams, lotions, gels, and inhalants, etc.

In some embodiments, the pharmaceutical composition is an oral preparation. The oral preparation includes, but is not limited to, capsules, sachets, pills, tablets, lozenges (the base for flavoring is often sucrose, Arabic gum or tragacanth), powders, granules, aqueous or non-aqueous solutions or suspensions, water-in-oil or oil-in-water emulsions, elixirs or syrups, pastilles (where suitable inert bases include, for example, gelatin and glycerin, or sucrose or gum arabic) and/or mouthwashes, or analogues thereof.

The oral solid preparation (such as capsules, tablets, pills, dragees, powders, and granules, etc.) comprises the active substance and one or more pharmaceutically acceptable aids, such as sodium citrate or dicalcium phosphate, and/or the following substances: (1) fillers or supplements, such as starch, lactose, sucrose, glucose, mannitol, and/or silicic acid; (2) binders, such as carboxymethyl cellulose, alginate, gelatin, polyvinylpyrrolidone, sucrose, and/or Arabic gum; (3) wetting agents, such as glycerol; (4) disintegrating agents, such as agar, calcium carbonate, potato or tapioca starch, alginic acid, some silicates and/or sodium carbonate; (5) retarder solutions, such as paraffin; (6) absorption accelerators, such as quaternary ammonium compounds; (7) lubricants, such as acetol and glyceryl monostearate; (8) absorbents, such as kaolin and bentonite; (9) glidants, such as talc, calcium stearate, magnesium stearate, solid polyethylene glycol, sodium dodecyl sulfate, and mixtures thereof; and (10) coloring agents.

The oral liquid preparation comprises pharmaceutically acceptable emulsions, microemulsions, solutions, suspensions, syrups and elixirs. In addition to the active substance, the liquid dosage form may also contain a commonly used inert diluent, for example, water or other solvents, solubilizers and emulsifiers, such as ethanol, isopropanol, ethyl carbonate, ethyl acetate, benzyl alcohol, benzyl benzoate, propylene glycol, 1,3-butanediol, oils (in particular cottonseed oil, peanut oil, corn oil, olive oil, castor oil and sesame oil), glycerin, tetrahydrofurfuryl alcohol, polyethylene glycol and fatty acid sorbitol ester, or a mixture of two or more thereof. In addition to the inert diluent, adjuvants such as wetting agents, emulsifying and suspending agents, sweetening agents, flavoring agents, pigments, perfumes and preservatives may also be added to the oral liquid preparation.

In some embodiments, the pharmaceutical composition is an injectable preparation. The injectable preparation includes sterile aqueous solutions, dispersions, suspensions or emulsions. In all instances, the injectable preparation shall be sterile and in a liquid state to facilitate the injection, remain stable under production and storage conditions, and shall be resistant to contamination by microorganisms (e.g., bacteria and fungi). The carrier may be a solvent or dispersing medium, including, for example, water, ethanol, polyhydroxy compounds (e.g., glycerol, propylene glycol, and liquid polyethylene glycol, etc.) and suitable mixtures thereof, and/or vegetable oils. The injectable preparation needs to have a proper fluidity which can be maintained by a variety of ways, for example, by the use of coatings such as lecithin, by the use of surfactants, etc. Resistance to microbial contamination can be achieved by adding various antibacterial and antifungal agents (e.g., paraben, chlorobutanol, phenol, sorbic acid, and thimerosal, etc.).

In some embodiments, the pharmaceutical composition is an oral spray preparation or a nasal spray preparation. The spray preparations include, but are not limited to, aqueous aerosols, non-aqueous suspensions, liposome preparations or solid particle preparations, etc. Aqueous aerosols are obtained by formulating an aqueous solution or suspension of the active agent with a conventional pharmaceutically acceptable excipient and stabilizer. The carrier and stabilizer vary according to the needs of the specific compound, and generally include non-ionic surfactants (Tweens, or polyethylene glycol), oleic acid, lecithin, amino acids such as glycine, buffers, salts, sugar or sugar alcohols. Aerosols are usually prepared from isotonic solutions and can be delivered via a nebulizer.

The pharmaceutical composition can be formulated to provide rapid release, sustained release or delayed release of the active ingredient after administration to a subject. In some embodiments, the pharmaceutical composition can be formulated into a sustained release form. As used herein, the term "sustained release form" indicates that the active agent is released from the pharmaceutical composition over an extended period of time (extended release) or at a certain location (controlled release), so that it is biologically absorbed in a subject (e.g., in the subject's gastrointestinal tract). In some embodiments, the extended period of time can be about 1 to 24 hrs, 2 to 12 hrs, 3 to 8 hrs, 4 to 6 hrs, 1 to 2 days or longer. In some embodiments, the extended period of time can be at least about 4 hrs, at least about 8 hrs, at least about 12 hrs, or at least about 24 hrs. In some embodiments, the pharmaceutical composition can be formulated into the form of a tablet. For example, the release rate of the active agent can not only be controlled by the active agent that dissolves in the gastrointestinal fluid independently of pH and then diffuses out of the tablet or pill, but also be affected by the physical process of disintegration and dissolution of the tablet. In some embodiments, the polymer materials disclosed in "Medical Applications of Controlled Release," Langer and Wise eds, CRC Pres., Boca Raton, Florida (1974), "Controlled Drug Bioavailability," Drug Product Design and Performance, Smolen and Ball eds, Wiley, New York (1984), Ranger and Peppas, 1983, J Macromol. Sci. Rev. Macromol Chem. 23:61, Levy et al., 1985, Science 228:190, During et al., 1989, Ann. Neurol. 25:351, and Howard et al., 1989, J. Neurosurg. 71:105 can be used for sustained release.

In some embodiments, the pharmaceutical composition comprises 1-99 wt% of the compound of Formula I or a pharmaceutically acceptable salt thereof. In some embodiments, the pharmaceutical composition comprises 5-99 wt%, 10-99 wt%, 15-99 wt%, 20-99 wt%, 25-99 wt%, 30-99 wt%, 35-99 wt%, 40-99 wt%, 45-99 wt%, 50-99 wt%, 55-99 wt%, 60-99 wt%, or 65-99 wt% of the compound of Formula I or a pharmaceutically acceptable salt thereof.

In some embodiments, the pharmaceutical composition can be formulated into a unit dosage form, each containing 0.01-1000 mg, 0.01-900 mg, 0.01-800 mg, 0.01-700 mg, 0.01-600 mg, 0.01-500 mg, 0.01-400 mg, 0.01-300 mg, 0.01-200 mg, 0.01-100 mg, 0.01-50 mg, 0.05-900 mg, 0.05-800 mg, 0.05-700 mg, 0.05-600 mg, 0.05-500 mg, 0.05-400 mg, 0.05-300 mg, 0.05-200 mg, 0.05-100 mg, 0.05-50 mg, 0.1-1000 mg, 0.1-900 mg, 0.1-800 mg, 0.1-700 mg, 0.1-600 mg, 0.1-500 mg, 0.1-400 mg, 0.1-300 mg, 0.1-200 mg, 0.1-100 mg, or 0.1-50 mg of the compound of Formula I or a pharmaceutically acceptable salt thereof. The term "unit dosage form" as used herein refers to a physically discrete unit suitable as a unit dose, each unit containing a predetermined quantity of active ingredient calculated to produce the desired therapeutic effect, in association with a suitable pharmaceutical carrier. Examples of such unit dosage forms are tablets (including scored or coated tablets), capsules, pills, powders, wafers, suppositories, injectable solutions or suspensions, and similar dosage forms, and their divided multi-dose form. Of course, the daily dose of the compound of the present application will vary according to the compound used, the mode of administration, the desired treatment and the specific disease treated. In some embodiments, the daily dose of the compound of the present application is 0.01-200 mg/kg body weight administered once, or 0.01-100 mg/kg body weight administered seperately. Regardless of the administration method, the optimal dose for an individual depends on the specific treatment. Generally, starting from a small dose, the dose is gradually increased until the most suitable dose is found.

### Drug Combination

In one aspect, the present application provides a drug combination comprising the compound of the present application or a pharmaceutically acceptable salt thereof and at least one additional active agent.

In some embodiments, the drug combination is used to treat or prevent RIP 1 kinase-mediated diseases or disorders, or diseases or disorders caused by programmed cell necrosis.

In some embodiments, the at least one additional active agent includes, but is not limited to, a thrombolytic agent, a tissue-type plasminogen activator, an anticoagulant, a platelet aggregation inhibitor, an antimicrobial agent (antibiotics, broad-spectrum antibiotics, β-lactam, anti-mycobacterial drugs, bactericidal antibiotics, and anti-MRSA therapy), a long-acting beta agonist, combination of an inhalation corticosteroid and a long-acting beta agonist, a short-acting beta agonist, a leukotriene modulator, anti-IgE, a methylxanthine bronchodilator, a mast cell inhibitor, a protein tyrosine kinase inhibitor, a CRTH2/D-type prostaglandin receptor antagonist, an adrenaline inhalation aerosol, a phosphodiesterase inhibitor, combination of a phosphodiesterase-3 inhibitor and a phosphodiesterase-4 inhibitor, a long-acting inhalation anticholinergic drug, a muscarinic antagonist, a long-acting muscarinic antagonist, a low-dose steroids, an inhalation corticosteroid, an oral corticosteroid, a topical corticosteroid, an antithymocyte globulin, thalidomide, chlorambucil, a calcium channel blocker, a topical skin moisturizer, an ACE inhibitor, a serotonin reuptake inhibitor, an endothelin-1 receptor inhibitors, an anti-fibrosis agent, a proton pump inhibitor, cystic fibrosis transmembrane conductance regulator, mucolytics, a pancreatin, a bronchodilator, an intravitreal injection, an anti-vascular endothelial growth factor inhibitor, a ciliary neurotrophic growth factor, a trivalent (IIV3) inactivated influenza vaccine, a quadrivalent (IIV4) inactivated influenza vaccine, a trivalent recombinant influenza vaccine, a tetravalent live attenuated influenza vaccine, an antiviral agent, an inactivated influenza vaccine, a ciliary neurotrophic growth factor, a gene transfer agent, an immunomodulator, a calcineurin inhibitor, interferon y, antihistamine, a PD-1 inhibitor, a PD-L1 inhibitor, a monoclonal antibody, a polyclonal anti-T cell antibody, an anti-thymocyte gamma globulin horse antibody, an anti-thymocyte globulin rabbit antibody, an anti-CD40 antagonist, a JAK inhibitor and an anti-TCR mouse mAb.

In some embodiments, the compound of the present application or a pharmaceutically acceptable salt thereof and the at least one additional active agent are administered separately, simultaneously, or sequentially in any order.

### Treatment methods and indications

The compound of the present application or a pharmaceutically acceptable salt thereof is capable of inhibiting the activity of RIP1 kinase. Therefore, in one aspect, the present application provides a method for treating or preventing RIP1 kinase-mediated diseases or disorders, or diseases or disorders caused by programmed cell necrosis, which comprises administering to a subject an effective amount of the compound of the present application or a pharmaceutically acceptable salt thereof, or the pharmaceutical composition or the drug combination of the present application.

The term "subject" as used herein refers to an organism, tissue or cell. The subjects may include human subjects for medical purposes (e.g., diagnosis and/or treatment of existing conditions or diseases, or prophylactic treatment to prevent the onset of conditions or diseases), or animal subjects for medical or veterinary purposes or development purposes. The subjects also include sample materials from tissue culture, cell culture, organ replication, and stem cell production, etc. Suitable animal subjects include mammals and birds. The term "mammal" as used herein includes, but is not limited to, primates (such as humans, monkeys, and apes, etc.), bovine (such as bulls, etc.), sheep (such as sheep, goats, etc.), pigs, horses, cats, dogs, rabbits, rodents (e.g. mice, rats, etc.), and the like. The term "birds" as used herein includes, but is not limited to, chickens, ducks, geese, quail, turkeys, and pheasants, etc. In some embodiments, the subject is a mammal or a mammalian cell. In some embodiments, the subject is a human or human cell. The human subjects include, but are not limited to, fetus, newborn, toddler, adolescent and adult subjects. In addition, the "subject" may include patients suffering from or suspected of suffering from a certain condition or disease. Therefore, the terms "subject" and "patient" are used interchangeably herein. The subject can also refer to the cells in the laboratory or the bioprocessing media in the tests.

The term "effective amount" as used herein refers to an amount of a drug or pharmaceutical agent that will cause a biological or medical response pursued, for example, by a researcher or clinician, in a tissue, system, animal or human. In addition, the term "therapeutically effective amount" means any amount that results in improved treatment, healing, prevention or alleviation of a disease, disorder, or side effect, or decreased development of a disease or disorder, compared to a corresponding subject that does not receive such an amount. The term also includes, within its scope, an amount effective to enhance normal physiological functions. The therapeutically effective amount of one or more compounds of the present application is known to a skilled person, or can be easily determined by standard methods known in the art.

In some embodiments, the RIP1 kinase-mediated diseases or disorders or diseases or disorders caused by programmed cell necrosis are selected from the group consisting of inflammatory bowel disease, Crohn's disease, ulcerative colitis, psoriasis, retinal degenerative disease, retinal detachment, retinitis pigmentosa, macular degeneration, pancreatitis, atopic dermatitis, rheumatoid arthritis, spondyloarthritis, gout, SoJIA, systemic lupus erythematosus, Sjogren's syndrome, systemic scleroderma, antiphospholipid syndrome, vasculitis, osteoarthritis, non-alcoholic steatohepatitis, alcoholic steatohepatitis, autoimmune hepatitis, hepatitis B, hepatitis C, autoimmune hepatobiliary disease, primary sclerosing cholangitis, acetaminophen poisoning, liver toxicity, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic disease, asthma, atopic dermatitis, multiple sclerosis, Diabetes Type I, Wegener's granuloma, pulmonary sarcoidosis, Behcet's disease, Interleukin-1 converting enzyme-related fever syndrome, chronic obstructive pulmonary disease, tumor necrosis factor receptor related periodic syndrome, periodontitis, stroke, burns, burn shock, traumatic brain injury, atherosclerosis, cisplatin-induced kidney injury, acute kidney injury, pancreatitis, chronic kidney disease, acute respiratory distress syndrome, chronic obstructive pulmonary disease, Gaucher disease, Niemann Pick's disease, acute liver failure, cancers (e.g. pancreatic cancer), bacterial infection, smoking-induced injury, cystic fibrosis, NF-κ-B key regulatory gene mutation, heme-oxidized IRP2 ubiquitin ligase-1 deficiency, chain ubiquitin chain assembly complex deficiency syndrome, hematological malignancies, solid organ malignancies, influenza, staphylococcal infections, mycobacterial infections, lysosomal storage diseases, GM2 gangliosidosis, α-mannosidosis, aspartylglucosaminuria, cholesterol ester storage disease, chronic hexosaminidase A deficiency, cystinosis, Danon disease, Fabry disease, Farber disease, fucosidosis, galactosialidosis, GM1 gangliosidosis, mucolipid accumulation, infantile free sialic acid storage disease, juvenile hexosaminidase A deficiency, Krabbe disease, lysosomal acid lipase deficiency, metachromatic leukodystrophy, mucopolysaccharidosis, multiple sulfatase deficiency, neuronal ceroid lipofuscinoses, Pompe disease, pycondysostosis, Sandhoff's Disease, Schindler's Disease, Salla disease, Tay-Sachs disease, Wolman disease, Stevens-Johnson syndrome, and toxic epidermal necrolysis.

The present application also includes, but is not limited to, the following embodiments:

Item 1. A compound of Formula (I), or an optical isomer, a tautomer or a pharmaceutically acceptable salt thereof: 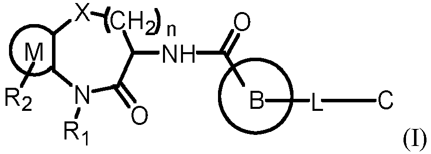 wherein
X is O, S or CH₂;
ring M has a structure of 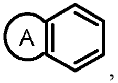 in which ring A is selected from the group consisting of substituted or unsubstituted 5- to 6-membered heteroaryl, and substituted or unsubstituted 5- to 6-membered heterocyclyl, wherein the ring skeleton of the heteroaryl group or heterocyclyl group has one or more heteroatoms selected from N, O or S;
n is selected from 1, 2 or 3;
B is selected from the group consisting of substituted or unsubstituted phenyl, substituted or unsubstituted 5- to 6-membered heteroaryl, and substituted or unsubstituted 5-to 6-membered heterocyclyl;
L is selected from the group consisting of O, S, NH, N(CH₃), substituted or unsubstituted C₁-C₆ alkylene, substituted or unsubstituted C₁-C₆ alkylene-O-, substituted or unsubstituted C₁-C₆ alkylene-NH-, (substituted or unsubstituted C₁-C₆ alkylene)₂-N-, substituted or unsubstituted C₃-C₆ alkenylene, and substituted or unsubstituted C₃-C₆ alkenylene-O-;
C is selected from the group consisting of H, substituted or unsubstituted (C₃-C₆) cycloalkyl, substituted or unsubstituted phenyl, substituted or unsubstituted 5- to 6-membered heteroaryl, and substituted or unsubstituted 5- to 6-membered heterocyclyl;
R₁ is selected from H, or substituted or unsubstituted C₁-C₆ alkyl;
R₂ is one or more substituents on the phenyl ring selected from the group consisting of H, halo, halo substituted or unsubstituted C₁-C₆ alkyl, and C₁-C₆ acyl;
wherein unless otherwise indicated, "substituted" refers to the substitution of one or more hydrogen atoms on the group with a substituent selected from the group consisting of halo, cyano, alkyl, acyl, sulfonyl, hydroxyl, amino, benzyl, oxo (=O), (C₁-C₄) alkyl, halo(C₁-C₄) alkyl, (C₁-C₄)alkoxy, halo(C₁-C₄)alkoxy, nitro, and (C₁-C₄) alkylC(O)-; and
the configuration of each chiral center is independently R-configuration or S-configuration.

Item 2. The compound according to Item 1, where C is substituted or unsubstituted phenyl, or substituted or unsubstituted 5- to 6-membered heteroaryl.

Item 3. The compound according to Item 1, where L is substituted or unsubstituted C₁-C₆ alkylene.

Item 4. The compound according to Item 1, where ring A is a 5-membered ring having one or more N atoms on the ring skeleton.

Item 5. A method for preparing the compound according to Item 1, comprising Step (a) or (b): 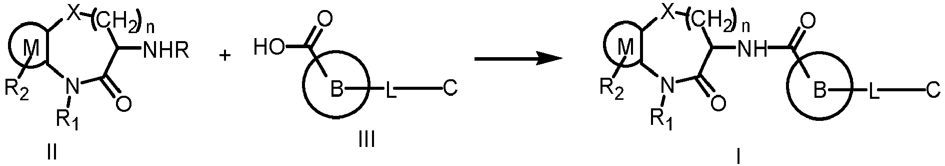
(a) reacting a compound of Formula II with a compound of Formula III in an inert solvent in the presence of a condensation reagent and a base, to obtain a compound of Formula I, wherein R is H; or
(b) deprotecting the amino protecting group from the compound of Formula II under an acidic condition, and then reacting with the compound of Formula III in an inert solvent in the presence of a condensation reagent and a base, to obtain a compound of Formula I, wherein R is an amino protecting group (preferably Boc), and other groups are as defined in Item 1.

Item 6. A pharmaceutical composition, comprising (a) a therapeutically effective amount of the compound of Formula I, or an optical isomer, a tautomer, or a pharmaceutically acceptable salt thereof, or a combination thereof; and (b) a pharmaceutically acceptable carrier.

Item 7. Use of the compound of Formula I according to Item 1, or a pharmaceutically acceptable salt thereof, a racemate, a R-isomer, an S-isomer or a mixture thereof in the manufacture of a pharmaceutical composition for treating or preventing RIP 1 kinase-mediated diseases or disorders; or in the manufacture of a pharmaceutical composition for treating or preventing diseases or disorders caused by programmed cell necrosis.

Item 8. The use according to Item 7, where the diseases or disorders are selected from the group consisting of inflammatory bowel disease, Crohn's disease, ulcerative colitis, psoriasis, retinal detachment, retinitis pigmentosa, macular degeneration, pancreatitis, atopic dermatitis, rheumatoid arthritis, spondyloarthritis, gout, SoJIA, systemic lupus erythematosus, Sjogren's syndrome, systemic scleroderma, antiphospholipid syndrome, vasculitis, osteoarthritis, non-alcoholic steatohepatitis, alcoholic steatohepatitis, autoimmune hepatitis, autoimmune hepatobiliary disease, primary sclerosing cholangitis, acetaminophen poisoning, liver toxicity, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic disease, asthma, atopic dermatitis, multiple sclerosis, Diabetes Type I, Wegener's granuloma, pulmonary sarcoidosis, Behcet's disease, Interleukin-1 converting enzyme-related fever syndrome, chronic obstructive pulmonary disease, tumor necrosis factor receptor related periodic syndrome, and periodontitis.

Item 9. An intermediate compound of Formula II below: 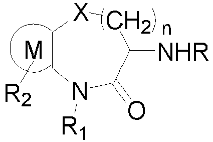 wherein R is selected from the group consisting of H and an amino protecting group (preferably Boc); and other groups are as defined in claim 1.

Item 10. The intermediate compound according to Item 9, selected from the group consisting of:

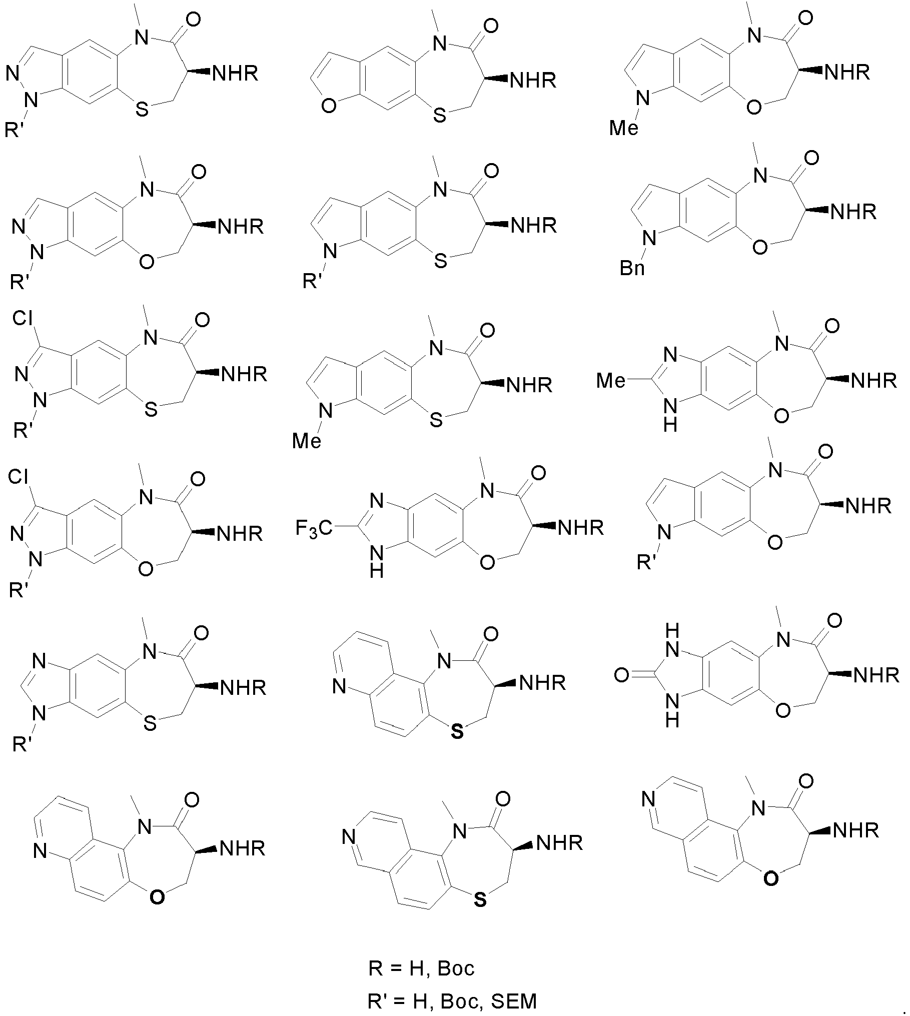

The present application provides a new class of compounds that inhibit the activity of RIP1 kinase. Compared with the existing compounds, the compound of the present application has a better inhibitory effect against programmed cell necrosis, and has improved selectivity and pharmacokinetics.

### Examples

The present application is further described below in conjunction with specific examples. It is to be understood that these examples are merely illustrative of the present application and are not intended to limit the scope of the present application. In the experimental methods that do not indicate specific conditions in the following examples, conventional conditions or the conditions recommended by the manufacturer are employed. Unless otherwise stated, the percentages and parts are by weight.

### Example 1. Synthesis of exemplary Compounds RIP1-001, RIP1-003, RIP1-023, RIP1-136, and RIP1-182

**Method 1:**

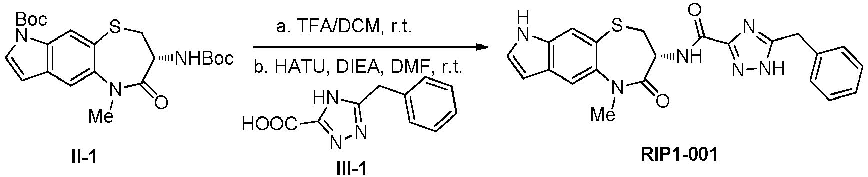

Compound **II-1** (27.8 mg, 0.08 mmol) was placed into a 25 mL single-neck flask, and then TFA (1 mL) and DCM (4 mL) were added and reacted at room temperature for 30 min, until the reaction was completed as indicated by TLC. The solvent was removed under reduced pressure, and the residue was dried under vacuum and re-dissolved in DMF (4 mL). HATU (38 mg, 0.1 mmol), DIEA (51.7 mg, 0.4 mmol), and 5-benzyl-4*H*-1,2,4-triazole-3-carboxylic acid **III-1** (20.3 mg, 0.1 mmol) were added, and reacted overnight at room temperature, until the reaction was completed as indicated by TLC. The reaction solution was extracted with EA, washed with deionized water and saturated NaCl, and dried over Na₂SO₄. The solvent was removed under reduced pressure, and the residue was separated by reversed-phase column chromatography, and freeze dried to obtain **RIP1-001:** white solid 14.0 mg (40.4%).

By changing the compound of Formula II and the compound of Formula III, Compounds RIP 1-002, RIP1-013 to RIP 1-020, RIP 1-026 to RIP 1-030, RIP1-036 to RIP1-040, RIP 1-092 to RIP1-135, RIP1-145 to RIP1-150, RIP1-156 to RIP1-165, RIP1-168 to RIP1-180, RIP1-187 to RIP1-189, RIP1-193 to RIP1-205, RIP1-207 to RIP1-210, and RIP1-214 to RIP1-215 could also be prepared by Method 1.

### Method 2:

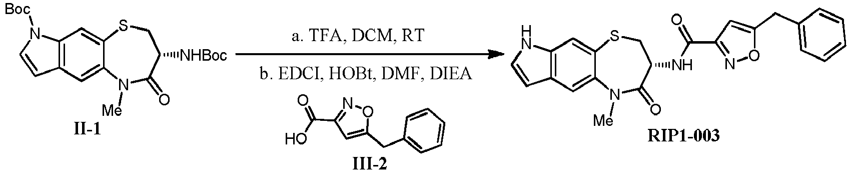

**II-1** (30.0 mg) was dissolved in dichloromethane (1 mL), and then TFA (0.3 mL) was added and stirred at room temperature for 3 h. The dichloromethane and TFA were removed by rotary evaporation to obtain a Boc-deprotected aminotrifluoroacetate.

The Boc-deprotected intermediate obtained in the previous step (17.7 mg) was dissolved in DMF (1 mL), and then DIEA (43.3 mg), EDCI (19.3 mg), and HOBt (13.6 mg) were added in sequence, stirred at normal temperature for 1 h, and then cooled to 0°C. The trifluoroacetate obtained in the previous step was dissolved in DMF (1 mL), and then slowly added dropwise into the reaction solution. The reaction solution was transferred to room temperature and continuously reacted overnight. After the reaction was completed, water (10 mL) was slowly added, stirred for 0.5 h, and extracted with ethyl acetate (10 mL). The ethyl acetate layer was collected, dried, rotary evaporated to dryness, and the residue was separated by column chromatography on silica gel (eluent: petroleum ether:ethyl acetate=3:1) to obtain **RIP1-003** (19.0 mg, yield 66%).

By changing the compound of Formula II and the compound of Formula III, Compounds RIP1-004 to RIP1-012, RIP1-041 to RIP1-058, and RIP1-061 to RIP1-088 could also be prepared by Method 2.

### Method 3:

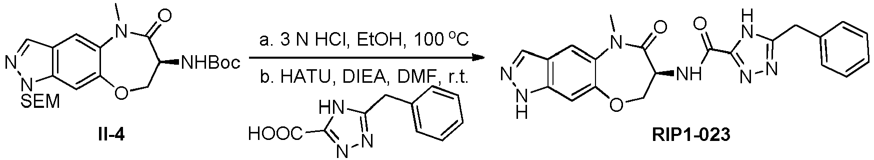

Compound **II-4** (27.8 mg, 0.06 mmol) was placed into a 15 mL sealing tube, and then 3 N HCl (1 mL) and EtOH (0.5 mL) were added, and reacted at 100°C for 2 h, until the reaction was completed as indicated by TLC. The solvent was removed under reduced pressure, and the residue was dried under vacuum and re-dissolved in DMF (4 mL). HATU (28.5 mg, 0.075 mmol), DIEA (38.8 mg, 0.3 mmol), and 5-benzyl-4*H*-1,2,4-triazole-3-carboxylic acid (15.2 mg, 0.075 mmol) were added, and reacted overnight at room temperature, until the reaction was completed as indicated by TLC. The reaction solution was extracted with EA, washed with deionized water and saturated NaCl, and dried over Na₂SO₄. The solvent was removed under reduced pressure, and the residue was separated by reversed-phase column chromatography, and freeze dried to obtain **RIP1-023:** white solid 10 mg (40%).

By changing the compound of Formula II and the compound of Formula III, Compounds RIP1-021 to RIP1-025, RIP1-031 to RIP1-035, RIP1-059 to RIP1-060, RIP1-089 to RIP1-091 and RIP1-190 could also be prepared by Method 3.

### Method 4:

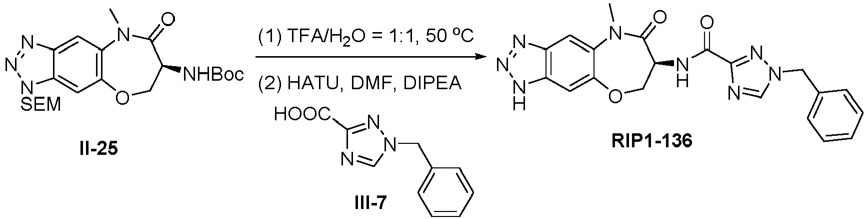

Compound **II-25** (1.0 equiv) was placed into a single-neck flask, and then a mixed solvent of TFA/H₂O (1:1) was added, and reacted at 50°C for 2 h, until the reaction was completed as indicated by TLC. The solvent was removed under reduced pressure, and the residue was dried under vacuum and re-dissolved in anhydrous DMF. HATU (1.2 equiv), DIPEA (5 equiv), and the crude product obtained after compound Step **1)** (1.1 equiv) were added, and reacted overnight at room temperature, until the reaction was completed as indicated by TLC. The reaction solution was extracted with ethyl acetate, washed with deionized water and saturated saline, and dried over anhydrous sodium sulfate. The solvent was removed under reduced pressure, and the residue was separated by reversed-phase column chromatography, and freeze dried to obtain Compound **RIP1-136.**

By changing the compound of Formula II and the compound of Formula III, Compounds RIP1-137 to RIP1-143, RIP1-153, RIP1-166 to RIP1-167 and RIP1-181 could also be prepared by Method 4.

### Method 5:

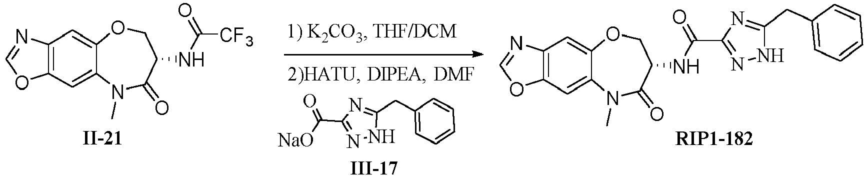

**II-21** (60 mg, 0.1823 mmol) and K₂CO₃ (51 mg, 0.3646 mmol) were added to a 50 mL reaction flask, and then THF (3 mL) and H₂O (3 mL) were added and reacted overnight at room temperature. After the reaction was completed, the solvent was removed by rotary evaporation. HATU (84 mg, 0.2187 mmol), **III-1** (43 mg, 0.1914 mmol), and DIPEA (150 µL, 0.9118 mmol) were added, and then DMF (4 mL) was added as a solvent. The reaction was continued at room temperature for 1 h. After the reaction was completed, reversed-phase column chromatography afforded **RIP1-182** (28 mg, yield 36.8%).

By changing the compound of Formula II and the compound of Formula III, Compounds RIP1-183 to RIP1-186, RIP1-206, RIP1-211 to RIP1-213, and RIP1-217 to RIP1-222 could also be prepared by Method 5.

The synthesis method, intermediates and yield of the compound of Formula (I) of the present application are summarized as follows:

| Synthesis method | Compound of Formula II | Compound of Formula III | Product (compound of Formula I) |
|---|---|---|---|
| Method 1 | 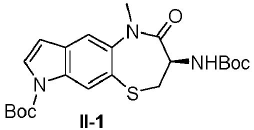 | 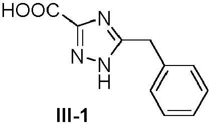 | 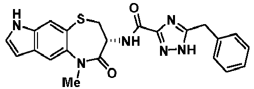 |
| | | | RIP1-001, yield: 32% |
| Method 1 | 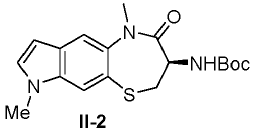 | 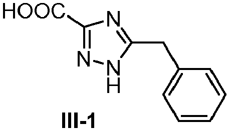 | 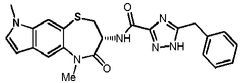 |
| | | | RIP1-002, yield: 30% |
| Method 2 | 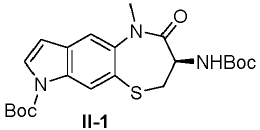 | 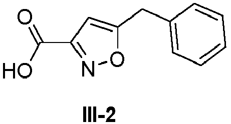 | 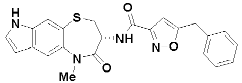 |
| | | | RIP1-003, yield: 65% |
| Method 2 | 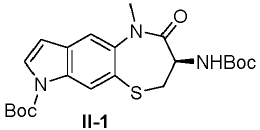 | 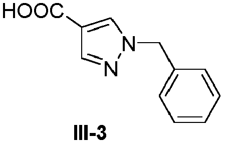 | 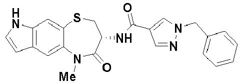 |
| | | | RIP1-004, yield: 60% |
| Method 2 | 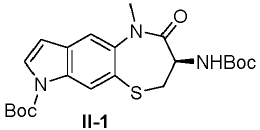 | 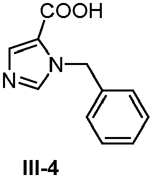 | 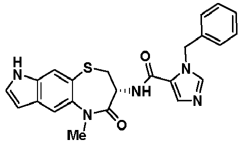 |
| | | | RIP1-005, yield: 49% |
| Method 2 | 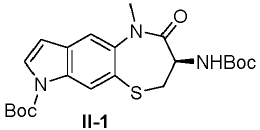 | 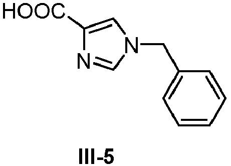 | 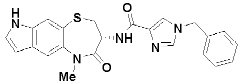 |
| | | | RIP1-006, yield: 49% |
| Method 2 | 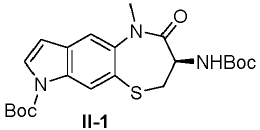 | 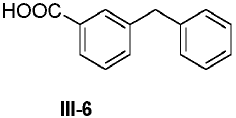 | 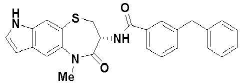 |
| | | | RIP1-007, yield: 62% |
| Method 2 | 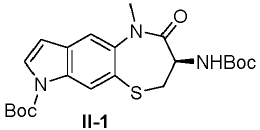 | 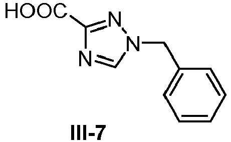 | 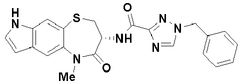 |
| | | | RIP 1-008, yield: 60% |
| Method 2 | 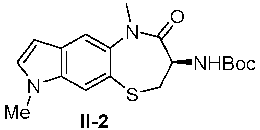 | 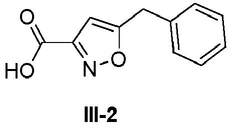 | 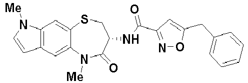 |
| | | | RIP1-009, yield: 61% |
| Method 2 | 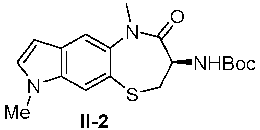 | 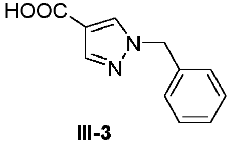 | 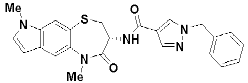 |
| | | | RIP1-010, yield: 69% |
| Method 2 | 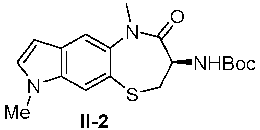 | 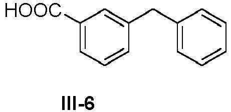 | 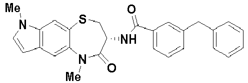 |
| | | | RIP 1-011, yield: 54% |
| Method 2 | 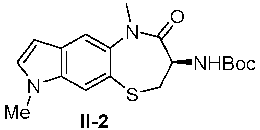 | 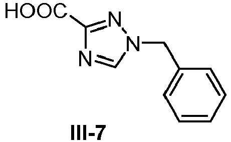 | 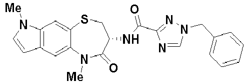 |
| | | | RIP1-012, yield: 68% |
| Method 1 | 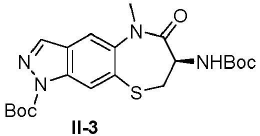 | 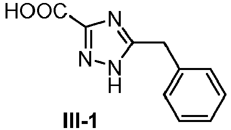 | 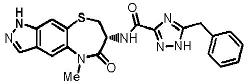 |
| | | | RIP1-013, yield: 40% |
| Method 1 | 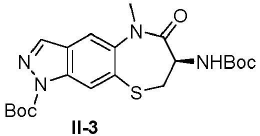 | 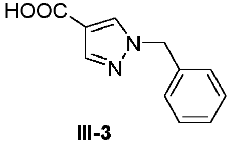 | 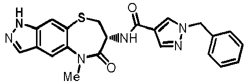 |
| | | | RIP1-014, yield: 43% |
| Method 1 | 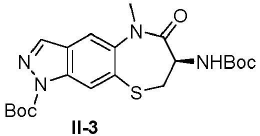 | 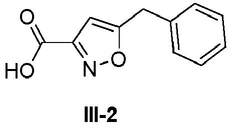 | 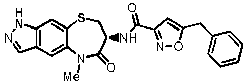 |
| | | | RIP 1-015, yield: 40% |
| Method 1 | 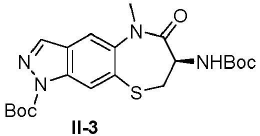 | 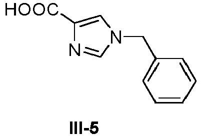 | 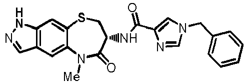 |
| | | | RIP1-016, yield: 58% |
| Method 1 | 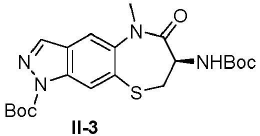 | 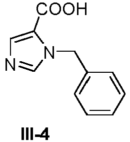 | 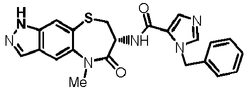 |
| | | | RIP1-017, yield: 66% |
| Method 1 | 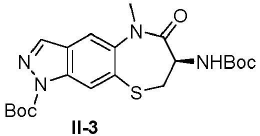 | 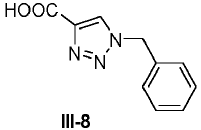 | 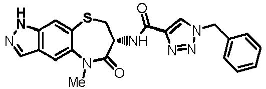 |
| | | | RIP1-019, yield: 38% |
| Method 1 | 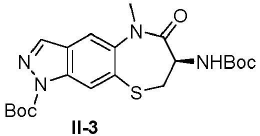 | 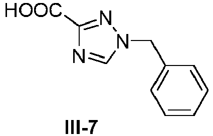 | 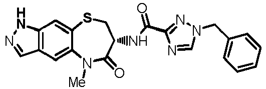 |
| | | | RIP1-020, yield: 49% |
| Method 3 | 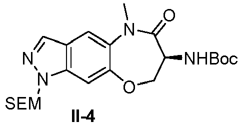 | 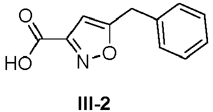 | 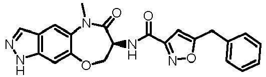 |
| | | | RIP1-021, yield: 36% |
| Method 3 | 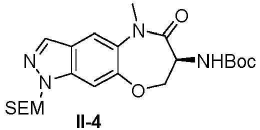 | 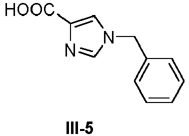 | 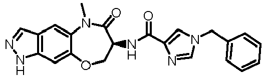 |
| | | | RIP1-022, yield: 31% |
| Method 3 | 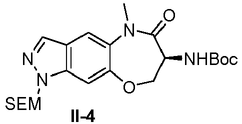 | 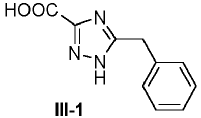 | 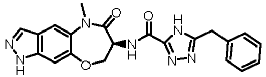 |
| | | | RIP1-023, yield: 40% |
| Method 3 | 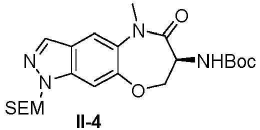 | 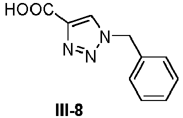 | 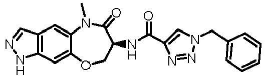 |
| | | | RIP1-024, yield: 41% |
| Method 3 | 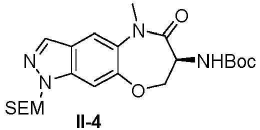 | 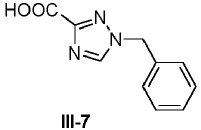 | 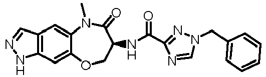 |
| | | | RIP1-025, yield: 37% |
| Method 1 | 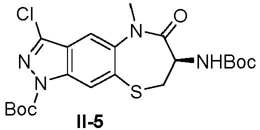 | 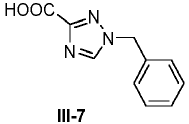 | 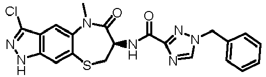 |
| | | | RIP1-026, yield: 35% |
| Method 1 | 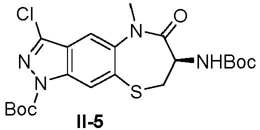 | 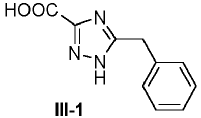 | 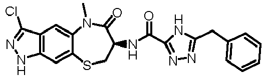 |
| | | | RIP1-027, yield: 35% |
| Method 1 | 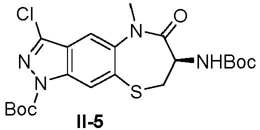 | 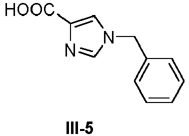 | 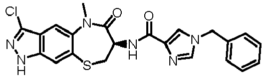 |
| | | | RIP1-028, yield: 17% |
| Method 1 | 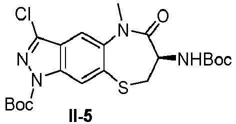 | 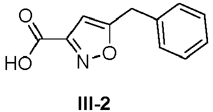 | 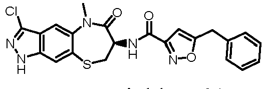 |
| | | | R1P1-029, yield: 21% |
| Method 1 | 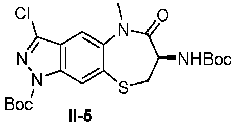 | 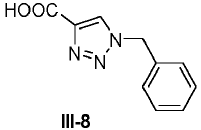 | 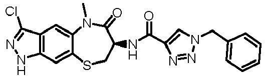 |
| | | | RIP1-030, yield: 31% |
| Method 3 | 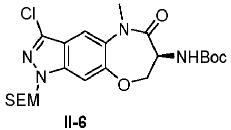 | 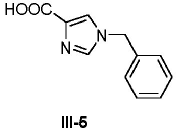 | 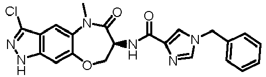 |
| | | | RIP1-031, yield: 43% |
| Method 3 | 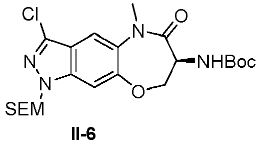 | 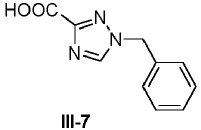 | 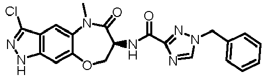 |
| | | | RIP1-032, yield: 39% |
| Method 3 | 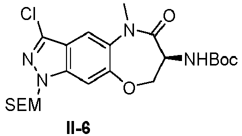 | 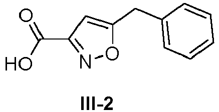 | 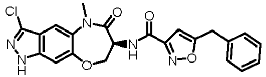 |
| | | | RIP1-033, yield: 40% |
| Method 3 | 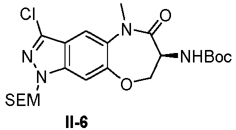 | 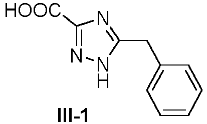 | 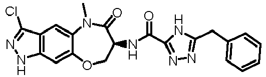 |
| | | | RIP1-034, yield: 34% |
| Method 3 | 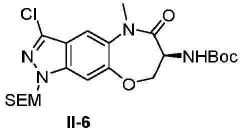 | 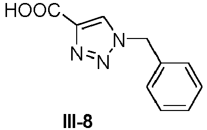 | 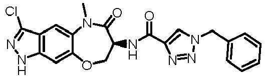 |
| | | | RIP1-035, yield: 47% |
| Method 1 | 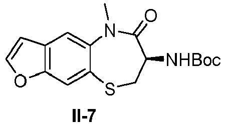 | 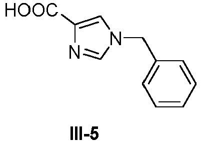 | 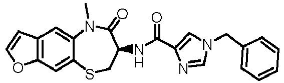 |
| | | | RIP1-036, yield: 77% |
| Method 1 | 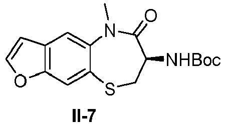 | 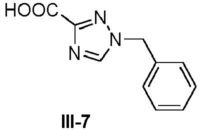 | 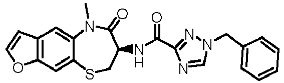 |
| | | | RIP1-037, yield: 87% |
| Method 1 | 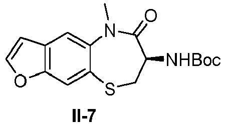 | 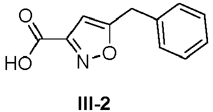 | 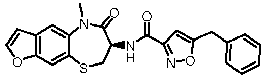 |
| | | | RIP1-038, yield: 65% |
| Method 1 | 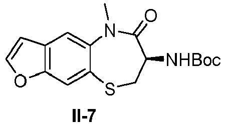 | 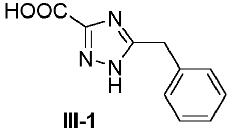 | 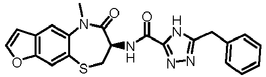 |
| | | | RIP1-039, yield: 59% |
| Method 1 | 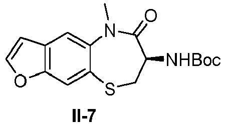 | 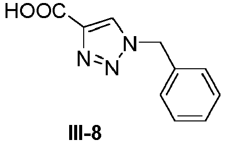 | 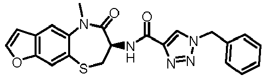 |
| | | | RIP 1-040, yield: 69% |
| Method 2 | 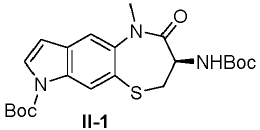 | 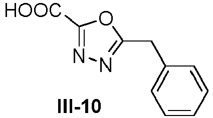 | 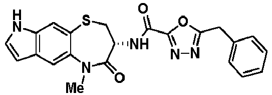 |
| | | | RIP1-041, yield: 50% |
| Method 2 | 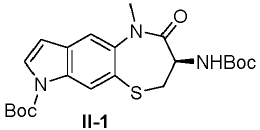 | 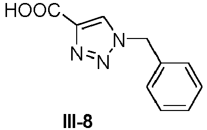 | 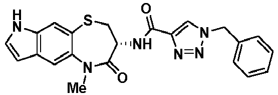 |
| | | | RIP1-042, yield: 68% |
| Method 2 | 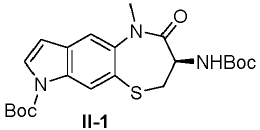 | 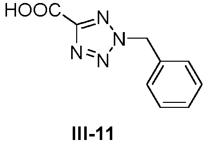 | 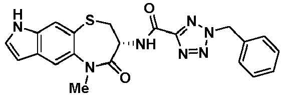 |
| | | | RIP1-043, yield: 60% |
| Method 2 | 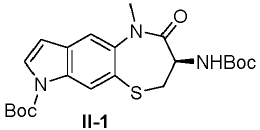 | 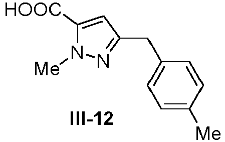 | 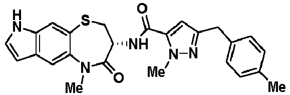 |
| | | | RIP1-044, yield: 56% |
| Method 2 | 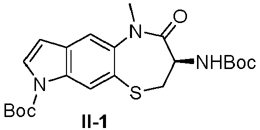 | 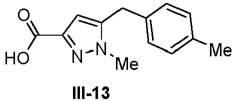 | 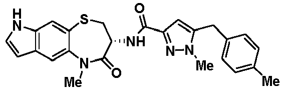 |
| | | | RIP1-045, yield: 61% |
| Method 2 | 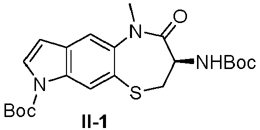 | 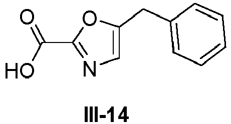 | 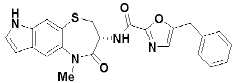 |
| | | | RIP1-046, yield: 65% |
| Method 2 | 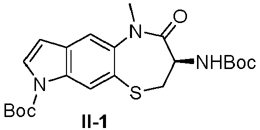 | 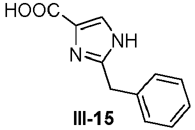 | 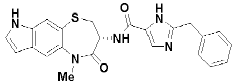 |
| | | | RIP1-047, yield: 43% |
| Method 2 | 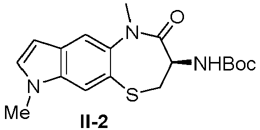 | 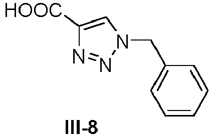 | 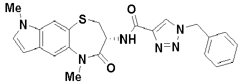 |
| | | | RIP1-048, yield: 60% |
| Method 2 | 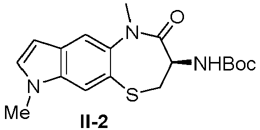 | 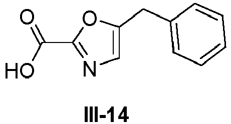 | 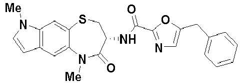 |
| | | | RIP1-049, yield: 47% |
| Method 2 | 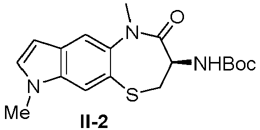 | 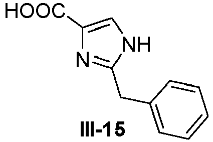 | 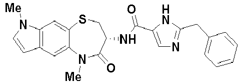 |
| | | | RIP1-050, yield: 45% |
| Method 2 | 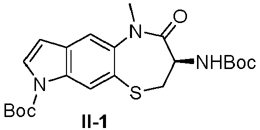 | 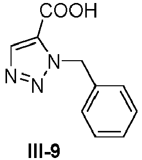 | 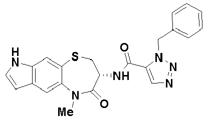 |
| | | | RIP1-051, yield: 51% |
| Method 2 | 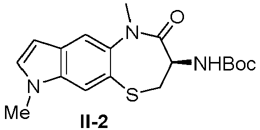 | 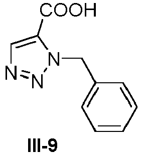 | 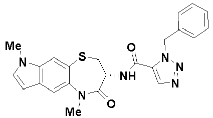 |
| | | | RIP1-052, yield: 68% |
| Method 2 | 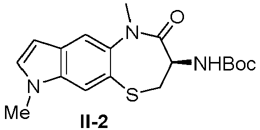 | 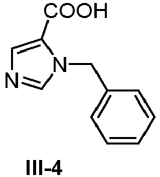 | 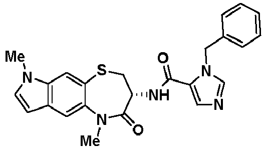 |
| | | | RIP1-054, yield: 61% |
| Method 2 | 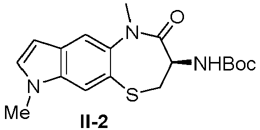 | 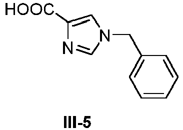 | 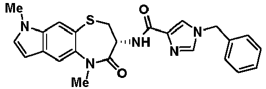 |
| | | | RIP1-055, yield: 55% |
| Method 2 | 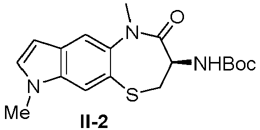 | 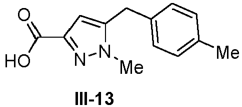 | 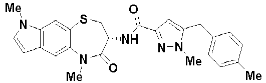 |
| | | | RIP1-056, yield: 63% |
| Method 2 | 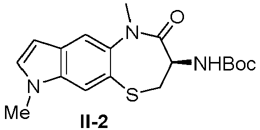 | 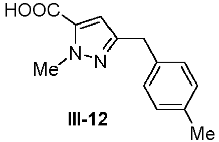 | 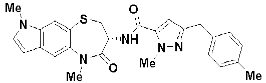 |
| | | | RIP1-057, yield: 60% |
| Method 2 | 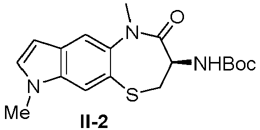 | 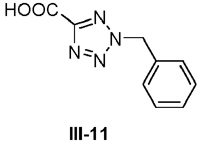 | 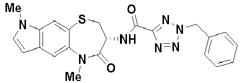 |
| | | | RIP1-058, yield: 50% |
| Method 3 | 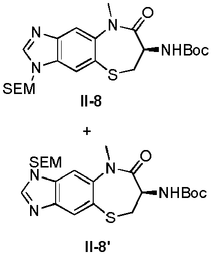 | 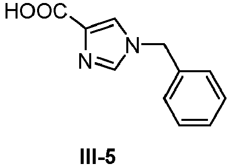 | 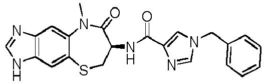 |
| | | | RIP1-059, yield: 17% |
| Method 3 | 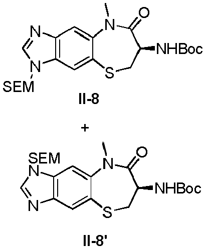 | 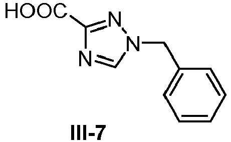 | 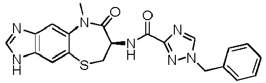 |
| | | | RIP1-060, yield: 32% |
| Method 2 | 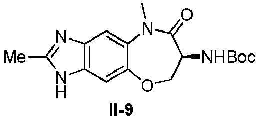 | 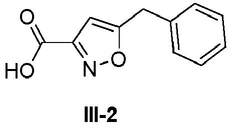 | 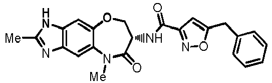 |
| | | | RIP1-061, yield: 54% |
| Method 2 | 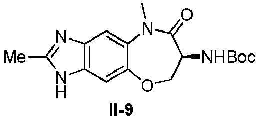 | 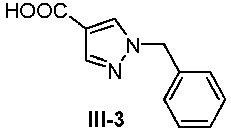 | 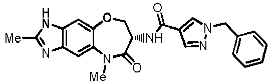 |
| | | | RIP 1-062, yield: 57% |
| Method 2 | 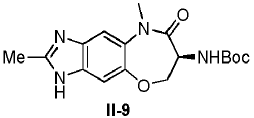 | 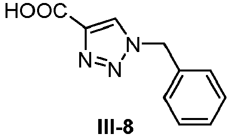 | 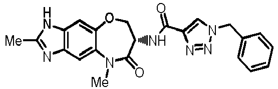 |
| | | | RIP1-063, yield: 65% |
| Method 2 | 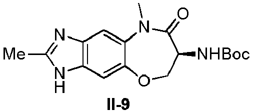 | 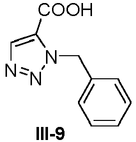 | 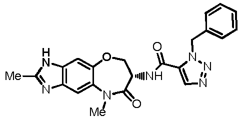 |
| | | | RIP1-064, yield: 62% |
| Method 2 | 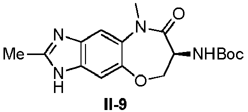 | 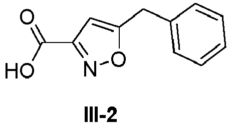 | 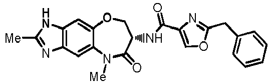 |
| | | | RIP1-065, yield: 54% |
| Method 2 | 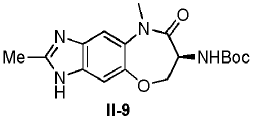 | 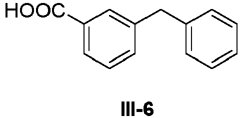 | 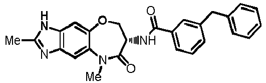 |
| | | | RIP1-066, yield: 51% |
| Method 2 | 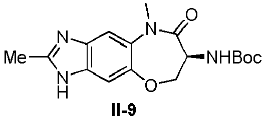 | 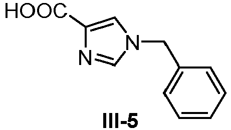 | 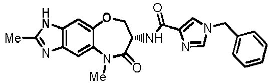 |
| | | | RIP1-067, yield: 68% |
| Method 2 | 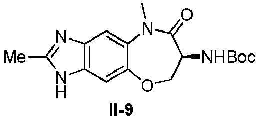 | 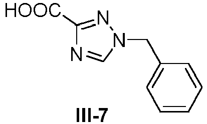 | 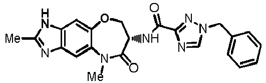 |
| | | | RIP1-068, yield: 70% |
| Method 2 | 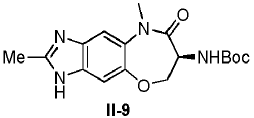 | 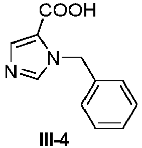 | 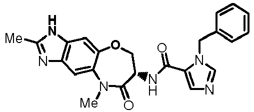 |
| | | | RIP1-069, yield: 68% |
| Method 2 | 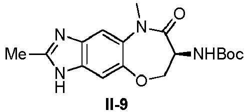 | 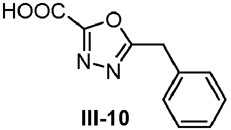 | 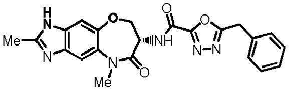 |
| | | | RIP1-070, yield: 54% |
| Method 2 | 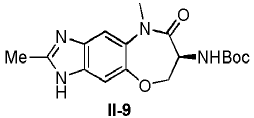 | 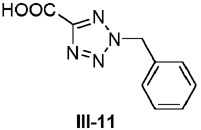 | 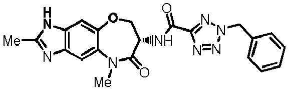 |
| | | | RIP1-071, yield: 59% |
| Method 2 | 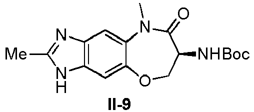 | 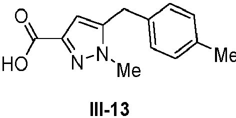 | 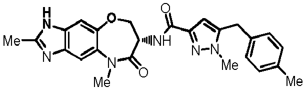 |
| | | | RIP1-072, yield: 68% |
| Method 2 | 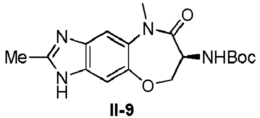 | 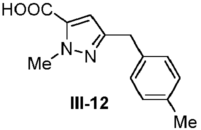 | 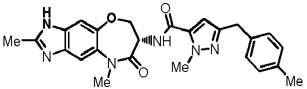 |
| | | | RIP1-073, yield: 63% |
| Method 2 | 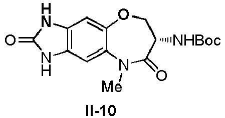 | 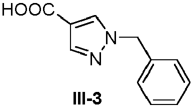 | 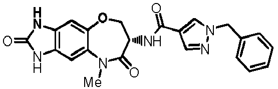 |
| | | | RIP1-074, yield: 61% |
| Method 2 | 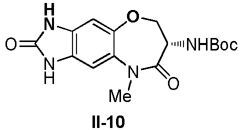 | 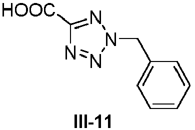 | 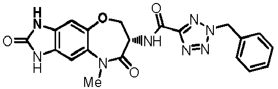 |
| | | | RIP1-075, yield: 52% |
| Method 2 | 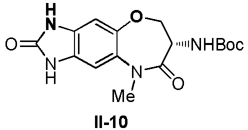 | 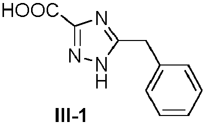 | 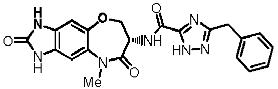 |
| | | | RIP1-076, yield: 30% |
| Method 2 | 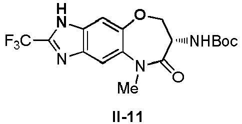 | 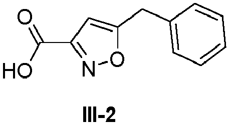 | 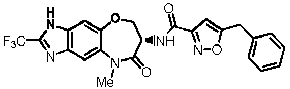 |
| | | | RIP 1-077, yield: 52% |
| Method 2 | 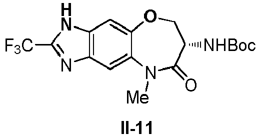 | 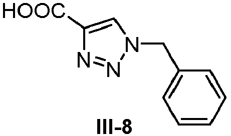 | 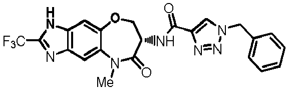 |
| | | | RIP1-078, yield: 65% |
| Method 2 | 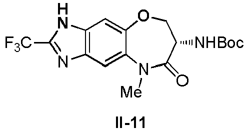 | 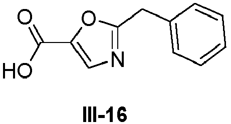 | 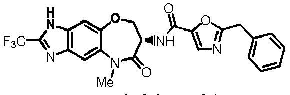 |
| | | | RIP1-079, yield: 62% |
| Method 2 | 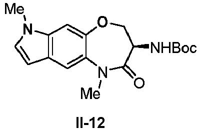 | 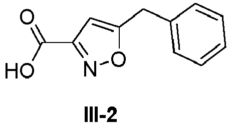 | 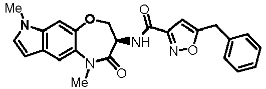 |
| | | | RIP1-080, yield: 63% |
| Method 2 | 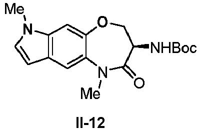 | 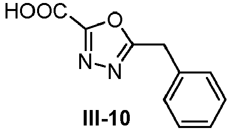 | 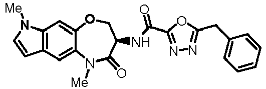 |
| | | | RIP1-081, yield: 55% |
| Method 2 | 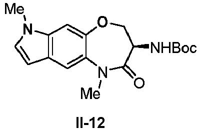 | 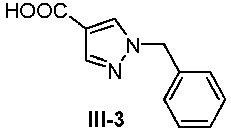 | 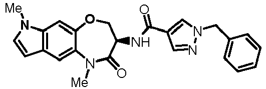 |
| | | | RIP1-082, yield: 66% |
| Method 2 | 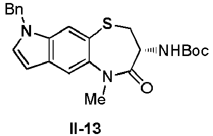 | 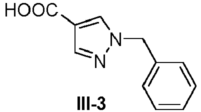 | 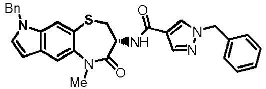 |
| | | | RIP1-083, yield: 55% |
| Method 2 | 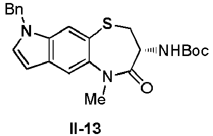 | 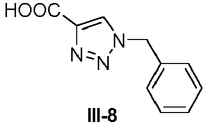 | 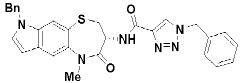 |
| | | | RIP1-084, yield: 64% |
| Method 2 | 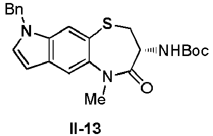 | 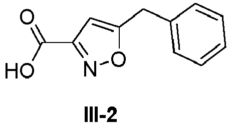 | 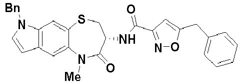 |
| | | | RIP1-085, yield: 53% |
| Method 2 | 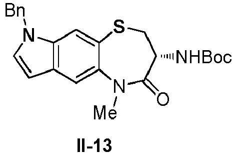 | 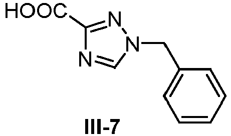 | 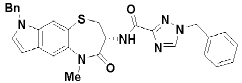 |
| | | | RIP1-086, yield: 64% |
| Method 2 | 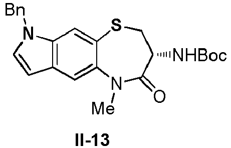 | 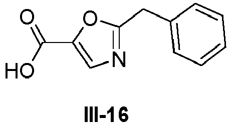 | 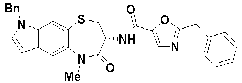 |
| | | | RIP1-087, yield: 64% |
| Method 2 | 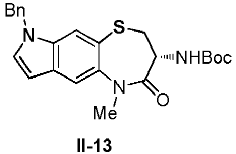 | 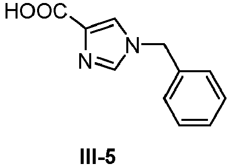 | 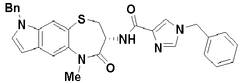 |
| | | | RIP1-088, yield: 72% |
| Method 3 | 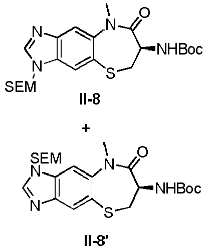 | 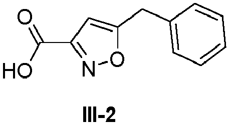 | 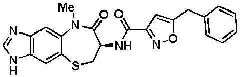 |
| | | | RIP1-089, yield: 30% |
| Method 3 | 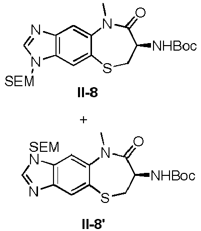 | 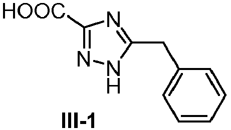 | 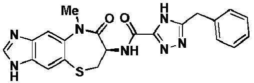 |
| | | | RIP1-090, yield: 32% |
| Method 3 | 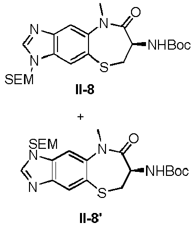 | 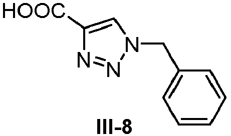 | 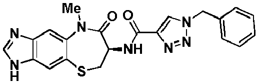 |
| | | | RIP1-091, yield: 36% |
| Method 1 | 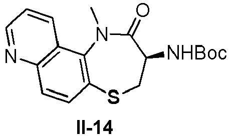 | 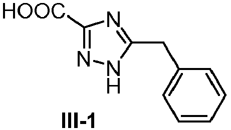 | 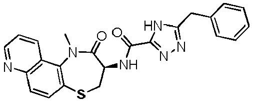 |
| | | | RIP1-092, yield: 55% |
| Method 1 | 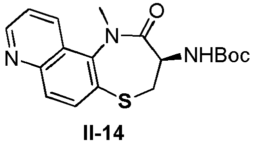 | 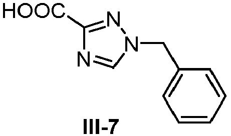 | 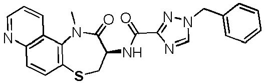 |
| | | | RIP1-093, yield: 63% |
| Method 1 | 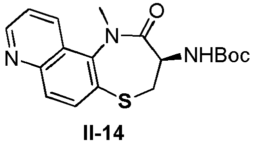 | 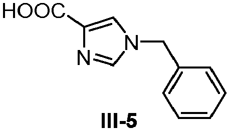 | 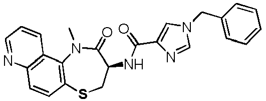 |
| | | | RIP1-094, yield: 61% |
| Method 1 | 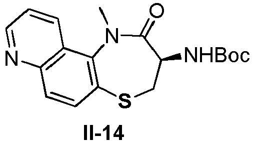 | 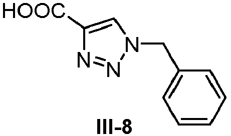 | 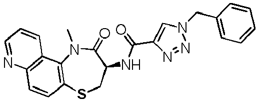 |
| | | | RIP1-095, yield: 67% |
| Method 1 | 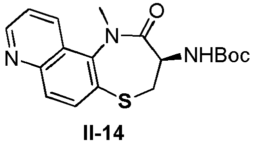 | 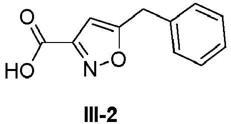 | 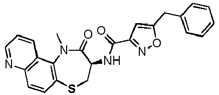 |
| | | | RIP1-096, yield: 59% |
| Method 1 | 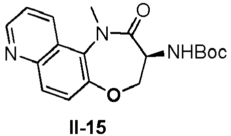 | 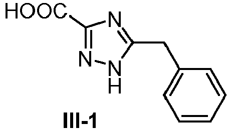 | 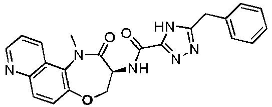 |
| | | | RIP1-097, yield: 56% |
| Method 1 | 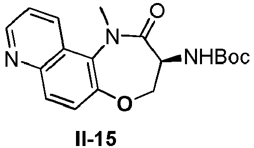 | 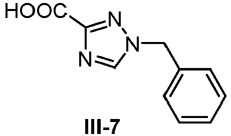 | 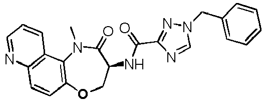 |
| | | | RIP1-098, yield: 50% |
| Method 1 | 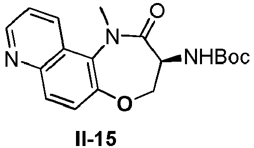 | 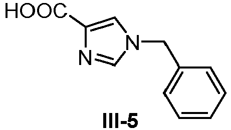 | 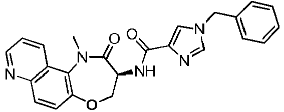 |
| | | | RIP1-099, yield: 58% |
| Method 1 | 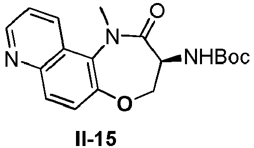 | 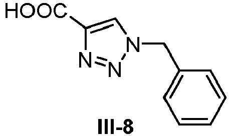 | 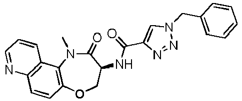 |
| | | | RIP1-100, yield: 62% |
| Method 1 | 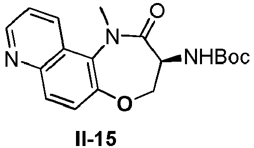 | 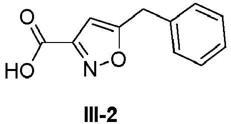 | 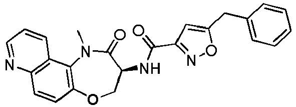 |
| | | | RIP1-101, yield: 68% |
| Method 1 | 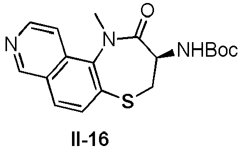 | 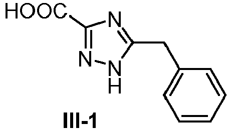 | 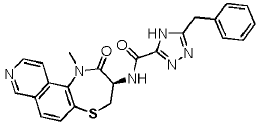 |
| | | | RIP1-102, yield: 61% |
| Method 1 | 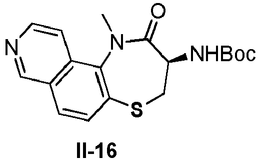 | 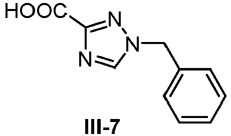 | 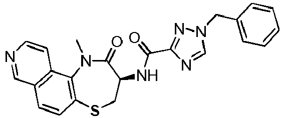 |
| | | | RIP1-103, yield: 51% |
| Method 1 | 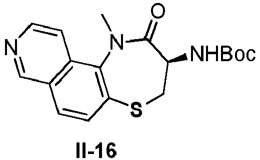 | 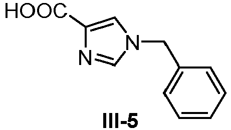 | 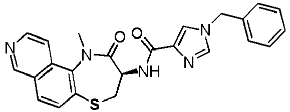 |
| | | | RIP1-104, yield: 63% |
| Method 1 | 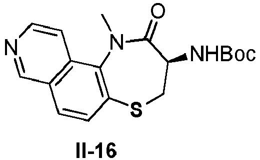 | 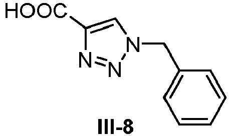 | 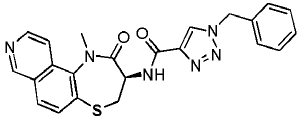 |
| | | | RIP1-105, yield: 54% |
| Method 1 | 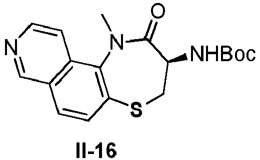 | 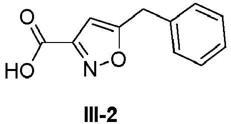 | 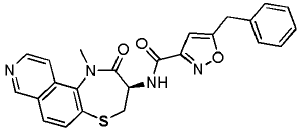 |
| | | | RIP1-106, yield: 65% |
| Method 1 | 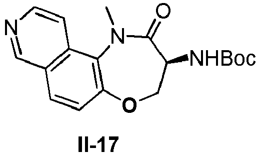 | 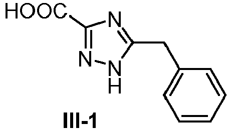 | 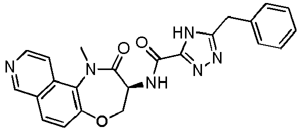 |
| | | | RIP1-107, yield: 53% |
| Method 1 | 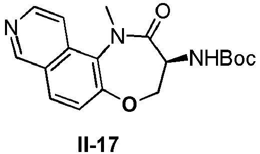 | 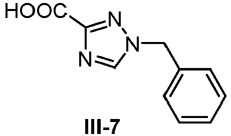 | 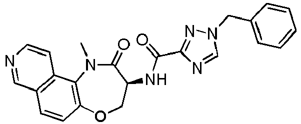 |
| | | | RIP1-108, yield: 68% |
| Method 1 | 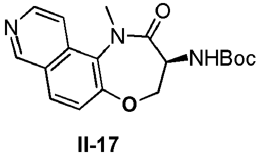 | 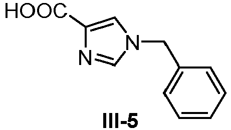 | 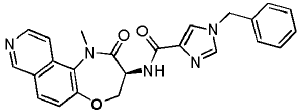 |
| | | | RIP1-109, yield: 60% |
| Method 1 | 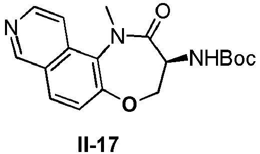 | 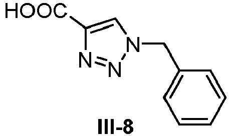 | 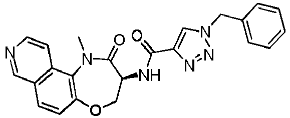 |
| | | | RIP1-110, yield: 65% |
| Method 1 | 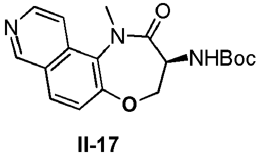 | 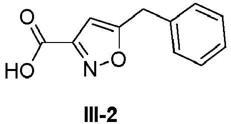 | 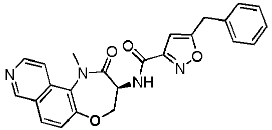 |
| | | | RIP1-111, yield: 68% |
| Method 1 | 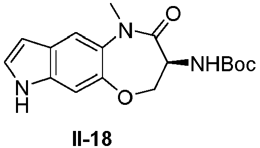 | 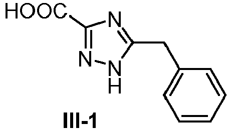 | 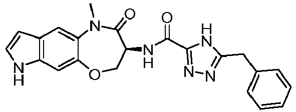 |
| | | | RIP1-112, yield: 35% |
| Method 1 | 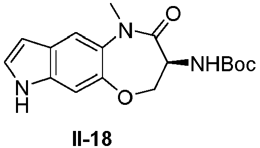 | 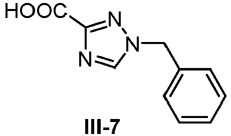 | 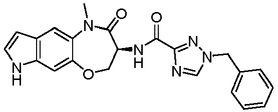 |
| | | | RIP1-113, yield: 41% |
| Method 1 | 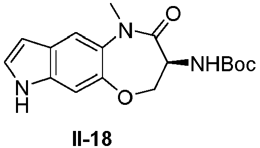 | 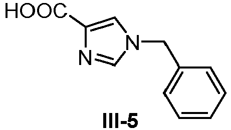 | 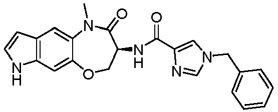 |
| | | | RIP1-114, yield: 38% |
| Method 1 | 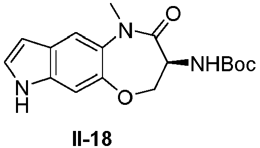 | 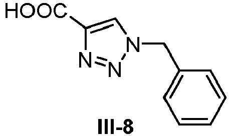 | 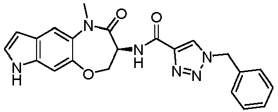 |
| | | | RIP1-115, yield: 47% |
| Method 1 | 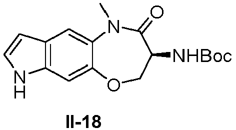 | 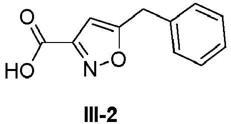 | 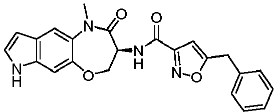 |
| | | | RIP1-116, yield: 54% |
| Method 1 | 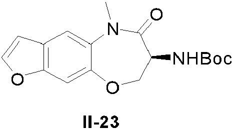 | 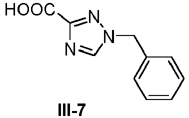 | 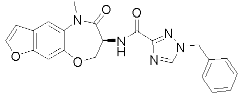 |
| | | | RIP1-119, yield: 95% |
| Method 1 | 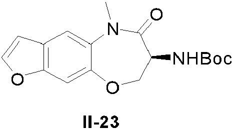 | 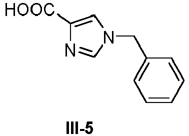 | 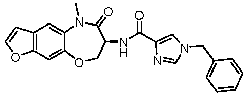 |
| | | | RIP1-120, yield: 96% |
| Method 1 | 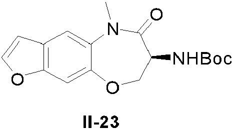 | 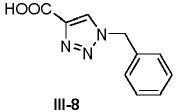 | 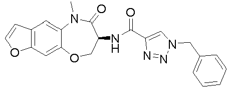 |
| | | | RIP1-121, yield: 92% |
| Method 1 | 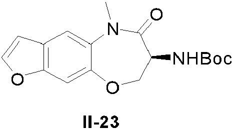 | 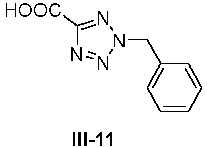 | 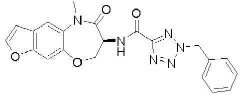 |
| | | | RIP1-122, yield: 92% |
| Method 1 | 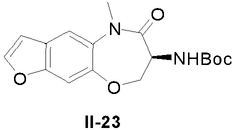 | 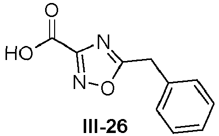 | 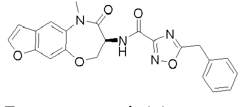 |
| | | | RIP1-123, yield: 82% |
| Method 1 | 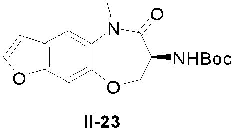 | 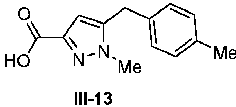 | 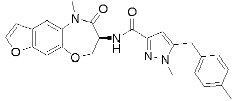 |
| | | | RIP1-124, yield: 90% |
| Method 1 | 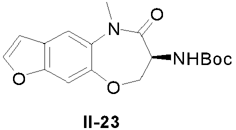 | 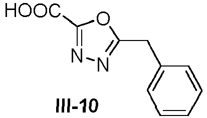 | 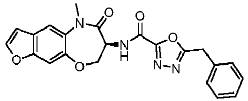 |
| | | | RIP1-125, yield: 56% |
| Method 1 | 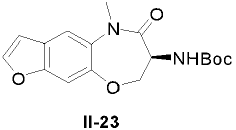 | 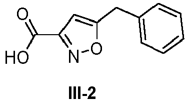 | 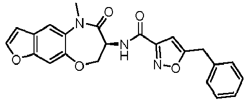 |
| | | | RIP1-126, yield: 93% |
| Method 1 | 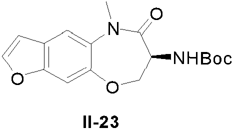 | 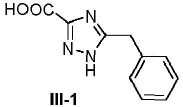 | 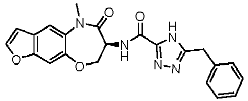 |
| | | | RIP1-127, yield: 69% |
| Method 1 | 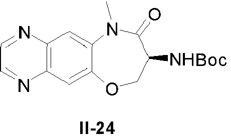 | 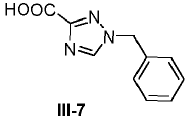 | 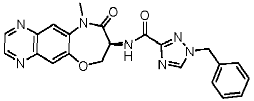 |
| | | | RIP1-128, yield: 56% |
| Method 1 | 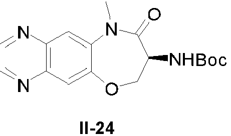 | 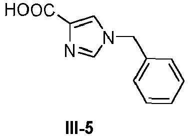 | 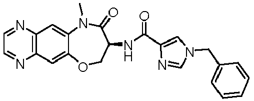 |
| | | | RIP1-129, yield: 67% |
| Method 1 | 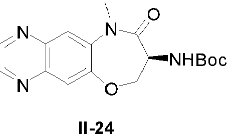 | 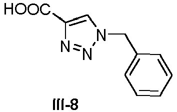 | 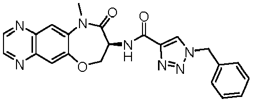 |
| | | | RIP1-130, yield: 64% |
| Method 1 | 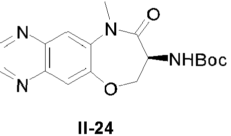 | 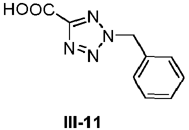 | 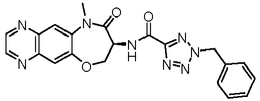 |
| | | | RIP1-131, yield: 64% |
| Method 1 | 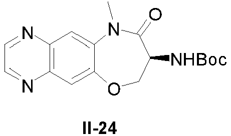 | 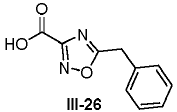 | 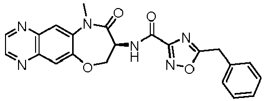 |
| | | | RIP1-132, yield: 59% |
| Method 1 | 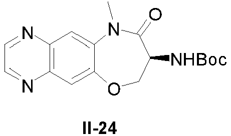 | 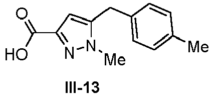 | 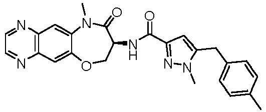 |
| | | | RIP1-133, yield: 58% |
| Method 1 | 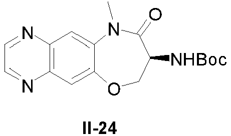 | 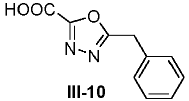 | 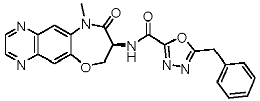 |
| | | | RIP1-134, yield: 48% |
| Method 1 | 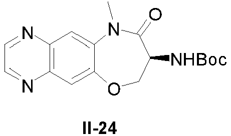 | 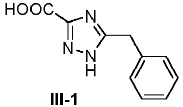 | 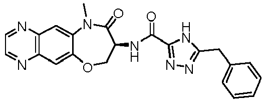 |
| | | | RIP1-135, yield: 52% |
| Method 4 | 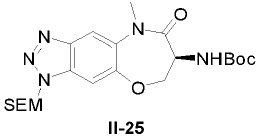 | 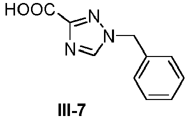 | 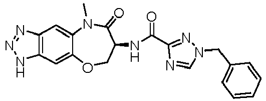 |
| | | | RIP1-136, yield: 56% |
| Method 4 | 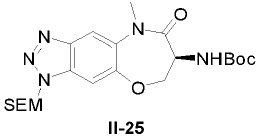 | 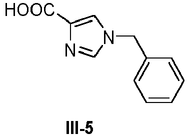 | 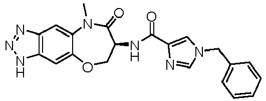 |
| | | | RIP1-137, yield: 39% |
| Method 4 | 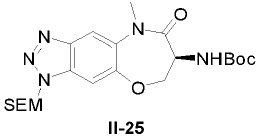 | 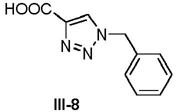 | 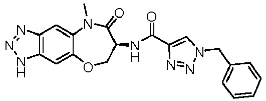 |
| | | | RIP1-138, yield: 58% |
| Method 4 | 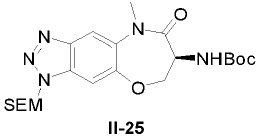 | 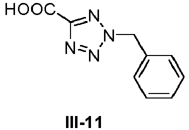 | 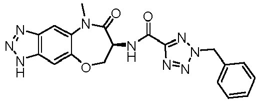 |
| | | | RIP1-139, yield: 35% |
| Method 4 | 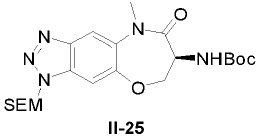 | 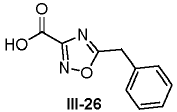 | 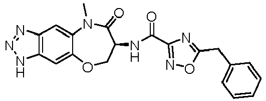 |
| | | | RIP1-140, yield: 66% |
| Method 4 | 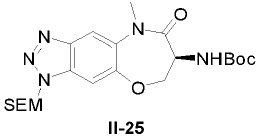 | 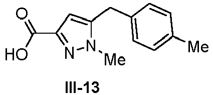 | 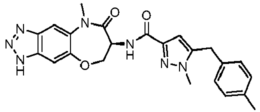 |
| | | | RIP1-141, yield: 58% |
| Method 4 | 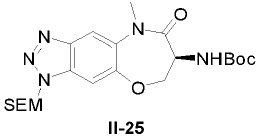 | 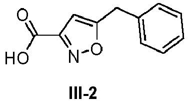 | 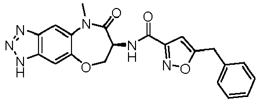 |
| | | | RIP1-142, yield: 66% |
| Method 4 | 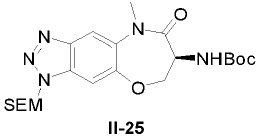 | 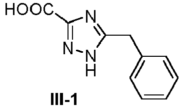 | 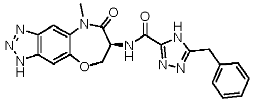 |
| | | | RIP1-143, yield: 55% |
| Method 1 | 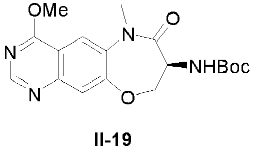 | 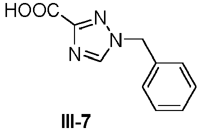 | 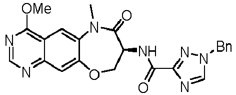 |
| | | | RIP1-145, yield: 73% |
| Method 1 | 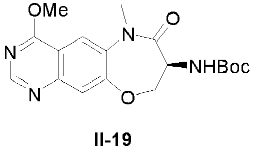 | 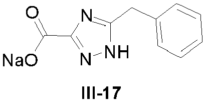 | 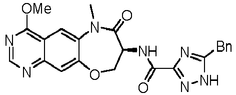 |
| | | | RIP1-146, yield: 75% |
| Method 1 | 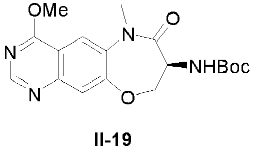 | 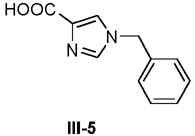 | 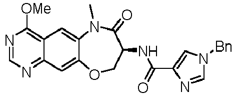 |
| | | | RIP1-147, yield: 69% |
| Method 1 | 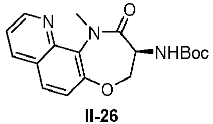 | 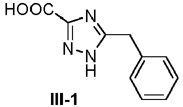 | 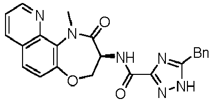 |
| | | | RIP1-148, yield: 64% |
| Method 1 | 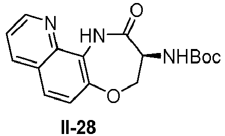 | 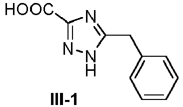 | 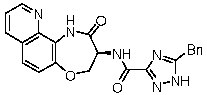 |
| | | | RIP1-149, yield: 65% |
| Method 1 | 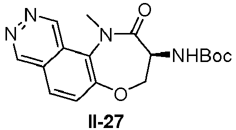 | 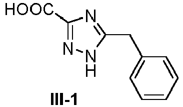 | 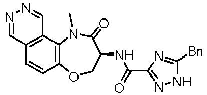 |
| | | | RIP1-150, yield: 8% |
| Method 4 | 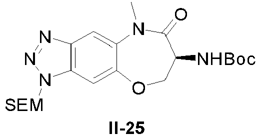 | 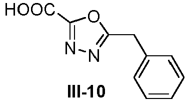 | 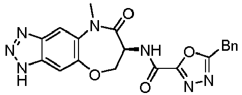 |
| | | | RIP1-153, yield: 41% |
| Method 1 | 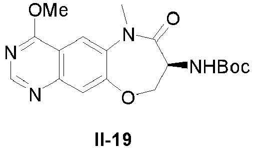 | 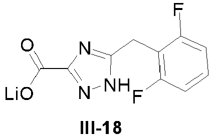 | 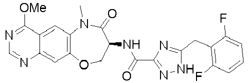 |
| | | | RIP1-156, yield: 33% |
| Method 1 | 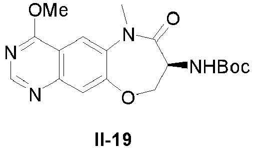 | 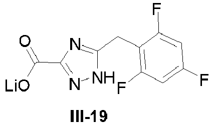 | 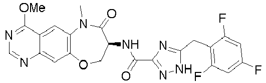 |
| | | | RIP1-157, yield: 27% |
| Method 1 | 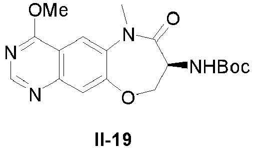 | 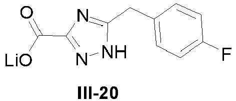 | 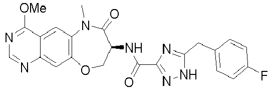 |
| | | | RIP1-158, yield: 21% |
| Method 1 | 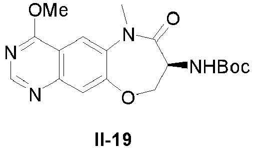 | 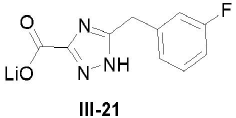 | 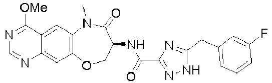 |
| | | | RIP1-159, yield: 25% |
| Method 1 | 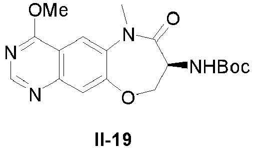 | 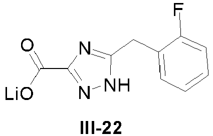 | 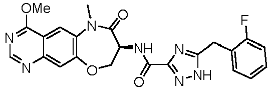 |
| | | | RIP1-160, yield: 21% |
| Method 1 | 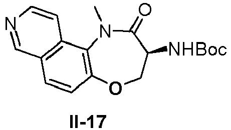 | 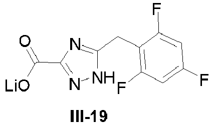 | 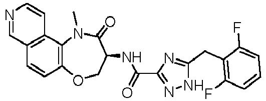 |
| | | | RIP1-161, yield: 62% |
| Method 1 | 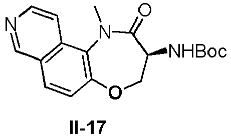 | 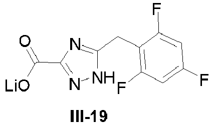 | 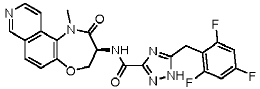 |
| | | | RIP1-162, yield: 52% |
| Method 1 | 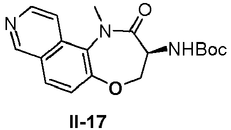 | 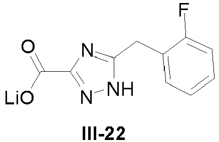 | 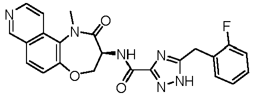 |
| | | | RIP1-163, yield: 72% |
| Method 1 | 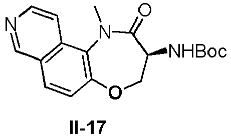 | 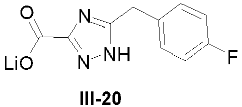 | 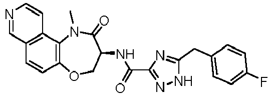 |
| | | | RIP1-164, yield: 69% |
| Method 1 | 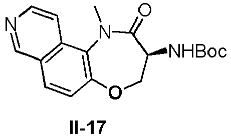 | 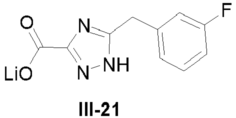 | 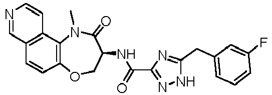 |
| | | | RIP1-165, yield: 72% |
| Method 4 | 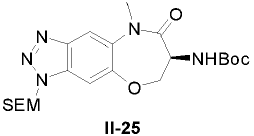 | 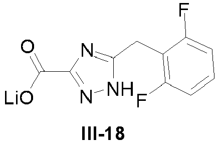 | 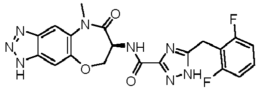 |
| | | | RIP1-166, yield: 36% |
| Method 4 | 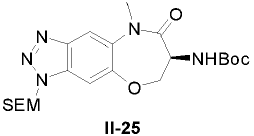 | 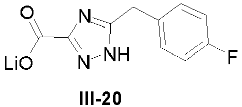 | 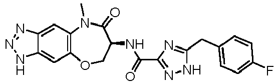 |
| | | | RIP1-167, yield: 40% |
| Method 1 | 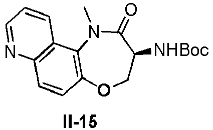 | 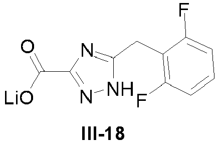 | 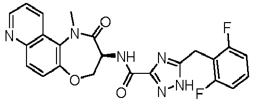 |
| | | | RIP1-168, yield: 59% |
| Method 1 | 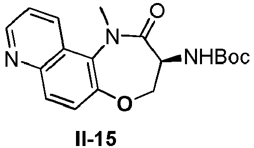 | 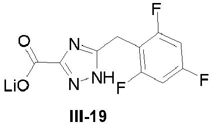 | 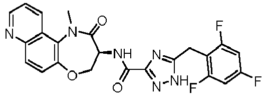 |
| | | | RIP1-169, yield: 59% |
| Method 1 | 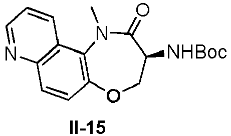 | 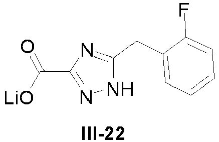 | 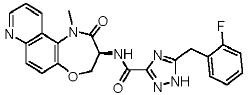 |
| | | | RIP1-170, yield: 72% |
| Method 1 | 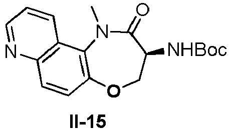 | 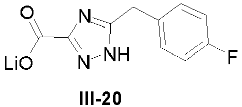 | 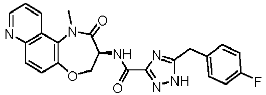 |
| | | | RIP1-171, yield: 74% |
| Method 1 | 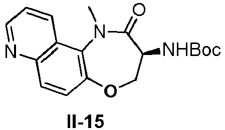 | 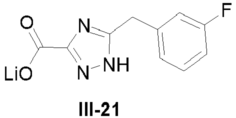 | 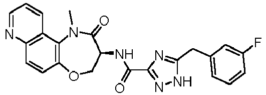 |
| | | | RIP1-172, yield: 64% |
| Method 1 | 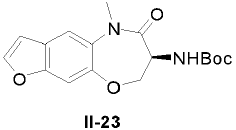 | 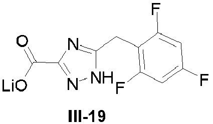 | 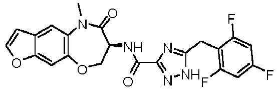 |
| | | | RIP1-173, yield: 74% |
| Method 1 | 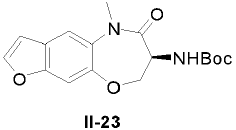 | 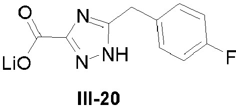 | 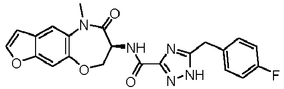 |
| | | | RIP1-174, yield: 73% |
| Method 1 | 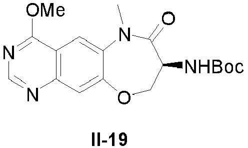 | 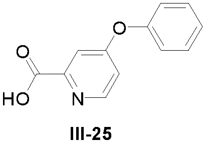 | 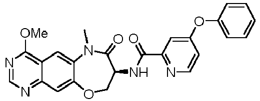 |
| | | | RIP1-175, yield: 40% |
| Method 1 | 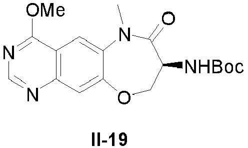 | 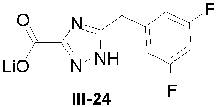 | 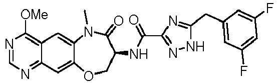 |
| | | | RIP1-176, yield: 36% |
| Method 1 | 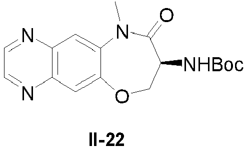 | 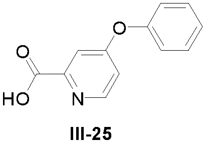 | 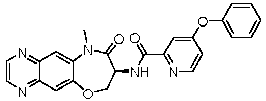 |
| | | | RIP1-177, yield: 59% |
| Method 1 | 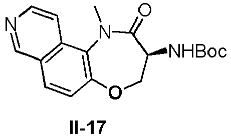 | 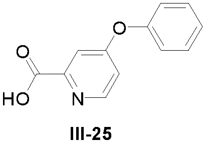 | 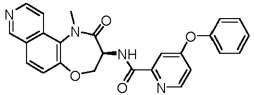 |
| | | | RIP1-178, yield: 51% |
| Method 1 | 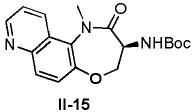 | 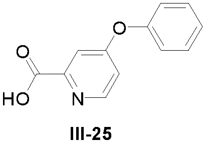 | 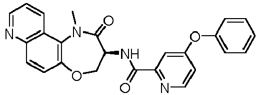 |
| | | | RIP1-179, yield: 50% |
| Method 1 | 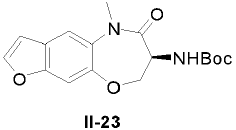 | 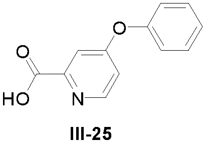 | 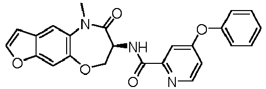 |
| | | | RIP1-180, yield: 62% |
| Method 4 | 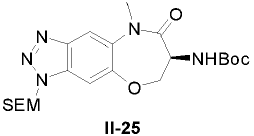 | 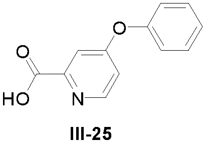 | 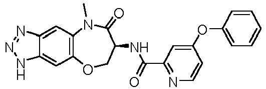 |
| | | | RIP1-181, yield: 80% |
| Method 5 | 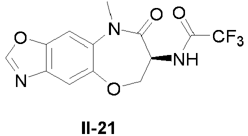 | 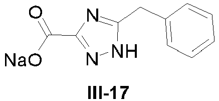 | 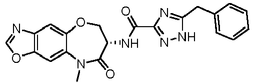 |
| | | | RIP1-182, yield: 37% |
| Method 5 | 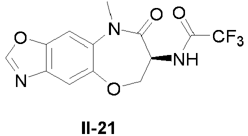 | 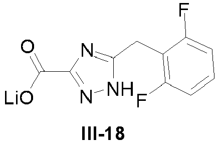 | 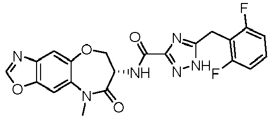 |
| | | | RIP1-183, yield: 19% |
| Method 5 | 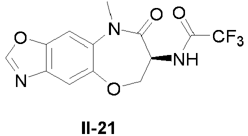 | 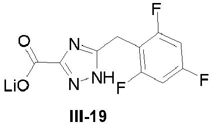 | 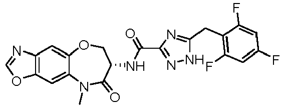 |
| | | | RIP1-184, yield: 24% |
| Method 5 | 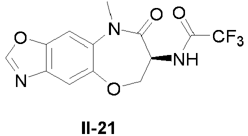 | 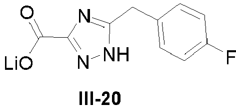 | 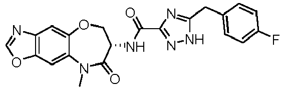 |
| | | | RIP1-185, yield: 33% |
| Method 5 | 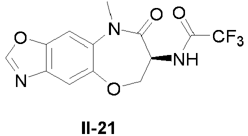 | 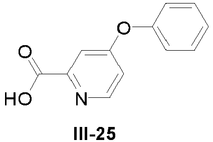 | 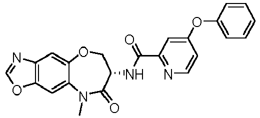 |
| | | | RIP1-186, yield: 62% |
| Method 1 | 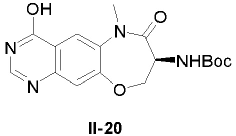 | 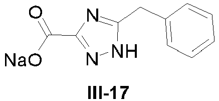 | 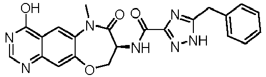 |
| | | | RIP1-187, yield: 20% |
| Method 1 | 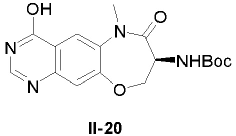 | 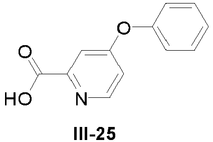 | 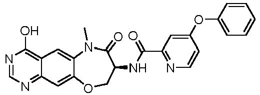 |
| | | | RIP1-188, yield: 39% |
| Method 1 | 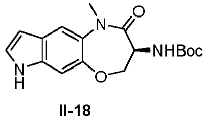 | 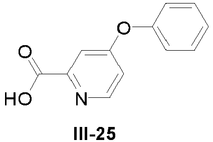 | 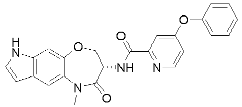 |
| | | | RIP1-189, yield: 15% |
| Method 3 | 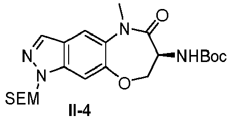 | 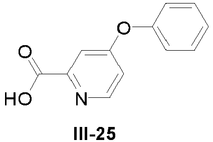 | 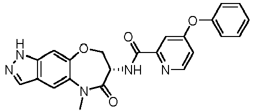 |
| | | | RIP1-190, yield: 39% |
| Method 1 | 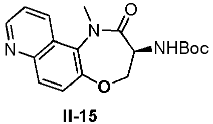 | 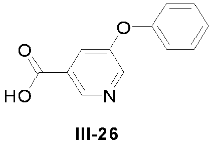 | 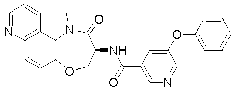 |
| | | | RIP1-193, yield: 39% |
| Method 1 | 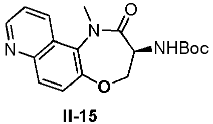 | 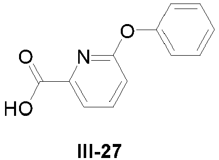 | 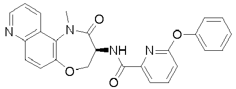 |
| | | | RIP1-194, yield: 82% |
| Method 1 | 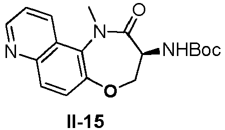 | 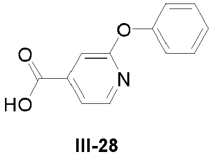 | 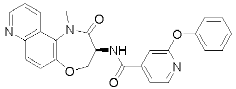 |
| | | | RIP1-195, yield: 97% |
| Method 1 | 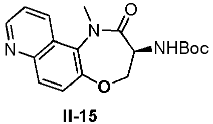 | 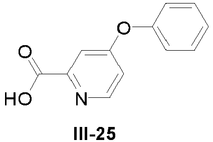 | 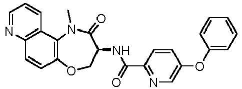 |
| | | | RIP1-196, yield: 97% |
| Method 1 | 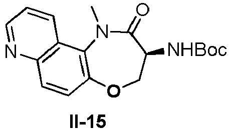 | 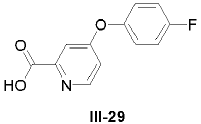 | 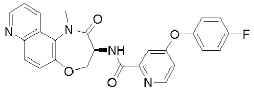 |
| | | | RIP1-197, yield: 67% |
| Method 1 | 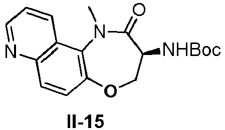 | 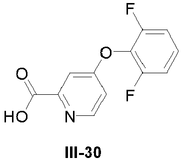 | 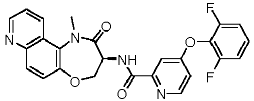 |
| | | | RIP1-198, yield: 62% |
| Method 1 | 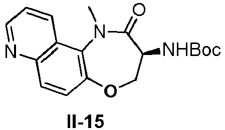 | 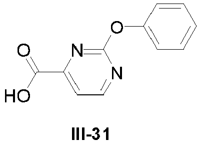 | 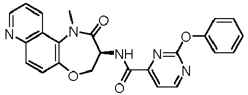 |
| | | | RIP1-199, yield: 70% |
| Method 1 | 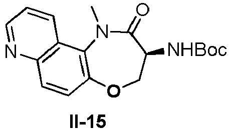 | 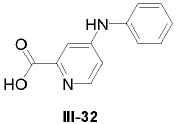 | 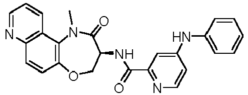 |
| | | | RIP 1-200, yield: 43% |
| Method 1 | 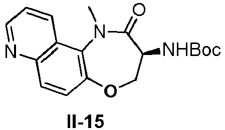 | 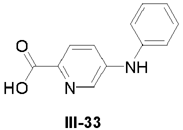 | 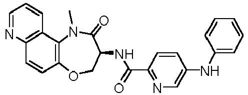 |
| | | | RIP1-201, yield: 66% |
| Method 1 | 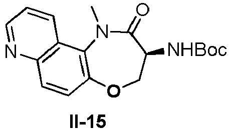 | 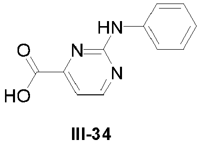 | 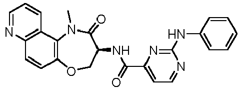 |
| | | | RIP 1-202, yield: 90% |
| Method 1 | 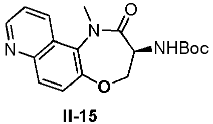 | 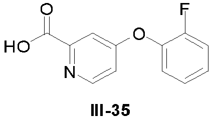 | 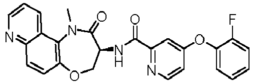 |
| | | | RIP1-203, yield: 79% |
| Method 1 | 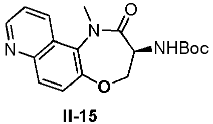 | 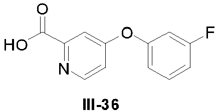 | 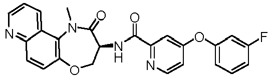 |
| | | | RIP1-204, yield: 71% |
| Method 1 | 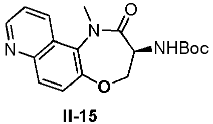 | 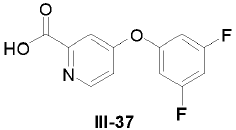 | 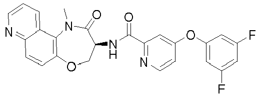 |
| | | | RIP1-205, yield: 83% |
| Method 5 | 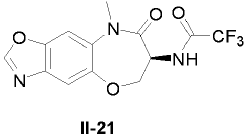 | 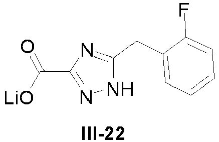 | 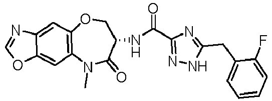 |
| | | | RIP1-206, yield: 53% |
| Method 1 | 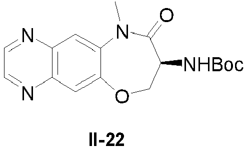 | 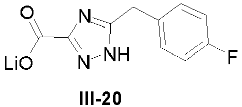 | 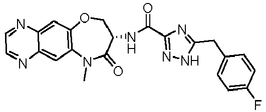 |
| | | | RIP1-207, yield: 47% |
| Method 1 | 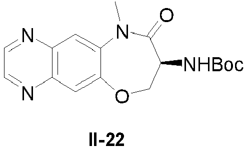 | 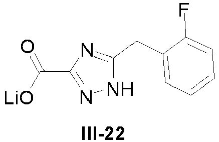 | 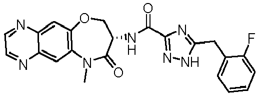 |
| | | | RIP1-208, yield: 21% |
| Method 1 | 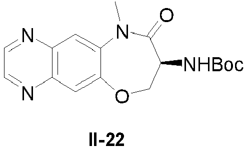 | 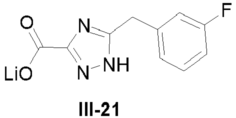 | 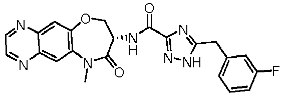 |
| | | | RIP 1-209, yield: 25% |
| Method 1 | 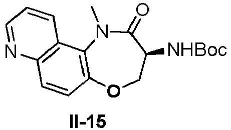 | 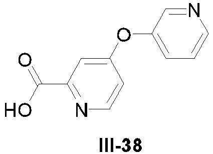 | 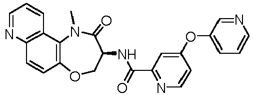 |
| | | | RIP1-210, yield: 66% |
| Method 5 | 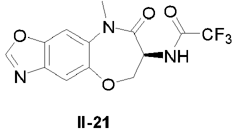 | 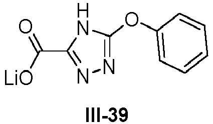 | 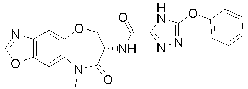 |
| | | | RIP1-211, yield: 14% |
| Method 5 | 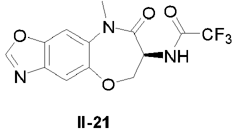 | 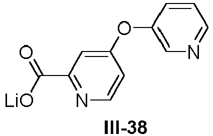 | 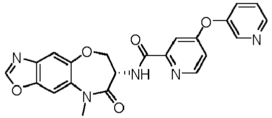 |
| | | | RIP1-212, yield: 29% |
| Method 5 | 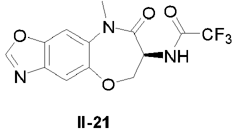 | 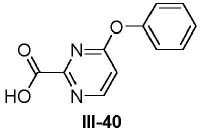 | 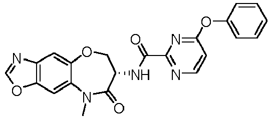 |
| | | | RIP1-213, yield: 35% |
| Method 1 | 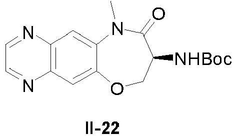 | 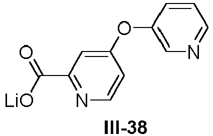 | 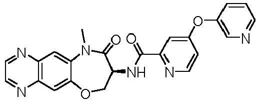 |
| | | | RIP1-214, yield: 32% |
| Method 1 | 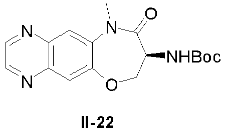 | 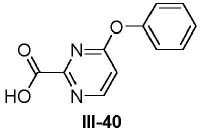 | 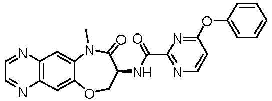 |
| | | | RIP1-215, yield: 31% |
| Method 5 | 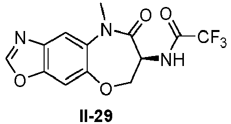 | 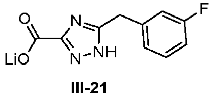 | 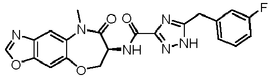 |
| | | | RIP1-217, yield: 16% |
| Method 5 | 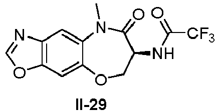 | 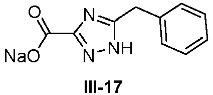 | 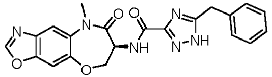 |
| | | | RIP1-218, yield: 32% |
| Method 5 | 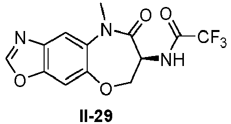 | 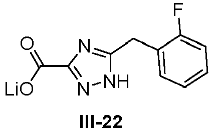 | 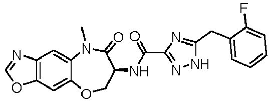 |
| | | | RIP1-219, yield: 19% |
| Method 5 | 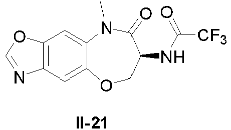 | 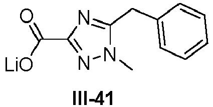 | 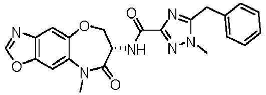 |
| | | | RIP1-220, yield: 21% |
| Method 5 | 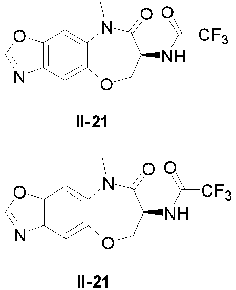 | 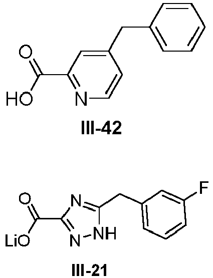 | 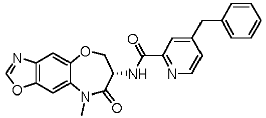 |
| | | | RIP 1-221, yield: 8% |
| Method 5 | | | 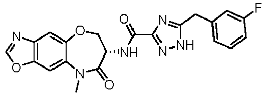 |
| | | | RIP1-222, yield: 22% |

The test data of each compound are shown below.

### RIP1-001

¹H NMR (500 MHz, CDCl₃) δ = 8.86 (s, 1H), 8.31 (d, *J* = 7.8, 1H), 7.63 (s, 1H), 7.49 (s, 1H), 7.31-7.26 (m, 2H), 7.25-7.17 (m, 5H), 6.50 (s, 1H), 4.83-4.76 (m, 1H), 4.08 (s, 2H), 3.76 (dd, *J* = 11.1, 6.9, 1H), 3.46 (s, 3H), 2.83 (t, *J* = 11.2, 1H); ESI-MS *m*/*z* 433.4 (M+H)⁺.

### RIP1-002

¹H NMR (400 MHz, CDCl₃) δ (ppm): 2.79 (t, J = 11.2 Hz, 1H), 3.45 (s, 3H), 3.75 (s, 3H), 3.76-3.80 (m, 1H), 4.06 (s, 2H),, 4.66-4.73 (m, 1H), 6.41 (s, 1H), 7.04-7.08 (m, 1H), 7.19-7.30 (m, 5H), 7.45 (s, 1H), 7.56 (s, 1H), 8.16 (brs, 1H); ESI-MS (M+H)⁺=447.1.

### RIP1-003

¹H NMR (500 MHz, CDCl₃) δ = 8.49 (s, 1H), 7.84 (d, *J* = 7.4, 1H), 7.71 (s, 1H), 7.54 (s, 1H), 7.34-7.29 (m, 3H), 7.28-7.24 (m, 1H), 7.21 (d, *J* = 7.1, 2H), 6.56-6.54 (m, 1H), 6.26 (s, 1H), 4.78-4.73 (m, 1H), 4.09 (s, 2H), 3.82 (dd, *J* = 11.2, 6.9, 1H), 3.48 (s, 3H), 2.84 (t, *J* = 11.2, 1H); ¹³C NMR (125 MHz, CDCl₃) δ 173.86, 169.89, 158.15, 158.01, 137.99, 135.24, 129.36, 128.88, 128.74, 127.31, 126.83, 119.90, 117.90, 116.81, 103.06, 101.54, 49.68, 38.12, 37.19, 33.16; MS-ESI: 433.3 (M+H)⁺.

### RIP1-004

¹H NMR (500 MHz, CDCl₃) δ = 8.73 (s, 1H), 7.83 (d, *J* = 0.4, 1H), 7.76 (s, 1H), 7.68 (s, 1H), 7.52 (s, 1H), 7.37-7.31 (m, 3H), 7.28-7.26 (m, 1H), 7.21 (dd, *J* = 7.7, 1.5, 2H), 7.03 (d, *J* = 7.3, 1H), 6.53-6.50 (m, 1H), 5.27 (s, 2H), 4.81-4.75 (m, 1H), 3.85 (dd, *J* = 11.2, 6.9, 1H), 3.47 (s, 3H), 2.80 (t, *J* = 11.1, 1H); ¹³C NMR (125 MHz, CDCl₃) δ 171.01, 161.45, 138.86, 137.98, 135.42, 134.74, 130.83, 129.45, 129.14, 128.63, 128.12, 126.99, 119.95, 118.52, 118.03, 116.86, 103.08, 56.63, 49.78, 38.65, 37.33; MS-ESI: 432.4 (M+H)⁺.

### RIP1-005

¹H NMR (500 MHz, CDCl₃) δ = 8.75 (s, 1H), 7.66 (s, 1H), 7.59 (d, *J* = 0.6, 1H), 7.55 (s, 1H), 7.53 (s, 1H), 7.30-7.23 (m, 4H), 7.19 (d, *J* = 7.3, 1H), 7.08 (d, *J* = 1.7, 1H), 7.06 (s, 1H), 6.53-6.51 (m, 1H), 5.44 (s, 2H), 4.75-4.69 (m, 1H), 3.77 (dd, *J* = 11.2, 6.8, 1H), 3.48 (s, 3H), 2.76 (t, *J* = 11.1, 1H); ¹³C NMR (125 MHz, CDCl₃) δ 170.55, 159.08, 141.63, 137.96, 136.58, 134.72, 132.64, 129.46, 128.94, 128.08, 127.32, 127.02, 125.32, 119.90, 117.97, 116.92, 103.08, 50.02, 49.66, 38.47, 37.33, 1.16; MS-ESI: 432.4 (M+H)⁺.

### RIP1-006

¹H NMR (500 MHz, CDCl₃) δ = 9.01 (s, 1H), 8.07 (d, *J* = 8.0, 1H), 7.65 (s, 1H), 7.48 (s, 1H), 7.47 (d, *J* = 1.2, 1H), 7.44 (d, *J* = 1.3, 1H), 7.36-7.31 (m, 3H), 7.23-7.22 (m, 1H), 7.13 (dd, *J* = 7.6, 1.7, 2H), 6.48-6.45 (m, 1H), 5.08 (s, 2H), 4.83 (ddd, *J* = 11.2, 7.8, 7.0, 1H), 3.80 (dd, *J*=11.1, 6.8, 1H), 3.47 (s, 3H), 2.88 (t, *J* = 11.2, 1H); ¹³C NMR (125 MHz, cdcl₃) δ 170.79, 161.75, 138.15, 137.17, 137.13, 135.27, 134.70, 129.39, 129.27, 128.74, 127.60, 126.93, 122.47, 119.90, 117.99, 116.74, 102.86, 51.44, 49.37, 38.68, 37.27; MS-ESI: 432.4 (M+H)⁺.

### RIP1-007

¹H NMR (500 MHz, CDCl₃) δ = 8.84 (s, 1H), 7.71 (s, 1H), 7.64 (s, 1H), 7.61 (dt, *J* = 7.1, 1.6, 1H), 7.54 (s, 1H), 7.42 (d, *J* = 7.2, 1H), 7.35-7.25 (m, 5H), 7.19 (t, *J* = 7.4, 1H), 7.15 (d, *J* = 7.1, 2H), 6.53-6.51 (m, 1H), 4.88-4.81 (m, 1H), 4.00 (s, 2H), 3.94 (dd, *J* = 11.1, 6.8, 1H), 3.50 (s, 3H), 2.85 (t, *J* = 11.1, 1H); ¹³C NMR (125 MHz, CDCl₃) δ 170.95, 166.48, 141.85, 140.53, 137.95, 134.79, 134.04, 132.55, 129.46, 129.01, 128.86, 128.70, 127.82, 127.07, 126.42, 124.99, 119.89, 118.08, 116.86, 103.00, 50.35, 41.87, 38.55, 37.32; MS-ESI: 442.4 (M+H)⁺.

### RIP1-008

¹H NMR (500 MHz, CDCl₃) δ = 9.03 (s, 1H), 8.22 (d, *J* = 7.6, 1H), 8.02 (s, 1H), 7.70 (s, 1H), 7.50 (s, 1H), 7.39-7.34 (m, 3H), 7.29-7.27 (m, 2H), 7.25 (d, *J* = 1.8, 1H), 6.49 (s, 1H), 5.36 (s, 2H), 4.87-4.81 (m, 1H), 3.92 (dd, *J* = 11.2, 6.9, 1H), 3.49 (s, 3H), 2.85 (t, *J* = 11.1, 1H); ¹³C NMR (125 MHz, CDCl₃) δ 170.36, 158.12, 157.01, 144.07, 137.82, 134.82, 133.91, 129.47, 129.31, 129.08, 128.37, 127.17, 119.62, 118.30, 116.78, 102.83, 54.45, 49.89, 38.52, 37.36; MS-ESI: 433.4 (M+H)⁺.

### RIP1-009

¹H NMR (500 MHz, CDCl₃) δ = 7.84 (d, *J* = 7.4, 1H), 7.65 (s, 1H), 7.52 (s, 1H), 7.32 (t, *J* = 7.3, 2H), 7.28 (s, 1H), 7.21 (d, *J* = 7.1, 2H), 7.15 (d, *J* = 3.1, 1H), 6.48 (d, *J* = 3.0, 1H), 6.26 (s, 1H), 4.77-4.70 (m, 1H), 4.08 (s, 2H), 3.85-3.80 (m, 4H), 3.48 (s, 3H), 2.84 (t, *J* = 11.1, 1H); ¹³C NMR (125 MHz, CDCl₃) δ 173.96, 170.03, 158.33, 158.12, 137.72, 135.63, 135.41, 131.53, 129.95, 129.02, 128.89, 127.46, 119.55, 117.07, 116.41, 101.70, 101.57, 49.83, 38.33, 37.31, 33.31, 31.77; MS-ESI: 447.4 (M+H)⁺.

### RIP1-010

¹H NMR (500 MHz, CDCl₃) δ = 7.82 (s, 1H), 7.75 (s, 1H), 7.64 (s, 1H), 7.51 (s, 1H), 7.37-7.31 (m, 3H), 7.23-7.20 (m, 2H), 7.14 (d, *J* = 3.1, 1H), 6.96 (d, *J* = 7.3, 1H), 6.47 (d, *J* = 3.0, 1H), 5.27 (s, 2H), 4.77-4.71 (m, 1H), 3.87-3.82 (m, 4H), 3.47 (s, 3H), 2.78 (t, *J* = 11.1, 1H); ¹³C NMR (125 MHz, CDCl₃) δ 171.00, 161.31, 138.80, 137.66, 135.66, 135.46, 131.49, 130.81, 129.91, 129.13, 128.61, 128.12, 119.63, 118.60, 117.04, 116.41, 101.55, 56.62, 49.75, 38.71, 37.30, 33.30; MS-ESI: 446.4 (M+H)⁺.

### RIP1-011

¹H NMR (500 MHz, CDCl₃) δ = 7.66 (s, 1H), 7.63 (s, 1H), 7.59 (d, *J* = 7.3, 1H), 7.53 (s, 1H), 7.35 (d, *J* = 7.1, 1H), 7.34-7.25 (m, 4H), 7.19 (t, *J* = 7.4, 1H), 7.16 (d, *J* = 1.2, 1H), 7.15 (d, *J* = 3.0, 2H), 6.48 (d, *J* = 3.7, 1H), 4.83-4.77 (m, 1H), 4.00 (s, 2H), 3.91 (dd, *J* = 11.1, 6.8, 1H), 3.84 (s, 3H), 3.50 (s, 3H), 2.82 (t, *J* = 11.1, 1H); ¹³C NMR (125 MHz, CDCl₃) δ 170.97, 166.28, 141.78, 140.56, 137.68, 135.67, 134.16, 132.43, 131.50, 129.92, 129.01, 128.81, 128.69, 127.81, 126.40, 124.97, 119.63, 117.06, 116.42, 101.56, 50.29, 41.87, 38.60, 37.27, 33.29; MS-ESI: 456.4 (M+H)⁺.

### RIP1-012

¹H NMR (500 MHz, CDCl₃) δ = 8.18 (d, *J* = 7.5, 1H), 7.99 (s, 1H), 7.65 (s, 1H), 7.51 (s, 1H), 7.39-7.34 (m, 3H), 7.27 (d, *J* = 2.5, 1H), 7.26 (d, *J* = 2.0, 1H), 7.15 (d, *J* = 3.1, 1H), 6.47 (dd, *J* = 3.1, 0.7, 1H), 5.35 (s, 2H), 4.83-4.77 (m, 1H), 3.91 (dd, *J* = 11.2, 6.9, 1H), 3.84 (s, 3H), 3.48 (s, 3H), 2.83 (t, *J* = 11.1, 1H); ¹³C NMR (125 MHz, CDCl₃) δ 170.36, 157.94, 157.08, 143.97, 137.67, 135.65, 133.95, 131.49, 129.94, 129.30, 129.06, 128.36, 119.55, 117.06, 116.53, 101.51, 54.42, 49.80, 38.53, 37.32, 33.29; MS-ESI: 447.4 (M+H)⁺.

### RIP1-013

¹H NMR (400 MHz, CDCl₃) δ 8.03 (s, 1H), 7.89 (s, 1H), 7.68 (s, 1H), 7.37-7.21 (m, 5H), 4.87-4.74 (m, 1H), 4.13 (s, 2H), 3.87-3.74 (m, 1H), 3.50 (s, 3H), 2.99-2.87 (m, 1H); ¹³C NMR (100 MHz, CDCl₃) δ 170.0, 158.2, 138.8, 138.6, 135.9, 134.2, 128.8, 127.2, 126.1, 123.8, 117.5, 116.8, 49.5, 37.5, 37.3, 33.2. ESI-MS: m/z 434.1 (M+H)⁺.

### RIP1-014

¹H NMR (400 MHz, CDCl₃) δ 11.82 (s, 1H), 7.92 (s, 1H), 7.87 (s, 1H), 7.81 (s, 1H), 7.66 (s, 1H), 7.60-7.54 (m, 2H), 7.38-7.31 (m, 3H), 7.24-7.19 (m, 2H), 5.25 (s, 2H), 4.90 (dt, *J* = 11.4, 7.4 Hz, 1H), 3.79 (dd, *J* = 11.3, 6.8 Hz, 1H), 3.43 (s, 3H), 2.93 (t, *J* = 11.4 Hz, 1H); ¹³C NMR (100 MHz, CDCl₃) δ 171.0, 162.1, 139.1, 138.6, 138.5, 135.3, 134.7, 131.1, 129.1, 128.6, 128.2, 126.0, 123.9, 118.0, 116.8, 116.8, 56.6, 49.6, 37.7, 37.6. ESI-MS: m/z 433.1 (M+H)⁺.

### RIP1-015

¹H NMR (400 MHz, CDCl₃) δ 10.97 (s, 1H), 8.01 (s, 1H), 7.92 (d, *J* = 7.1 Hz, 1H), 7.80 (s, 1H), 7.63 (s, 1H), 7.36-7.15 (m, 5H), 6.27 (s, 1H), 4.88-4.72 (m, 1H), 4.07 (s, 2H), 3.80 (dd, *J* = 11.0, 6.7 Hz, 1H), 3.47 (s, 3H), 2.89 (t, *J* = 11.2 Hz, 1H); ¹³C NMR (100 MHz, CDCl₃) δ 174.2, 169.7, 158.5, 158.1, 138.9, 138.7, 135.3, 135.0, 129.0, 128.9, 127.5, 126.5, 124.2, 117.0, 116.9, 101.7, 49.6, 37.6, 37.5, 33.3. ESI-MS: m/z 434.1 (M+H)⁺.

### RIP1-016

¹H NMR (400 MHz, CDCl₃) δ 11.94 (s, 1H), 8.17 (d, *J* = 8.2 Hz, 1H), 7.88 (d, *J* = 0.7 Hz, 1H), 7.68 (s, 1H), 7.52 (s, 1H), 7.49 (s, 1H), 7.48 (s, 1H), 7.35-7.29 (m, 3H), 7.15-7.10 (m, 2H), 5.08 (s, 2H), 4.91 (ddd, *J* = 11.4, 8.0, 7.0 Hz, 1H), 3.79 (dd, *J* = 11.1, 6.7 Hz, 1H), 3.43 (s, 3H), 2.96 (t, *J* = 11.3 Hz, 1H); ¹³C NMR (100 MHz, CDCl₃) δ 170.3, 162.2, 138.7, 138.6, 137.3, 136.7, 135.2, 134.5, 129.3, 128.7, 127.6, 126.0, 123.8, 122.8, 116.7, 116.6, 51.4, 49.1, 37.8, 37.4. ESI-MS: m/z 433.1 (M+H)⁺.

### RIP1-017

¹H NMR (400 MHz, CDCl₃) δ 11.61 (br s, 1H), 8.00 (s, 1H), 7.80-7.46 (m, 5H), 7.26-7.13 (m, 3H), 7.12-6.97 (m, 2H), 5.44 (q, *J* = 15.0 Hz, 2H), 4.77 (s, 1H), 3.79-3.66 (m, 1H), 3.48 (s, 3H), 2.86 (t, *J* = 11.0 Hz, 1H); ¹³C NMR (100 MHz, CDCl₃) δ 170.5, 159.4, 141.8, 138.7, 136.5, 134.8, 133.0, 128.9, 128.1, 127.3, 126.3, 125.1, 124.0, 117.0, 116.9, 53.6, 50.2, 49.4, 37.6. ESI-MS: m/z 433.1 (M+H)⁺.

### RIP1-019

¹H NMR (400 MHz, CDCl₃+CD₃OD) δ 7.99 (s, 1H), 7.92 (s, 1H), 7.81 (s, 1H), 7.64 (s, 1H), 7.38-7.30 (m, 3H), 7.25-7.19 (m, 2H), 5.58-5.45 (m, 2H), 4.78 (dd, *J* = 11.3, 6.7 Hz, 1H), 3.75 (dd, *J* = 11.1, 6.8 Hz, 1H), 3.46 (s, 3H), 2.91 (t, *J* = 11.3 Hz, 1H); ¹³C NMR (100 MHz, CDCl₃+CD₃OD) δ 170.0, 159.4, 142.8, 138.9, 138.7, 134.5, 133.8, 129.3, 129.2, 128.3, 126.4, 125.7, 124.0, 117.1, 116.9, 54.6, 49.2, 37.51, 37.49. ESI-MS: m/z 434.1 (M+H)⁺.

### RIP1-020

¹H NMR (400 MHz, CDCl₃+CD₃OD) δ 8.07 (s, 1H), 8.02 (s, 1H), 7.89 (s, 1H), 7.66 (s, 1H), 7.40-7.33 (m, 3H), 7.29-7.26 (m, 2H), 5.37 (s, 2H), 4.82 (dd, *J* = 11.2, 6.8 Hz, 1H), 3.86 (dd, *J* = 11.1, 6.8 Hz, 1H), 3.49 (s, 3H), 2.88 (t, *J* = 11.2 Hz, 1H); ¹³C NMR (100 MHz, CDCl₃+CD₃OD) δ 170.1, 158.1, 156.6, 144.2, 138.8, 138.6, 134.4, 133.8, 129.3, 129.1, 128.3, 126.1, 123.9, 117.5, 116.8, 54.4, 49.6, 49.5, 37.52, 37.47. ESI-MS: m/z 434.1 (M+H)⁺.

### RIP1-021

¹H NMR (400 MHz, CDCl₃) δ 10.49 (s, 1H), 8.05 (s, 1H), 7.80 (d, *J* = 6.3 Hz, 1H), 7.60 (s, 1H), 7.35-7.27 (m, 4H), 7.24-7.19 (m, 2H), 6.29 (s, 1H), 5.07-4.98 (m, 1H), 4.69 (dd, *J* = 9.8, 7.4 Hz, 1H), 4.24 (dd, *J* = 11.1, 9.9 Hz, 1H), 4.10 (s, 2H), 3.51 (s, 3H); ¹³C NMR (100 MHz, CDCl₃) δ 174.3, 168.4, 158.8, 158.0, 150.0, 138.6, 135.3, 135.2, 131.7, 129.1, 128.9, 127.5, 120.6, 115.4, 103.2, 101.7, 76.6, 49.2, 36.7, 33.3. ESI-MS: m/z 418.0 (M+H)⁺.

### RIP1-022

¹H NMR (400 MHz, CDCl₃) δ 11.00 (br s, 1H), 8.07-7.96 (m, 2H), 7.53 (s, 1H), 7.51-7.46 (m, 2H), 7.38-7.30 (m, 3H), 7.19-7.11 (m, 2H), 5.15-5.04 (m, 3H), 4.70-4.63 (m, 1H), 4.31-4.22 (m, 1H), 3.48 (s, 3H); ¹³C NMR (100 MHz, CDCl₃) δ 169.1, 162.4, 150.0, 137.3, 136.8, 135.2, 134.9, 131.8, 129.3, 128.8, 127.7, 122.7, 115.1, 103.0, 77.0, 51.5, 48.7, 36.5. ESI-MS: m/z 417.1 (M+H)⁺.

### RIP1-023

¹H NMR (400 MHz, CDCl₃) δ 8.00 (s, 1H), 7.56 (s, 1H), 7.31-7.27 (m, 2H), 7.26-7.17 (m, 4H), 5.01 (dd, *J* = 11.2, 7.4 Hz, 1H), 4.67-4.58 (m, 1H), 4.26-4.17 (m, 1H), 4.10 (s, 2H), 3.47 (s, 3H); ¹³C NMR (100 MHz, CDCl₃) δ 168.6, 158.7, 154.5, 149.7, 138.7, 135.9, 134.3, 131.4, 128.9, 127.3, 120.3, 115.2, 103.6, 76.4, 49.0, 36.6, 33.3. ESI-MS: m/z 418.1 (M+H)⁺.

### RIP1-024

¹H NMR (400 MHz, CDCl₃) δ 10.93 (br s, 1H), 8.03 (d, *J* = 6.8 Hz, 1H), 7.91 (s, 1H), 7.84 (s, 1H), 7.47 (s, 1H), 7.26 (s, 2H), 7.17 (s, 3H), 5.53-5.36 (m, 2H), 5.07-4.91 (m, 1H), 4.56 (t, *J* = 8.5 Hz, 1H), 4.19 (t, *J* = 10.4 Hz, 1H), 3.41 (s, 3H); ¹³C NMR (100 MHz, CDCl₃) δ 168.6, 159.9, 149.9, 142.8, 138.6, 134.9, 133.8, 131.7, 129.4, 129.2, 128.4, 125.7, 120.5, 115.2, 103.1, 76.7, 54.7, 48.9, 36.6. ESI-MS: m/z 418.1 (M+H)⁺.

### RIP1-025

¹H NMR (400 MHz, CDCl₃) δ 11.54 (br s, 1H), 8.17-7.86 (m, 3H), 7.47 (s, 1H), 7.33-7.26 (m, 3H), 7.23-7.16 (m, 2H), 5.30 (s, 2H), 5.09-4.96 (m, 1H), 4.71 (t, *J* = 8.2 Hz, 1H), 4.18 (t, *J* = 10.3 Hz, 1H), 3.43 (s, 3H); ¹³C NMR (100 MHz, CDCl₃) δ 168.6, 158.8, 156.7, 149.6, 144.3, 138.8, 134.6, 133.8, 131.3, 129.3, 129.1, 128.5, 120.5, 115.0, 103.7, 76.7, 54.5, 49.4, 36.6. ESI-MS: m/z 418.1 (M+H)⁺.

### RIP1-026

¹H NMR (400 MHz, CDCl₃) δ 11.81 (s, 1H), 8.24 (s, 1H), 8.08 (s, 1H), 7.81 (s, 1H), 7.51 (s, 1H), 7.41-7.34 (m, 3H), 7.33-7.27 (m, 2H), 5.38 (s, 2H), 4.88-4.75 (m, 1H), 3.96 (dd, *J* = 10.7, 6.7 Hz, 1H), 3.51 (s, 3H), 2.92 (t, *J* = 11.1 Hz, 1H); ¹³C NMR (100 MHz, CDCl₃) δ 169.7, 158.5, 156.7, 144.3, 139.9, 139.2, 134.8, 133.7, 129.4, 129.2, 128.5, 127.4, 121.3, 118.3, 115.2, 54.6, 49.8, 37.6, 37.4. ESI-MS: m/z 468.0 (M+H)⁺.

### RIP1-027

¹H NMR (400 MHz, CDCl₃) δ 8.23 (br s, 1H), 7.81 (s, 1H), 7.54 (s, 1H), 7.29-7.26 (m, 1H), 7.25-7.20 (m, 4H), 4.70 (dd, *J* = 11.2, 6.8 Hz, 1H), 4.10 (s, 2H), 3.72 (dd, *J* = 11.0, 6.8 Hz, 1H), 3.47 (s, 3H), 2.86 (t, *J* = 11.2 Hz, 1H); ¹³C NMR (100 MHz, CDCl₃) δ 169.8, 158.4, 139.9, 139.4, 135.8, 134.6, 129.0, 128.9, 127.9, 127.3, 121.2, 118.4, 115.3, 49.5, 37.6, 37.2, 33.2. ESI-MS: m/z 466.0 (M-H)⁻.

### RIP1-028

¹H NMR (400 MHz, CDCl₃) δ 11.69 (s, 1H), 8.16 (d, *J* = 7.9 Hz, 1H), 7.68 (s, 1H), 7.51-7.44 (m, 3H), 7.37-7.31 (m, 3H), 7.17-7.10 (m, 2H), 5.12 (d, *J* = 16.7 Hz, 2H), 4.87 (dt, *J* = 11.5, 7.2 Hz, 1H), 3.82 (dd, *J* = 11.0, 6.6 Hz, 1H), 3.47 (s, 3H), 2.97 (t, *J* = 11.3 Hz, 1H); ¹³C NMR (100 MHz, CDCl₃) δ 170.1, 162.3, 139.7, 139.6, 137.4, 136.6, 135.1, 135.0, 129.3, 128.8, 127.9, 127.7, 122.9, 121.2, 117.3, 115.3, 51.5, 49.1, 37.7, 37.5. ESI-MS: m/z 467.0 (M+H)⁺.

### RIP1-029

¹H NMR (400 MHz, CDCl₃) δ 10.90 (br s, 1H), 7.91 (s, 1H), 7.78 (s, 1H), 7.56 (s, 1H), 7.37-7.27 (m, 3H), 7.24-7.19 (m, 2H), 6.28 (s, 1H), 4.78 (dd, *J* = 11.2, 6.7 Hz, 1H), 4.09 (s, 2H), 3.81 (dd, *J* = 11.1, 6.7 Hz, 1H), 3.50 (s, 3H), 2.92 (t, *J* = 11.3 Hz, 1H); ¹³C NMR (100 MHz, CDCl₃) δ 174.3, 169.5, 158.6, 158.0, 139.7, 139.7, 135.5, 135.3, 129.1, 128.9, 128.2, 127.5, 121.5, 117.5, 115.6, 101.7, 49.5, 37.6, 37.3, 33.3. ESI-MS: m/z 468.0 (M+H)⁺.

### RIP1-030

¹H NMR (400 MHz, CDCl₃+CD₃OD) δ 11.85 (br s, 1H), 8.19 (d, *J* = 7.1 Hz, 1H), 7.91 (s, 1H), 7.80 (s, 1H), 7.57 (s, 1H), 7.41-7.31 (m, 3H), 7.26-7.19 (m, 2H), 5.57-5.46 (m, 2H), 4.84-4.68 (m, 1H), 3.76 (dd, *J* = 10.7, 6.7 Hz, 1H), 3.45 (d, *J* = 29.4 Hz, 3H), 2.92 (t, *J* = 11.2 Hz, 1H); ¹³C NMR (100 MHz, CDCl₃+CD₃OD) δ 169.9, 159.4, 142.8, 139.7, 139.6, 134.9, 133.8, 129.4, 129.2, 128.3, 128.1, 125.7, 121.3, 117.7, 115.5, 54.6, 49.2, 37.6, 37.4. ESI-MS: m/z 468.1 (M+H)⁺.

### RIP1-031

¹H NMR (400 MHz, CDCl₃) δ 11.05 (s, 1H), 8.03 (d, *J* = 7.3 Hz, 1H), 7.53-7.46 (m, 2H), 7.43 (s, 1H), 7.39-7.30 (m, 3H), 7.18 (s, 1H), 7.17-7.11 (m, 2H), 5.21-5.04 (m, 3H), 4.72-4.63 (m, 1H), 4.29 (dd, *J* = 11.3, 9.9 Hz, 1H), 3.51 (s, 3H); ¹³C NMR (100 MHz, CDCl₃) δ 168.8, 162.8, 150.7, 139.7, 137.4, 136.4, 135.1, 134.7, 132.3, 129.3, 128.8, 127.7, 122.9, 117.6, 113.6, 103.5, 76.8, 51.5, 48.7, 36.5. ESI-MS: m/z 451.1 (M+H)⁺.

### RIP1-032

¹H NMR (400 MHz, CDCl₃) s 11.53 (s, 1H), 8.15 (d, *J* = 6.4 Hz, 1H), 8.08 (s, 1H), 7.46 (s, 1H), 7.40-7.28 (m, 5H), 5.37 (s, 2H), 5.07 (dt, *J* = 11.1, 6.9 Hz, 1H), 4.85 (dd, *J* = 9.6, 7.4 Hz, 1H), 4.35-4.25 (m, 1H), 3.52 (s, 3H); ¹³C NMR (100 MHz, CDCl₃) δ 168.4, 159.0, 156.5, 150.3, 144.4, 139.9, 134.4, 133.7 131.8, 129.3, 129.2, 128.5, 117.7, 113.5, 104.6, 76.6, 54.6, 49.5, 36.6. ESI-MS: m/z 452.1 (M+H)⁺.

### RIP1-033

¹H NMR (400 MHz, CDCl₃) δ 11.38 (br s, 1H), 7.83 (br s, 1H), 7.40 (s, 1H), 7.30-7.11 (m, 6H), 6.24 (s, 1H), 5.07-4.93 (m, 1H), 4.67-4.55 (m, 1H), 4.21 (t, *J* = 10.5 Hz, 1H), 4.02 (s, 2H), 3.45 (s, 3H); ¹³C NMR (100 MHz, CDCl₃) δ 174.4, 168.2, 159.1, 157.8, 150.8, 139.7, 135.2, 135.1, 132.3, 129.0, 128.9, 127.5, 117.8, 114.0, 103.8, 101.7, 76.4, 49.1, 36.7, 33.3. ESI-MS: m/z 452.1 (M+H)⁺.

### RIP1-034

¹H NMR (400 MHz, CDCl₃+CD₃OD) δ 7.45 (s, 1H), 7.32-7.12 (m, 6H), 5.05-4.92 (m, 1H), 4.61 (t, *J* = 8.4 Hz, 1H), 4.22 (t, *J* = 10.4 Hz, 1H), 4.08 (s, 2H), 3.46 (s, 3H); ¹³C NMR (100 MHz, CDCl₃+CD₃OD) δ 168.5, 158.8, 150.5, 139.9, 135.7, 134.3, 132.0, 128.84, 128.80, 127.2, 117.6, 113.7, 104.2, 76.3, 49.0, 36.6, 33.1. ESI-MS: m/z 452.1 (M+H)⁺.

### RIP1-035

¹H NMR (400 MHz, CDCl₃) δ 7.92 (s, 1H), 7.49 (s, 1H), 7.36-7.32 (m, 2H), 7.29-7.18 (m, 5H), 5.51 (s, 2H), 5.07-4.95 (m, 1H), 4.61 (dd, *J* = 9.7, 7.4 Hz, 1H), 4.29-4.20 (m, 1H), 3.49 (s, 3H); ¹³C NMR (100 MHz, CDCl₃) δ 168.6, 159.8, 150.6, 142.3, 139.8, 134.3, 133.8, 132.1, 129.2, 129.0, 128.1, 125.8, 117.5, 113.8, 103.9, 76.3, 54.4, 48.6, 36.5. ESI-MS: m/z 452.1 (M+H)⁺.

### RIP1-036

¹H NMR (400 MHz, CDCl₃) δ 8.01 (d, *J* = 7.7 Hz, 1H), 7.81 (s, 1H), 7.69 (d, *J* = 1.8 Hz, 1H), 7.50 (s, 1H), 7.44 (d, *J* = 7.7 Hz, 2H), 7.38-7.29 (m, 3H), 7.17-7.07 (m, 2H), 6.76 (s, 1H), 5.08 (s, 2H), 4.77 (dt, *J* = 11.1, 7.5 Hz, 1H), 3.79 (dd, *J* = 11.0, 6.8 Hz, 1H), 3.45 (s, 3H), 2.89 (t, *J* = 11.2 Hz, 1H); ¹³C NMR (100 MHz, CDCl₃) δ 170.6, 161.6, 153.1, 147.4, 141.4, 137.1, 135.3, 129.6, 129.2, 128.7, 127.6, 123.3, 122.4, 118.3, 117.3, 106.8, 51.4, 49.1, 38.7, 37.1. ESI-MS: m/z 433.1 (M+H)⁺.

### RIP1-037

¹H NMR (400 MHz, CDCl₃) δ 8.15 (d, *J* = 7.7 Hz, 1H), 8.02 (s, 1H), 7.82 (s, 1H), 7.70 (d, *J* = 2.2 Hz, 1H), 7.50 (s, 1H), 7.38-7.32 (m, 3H), 7.27-7.22 (m, 2H), 6.77 (dd, *J* = 2.1, 0.9 Hz, 1H), 5.35 (s, 2H), 4.80 (dt, *J* = 11.1, 7.2 Hz, 1H), 3.89 (dd, *J* = 11.2, 6.9 Hz, 1H), 3.45 (s, 3H), 2.87 (t, *J* = 11.2 Hz, 1H); ¹³C NMR (100 MHz, CDCl₃) δ 170.2, 157.9, 156.9, 153.1, 147.5, 144.0, 141.0, 133.9, 129.6, 129.2, 129.0, 128.3, 123.0, 118.4, 117.3, 106.7, 54.3, 49.5, 38.5, 37.2. ESI-MS: m/z 434.1 (M+H)⁺.

### RIP1-038

¹H NMR (400 MHz, CDCl₃) δ 7.87-7.79 (m, 2H), 7.71 (d, *J* = 2.1 Hz, 1H), 7.52 (s, 1H), 7.35-7.19 (m, 5H), 6.79 (d, *J* = 1.3 Hz, 1H), 6.27 (s, 1H), 4.74 (dt, *J* = 11.2, 7.2 Hz, 1H), 4.08 (s, 2H), 3.83 (dd, *J* = 11.1, 6.9 Hz, 1H), 3.46 (s, 3H), 2.88 (t, *J* = 11.2 Hz, 1H); ¹³C NMR (100 MHz, CDCl₃) δ 174.0, 169.9, 158.2, 158.1, 153.1, 147.5, 141.1, 135.3, 129.7, 129.0, 128.9, 127.4, 123.1, 118.4, 117.4, 106.8, 101.7, 49.6, 38.3, 37.2, 33.3. ESI-MS: m/z 434.1 (M+H)⁺.

### RIP1-039

¹H NMR (400 MHz, CDCl₃) δ 8.25 (d, *J* = 7.9 Hz, 1H), 7.80 (s, 1H), 7.70 (d, *J* = 2.1 Hz, 1H), 7.46 (s, 1H), 7.15 (s, 5H), 6.76 (d, *J* = 1.3 Hz, 1H), 4.77 (dt, *J* = 11.3, 7.4 Hz, 1H), 4.06 (s, 2H), 3.74 (dd, *J* = 11.2, 6.9 Hz, 1H), 3.42 (s, 3H), 2.87 (t, *J* = 11.3 Hz, 1H); ¹³C NMR (100 MHz, CDCl₃) δ 170.4, 158.7, 153.1, 147.5, 141.1, 136.1, 129.8, 128.9, 128.7, 127.0, 123.0, 118.3, 117.4, 106.8, 49.6, 38.3, 37.3, 32.9. ESI-MS: m/z 434.1 (M+H)⁺.

### RIP1-040

¹H NMR (400 MHz, CDCl₃) δ 8.11 (d, *J* = 7.5 Hz, 1H), 7.87 (s, 1H), 7.82 (s, 1H), 7.70 (d, *J* = 1.7 Hz, 1H), 7.51 (s, 1H), 7.39-7.31 (m, 3H), 7.26-7.20 (m, 2H), 6.78 (s, 1H), 5.59-5.45 (m, 2H), 4.77 (dt, *J* = 11.1, 7.3 Hz, 1H), 3.81 (dd, *J* = 11.1, 6.9 Hz, 1H), 3.46 (s, 3H), 2.91 (t, *J* = 11.2 Hz, 1H); ¹³C NMR (100 MHz, CDCl₃) δ 170.2, 159.1, 153.1, 147.5, 143.1, 141.3, 133.9, 129.7, 129.4, 129.2, 128.3, 125.4, 123.2, 118.3, 117.3, 106.8, 54.6, 49.3, 38.5, 37.2. ESI-MS: m/z 434.1 (M+H)⁺.

### RIP1-041

¹H NMR (500 MHz, CDCl₃) δ = 8.59 (s, 1H), 8.12 (brs, 1H), 7.72 (s, 1H), 7.55 (s, 1H), 7.34-7.27 (m, 6H), 6.56 (s, 1H), 4.77-4.70 (m, 1H), 4.23 (s, 2H), 3.84 (dd, *J* = 11.2, 6.9, 1H), 3.49 (s, 3H), 2.84 (t, *J* = 11.1, 1H). MS-ESI: 434.1 (M+H)⁺.

### RIP1-042

¹H NMR (500 MHz, CDCl₃) δ = 8.83 (s, 1H), 8.16 (d, *J* = 7.8, 1H), 7.86 (s, 1H), 7.67 (s, 1H), 7.51 (s, 1H), 7.37-7.33 (m, 3H), 7.26-7.21 (m, 3H), 6.50 (s, 1H), 5.51 (d, *J* = 7.1, 2H), 4.85-4.79 (m, 1H), 3.81 (dd, *J* = 11.2, 6.9, 1H), 3.48 (s, 3H), 2.88 (t, *J* = 11.2, 1H); ^{l13}C NMR (125 MHz, cdcl₃) δ 170.33, 159.19, 143.19, 138.10, 134.68, 133.93, 129.45, 129.37, 129.17, 128.31, 127.00, 125.44, 119.89, 118.01, 116.83, 102.98, 54.59, 49.56, 38.48, 37.33; MS-ESI: 433.2 (M+H)⁺.

### RIP1-043

¹H NMR (500 MHz, CDCl₃) δ = 8.83 (s, 1H), 8.19 (d, *J=* 7.4, 1H), 7.72 (s, 1H), 7.53 (s, 1H), 7.39-7.34 (m, 5H), 7.31-7.29 (m, 1H), 6.54-6.52 (m, 1H), 5.79 (s, 2H), 4.86-4.81 (m, 1H), 3.92 (dd, *J* = 11.2, 6.9, 1H), 3.49 (s, 3H), 2.85 (t, *J* = 11.1, 1H); ¹³C NMR (125 MHz, cdcl₃) δ 169.94, 159.74, 155.64, 137.78, 134.80, 132.51, 129.54, 129.43, 129.26, 128.73, 127.21, 119.64, 118.26, 116.88, 103.00, 57.59, 50.07, 38.32, 37.40; MS-ESI: 434.1 (M+H)⁺.

### RIP1-044

¹H NMR (400 MHz, CDCl₃) δ = 9.17 (s, 1H), 7.89 (d, *J* = 7.8, 1H), 7.66 (s, 1H), 7.49 (s, 1H), 7.23 (t, *J* = 2.7, 1H), 7.08 (d, *J* = 7.8, 2H), 6.98 (d, *J* = 7.9, 2H), 6.46 (s, 1H), 6.46 (s, 1H), 4.88-4.80 (m, 1H), 3.91 (s, 2H), 3.86 (dd, *J* = 11.1, 6.8, 1H), 3.69 (s, 3H), 3.48 (s, 3H), 2.87 (t, *J* = 11.2, 1H), 2.30 (s, 3H); ¹³C NMR(100 MHz, CDCl₃) δ 170.84, 161.46, 144.46, 143.36, 137.92, 136.61, 134.74, 133.66, 129.58, 129.36, 128.34, 127.05, 119.66, 118.07, 116.69, 106.81, 102.72, 77.48, 77.16, 76.84, 49.50, 38.65, 37.27, 37.12, 31.56, 21.12; MS-ESI: 460.2 (M+H)⁺.

### RIP1-045

¹H NMR (500 MHz, CDCl₃) δ = 8.68 (s, 1H), 7.69 (s, 1H), 7.54 (s, 1H), 7.28 (s, 1H), 7.14-7.07 (m, 5H), 6.54 (s, 1H), 6.30 (s, 1H), 4.75-4.68 (m, 1H), 4.01 (s, 3H), 3.89 (s, 2H), 3.81 (dd, *J* = 11.0, 6.8, 1H), 3.47 (s, 3H), 2.78 (t, *J* = 11.1, 1H), 2.33 (s, 3H); MS-ESI: 460.15 (M+H)⁺.

### RIP1-046

¹H NMR (500 MHz, CDCl₃) δ = 8.85 (s, 1H), 8.00 (s, 1H), 7.99 (d, *J* = 8.4, 1H), 7.68 (s, 1H), 7.52 (s, 1H), 7.35-7.31 (m, 2H), 7.30-7.25 (m, 3H), 6.50 (s, 1H), 4.83-4.77 (m, 1H), 4.10 (s, 2H), 3.83 (dd, *J* = 11.1, 6.8, 1H), 3.49 (s, 3H), 2.87 (t, *J* = 11.2, 1H); ¹³C NMR (125 MHz, CDCl₃) δ 170.31, 162.98, 159.70, 141.31, 137.89, 135.78, 134.64, 134.58, 129.33, 128.80, 128.80, 127.31, 126.89, 119.70, 117.90, 116.70, 102.85, 49.36, 38.37, 37.16, 34.49; MS-ESI: 433.1 (M+H)⁺.

### RIP1-047

¹H NMR (500 MHz, CD₃OD) δ = 7.74 (s, 1H), 7.69 (s, 1H), 7.59 (s, 1H), 7.40 (d, *J*=3.1, 1H), 7.34 - 7.29 (m, 2H), 7.26 - 7.21 (m, 3H), 6.55 (dd, *J*=3.1, 0.7, 1H), 4.10 (s, 2H), 3.94 - 3.90 (m, 1H), 3.64 (dd, *J*=11.2, 6.9, 1H), 3.45 (s, 3H), 2.95 (t, *J*=11.4, 1H); MS-ESI: 432.1 (M+H)⁺.

### RIP1-048

¹H NMR (500 MHz, CDCl₃) δ = 8.15 (d, *J* = 7.5, 1H), 7.86 (s, 1H), 7.64 (s, 1H), 7.52 (s, 1H), 7.38-7.34 (m, 3H), 7.26-7.22 (m, 2H), 7.14 (d, *J* = 2.8, 1H), 6.47 (d, *J* = 3.0, 1H), 5.52 (d, *J* = 7.6, 2H), 4.81-1.74 (m, 1H), 3.82 (s, 3H), 3.80 (dd, *J*=7.0, 4.2, 1H), 3.48 (s, 3H), 2.87 (t, *J*=11.1, 1H); ^{l13}C NMR (125 MHz, CDCl₃) δ 170.30, 159.10, 143.24, 137.88, 135.60, 133.96, 131.45, 129.92, 129.38, 129.18, 128.32, 125.43, 119.69, 117.06, 116.37, 101.54, 54.60, 49.56, 38.52, 37.30, 33.29; MS-ESI: 447.4 (M+H)⁺.

### RIP1-049

¹H NMR (500 MHz, CDCl₃) δ = 8.00 (s, 1H), 7.95 (d, *J* = 7.6, 1H), 7.65 (s, 1H), 7.53 (s, 1H), 7.37-7.32 (m, 2H), 7.31-7.27 (m, 3H), 7.15 (d, *J* = 3.0, 1H), 6.48 (d, *J* = 3.0, 1H), 4.79-4.73 (m, 1H), 4.11 (s, 2H), 3.85-3.81 (m, 4H), 2.86 (t, *J* = 11.1, 1H); ¹³C NNM (125 MHz, CDCl₃) δ 170.47, 163.01, 159.70, 141.36, 137.83, 136.05, 135.63, 134.85, 131.47, 129.94, 128.94, 127.44, 119.65, 119.54, 117.06, 116.40, 101.55, 49.45, 38.56, 37.26, 34.65, 33.30; MS-ESI: 447.4 (M+H)⁺.

### RIP1-050

¹H NMR (500 MHz, CDCl₃) δ = 8.85 (brs, 1H), 7.60 (s, 1H), 7.50 (s, 1H), 7.34 (d, *J* = 7.0, 2H), 7.23 (t, *J* = 7.3, 2H), 7.20-7.16 (m, 2H), 7.14 (d, *J* = 3.0, 1H), 7.02 (d, *J* = 5.9, 1H), 6.47 (d, *J* = 3.1, 1H), 5.29 (s, 2H), 4.72-4.67 (m, 1H), 3.80 (s, 3H), 3.68 (dd, *J* = 11.4, 7.0, 1H), 3.41 (s, 3H), 3.17 (t, *J* = 11.4, 1H); ^{l13}C NMR (126 MHz, CDCl₃) δ 171.54, 159.75, 148.15, 137.59, 135.62, 131.65, 129.99, 129.25, 129.17, 128.78, 128.60, 127.73, 119.59, 117.19, 116.18, 101.75, 101.54, 53.56, 50.53, 49.68, 37.71, 33.29; MS-ESI: 447.3 (M+H)⁺.

### RIP1-051

¹H NMR (500 MHz, CDCl₃) δ = 8.64 (s, 1H), 8.06 (s, 1H), 7.69 (s, 1H), 7.65 (d, *J* = 7.3, 1H), 7.56 (s, 1H), 7.32-7.30 (m, 1H), 7.25-7.19 (m, 5H), 6.57-6.55 (m, 1H), 5.82 (d, *J* = 3.1, 2H), 4.76-4.70 (m, 1H), 3.75 (dd, *J* = 11.3, 7.0, 1H), 3.48 (s, 3H), 2.79 (t, *J* = 11.2, 1H); ^{l13}C NMR (125 MHz, CDCl₃) δ 170.38, 156.74, 137.78, 135.46, 134.75, 134.24, 129.92, 129.57, 128.78, 128.31, 128.05, 127.16, 119.75, 118.03, 117.03, 103.24, 53.28, 50.04, 38.17, 37.49 MS-ESI: 433.1 (M+H)⁺.

### RIP1-052

¹H NMR (500 MHz, CDCl₃) δ = 8.02 (s, 1H), 7.64 (s, 1H), 7.54 (s, 1H), 7.41 (d, *J* = 7.2, 1H), 7.26-7.20 (m, 5H), 7.17 (d, *J*=3.0, 1H), 6.50 (d, *J*=3.0, 1H), 5.82 (s, 2H), 4.71-4.65 (m, 1H), 3.84 (s, 3H), 3.77 (dd, *J* = 11.2, 6.9, 1H), 3.48 (s, 3H), 2.75 (t, *J* = 11.1, 1H); ¹³C NMR (125 MHz, CDCl₃) δ 170.15, 156.66, 137.40, 135.98, 135.69, 135.45, 134.14, 131.69, 130.00, 128.78, 128.34, 128.11, 119.33, 117.14, 116.43, 101.64, 53.24, 50.03, 38.26, 37.39, 33.32; MS-ESI: 447.3 (M+H)⁺.

### RIP1-054

¹H NMR (500 MHz, CDCl₃) δ = 7.62 (s, 1H), 7.57 (s, 1H), 7.53 (s, 1H), 7.52 (s, 1H), 7.27 (s, 1H), 7.26-7.23 (m, 2H), 7.16 (d, *J* = 7.1, 1H), 7.14 (d, *J* = 2.9, 1H), 7.07 (d, *J* = 7.0, 2H), 6.47 (d, *J* = 3.0, 1H), 5.44 (s, 2H), 4.71-4.65 (m, 1H), 3.81 (s, 3H), 3.77 (dd, *J* = 11.1,6.7, 1H), 3.47 (s, 1H), 2.75 (t, *J* = 11.1, 1H); ¹³C NMR (125 MHz, CDCl₃) δ 170.53, 159.00, 141.58, 137.61, 136.60, 135.62, 132.56, 131.51, 129.90, 128.91, 128.06, 127.35, 127.27, 119.52, 117.06, 116.35, 101.55, 49.97, 49.63, 38.50, 37.26, 33.25; MS-ESI: 446.4 (M+H)⁺.

### RIP1-055

¹H NMR (500 MHz, CDCl₃) δ = 8.03 (d, *J* = 7.7, 1H), 7.63 (s, 1H), 7.50 (s, 1H), 7.45 (s, 1H), 7.43 (s, 1H), 7.35-7.30 (m, 3H), 7.13 (d, *J* = 3.5, 3H), 6.46 (d, *J* = 3.0, 1H), 5.07 (s, 2H), 4.80-4.75 (m, 1H), 3.82-3.78 (m, 4H), 3.47 (s, 3H), 2.86 (t, *J* = 11.1, 1H); ¹³C NMR (125 MHz, CDCl₃) δ 170.77, 161.55, 138.01, 137.24, 137.06, 135.56, 135.31, 131.33, 129.86, 129.23, 128.69, 127.57, 122.37, 119.81, 117.00, 116.33, 101.48, 51.40, 49.30, 38.69, 37.21, 33.25; MS-ESI: 446.4 (M+H)⁺.

### RIP1-056

¹H NMR (500 MHz, CDCl₃) δ = 7.84 (d, *J* = 7.6, 1H), 7.64 (s, 1H), 7.51 (s, 1H), 7.14 (d, *J* = 2.9, 1H), 7.08 (d, *J* = 7.8, 2H), 6.98 (d, *J* = 7.8, 2H), 6.47 (d, *J* = 3.0, 1H), 6.45 (s, 1H), 4.81-4.75 (m, 1H), 3.90 (s, 2H), 3.86-3.82 (m, 4H), 3.69 (s, 3H), 3.48 (s, 3H), 2.84 (t, *J* = 11.1, 1H), 2.31 (s, 3H); ¹³C NMR (125 MHz, CDCl₃) δ 170.86, 161.24, 144.67, 143.21, 137.91, 136.60, 135.60, 133.73, 131.38, 129.89, 129.58, 128.34, 119.76, 117.01, 116.39, 106.82, 101.50, 49.46, 38.69, 37.22, 37.09, 33.27, 31.58, 21.12; MS-ESI: 474.4 (M+H)⁺.

### RIP1-057

¹H NMR (500 MHz, CDCl₃) δ = 7.63 (s, 1H), 7.52 (s, 1H), 7.14 (d, *J* = 3.0, 1H), 7.13-7.09 (m, 4H), 7.07 (d, *J* = 7.3, 1H), 6.47 (d, *J* = 3.0, 1H), 6.29 (s, 1H), 4.72-4.66 (m, 1H), 4.01 (s, 3H), 3.89 (s, 2H), 3.83-3.78 (m, 4H), 3.47 (s, 3H), 2.77 (t, *J* = 11.1, 1H), 2.33 (s, 3H); ¹³C NMR (125 MHz, CDCl₃) δ 170.44, 158.73, 150.97, 137.56, 136.44, 136.02, 135.64, 135.30, 131.55, 129.94, 129.42, 128.74, 119.42, 117.01, 116.37, 105.91, 101.57, 49.71, 39.07, 38.45, 37.18, 34.20, 33.26, 21.17; MS-ESI: 474.4 (M+H)⁺.

### RIP1-058

¹H NMR (500 MHz, CDCl₃) δ = 8.32 (s, 1H), 8.12 (d, *J=* 7.4, 1H), 7.65 (s, 1H), 7.53 (s, 1H), 7.36-7.29 (m, 4H), 7.16 (d, *J* = 3.0, 1H), 6.49 (d, *J* = 3.0, 1H), 4.74-4.68 (m, 1H), 4.23 (s, 2H), 3.86-3.81 (m, 4H), 3.48 (s, 3H), 2.85 (t, *J* = 11.1, 1H); MS-ESI: 448.4 (M+H)⁺.

### RIP1-059

¹H NMR (400 MHz, Methanol-d4) δ 8.31 (s, 1H), 7.97 (s, 1H), 7.81 (dd, J = 9.0, 1.4 Hz, 1H), 7.79 - 7.73 (m, 2H), 7.61 (d, J = 1.3 Hz, 1H), 7.39 - 7.24 (m, 6H), 5.26 (s, 1H), 5.22 (s, 2H), 4.69 (dd, J = 11.5, 6.9 Hz, 1H), 3.68 (dd, J = 11.3, 6.9 Hz, 1H), 3.46 (s, 3H), 2.98 (t, J = 11.4 Hz, 1H); ESI: m/z 433.1 (M+H)⁺.

### RIP1-060

¹H NMR (400 MHz, Methanol-d4) δ 8.55 (s, 1H), 8.32 (s, 1H), 7.97 (s, 1H), 7.76 (s, 1H), 7.36 - 7.34 (m, 4H), 7.33 (d, J = 3.5 Hz, 5H), 5.44 (s, 2H), 5.42 (s, 1H), 4.71 (dd, J = 11.6, 6.9 Hz, 1H), 3.68 (dd, J = 11.4, 6.9 Hz, 1H), 3.45 (s, 2H), 3.15 - 2.95 (m, 1H); ESI: m/z 434.1 (M+H)⁺.

### RIP1-061

¹H NMR (500 MHz, CDCl₃) δ = 7.82 (d, *J* = 7.0, 1H), 7.38 (brs, 1H), 7.35-7.24 (m, 5H), 7.21 (d, *J* = 7.3, 2H), 6.27 (s, 1H), 4.99-4.93 (m, 1H), 4.66 (dd, *J* = 9.4, 7.9, 1H), 4.22 (t, *J* = 10.5, 1H), 4.09 (s, 2H), 3.42 (s, 3H), 2.61 (s, 3H); MS-ESI: 432.1 (M+H)⁺; HRMS (ESI) Calcd. for C₂₃H₂₂O₄N₅ (M+H)⁺: 432.1666, found: 432.1662.

### RIP1-062

¹H NMR (500 MHz, CDCl₃) δ = 7.87 (s, 1H), 7.79 (s, 1H), 7.37-7.27 (m, 4H), 7.24-7.20 (m, 3H), 7.17 (d, *J* = 6.8, 1H), 5.27 (s, 2H), 5.00-4.94 (m, 1H), 4.66 (dd, *J* = 9.6, 7.6, 1H), 4.21 (t, *J* = 10.5, 1H), 3.40 (s, 3H), 2.57 (s, 3H); MS-ESI: 431.4 (M+H)⁺; HRMS (ESI) Calcd. for C₂₃H₂₃O₃N₆ (M+H)⁺: 431.1826, found: 431.1827.

### RIP1-063

¹H NMR (500 MHz, CDCl₃) δ = 8.09 (d, *J* = 7.3, 1H), 7.91 (s, 1H), 7.36-7.31 (m, 3H), 7.29 (s, 1H), 7.25-7.20 (m, 3H), 5.51 (d, *J* = 3.9, 2H), 5.03-4.97 (m, 1H), 4.62 (dd, *J* = 9.3, 7.8, 1H), 4.24 (t, *J* = 0.5, 1H), 3.39 (s, 3H), 2.56 (s, 3H); MS-ESI: 432.3 (M+H)⁺; HRMS (ESI) Calcd. for C₂₂H₂₂O₃N₇ (M+H)⁺: 432.1779, found: 432.1778.

### RIP1-064

¹H NMR (500 MHz, CDCl₃) δ = 8.10 (s, 1H), 7.74 (d, *J* = 7.0, 1H), 7.37 (s, 1H), 7.30 (s, 1H), 7.26-7.18 (m, 5H), 5.80 (s, 2H), 5.05-4.97 (m, 1H), 4.65-4.53 (m, 1H), 4.20 (t, *J* = 10.4, 1H), 3.44 (s, 3H), 2.62 (s, 3H); MS-ESI: 432.4 (M+H)⁺; HRMS (ESI) Calcd. for C₂₂H₂₂O₃N₇ (M+H)⁺: 432.1779, found: 432.1779.

### RIP1-065

¹H NMR (500 MHz, CDCl₃) δ = 8.04 (s, 1H), 7.94 (d, *J* = 7.3, 1H), 7.35-7.26 (m, 6H), 7.24 (s, 1H), 5.00-4.94 (m, 1H), 4.64 (dd, *J* = 9.6, 7.6, 1H), 4.24 (t, *J* = 10.5, 1H), 4.11 (s, 2H), 3.41 (s, 3H), 2.58 (s, 3H); ^{l13}C NMR (125 MHz, CDCl₃) δ 168.70, 163.42, 160.70, 153.52, 146.05, 141.68, 136.01, 135.52, 134.61, 131.65, 128.96, 128.92, 128.67, 127.51, 109.29, 107.33, 77.16, 49.14, 36.31, 34.60, 14.95; MS-ESI: 432.3 (M+H)⁺; HRMS (ESI) Calcd. for C₂₃H₂₂O₄N₅ (M+H)⁺: 432.1666, found: 432.1668.

### RIP1-066

¹H NMR (500 MHz, CDCl₃) δ = 7.68-7.62 (m, 2H), 7.48 (d, *J* = 6.5, 1H), 7.39-7.27 (m, 4H), 7.24 (d, *J* = 6.5, 2H), 7.18 (t, *J* = 7.3, 1H), 7.15 (d, *J* = 7.6, 2H), 5.07-4.96 (m, 1H), 4.77 (dd, *J* = 9.5, 7.6, 1H), 4.25 (t, *J* = 10.4, 1H), 4.00 (s, 2H), 3.44 (s, 3H), 2.55 (s, 3H); ¹³C NMR (125 MHz, CDCl₃) δ 169.13, 167.46, 153.51, 146.01, 142.09, 140.34, 133.42, 132.99, 131.31, 129.02, 128.96, 128.72, 127.82, 126.49, 125.02, 109.75, 109.71, 106.91, 106.87, 77.41, 50.00, 41.83, 36.38, 14.96; MS-ESI: 431.3 (M+H)⁺; HRMS (ESI) Calcd. for C₂₆H₂₅O₃N₄ (M+H)⁺: 431.1921, found: 432.1922.

### RIP1-067

¹H NMR (500 MHz, CD₃OD) δ = 7.76 (s, 1H), 7.63 (s, 1H), 7.51 (s, 1H), 7.36-7.27 (m, 4H), 7.24 (d, *J* = 7.7, 2H), 5.21 (s, 2H), 5.00-4.94 (m, 1H), 4.53 (dd, *J* = 9.7, 7.8, 1H), 4.29 (t, *J* = 10.6, 1H), 3.43 (s, 3H), 2.58 (s, 3H); ¹³C NMR (125 MHz, CD₃OD) δ 171.00, 164.12, 155.23, 147.60, 139.43, 137.54, 136.79, 133.32, 130.05, 129.42, 128.86, 124.35, 110.26, 110.25, 108.27, 108.24, 78.21, 52.01, 50.22, 36.48, 14.35; MS-ESI: 431.4 (M+H)⁺; HRMS (ESI) Calcd. for C₂₃H₂₃O₃N₆ (M+H)⁺: 431.1826, found: 431.1829.

### RIP1-068

¹H NMR (500 MHz, CDCl₃) δ = 8.18 (d, *J* = 6.8, 1H), 8.10 (s, 1H), 7.38-7.32 (m, 4H), 7.26-7.22 (m, 3H), 5.36 (s, 2H), 5.03-4.97 (m, 1H), 4.77 (dd, *J* = 9.2, 7.9, 1H), 4.25 (t, *J* = 10.4, 1H), 3.42 (s, 3H), 2.56 (s, 3H); ¹³C NMR (125 MHz, CDCl₃) δ 168.59, 159.01, 156.58, 153.60, 145.85, 144.35, 133.69, 131.17, 129.35, 129.17, 128.33, 109.89, 109.84, 107.10, 107.05, 77.36, 54.54, 49.69, 36.35, 14.92; MS-ESI: 432.4 (M+H)⁺; HRMS (ESI) Calcd. for C₂₂H₂₂O₃N₇ (M+H)⁺: 432.1779, found: 432.1777.

### RIP1-069

¹H NMR (500 MHz, CDCl₃) δ = 7.65 (s, 1H), 7.56 (s, 1H), 7.30-7.26 (m, 2H), 7.26-7.19 (m, 4H), 7.07 (d, *J* = 1.3, 1H), 7.06 (s, 1H), 5.44 (s, 2H), 4.98-4.91 (m, 1H), 4.60 (dd, *J* = 9.7, 7.6, 1H), 4.23- 4.09 (m, 1H), 3.41 (s, 3H), 2.57 (s, 3H); ¹³C NMR (125 MHz, CDCl₃) δ 169.19, 159.69, 153.28, 146.06, 141.71, 136.46, 132.94, 131.44, 128.97, 128.16, 127.25, 125.03, 109.68, 109.66, 107.23, 107.22, 77.36, 50.14, 49.18, 36.39, 15.06; MS-ESI: 431.4 (M+H)⁺; HRMS (ESI) Calcd. for C₂₃H₂₃O₆N₃ (M+H)⁺: 431.1826, found: 431.1829.

### RIP1-070

¹H NMR (500 MHz, CDCl₃) δ = 8.11 (d, *J* = 6.9, 1H), 7.41 (s, 1H), 7.34-7.26 (m, 6H), 4.99-4.93 (m, 1H), 4.68 (dd, *J* = 9.6, 7.8, 1H), 4.26-4.20 (m, 4H), 3.43 (s, 3H), 2.61 (s, 3H); MS-ESI: 433.4 (M+H)⁺; HRMS (ESI) Calcd. for C₂₂H₂₁O₄N₆ (M+H)⁺: 433.1619, found: 433.1613.

### RIP1-071

¹H NMR (500 MHz, CDCl₃) δ = 8.16 (d, *J* = 6.7, 1H), 7.40 (s, 1H), 7.39-7.33 (m, 5H), 7.29 (s, 1H), 5.80 (s, 2H), 5.06-5.00 (m, 1H), 4.77 (dd, *J* = 9.6, 7.7, 1H), 4.25 (t, *J* = 10.4, 1H), 3.44 (s, 3H), 2.60 (s, 3H); ^{l13}C NMR (125 MHz, CDCl₃) δ 168.24, 159.37, 156.51, 153.65, 146.04, 132.32, 131.31, 129.55, 129.32, 128.76, 110.18, 110.14, 107.06, 107.01, 77.04, 57.75, 49.82, 36.42, 14.96; MS-ESI: 433.3 (M+H)⁺; HRMS (ESI) Calcd. for C₂₁H₂₁O₃N₈(M+H)⁺: 433.1731, found: 433.1733.

### RIP1-072

¹H NMR (500 MHz, CDCl₃) δ = 7.86 (d, *J* = 7.1, 1H), 7.28 (s, 1H), 7.24 (s, 1H), 7.08 (d, *J* = 7.8, 2H), 6.98 (d, *J* = 7.7, 2H), 6.45 (s, 1H), 5.02-4.93 (m, 1H), 4.68 (t, *J*= 8.6, 1H), 4.24 (t, *J*=10.4, 1H), 3.91 (s, 2H), 3.72 (s, 3H), 3.40 (s, 3H), 2.57 (s, 3H), 2.30 (s, 3H); ^{l13}C NMR (125 MHz, CDCl₃) δ 168.97, 162.35, 153.60, 145.99, 143.87, 143.82, 136.74, 133.46, 131.58, 129.62, 128.35, 109.21, 109.16, 107.33, 107.25, 106.77, 77.36, 49.22, 37.21, 36.26, 31.58, 21.10, 14.93; MS-ESI: 459.4 (M+H)⁺; HRMS (ESI) Calcd. for C₂₅H₂₇O₃N₆ (M+H)⁺: 459.2139, found: 59.2141.

### RIP1-073

¹H NMR (500 MHz, CDCl₃) δ = 7.37 (s, 1H), 7.26 (s, 1H), 7.13-7.08 (m, 5H), 6.36 (s, 1H), 4.96-4.89 (m, 1H), 4.64 (dd, *J* = 9.6, 7.6, 1H), 4.16 (t, *J* = 10.5, 1H), 4.01 (s, 3H), 3.89 (s, 2H), 3.42 (s, 3H), 2.61 (s, 3H), 2.31 (s, 3H); ¹³C NMR (126 MHz, CDCl₃) δ 168.86, 159.53, 153.46, 151.15, 146.25, 136.27, 136.10, 134.95, 131.73, 129.43, 128.71, 107.19, 107.17, 107.13, 107.13, 106.33, 77.29, 49.32, 39.10, 36.34, 34.14, 21.14, 15.01; MS-ESI: 459.4 (M+H)⁺; HRMS (ESI) Calcd. for C₂₅H₂₇O₃N₆ (M+H)⁺: 459.2139, found: 459.2140.

### RIP 1-074

¹H NMR (500 MHz, CD₃OD) δ = 8.18 (s, 1H), 7.98 (s, 1H), 7.40-7.33 (m, 3H), 7.31-7.28 (m, 2H), 7.11 (s, 1H), 6.93 (s, 1H), 5.38 (s, 2H), 5.08-5.03 (m, 1H), 4.48 (dd, *J* = 9.8, 7.8, 1H), 4.41 (dd, *J* = 11.5, 9.9, 1H), 3.41 (s, 3H); ESI-MS *m*/*z* 433.0 (M + H)⁺.

### RIP 1-075

¹H NMR (500 MHz, CD₃OD) δ = 7.46-7.43 (m, 1H), 7.43-7.38 (m, 2H), 7.11 (s, 1H), 6.95 (s, 1H), 5.96 (s, 1H), 5.38-5.35 (m, 1H), 5.07 (dd, *J* = 11.4, 7.7, 1H), 4.46 (dd, *J* = 11.3, 10.0, 1H), 3.42 (s, 2H); ESI-MS *m*/*z* 435.3 (M + H)⁺.

### RIP 1-076

¹H NMR (500 MHz, CD₃OD) δ = 7.35-7.28 (m, 5H), 7.10 (s, 1H), 6.95 (s, 1H), 5.03 (dd, *J* = 11.2, 7.5, 1H), 4.37 (dd, *J* = 11.1, 10.1, 1H), 4.18 (s, 2H), 4.09-4.03 (m, 1H), 3.42 (s, 3H); ESI-MS *m*/*z* 434.4 (M + H)⁺.

### RIP1-077

¹H NMR (400 MHz, CDCl₃) δ = 7.86 (d, *J* = 8.3, 1H), 7.67 (s, 1H), 7.51-7.41 (m, 1H), 7.33-7.25 (m, 3H), 7.21 (d, *J* = 7.0, 2H), 6.27 (s, 1H), 5.02-4.86 (m, 1H), 4.73 (dd, *J* = 11.6, 5.7, 1H), 4.27 (t, *J* = 10.5, 1H), 4.09 (s, 2H), 3.47 (s, 3H). HRMS (ESI) Calcd. for C₂₃H₁₈F₃N₅O₄ (M+H)⁺: 486.1389, found: 486.1392.

### RIP1-078

¹H NMR (500 MHz, CDCl₃) δ = 12.22 (s, 1H), 8.23 (s, 2H), 7.90 (s, 1H), 7.65 (s, 1H), 7.40-7.34 (m, 4H), 7.27 (d, *J* = 3.3, 2H), 5.54 (s, 2H), 5.06-4.98 (m, 1H), 4.33 (t, *J*=10.5, 1H), 3.67-3.33 (m, 4H); HRMS (ESI) Calcd. for C₂₂H₁₈F₃N₇O₃Na (M+Na)⁺: 508.1321, found: 508.1315.

### RIP1-079

¹H NMR (500 MHz, CDCl₃) δ 12.15 (s, 1H), 8.13 (d, *J* = 6.5 Hz, 1H), 8.05 (s, 1H), 7.70 (s, 1H), 7.35 (d, *J* = 7.2 Hz, 2H), 7.33-7.30 (m, 4H), 5.04-4.97 (m, 1H), 4.85-4.80 (m, 1H), 4.36 (t, *J* = 10.4 Hz, 1H), 4.14 (s, 3H), 3.54 (s, 2H); HRMS (ESI) Calcd. for C₂₃H₁₈F₃N₅O₄ (M+H)⁺: 486.1389, found: 486.1390.

### RIP1-080

¹H NMR (500 MHz, CDCl₃) δ = 7.84 (d, *J* = 7.3, 1H), 7.64 (s, 1H), 7.52 (s, 1H), 7.35-7.27 (m, 3H), 7.21 (d, *J* = 7.5, 2H), 7.15 (d, *J* = 2.7, 1H), 6.48 (d, *J* = 2.9, 1H), 6.26 (s, 1H), 4.77-4.70 (m, 1H), 4.08 (s, 2H), 3.86-3.77 (m, 4H), 3.47 (s, 3H), 2.84 (t, *J* = 11.1, 1H); ¹³C NMR (125 MHz, CDCl₃) δ 173.97, 170.04, 158.30, 158.11, 137.68, 135.62, 135.39, 131.53, 129.94, 129.01, 128.88, 127.45, 119.51, 117.06, 116.39, 101.69, 101.55, 49.82, 38.31, 37.30, 33.29, 29.45; MS-ESI: 447.3 (M+H)⁺.

### RIP1-081

¹H NMR (500 MHz, CDCl₃) δ = 8.12 (d, *J=* 7.2, 1H), 7.65 (s, 1H), 7.53 (s, 1H), 7.34-7.27 (m, 5H), 7.16 (d, *J* = 3.0, 1H), 6.49 (d, *J* = 3.1, 1H), 4.74-4.68 (m, 1H), 4.23 (s, 2H), 3.86-3.81 (m, 4H), 3.48 (s, 3H), 2.85 (t, *J* = 11.1, 1H); ¹³C NMR (125 MHz, CDCl₃) δ 169.44, 167.63, 158.80, 152.21, 137.46, 135.66, 133.05, 131.67, 130.02, 129.14, 129.00, 127.92, 119.28, 117.11, 116.47, 101.61, 50.24, 38.13, 37.39, 33.32, 31.96; ; MS-ESI: 448.4 (M+H)⁺.

### RIP1-082

¹H NMR (500 MHz, CDCl₃) δ = 7.82 (s, 1H), 7.77 (s, 1H), 7.64 (s, 1H), 7.52 (s, 1H), 7.36-7.29 (m, 3H), 7.21 (d, *J* = 7.3, 2H), 7.14 (d, *J* = 2.4, 1H), 7.07 (d, *J* = 7.2, 1H), 6.47 (d, *J* = 3.0, 1H), 5.25 (s, 2H), 4.78-4.72 (m, 1H), 3.85-3.80 (m, 4H), 3.46 (s, 3H), 2.79 (t, *J* = 11.1, 1H); ¹³C NMR (125 MHz, CDCl₃) δ 171.11, 161.37, 138.86, 137.65, 135.64, 135.46, 131.49, 130.83, 129.90, 129.09, 128.57, 128.10, 119.60, 118.55, 117.04, 116.38, 101.54, 56.57, 49.73, 38.66, 37.31, 33.27; MS-ESI: 446.4 (M+H)⁺.

### RIP1-083

¹H NMR (500 MHz, CDCl₃) δ = 7.83 (s, 1H), 7.78 (s, 1H), 7.61 (s, 1H), 7.54 (s, 1H), 7.37-7.28 (m, 7H), 7.22 (d, *J* = 1.5, 1H), 7.21 (d, *J* = 3.1, 2H), 7.16 (d, *J* = 0.7, 1H), 7.15 (s, 1H), 6.54 (d, *J* = 3.1, 1H), 5.32 (s, 2H), 5.25 (s, 2H), 4.82-4.75 (m, 1H), 3.78 (dd, *J* = 11.2, 6.9, 1H), 3.45 (s, 3H), 2.78 (t, *J* = 11.2, 1H); ¹³C NMR(125 MHz, CDCl₃) δ 171.18, 161.43, 138.92, 137.87, 136.76, 135.45, 135.23, 130.86, 130.83, 130.12, 129.09, 129.07, 129.04, 128.54, 128.09, 126.97, 119.92, 118.49, 117.19, 116.52, 102.27, 56.53, 50.44, 49.70, 38.59, 37.36, 1.14; MS-ESI: 522.4 (M+H)⁺.

### RIP1-084

¹H NMR (500 MHz, CDCl₃) δ = 8.15 (d, *J* = 7.9, 1H), 7.88 (s, 1H), 7.61 (s, 1H), 7.53 (s, 1H), 7.36-7.33 (m, 5H), 7.26 -7.22 (m, 3H), 7.20 (d, *J* = 3.0, 1H), 7.15 (d, *J* = 7.3, 2H), 6.53 (d, *J* = 3.1, 1H), 5.51 (d, *J* = 6.4, 2H), 5.32 (s, 2H), 4.83-4.76 (m, 1H), 3.78 (dd, *J* = 11.2, 6.8, 1H), 3.47 (s, 3H), 2.85 (t, *J* = 11.2, 1H); ¹³C NMR (125 MHz, CDCl₃) δ 170.31, 159.09, 143.19, 138.04, 136.77, 135.15, 133.95, 130.82, 130.10, 129.32, 129.10, 129.06, 128.26, 128.06, 126.96, 125.43, 119.94, 117.17, 116.48, 102.23, 54.53, 50.42, 49.49, 38.45, 37.32; ; MS-ESI: 523.4 (M+H)⁺.

### RIP1-085

¹H NMR (500 MHz, CDCl₃) δ = 7.85 (d, *J* = 7.7, 1H), 7.61 (s, 1H), 7.54 (d, *J* = 1.1, 1H), 7.37-7.30 (m, 6H), 7.23-7.20 (m, 3H), 7.16 (d, *J* = 7.6, 2H), 6.55 (s, 1H), 6.27 (s, 1H), 5.33 (s, 2H), 4.79-4.73 (m, 1H), 4.08 (s, 2H), 3.79 (dd, *J* = 11.3, 6.5, 1H), 3.47 (s, 3H), 2.81 (t, *J* = 11.1, 1H); ¹³C NMR (125 MHz, CDCl₃) δ 173.96, 170.04, 158.28, 158.11, 137.89, 136.75, 135.36, 135.20, 130.91, 130.15, 129.10, 128.99, 128.86, 128.10, 127.43, 126.98, 119.82, 117.20, 116.54, 102.27, 101.67, 50.45, 49.79, 38.26, 37.34, 33.26; ; MS-ESI: 523.4 (M+H)⁺.

### RIP1-086

¹H NMR (500 MHz, CDCl₃) δ = 8.18 (d, *J* = 7.6, 1H), 8.01 (s, 1H), 7.62 (s, 1H), 7.53 (s, 1H), 7.37-7.33 (m, 5H), 7.30 (d, *J* = 7.0, 1H), 7.26 (t, *J* = 3.7, 2H), 7.21 (d, *J* = 3.1, 1H), 7.17 (d, *J* = 7.4, 2H), 6.54 (d, *J* = 3.1, 1H), 5.35 (s, 2H), 5.33 (s, 2H), 4.86-4.80 (m, 1H), 3.87 (dd, *J* = 11.1,7.0, 1H), 3.47 (s, 3H), 2.81 (t, *J* = 11.1, 1H); ¹³C NMR (125 MHz, CDCl₃) δ 170.34, 157.91, 156.99, 144.00, 137.84, 136.77, 135.22, 133.92, 130.85, 130.11, 129.25, 129.08, 129.00, 128.31, 128.07, 127.00, 119.84, 117.19, 116.62, 102.23, 54.37, 50.43, 49.74, 38.48, 37.36; MS-ESI: 523.5 (M+H)⁺.

### RIP1-087

¹H NMR (500 MHz, CDCl₃) δ = 8.00 (s, 1H), 7.95 (d, *J* = 7.7, 1H), 7.62 (s, 1H), 7.54 (s, 1H), 7.36-7.33 (m, 4H), 7.32 (s, 1H), 7.30-7.28 (m, 3H), 7.21 (d, *J* = 3.1, 1H), 7.16 (d, *J* = 7.2, 2H), 6.54 (d, *J* = 3.1, 1H), 5.33 (s, 2H), 4.81-4.75 (m, 1H), 4.10 (s, 2H), 3.79 (dd, *J* = 11.0, 6.9, 1H), 3.48 (s, 3H), 2.83 (t, *J* = 11.1, 1H); ¹³C NMR (126 MHz, CDCl₃) δ 170.45, 163.02, 159.69, 141.37, 138.01, 136.78, 135.99, 135.20, 134.80, 130.84, 130.13, 129.10, 128.91, 128.09, 127.90, 127.41, 126.97, 119.94, 117.18, 116.53, 102.26, 50.45, 49.41, 38.50, 37.29, 34.61; MS-ESI: 523.4 (M+H)⁺.

### RIP1-088

¹H NMR (500 MHz, CDCl₃) δ = 8.04 (d, *J* = 7.9, 1H), 7.60 (s, 1H), 7.52 (s, 1H), 7.47 (s, 1H), 7.44 (s, 1H), 7.36-7.31 (m, 6H), 7.19 (d, *J* = 2.2, 1H), 7.15 (d, *J* = 7.8, 2H), 7.13 (d, *J* = 7.0, 2H), 6.53 (d, *J* = 2.9, 1H), 5.32 (s, 2H), 5.07 (s, 2H), 4.83-4.77 (m, 1H), 3.76 (dd, *J* = 11.0, 6.8, 1H), 3.45 (s, 3H), 2.84 (t, *J* = 11.2, 1H); ¹³C NMR (125 MHz, CDCl₃) δ 170.77, 161.52, 138.16, 137.13, 137.02, 136.80, 135.23, 135.12, 130.71, 130.05, 129.20, 129.04, 128.66, 128.02, 127.56, 126.95, 122.43, 120.07, 117.13, 116.45, 102.20, 51.38, 50.39, 49.29, 38.59, 37.25; ; MS-ESI: 522.3 (M+H)⁺.

### RIP1-089

¹H NMR (400 MHz, CD₃OD_SPE) δ 8.30 (s, 1H), 8.00 (d, J = 18.6 Hz, 1H), 7.75 (s, 1H), 7.26 (dd, J = 18.8, 6.7 Hz, 5H), 6.33 (s, 1H), 4.66 (dd, J = 11.7, 7.0 Hz, 1H), 4.11 (s, 2H), 3.69 - 3.51 (m, 1H), 3.43 (s, 3H), 3.30 (dt, J = 3.3, 1.6 Hz, 2H), 3.05 (t, J = 11.5 Hz, 1H). ¹³C NMR (101 MHz, CD₃OD_SPE) δ 174.48 (s), 170.74 (s), 159.07 (s), 157.90 (s), 143.94 (s), 141.00 (s), 135.77 (s), 128.47 (s), 128.45 (s), 126.85 (s), 121.08 (s), 49.89 (s), 36.95 (s), 36.10 (s), 32.22 (s). ESI: m/z 434.1 (M+H)⁺.

### RIP1-090

¹H NMR (400 MHz, CD₃OD_SPE) δ 8.30 (s, 1H), 7.94 (s, 1H), 7.74 (s, 1H), 7.25 (dd, J = 14.0, 6.9 Hz, 5H), 4.70 (dd, J = 11.6, 6.9 Hz, 1H), 4.12 (s, 2H), 3.66 (dt, J = 16.1, 8.1 Hz, 1H), 3.44 (s, 3H), 3.05 - 2.96 (m, 1H). ESI: m/z 434.1 (M+H)⁺.

### RIP1-091

¹H NMR (400 MHz, CDCl₃) δ 8.14 (s, 1H), 7.98 (d, J = 2.0 Hz, 1H), 7.95 (s, 1H), 7.62 (s, 1H), 7.32 (dt, J = 28.9, 5.7 Hz, 5H), 5.54 (s, 2H), 4.81 - 4.71 (m, 1H), 3.76 (dd, J = 10.9, 7.1 Hz, 1H), 3.48 (s, 3H), 2.93 (t, J = 11.3 Hz, 1H). ¹³C NMR (101 MHz, CDCl₃) δ 170.35 (s), 159.33 (s), 159.26 (s), 143.32 (s), 142.62 (s), 142.59 (s), 140.73 (s), 139.38 (s), 136.30 (s), 133.76 (s), 129.19 (s), 129.01 (s), 128.12 (s), 125.65 (s), 121.87 (s), 121.19 (s), 112.26 (s), 63.44 (s), 54.43 (s), 38.09 (s), 37.12 (s). ESI: m/z 434.1 (M+H)⁺.

### RIP1-092

¹H NMR (400 MHz, CDCl₃) δ 8.96 (dd, *J* = 4.2, 1.5 Hz, 1H), 8.13 (d, *J* = 8.4 Hz, 1H), 8.08 (d, *J* = 8.8 Hz, 1H), 7.90 (d, *J* = 8.8 Hz, 1H), 7.47 (dd, *J* = 8.6, 4.2 Hz, 1H), 7.24 - 7.15 (m, 5H), 4.77 (dd, *J* = 10.5, 7.4 Hz, 1H), 4.09 (s, 2H), 3.87 (dd, *J* = 11.4, 7.2 Hz, 1H), 3.46 (s, 2H), 3.38 (s, 3H), 3.03 (t, *J* = 11.2 Hz, 1H). ¹³C NMR (100 MHz, CDCl₃) δ 172.01, 158.15, 151.46, 149.38, 141.76, 135.89, 135.02, 131.82, 130.26, 128.87, 128.80, 127.13, 127.06, 124.16, 122.32, 60.46, 50.79, 49.72, 39.25, 36.93, 33.20. MS (ESI): m/z 445.1 (M+H)⁺.

### RIP1-093

¹H NMR (400 MHz, CDCl₃) δ 9.00 (dd, *J* = 4.2, 1.6 Hz, 1H), 8.19 - 8.13 (m, 2H), 8.10 (dd, *J* = 8.8, 0.5 Hz, 1H), 8.03 (s, 1H), 7.93 (d, *J* = 8.7 Hz, 1H), 7.51 (dd, *J* = 8.6, 4.2 Hz, 1H), 7.39 - 7.34 (m, 3H), 7.29-7.25 (m, 2H), 5.37 (s, 2H), 4.84 (dt, *J* = 10.7, 7.4 Hz, 1H), 4.01 (dd, *J* = 11.4, 7.2 Hz, 1H), 3.42 (s, 3H), 3.03 (t, *J* = 11.1 Hz, 1H). ¹³C NMR (100 MHz, CDCl₃) δ 171.94, 158.02, 156.69, 151.46, 149.52, 143.95, 141.71, 135.07, 133.77, 131.71, 130.33, 129.20, 128.98, 128.24, 127.10, 124.19, 122.23, 54.34, 49.68, 39.52, 36.90. MS (ESI): m/z 445.1 (M+H)⁺.

### RIP1-094

¹H NMR (400 MHz, CDCl₃) 8.96 (dd, *J* = 4.1, 1.5 Hz, 1H), 8.15 (d, *J* = 8.5 Hz, 1H), 8.05 (d, *J* = 8.8 Hz, 1H), 7.99 (d, *J* = 7.9 Hz, 1H), 7.90 (d, *J* = 8.7 Hz, 1H), 7.51 - 7.41 (m, 3H), 7.36 - 7.28 (m, 3H), 7.15 - 7.09 (m, 2H), 5.07 (s, 2H), 4.77 (dt, *J* = 10.9, 7.5 Hz, 1H), 3.87 (dd, *J* = 11.3, 7.1 Hz, 1H), 3.38 (s, 3H), 3.04 (t, *J* = 11.1 Hz, 1H). ¹³C NMR (100 MHz, CDCl₃) δ 172.38, 161.63, 151.35, 149.45, 142.03, 137.07, 136.74, 135.08, 135.07, 131.86, 130.06, 129.16, 128.66, 127.50, 127.19, 124.23, 122.54, 122.20, 51.35, 49.27, 39.62, 36.80. MS (ESI): m/z 444.1 (M+H)⁺.

### RIP1-095

¹H NMR (400 MHz, CDCl₃) δ 8.99 (dd, *J* = 4.2, 1.6 Hz, 1H), 8.18 (dd, *J* = 8.5, 0.6 Hz, 1H), 8.13 - 8.05 (m, 2H), 7.93 (d, *J* = 8.7 Hz, 1H), 7.87 (s, 1H), 7.51 (dd, *J* = 8.6, 4.2 Hz, 1H), 7.38 - 7.34 (m, 3H), 7.27 - 7.22 (m, 2H), 5.53 (d, *J* = 1.3 Hz, 2H), 4.79 (dt, *J* = 10.9, 7.4 Hz, 1H), 3.42 (s, 3H), 3.07 (t, *J* = 11.1 Hz, 1H). ¹³C NMR (100 MHz, CDCl₃) δ 171.86, 159.14, 151.44, 149.50, 142.86, 141.91, 135.03, 133.75, 131.77, 130.22, 129.28, 129.11, 128.22, 127.10, 125.42, 124.21, 122.25, 54.52, 49.45, 39.45, 36.86. MS (ESI): m/z 445.1 (M+H)⁺.

### RIP1-096

¹H NMR (400 MHz, CDCl₃) δ 9.00 (dd, *J* = 4.1, 1.5 Hz, 1H), 8.17 (d, *J* = 8.1 Hz, 1H), 8.10 (d, *J* = 8.8 Hz, 1H), 7.93 (d, *J* = 8.7 Hz, 1H), 7.81 (d, *J* = 7.4 Hz, 1H), 7.52 (dd, *J* = 8.6, 4.2 Hz, 1H), 7.35 - 7.18 (m, 5H), 6.26 (s, 1H), 4.75 (dt, *J* = 10.7, 7.3 Hz, 1H), 4.09 (s, 2H), 3.93 (dd, *J* = 11.3, 7.1 Hz, 1H), 3.42 (s, 3H), 3.03 (t, *J* = 11.1 Hz, 1H). ¹³C NMR (100 MHz, CDCl₃) δ 174.10, 171.58, 158.19, 157.97, 151.51, 149.51, 141.74, 135.18, 135.01, 131.70, 130.36, 128.93, 128.75, 127.39, 127.03, 124.18, 122.30, 101.58, 49.72, 39.27, 36.88, 33.18. MS (ESI): m/z 445.1 (M+H)⁺.

### RIP1-097

¹H NMR (400 MHz, MeOD) δ 8.80 (d, *J* = 3.5 Hz, 1H), 8.30 (d, *J* = 8.3 Hz, 1H), 7.97 (d, *J* = 9.0 Hz, 1H), 7.61 - 7.51 (m, 2H), 7.25 - 7.07 (m, 5H), 4.95 (d, *J* = 7.7 Hz, 1H), 4.63 - 4.52 (m, 1H), 4.45 (t, *J* = 9.9 Hz, 1H), 4.14 - 3.97 (m, 2H), 3.32 (s, 3H). ¹³C NMR (100 MHz, MeOD) δ 171.13, 149.77, 149.28, 145.94, 132.31, 130.26, 129.57, 128.49, 128.36, 126.82, 125.23, 124.19, 121.90, 77.28, 49.09, 36.43. MS (ESI): m/z 429.1 (M+H)⁺.

### RIP1-098

¹H NMR (400 MHz, CDCl₃) δ 8.96 (dd, *J* = 4.2, 1.6 Hz, 1H), 8.17 - 8.06 (m, 3H), 8.02 (s, 1H), 7.59 (d, *J* = 9.0 Hz, 1H), 7.50 (dd, *J* = 8.6, 4.2 Hz, 1H), 7.41 - 7.36 (m, 3H), 7.29 (dd, *J* = 7.1, 2.4 Hz, 2H), 5.38 (s, 2H), 5.17 (dt, *J* = 10.9, 7.5 Hz, 1H), 4.85 (dd, *J* = 9.9, 7.6 Hz, 1H), 4.41 - 4.34 (m, 1H), 3.47 (s, 3H). ¹³C NMR (100 MHz, CDCl₃) δ 170.89, 158.47, 156.57, 150.04, 149.22, 146.78, 143.98, 133.72, 131.10, 131.08, 129.57, 129.22, 129.01, 128.27, 125.30, 124.07, 121.71, 78.16, 54.39, 48.95, 37.41. MS (ESI): m/z 429.1 (M+H)⁺.

### RIP1-099

¹H NMR (400 MHz, CDCl₃) δ 8.94 (dd, *J* = 4.1, 1.5 Hz, 1H), 8.12 (dd, *J* = 20.9, 8.5 Hz, 2H), 7.94 (d, *J* = 7.5 Hz, 1H), 7.58 (d, *J* = 9.0 Hz, 1H), 7.52 - 7.44 (m, 3H), 7.39 - 7.32 (m, 3H), 7.15 (dd, *J* = 7.3, 1.9 Hz, 2H), 5.16 - 5.06 (m, 3H), 4.76 (dd, *J* = 9.9, 7.6 Hz, 1H), 4.44 - 4.34 (m, 1H), 3.46 (s, 3H).¹³C NMR (100 MHz, CDCl₃) δ 171.28, 162.04, 149.96, 149.25, 146.77, 137.07, 135.05, 131.19, 130.96, 130.87, 130.27, 129.88, 129.19, 128.70, 127.52, 126.58, 125.30, 124.43, 124.15, 123.92, 122.51, 121.61, 78.41, 51.39, 48.52, 37.33. MS (ESI): m/z 428.1 (M+H)⁺.

### RIP1-100

¹H NMR (400 MHz, CDCl₃) δ 8.95 (dd, *J* = 4.2, 1.6 Hz, 1H), 8.19 - 8.13 (m, 1H), 8.11 (d, *J*= 9.1 Hz, 1H), 8.05 (d, *J* = 7.4 Hz, 1H), 7.89 (s, 1H), 7.58 (d, *J* = 9.1 Hz, 1H), 7.49 (dd, *J* = 8.6, 4.2 Hz, 1H), 7.40 - 7.34 (m, 3H), 7.29 - 7.24 (m, 3H), 5.54 (d, *J* = 4.1 Hz, 2H), 5.11 (dt, *J* = 10.9, 7.5 Hz, 1H), 4.78 (dd, *J* = 9.9, 7.6 Hz, 1H), 4.40 (dd, *J* = 10.8, 10.1 Hz, 1H), 3.47 (s, 3H). ¹³C NMR (100 MHz, CDCl₃) δ 170.80, 159.56, 150.04, 149.20, 146.78, 142.75, 133.71, 131.14, 131.00, 129.74, 129.31, 129.14, 128.26, 125.45, 125.24, 124.10, 121.70, 78.13, 54.55, 48.74, 37.40. MS (ESI): m/z 429.1 (M+H)⁺.

### RIP1-101

¹H NMR (400 MHz, CDCl₃) δ 8.96 (dd, *J* = 4.2, 1.6 Hz, 1H), 8.15 (d, *J* = 7.9 Hz, 1H), 8.12 (d, *J* = 9.4 Hz, 1H), 7.75 (d, *J* = 7.1 Hz, 1H), 7.59 (d, *J* = 9.1 Hz, 1H), 7.50 (dd, *J* = 8.6, 4.2 Hz, 1H), 7.36 - 7.27 (m, 3H), 7.25 - 7.20 (m, 2H), 6.28 (s, 1H), 5.07 (dt, *J* = 10.9, 7.4 Hz, 1H), 4.79 (dd, *J* = 9.9, 7.6 Hz, 1H), 4.45 - 4.32 (m, 1H), 4.10 (s, 2H), 3.47 (s, 3H).¹³C NMR (100 MHz, CDCl₃) δ 174.17, 170.51, 158.63, 157.86, 150.10, 149.17, 146.79, 135.16, 131.14, 131.06, 129.57, 128.94, 128.77, 127.40, 125.19, 124.05, 121.75, 101.59, 49.03, 37.41, 33.20, 29.33. MS (ESI): m/z 429.1 (M+H)⁺.

### RIP1-102

¹H NMR (400 MHz, CDCl₃) δ 9.30 (s, 1H), 8.59 (d, *J* = 6.0 Hz, 1H), 8.22 (d, *J* = 7.7 Hz, 1H), 7.94 (d, *J* = 8.4 Hz, 1H), 7.83 (d, *J* = 8.4 Hz, 1H), 7.59 (d, *J* = 5.9 Hz, 1H), 7.25 - 7.18 (m, 5H), 4.77 (dt, *J* = 10.8, 7.5 Hz, 1H), 4.11 (s, 2H), 3.90 (dd, *J* = 11.3, 7.1 Hz, 1H), 3.40 (s, 3H), 3.07 (t, *J* = 11.1 Hz, 1H). ¹³C NMR (101 MHz, CDCl₃) δ 171.58, 153.09, 144.48, 140.90, 135.87, 133.17, 131.66, 131.47, 129.76, 128.89, 128.83, 128.29, 127.17, 116.00, 100.00, 77.25, 49.55, 39.17, 36.59, 33.25. MS (ESI): m/z 445.0 (M+H)⁺.

### RIP1-103

¹H NMR (400 MHz, CDCl₃) δ 9.34 (s, 1H), 8.64 (d, *J* = 5.9 Hz, 1H), 8.18 (d, *J* = 7.5 Hz, 1H), 8.04 (s, 1H), 7.97 (d, *J* = 8.4 Hz, 1H), 7.85 (d, *J* = 8.4 Hz, 1H), 7.60 (d, *J* = 5.9 Hz, 1H), 7.41 - 7.33 (m, 3H), 7.27 (d, *J* = 5.2 Hz, 2H), 5.37 (s, 2H), 4.91 - 4.78 (m, 1H), 4.02 (dd, *J* = 11.3, 7.1 Hz, 1H), 3.43 (s, 3H), 3.06 (t, *J* = 11.1 Hz, 1H).¹³C NMR (100 MHz, CDCl₃) δ 171.60, 158.03, 156.64, 153.19, 144.62, 143.97, 140.85, 133.73, 133.21, 131.64, 131.47, 129.79, 129.21, 129.00, 128.24, 128.23, 115.91, 54.36, 49.55, 39.37, 36.56. MS (ESI): m/z 445.0 (M+H)⁺.

### RIP1-104

¹H NMR (400 MHz, CDCl₃) δ 9.33 (s, 1H), 8.62 (d, *J* = 5.9 Hz, 1H), 8.02 (d, *J* = 7.8 Hz, 1H), 7.94 (d, *J* = 8.4 Hz, 1H), 7.84 (d, *J* = 8.4 Hz, 1H), 7.61 (d, *J* = 5.9 Hz, 1H), 7.46 (d, *J* = 13.1 Hz, 2H), 7.39 - 7.30 (m, 3H), 7.18 - 7.09 (m, 2H), 5.09 (s, 2H), 4.79 (dt, *J* = 10.9, 7.4 Hz, 1H), 3.91 (dd, *J* = 11.2, 7.0 Hz, 1H), 3.41 (s, 3H), 3.09 (t, *J* = 11.1 Hz, 1H). ¹³C NMR (100 MHz, CDCl₃) δ 172.03, 161.63, 153.10, 144.56, 141.18, 137.06, 136.75, 135.05, 133.20, 131.72, 131.53, 129.77, 129.18, 128.69, 128.00, 127.49, 122.53, 116.02, 51.37, 49.12, 39.52, 36.45. MS (ESI): m/z 444.1 (M+H)⁺.

### RIP1-105

¹H NMR (400 MHz, CDCl₃) δ 9.33 (s, 1H), 8.63 (d, *J* = 6.0 Hz, 1H), 8.13 (d, *J* = 7.7 Hz, 1H), 7.95 (d, *J* = 8.4 Hz, 1H), 7.89 - 7.81 (m, 2H), 7.61 (d, *J* = 6.0 Hz, 1H), 7.40 - 7.33 (m, 3H), 7.27 - 7.22 (m, 2H), 5.53 (d, *J* = 1.8 Hz, 2H), 4.79 (dt, *J* = 10.9, 7.3 Hz, 1H), 3.92 (dd, *J* = 11.3, 7.0 Hz, 1H), 3.43 (s, 3H), 3.10 (t, *J* = 11.1 Hz, 1H). ¹³C NMR (100 MHz, CDCl₃) δ 171.53, 159.16, 153.16, 144.66, 142.83, 141.07, 133.73, 133.16, 131.61, 131.50, 129.78, 129.30, 129.13, 128.22, 128.11, 125.42, 115.95, 54.54, 49.29, 39.33, 36.52. MS (ESI): m/z 445.1 (M+H)⁺.

### RIP1-106

¹H NMR (400 MHz, CDCl₃) δ 9.34 (s, 1H), 8.65 (d, *J* = 6.0 Hz, 1H), 7.98 - 7.94 (m, 1H), 7.87 - 7.80 (m, 2H), 7.61 (d, *J* = 6.0 Hz, 1H), 7.35 - 7.27 (m, 3H), 7.24 - 7.19 (m, 2H), 6.26 (s, 1H), 4.75 (dt, *J* = 10.9, 7.3 Hz, 1H), 4.09 (s, 2H), 3.94 (dd, *J* = 11.3, 7.1 Hz, 1H), 3.42 (s, 3H), 3.06 (t, *J* = 11.1 Hz, 1H). ¹³C NMR (100 MHz, CDCl₃) δ 174.13, 171.24, 158.20, 157.94, 153.21, 144.73, 140.90, 135.17, 133.13, 131.53, 131.45, 129.78, 128.93, 128.76, 128.23, 127.40, 115.88, 101.57, 49.56, 39.14, 36.54, 33.18. MS (ESI): m/z 445.0 (M+H)⁺.

### RIP1-107

¹H NMR (400 MHz, CDCl₃) δ 9.25 (s, 1H), 8.56 (d, *J* = 6.0 Hz, 1H), 8.11 (d, *J* = 5.3 Hz, 1H), 7.96 (d, *J* = 8.8 Hz, 1H), 7.58 (d, *J* = 6.0 Hz, 1H), 7.43 (d, *J* = 8.7 Hz, 1H), 7.25 - 7.16 (m, 5H), 5.06 (s, 1H), 4.75 (dd, *J* = 9.7, 7.7 Hz, 1H), 4.40 (t, *J* = 10.5 Hz, 1H), 4.12 (s, 2H), 3.45 (s, 3H). ¹³C NMR (100 MHz, CDCl₃) δ 170.42, 158.63, 152.90, 152.36, 143.92, 135.85, 132.00, 129.55, 129.07, 128.89, 128.83, 127.20, 127.18, 123.58, 115.79, 77.93, 48.81, 37.08, 33.24. MS (ESI): m/z 429.1 (M+H)⁺.

### RIP1-108

¹H NMR (400 MHz, CDCl₃) δ 9.31 (s, 1H), 8.61 (d, *J* = 6.0 Hz, 1H), 8.10 (d, *J* = 7.2 Hz, 1H), 8.03 (s, 1H), 8.00 (d, *J* = 8.7 Hz, 1H), 7.60 (d, *J* = 6.0 Hz, 1H), 7.48 (d, *J* = 8.7 Hz, 1H), 7.43 - 7.34 (m, 3H), 7.32 - 7.27 (m, 2H), 5.38 (s, 2H), 5.14 (dt, *J* = 10.9, 7.4 Hz, 1H), 4.86 (dd, *J* = 9.9, 7.5 Hz, 1H), 4.45 - 4.37 (m, 1H), 3.48 (s, 3H). ¹³C NMR (100 MHz, CDCl₃) δ 170.55, 158.49, 156.55, 153.04, 152.36, 144.16, 143.97, 133.69, 131.99, 129.46, 129.24, 129.06, 129.04, 128.28, 127.25, 123.64, 115.67, 78.14, 54.42, 48.83, 37.05. MS (ESI): m/z 429.1 (M+H)⁺.

### RIP1-109

¹H NMR (400 MHz, CDCl₃) δ 9.22 (s, 1H), 8.52 (d, *J* = 6.0 Hz, 1H), 7.89 (m, 2H), 7.53 (d, *J* = 5.9 Hz, 1H), 7.40 (dd, *J* = 9.4, 6.4 Hz, 3H), 7.28-7.22 (m, *J* = 8.8 Hz, 3H), 7.12 - 7.05 (m, 2H), 5.10 - 4.98 (m, 2H), 4.70 (dd, *J* = 9.8, 7.5 Hz, 1H), 4.34 (t, *J* = 10.5 Hz, 1H), 3.39 (s, 3H). ¹³C NMR (100 MHz, CDCl₃) δ 170.95, 162.06, 152.96, 152.39, 144.04, 137.10, 136.67, 135.04, 132.06, 129.35, 129.25, 129.19, 128.70, 127.52, 127.21, 123.61, 122.56, 115.78, 78.39, 51.39, 48.38, 36.97. MS (ESI): m/z 428.0 (M+H)⁺.

### RIP1-110

¹H NMR (400 MHz, CDCl₃) δ 9.29 (s, 1H), 8.60 (d, *J* = 5.9 Hz, 1H), 8.07 (d, *J* = 7.1 Hz, 1H), 7.98 (d, *J* = 8.7 Hz, 1H), 7.89 (s, 1H), 7.61 (d, *J* = 5.9 Hz, 1H), 7.47 (d, *J* = 8.7 Hz, 1H), 7.42 - 7.33 (m, 3H), 7.29 - 7.22 (m, 2H), 5.65 - 5.46 (m, 2H), 5.10 (dt, *J* = 10.9, 7.4 Hz, 1H), 4.78 (dd, *J* = 9.6, 7.7 Hz, 1H), 4.43 (t, *J* = 10.5 Hz, 1H), 3.48 (s, 3H). ¹³C NMR (100 MHz, CDCl₃) δ 170.47, 159.59, 153.00, 152.33, 144.13, 142.70, 133.71, 132.02, 129.37, 129.31, 129.23, 129.15, 128.25, 127.22, 125.47, 123.55, 115.73, 78.10, 54.56, 48.60, 37.04. MS (ESI): m/z 429.1 (M+H)⁺.

### RIP1-111

¹H NMR (400 MHz, CDCl₃) δ 9.30 (s, 1H), 8.61 (d, *J* = 6.0 Hz, 1H), 7.99 (d, *J* = 8.7 Hz, 1H), 7.77 (d, *J* = 70 Hz, 1H), 7.60 (d, *J* = 60 Hz, 1H), 7.47 (d, *J* = 8.7 Hz, 1H), 7.36 - 7.28 (m, 3H), 7.25 - 7.20 (m, 2H), 6.28 (s, 1H), 5.05 (dt, *J* = 11.0, 7.3 Hz, 1H), 4.80 (dd, *J* = 9.9, 7.4 Hz, 1H), 4.40 (dd, *J* = 10.8, 10.1 Hz, 1H), 4.10 (s, 2H), 3.48 (s, 3H). ¹³C NMR (100 MHz, CDCl₃) δ 174.21, 170.18, 158.65, 157.82, 153.04, 152.30, 144.20, 135.15, 131.97, 129.50, 129.07, 128.95, 128.78, 127.41, 127.23, 123.51, 115.67, 101.57, 77.88, 48.89, 37.05, 33.20. MS (ESI): m/z 429.1 (M+H)⁺.

### RIP1-112

¹H NMR (400 MHz, DMSO) δ 11.10 (s, 1H), 8.29 (s, 1H), 7.43 - 7.01 (m, 9H), 6.27 (s, 1H), 4.79 (d, *J* = 9.3 Hz, 1H), 4.49 - 4.16 (m,2H), 4.05 (s, 2H), 3.44 (s, 3H). MS (ESI): m/z 417.1 (M+H)⁺. MS (ESI): m/z 417.1 (M+H)⁺.

### RIP1-113

¹H NMR (400 MHz, CDCl₃) δ 9.04 (d, *J* = 91.0 Hz, 1H), 8.08 (d, *J* = 17.6 Hz, 4H), 7.44 -7.35 (m, 5H), 6.94 (s, 1H), 6.46 (s, 1H), 5.36 (s, 2H), 4.72 (s, 2H), 4.19 (s, 1H), 3.37 (s, 3H). ¹³C NMR (100 MHz, CDCl₃) δ 168.92, 168.71, 158.53, 156.72, 150.46, 150.11, 146.25, 135.34, 133.81, 129.20, 128.96, 128.28, 125.99, 119.43, 105.15, 103.10, 57.00, 54.33, 49.26, 36.36. MS (ESI): m/z 417.1 (M+H)⁺. MS (ESI): m/z 417.1 (M+H)⁺.

### RIP1-114

¹H NMR (400 MHz, CDCl₃) δ 8.27 (s, 1H), 7.89 (d, *J* = 7.2 Hz, 1H), 7.42 - 7.37 (m, 3H), 7.31 - 7.25 (m, 3H), 7.19 - 7.16 (m, 1H), 7.14 (s, 1H), 7.08 (dd, *J* = 7.3, 1.9 Hz, 2H), 6.47 - 6.44 (m, 1H), 5.02 (s, 2H), 4.99 (dd, *J* = 7.5, 3.6 Hz, 1H), 4.59 (dd, *J* = 9.7, 7.5 Hz, 1H), 4.15 (dd, *J* = 11.0, 9.8 Hz, 1H), 3.43 (s, 3H). ¹³C NMR (100 MHz, CDCl₃) δ 174.49, 168.24, 160.97, 145.31, 135.99, 134.09, 133.06, 129.08, 128.90, 128.14, 127.62, 126.49, 124.60, 124.15, 121.32, 113.94, 103.45, 101.71, 50.32, 47.74, 35.24, 34.91. MS (ESI): m/z 416.1 (M+H)⁺. MS (ESI): m/z 416.1 (M+H)⁺.

### RIP1-115

¹H NMR (400 MHz, CDCl₃) δ 8.44 (s, 1H), 8.15 - 8.01 (m, 2H), 7.96 - 7.82 (m, 2H), 7.45 - 7.31 (m, 5H), 6.96 (d, *J* = 6.5 Hz, 1H), 6.45 (s, 1H), 5.26 (s, 1H), 5.08 (dd, *J* = 16.4, 8.7 Hz, 2H), 4.65 (s, 2H), 3.37 (s, 3H). ¹³C NMR (100 MHz, CDCl₃) δ 168.88, 159.57, 150.15, 146.56, 142.94, 135.17, 133.78, 129.29, 129.10, 128.25, 125.36, 119.51, 118.72, 104.95, 102.90, 99.99, 57.17, 54.51, 49.02, 36.52. MS (ESI): m/z 417.1 (M+H)⁺. MS (ESI): m/z 417.1 (M+H)⁺.

### RIP1-116

¹H NMR (400 MHz, CDCl₃) δ 8.37 (s, 1H), 7.77 (d, *J* = 6.4 Hz, 2H), 7.38 - 7.29 (m, 5H), 6.96 (d, *J* = 6.4 Hz, 1H), 6.46 (d, *J* = 4.3 Hz, 1H), 6.31 (d, *J* = 5.0 Hz, 1H), 6.28 (d, *J* = 3.0 Hz, 1H), 5.13 - 4.97 (m, 2H), 4.31 (t, *J* = 6.7 Hz, 1H), 4.09 (s, 2H), 3.38 (s, 3H). ¹³C NMR (100 MHz, CDCl₃) δ 174.04, 168.59, 158.61, 150.13, 146.45, 135.23, 130.95, 128.93, 128.79, 127.38, 126.20, 122.97, 119.48, 118.68, 114.00, 104.97, 101.55, 65.61, 57.20, 49.22, 33.19. MS (ESI): m/z 417.1 (M+H)⁺. MS (ESI): m/z 417.1 (M+H)⁺.

### RIP1-119

¹H NMR (400 MHz, CDCl₃) δ 8.08 (d, *J* = 7.2 Hz, 1H), 8.03 (s, 1H), 7.68 (d, *J* = 2.2 Hz, 1H), 7.43 (s, 1H), 7.40-7.34 (m, 4H), 7.31-7.27 (m, 2H), 6.77 (dd, *J* = 2.2, 0.8 Hz, 1H), 5.37 (s, 2H), 5.10 (dt, *J* = 11.1, 7.4 Hz, 1H), 4.77 (dd, *J* = 9.8, 7.5 Hz, 1H), 4.25 (dd, 7 = 11.0, 9.9 Hz, 1H), 3.49 (s, 3H); ESI-MS: m/z 418.0 (M+H)⁺.

### RIP1-120

¹H NMR (400 MHz, CDCl₃) δ 7.93 (d, *J* = 7.3 Hz, 1H), 7.66 (d, *J* = 1.7 Hz, 1H), 7.47 (d, *J* = 4.2 Hz, 2H), 7.41 (s, 1H), 7.38-7.28 (m, 4H), 7.14 (d, *J* = 5.8 Hz, 2H), 6.75 (s, 1H), 5.09 (s, 2H), 5.07-4.98 (m, 1H), 4.71-4.63 (m, 1H), 4.25 (t, *J* = 10.5 Hz, 1H), 3.47 (s, 3H); ESI-MS: m/z 417.0 (M+H)⁺.

### RIP1-121

¹H NMR (400 MHz, CDCl₃) δ 8.04 (d, *J* = 7.2 Hz, 1H), 7.89 (s, 1H), 7.67 (d, *J* = 2.2 Hz, 1H), 7.43 (s, 1H), 7.41-7.33 (m, 4H), 7.28-7.24 (m, 2H), 6.77 (dd, *J* = 2.1, 0.8 Hz, 1H), 5.54 (d, *J* = 3.3 Hz, 2H), 5.06 (dt, *J* = 11.1, 7.4 Hz, 1H), 4.69 (dd, *J* = 9.7, 7.5 Hz, 1H), 4.27 (dd, *J* = 11.1, 9.9 Hz, 1H), 3.49 (s, 3H); ESI-MS: m/z 418.1 (M+H)⁺.

### RIP1-122

¹H NMR (400 MHz, CDCl₃) δ 8.07 (d, *J* = 6.9 Hz, 1H), 7.69 (d, *J* = 2.2 Hz, 1H), 7.44 (s, 1H), 7.42-7.34 (m, 6H), 6.77 (dd, *J* = 2.2, 0.9 Hz, 1H), 5.81 (s, 2H), 5.09 (dt, *J* = 11.0, 7.3 Hz, 1H), 4.78 (dd, *J* = 9.8, 7.5 Hz, 1H), 4.26 (dd, *J* = 11.0, 9.9 Hz, 1H), 3.50 (s, 3H); ESI-MS: m/z 419.1 (M+H)⁺.

### RIP1-123

¹H NMR (400 MHz, CDCl₃) δ 8.00 (d, *J* = 6.8 Hz, 1H), 7.69 (d, *J* = 2.2 Hz, 1H), 7.44 (s, 1H), 7.39-7.29 (m, 6H), 6.77 (dd, *J* = 2.2, 0.9 Hz, 1H), 5.05 (dt, *J* = 11.0, 7.3 Hz, 1H), 4.77 (dd, *J* = 9.8, 7.5 Hz, 1H), 4.28 (s, 2H), 4.24 (dd, *J* = 11.0, 9.9 Hz, 1H), 3.50 (s, 3H); ¹³C NMR (100 MHz, CDCl₃) δ 207.1, 179.5, 168.2, 163.1, 155.7, 153.0, 147.6, 146.8, 132.6, 132.1, 129.1, 129.0, 128.0, 125.1, 115.3, 106.4, 106.0, 49.4, 36.4, 33.0, 31.0; ESI-MS: m/z 419.1 (M+H)⁺.

### RIP1-124

¹H NMR (400 MHz, CDCl₃) δ 7.74 (d, *J* = 7.2 Hz, 1H), 7.67 (d, *J* = 2.2 Hz, 1H), 7.43 (s, 1H), 7.36 (d, *J* = 0.5 Hz, 1H), 7.10 (d, *J* = 7.8 Hz, 2H), 6.99 (d, *J* = 8.0 Hz, 2H), 6.76 (dd, *J* = 2.2, 0.9 Hz, 1H), 6.48 (s, 1H), 5.06 (dt, *J* = 11.1, 7.4 Hz, 1H), 4.72 (dd, *J* = 9.7, 7.5 Hz, 1H), 4.24 (dd, *J* = 11.1, 9.8 Hz, 1H), 3.92 (s, 2H), 3.71 (s, 3H), 3.48 (s, 3H), 2.31 (s, 3H); ESI-MS: m/z 445.1 (M+H)⁺.

### RIP1-125

¹H NMR (400 MHz, CDCl₃) δ 8.04 (s, 1H), 7.69 (d, *J* = 2.2 Hz, 1H), 7.45 (s, 1H), 7.40-7.36 (m, 1H), 7.35-7.28 (m, 5H), 6.78 (d, *J* = 1.3 Hz, 1H), 4.99 (t, *J* = 8.9 Hz, 1H), 4.73 (dd, *J* = 9.8, 7.5 Hz, 1H), 4.31-4.21 (m, 3H), 3.49 (s, 3H); ESI-MS: m/z 419.1 (M+H)⁺.

### RIP1-126

¹H NMR (400 MHz, CDCl₃) δ 7.76 (d, *J* = 6.9 Hz, 1H), 7.68 (d, *J* = 2.2 Hz, 1H), 7.43 (s, 1H), 7.38-7.27 (m, 4H), 7.23 (d, *J* = 6.9 Hz, 2H), 6.77 (d, *J* = 1.4 Hz, 1H), 6.29 (s, 1H), 5.01 (dt, *J* = 11.1, 7.2 Hz, 1H), 4.71 (dd, *J* = 9.7, 7.5 Hz, 1H), 4.24 (dd, *J* = 10.9, 10.0 Hz, 1H), 4.10 (s, 2H), 3.48 (s, 3H); ESI-MS: m/z 419.0 (M+H)⁺.

### RIP1-127

¹H NMR (400 MHz, CDCl₃) δ 8.17 (d, *J* = 7.2 Hz, 1H), 7.73 (d, *J* = 2.2 Hz, 1H), 7.48 (d, *J* = 9.5 Hz, 1H), 7.39 (s, 1H), 7.31 (s, 1H), 7.29-7.21 (m, 4H), 6.82 (dd, *J* = 2.1, 0.8 Hz, 1H), 5.10 (dt, *J* = 10.9, 7.3 Hz, 1H), 4.71 (dd, *J* = 9.7, 7.6 Hz, 1H), 4.35-4.25 (m, 1H), 4.17 (s, 2H), 3.51 (s, 3H); ESI-MS: m/z 418.0 (M+H)⁺.

### RIP1-128

¹H NMR (400 MHz, CDCl₃) δ 8.87 (d, *J* = 1.8 Hz, 1H), 8.84 (d, *J* = 1.8 Hz, 1H), 8.08 (d, *J* = 7.1 Hz, 1H), 8.03 (s, 1H), 7.95 (s, 1H), 7.92 (s, 1H), 7.42 - 7.36 (m, 3H), 7.31-7.27 (m, 2H), 5.39 (s, 2H), 5.18 (dt, *J* = 11.3, 7.3 Hz, 1H), 4.84 (dd, *J* = 9.8, 7.4 Hz, 1H), 4.35 (dd, *J* = 11.2, 9.9 Hz, 1H), 3.61 (s, 3H); ¹³C NMR (100 MHz, CDCl₃) δ 168.5, 158.5, 156.5, 151.4, 145.4, 145.2, 144.0, 142.0, 141.0, 140.0, 133.7, 129.3, 129.1, 128.3, 122.6, 121.6, 76.4, 54.4, 49.1, 36.3; ESI-MS: m/z 430.0 (M+H)⁺.

### RIP1-129

¹H NMR (400 MHz, DMSO) δ 8.94 (dd, *J* = 5.1, 1.8 Hz, 2H), 8.19 (s, 1H), 8.13 (d, *J* = 7.9 Hz, 1H), 7.98 (d, *J* = 1.1 Hz, 1H), 7.92 (s, 2H), 7.89 (s, 1H), 7.76 (s, 1H), 7.40-7.27 (m, 3H), 5.26 (s, 2H), 4.98-4.87 (m, 1H), 4.66-4.56 (m, 1H), 4.51 (dd, *J* = 9.8, 7.6 Hz, 1H), 3.72 (s,3H); ¹³C NMR (100 MHz, DMSO) δ 169.3, 163.1, 162.0, 151.6, 146.2, 146.2, 141.4, 140.8, 140.7, 139.4, 138.3, 137.6, 137.5, 136.1, 132.8, 129.3, 129.2, 128.5, 128.4, 128.2, 128.2, 126.8, 123.4, 122.7, 120.8, 76.0, 51.5, 50.3, 36.0; ESI-MS: m/z 429.0 (M+H)⁺.

### RIP1-130

¹H NMR (400 MHz, CDCl₃) δ 8.84 (d, *J* = 5.5 Hz, 2H), 8.05 (d, *J* = 6.9 Hz, 1H), 8.00-7.85 (m, 3H), 7.43-7.23 (m, 5H), 5.54 (s, 2H), 5.14 (dt, *J* = 11.2, 7.2 Hz, 1H), 4.82-4.70 (m, 1H), 4.38 (t, *J* = 10.6 Hz, 1H), 3.60 (s, 3H); ¹³C NMR (100 MHz, CDCl₃) δ 168.4, 159.6, 151.5, 145.4, 145.1, 142.7, 141.9, 141.0, 140.2, 133.7, 129.3, 129.2, 128.3, 125.5, 122.5, 121.4, 76.4, 54.6, 48.9, 36.3; ESI-MS: m/z 430.0 (M+H)⁺.

### RIP1-131

¹H NMR (400 MHz, CDCl₃) δ 8.88 (d, *J* = 7.4 Hz, 2H), 8.11 (d, *J* = 6.6 Hz, 1H), 7.94 (d, *J* = 16.3 Hz, 2H), 7.43-7.34 (m, 5H), 5.81 (s, 2H), 5.18 (dt, *J* = 11.3, 7.2 Hz, 1H), 4.88-4.79 (m, 1H), 4.37 (t, *J* = 10.6 Hz, 1H), 3.61 (s, 3H); ¹³C NMR (100 MHz, CDCl₃) δ 168.1, 159.3, 156.1, 151.3, 145.5, 145.2, 142.0, 141.0, 139.8, 132.3, 129.4, 129.2, 128.7, 122.7, 121.6, 76.1, 57.6, 49.3, 36.3; ESI-MS: m/z 431.0 (M+H)⁺.

### RIP1-132

¹H NMR (400 MHz, CDCl₃) δ 8.85 (d, *J* = 7.3 Hz, 2H), 8.00 (d, *J* = 6.9 Hz, 1H), 7.93 (d, *J* = 16.7 Hz, 2H), 7.39-7.28 (m, 5H), 5.12 (dt, *J* = 11.3, 7.2 Hz, 1H), 4.82 (dd, *J* = 9.8, 7.4 Hz, 1H), 4.34 (dd, *J* = 11.1, 10.1 Hz, 1H), 4.28 (s, 2H), 3.60 (s, 3H); ¹³C NMR (100 MHz, CDCl₃) δ 179.7, 167.9, 162.9, 155.8, 151.3, 145.6, 145.3, 142.0, 141.0, 139.7, 132.6, 129.1, 129.0, 128.0, 122.8, 121.6, 76.0, 49.4, 36.3, 33.0; ESI-MS: m/z 431.1 (M+H)⁺.

### RIP1-133

¹H NMR (400 MHz, CDCl₃) δ 8.83 (d, *J* = 6.8 Hz, 2H), 7.91 (d, *J* = 16.9 Hz, 2H), 7.74 (d, *J* = 7.1 Hz, 1H), 7.09 (d, *J* = 7.7 Hz, 2H), 6.98 (d, *J* = 7.8 Hz, 2H), 6.47 (s, 1H), 5.13 (dt, *J* = 11.3, 7.2 Hz, 1H), 4.77 (dd, *J* = 9.4, 7.5 Hz, 1H), 4.33 (t, *J* = 10.6 Hz, 1H), 3.91 (s, 2H), 3.71 (s, 3H), 3.59 (s, 3H), 2.30 (s, 3H); ¹³C NMR (100 MHz, CDCl₃) δ 169.0, 161.7, 151.6, 145.3, 145.1, 144.0, 143.4, 141.9, 141.0, 140.3, 136.6, 133.5, 129.5, 128.3, 122.5, 121.4, 106.9, 80.1, 65.6, 48.8, 37.1, 36.2, 31.5, 21.0; ESI-MS: m/z 457.1 (M+H)⁺.

### RIP1-134

¹H NMR (400 MHz, CDCl₃) δ 8.86 (d, *J* = 6.4 Hz, 2H), 8.06 (d, *J* = 6.7 Hz, 1H), 7.94 (d, *J* = 16.9 Hz, 2H), 7.39-7.27 (m, 5H), 5.07 (dt, *J* = 11.3, 7.1 Hz, 1H), 4.83-4.74 (m, 1H), 4.37 (t, *J* = 10.6 Hz, 1H), 4.26 (s, 2H), 3.61 (s, 3H); ¹³C NMR (100 MHz, CDCl₃) δ 167.8, 167.5, 158.4, 152.7, 151.2, 145.6, 145.3, 142.0, 141.0, 139.7, 132.8, 129.1, 128.9, 127.9, 122.8, 121.6, 75.8, 49.5, 36.4, 31.9; ESI-MS: m/z 431.1 (M+H)⁺.

### RIP1-135

¹H NMR (400 MHz, CDCl₃) δ 8.82 (d, *J* = 6.8 Hz, 2H), 8.13 (d, *J* = 7.3 Hz, 1H), 7.93 (s, 1H), 7.87 (s, 1H), 7.19 (d, *J* = 13.4 Hz, 5H), 5.09 (dt, *J* = 11.3, 7.4 Hz, 1H), 4.75-4.66 (m, 1H), 4.40-4.30 (m, 1H), 4.11 (s, 2H), 3.56 (s, 3H); ¹³C NMR (100 MHz, CDCl₃) δ 168.4, 162.7, 158.7, 151.4, 145.4, 145.2, 141.9, 140.9, 140.0, 135.8, 128.8, 128.8, 127.1, 122.6, 121.4, 76.2, 49.1, 36.3, 33.1; ESI-MS: m/z 430.1 (M+H)⁺.

### RIP1-136

¹H NMR (400 MHz, CDCl₃) δ 8.21 (s, 1H), 8.18 (d, *J* = 6.2 Hz, 1H), 7.81 (s, 1H), 7.61 (s, 1H), 7.41-7.35 (m, 3H), 7.34-7.28 (m, 2H), 5.42 (s, 2H), 5.10-4.96 (m, 1H), 4.86 (s, 1H), 4.32 (t, *J* = 10.4 Hz, 1H), 3.52 (s, 3H); ¹³C NMR (100 MHz, CDCl₃) δ 168.3, 158.8, 156.3, 144.5, 133.4, 129.3, 128.4, 76.4, 54.6, 49.6, 36.5; ESI-MS: m/z 419.0 (M+H)⁺.

### RIP1-137

¹H NMR (400 MHz, CDCl₃) δ 8.05 (d, *J* = 7.5 Hz, 1H), 7.65 (s, 1H), 7.58-7.47 (m, 3H), 7.40-7.31 (m, 3H), 7.20-7.13 (m, 2H), 5.12 (s, 2H), 5.07-4.97 (m, 1H), 4.73 (dd, *J* = 9.8, 7.5 Hz, 1H), 4.35-4.26 (m, 1H), 3.50 (s, 3H); ESI-MS: m/z 418.1 (M+H)⁺.

### RIP1-138

¹H NMR (400 MHz, CDCl₃) δ 8.15 (d, *J* = 7.1 Hz, 1H), 7.96 (s, 1H), 7.72 (s, 1H), 7.57 (s, 1H), 7.43-7.32 (m, 3H), 7.31-7.27 (m, 2H), 5.55 (s, 2H), 5.05 (dt, *J* = 14.7, 7.3 Hz, 1H), 4.76-4.67 (m, 1H), 4.31 (t, *J* = 10.5 Hz, 1H), 3.51 (s, 3H); ESI-MS: m/z 419.1 (M+H)⁺.

### RIP1-139

¹H NMR (400 MHz, CDCl₃) δ 8.19 (d, *J* = 6.6 Hz, 1H), 7.82 (s, 1H), 7.63 (s, 1H), 7.45-7.32 (m, 5H), 5.83 (s, 2H), 5.08 (dt, *J* = 11.1, 7.1 Hz, 1H), 4.86-4.75 (m, 1H), 4.30 (t, *J=* 10.5 Hz, 1H), 3.53 (s, 3H); ESI-MS: m/z 420.0 (M+H)⁺.

### RIP1-140

¹H NMR (400 MHz, CDCl₃) δ 8.08 (s, 1H), 7.81 (s, 1H), 7.61 (s, 1H), 7.41-7.28 (m, 5H), 5.03 (s, 1H), 4.85-4.67 (m, 1H), 4.31 (s, 2H), 4.30-4.25 (m, 1H), 3.54 (s, 3H); ESI-MS: m/z 420.0 (M+H)⁺.

### RIP1-141

¹H NMR (400 MHz, CDCl₃) δ 7.89 (d, *J* = 7.1 Hz, 1H), 7.75-7.45 (d, *J* = 47.5 Hz, 2H), 7.10 (d, *J* = 7.9 Hz, 2H), 7.00 (d, *J* = 7.9 Hz, 2H), 6.52 (s, 1H), 5.01 (dt, *J* = 14.3, 7.2 Hz, 1H), 4.80 (dd, *J* = 9.7, 7.4 Hz, 1H), 4.34-4.24 (m, 1H), 3.94 (s, 2H), 3.75 (s, 3H), 3.50 (s, 3H), 2.31 (s, 3H); ESI-MS: m/z 446.1 (M+H)⁺.

### RIP1-142

¹H NMR (400 MHz, CDCl₃) δ 7.84 (d, *J* = 7.0 Hz, 1H), 7.77 (s, 1H), 7.60 (s, 1H), 7.36-7.27 (m, 3H), 7.25-7.19 (m, 2H), 6.31 (s, 1H), 5.02 (dt, *J* = 11.2, 7.2 Hz, 1H), 4.73 (dd, *J* = 9.7, 7.5 Hz, 1H), 4.34-4.23 (m, 1H), 4.11 (s, 2H), 3.53 (s, 3H); ESI-MS: m/z 419.0 (M+H)⁺.

### RIP1-143

¹H NMR (400 MHz, DMSO) δ 8.50 (s, 1H), 8.13-7.56 (m, 2H), 7.37-7.10 (m, 6H), 4.86 (d, *J* = 9.8 Hz, 1H), 4.63-4.29 (m, 2H), 4.13 (s, 2H), 3.39 (s, 3H); ESI-MS: m/z 419.0 (M+H)⁺.

### RIP1-145

26.9 mg, yield 73%. ¹H NMR (400 MHz, DMSO) δ 8.90 (s, 1H), 8.59 (brs, 1H), 8.44 (s, 1H), 8.18 (s, 1H), 7.52 (s, 1H), 7.48 - 7.25 (m, 5H), 5.54 (s, 2H), 5.05 - 4.84 (m, 1H), 4.80 - 4.64 (m, 1H), 4.63 - 4.44 (m, 1H), 3.56 (s, 3H), 3.45 (s, 3H). ¹³C NMR (100 MHz, CDCl₃) δ 168.48, 160.64, 158.47, 156.53, 154.79, 147.83, 147.60, 143.99, 136.34, 133.73, 129.22, 129.01, 128.27, 121.13, 120.88, 119.66, 77.27, 54.39, 48.96, 36.14, 34.24. ESI-MS *m*/*z* 460.1 (M+H)⁺.

### RIP1-146

27.6 mg yield 75%. ¹H NMR (400 MHz, CDCl₃) δ 8.12 - 8.00 (m, 2H), 7.97 (s, 1H), 7.39 (s, 1H), 7.17 - 7.03 (m, 5H), 5.03 - 4.85 (m, 1H), 4.68 - 4.52 (m, 1H), 4.32 - 4.20 (m, 1H), 4.04 (s, 2H), 3.52 (s, 3H), 3.41 (s, 3H). ¹³C NMR (100 MHz, CDCl₃) δ 168.34, 160.62, 158.75, 154.74, 147.71, 147.67, 136.33, 135.91, 128.83, 128.73, 127.05, 120.91, 120.87, 119.61, 76.51, 48.97, 36.14, 34.30, 33.04. ESI-MS *m*/*z* 460.1 (M+H)⁺.

### RIP1-147

25.3 mg yield 69%. ¹H NMR (400 MHz, CDCl₃) δ 8.13 (s, 1H), 8.05 (s, 1H), 7.93 (brs, *J* = 7.2 Hz, 1H), 7.51 - 7.44 (m, 3H), 7.39 - 7.32 (m, 3H), 7.18 - 7.10 (m, 2H), 5.10 (s, 2H), 5.09 - 5.01 (m, 1H), 4.76 - 4.68 (m, 1H), 4.39 - 4.30 (m, 1H), 3.61 (s, 3H), 3.51 (s, 3H). ¹³C NMR (100 MHz, CDCl₃) δ 168.88, 162.02, 160.69, 154.95, 147.69, 147.44, 137.08, 136.72, 136.68, 135.06, 129.19, 128.70, 127.53, 122.53, 120.97, 120.74, 119.56, 51.38, 48.52, 36.03, 34.23. ESI-MS *m*/*z* 459.1 (M+H)⁺.

### RIP1-148

ESI-MS: m/z 429.1 (M+H)⁺.

### RIP1-149

¹H NMR (400 MHz, CDCl₃+CD₃OD) δ 8.76 (dd, *J* = 4.2, 1.6 Hz, 1H), 8.08 (dd, *J* = 8.2, 1.6 Hz, 1H), 7.46 (d, *J* = 9.1 Hz, 1H), 7.36 (dd, *J* = 8.2, 4.3 Hz, 1H), 7.29-7.18 (m, 6H), 4.98 (dd, *J* = 8.5, 2.4 Hz, 1H), 4.70 (dd, *J* = 11.2, 2.4 Hz, 1H), 4.30 (dd, *J* = 11.2, 8.7 Hz, 1H), 4.09 (s, 2H); ESI-MS: m/z 415.1 (M+H)⁺.

### RIP1-150

ESI-MS: m/z 430.0 (M+H)⁺.

### RIP1-153

¹H NMR (400 MHz, DMSO) δ 8.12 (s, 1H), 7.75 (s, 1H), 7.42-7.23 (m, 5H), 4.90-4.80 (m, 1H), 4.65 (t, *J* = 10.7 Hz, 1H), 4.48-4.40 (m, 1H), 4.37 (s, 2H), 3.42 (s, 3H); ¹³C NMR (100 MHz, DMSO) δ 168.3, 167.9, 158.8, 153.5, 148.6, 136.0, 134.4, 129.4, 129.3, 127.8, 110.3, 107.4, 75.1, 49.7, 36.4, 31.2; ESI-MS: m/z 420.0 (M+H)⁺.

### RIP1-156

13.1 mg yield 33%.¹H NMR (400 MHz, DMSO) δ 8.40 (s, 1H), 8.13 (s, 1H), 7.48 (s, 1H), 7.45 - 7.31 (m, 1H), 7.17 - 7.03 (m, 2H), 4.88 (dt, *J* = 11.6, 7.7 Hz, 1H), 4.70 (t, *J* = 10.7 Hz, 1H), 4.48 (dd, *J* = 9.7, 7.6 Hz, 1H), 4.14 (s, 2H), 3.50 (s, 3H), 3.40 (s, 3H). ESI-MS *m*/*z* 496.0 (M+H)⁺.

### RIP1-157

11.0 mg' yield 27%. ¹H NMR (400 MHz, DMSO) δ 8.57 (s, 1H), 8.40 (s, 1H), 8.13 (s, 1H), 7.48 (s, 1H), 7.23 (t, *J* = 8.5 Hz, 2H), 4.89 (dt, *J* = 11.4, 7.6 Hz, 1H), 4.70 (t, *J* = 10.7 Hz, 1H), 4.49 (dd, *J* = 9.5, 7.7 Hz, 1H), 4.11 (s, 2H), 3.51 (s, 3H), 3.40 (s, 3H). ESI-MS *m*/*z* 514.0(M+H)⁺.

### RIP1-158

5.4 mg' yield 21%. ¹H NMR (400 MHz, DMSO) δ 8.46 (s, 1H), 8.20 (s, 1H), 7.54 (s, 1H), 7.37 (dd, *J* = 8.2, 5.7 Hz, 2H), 7.22 (t, *J* = 8.7 Hz, 2H), 4.96 (dt, *J* = 11.5, 7.7 Hz, 1H), 4.76 (t, *J* = 10.7 Hz, 1H), 4.56 (dd, *J* = 9.5, 7.7 Hz, 1H), 4.18 (s, 2H), 3.57 (s, 3H), 3.46 (s, 3H). ¹³C NMR (100 MHz, DMSO) δ 168.75, 160.43, 154.61, 149.50, 147.72, 136.72, 131.04, 130.95, 120.69, 120.44, 119.66, 115.89, 115.68, 75.91, 49.05, 35.86, 34.13. ESI-MS *m*/*z* 478.1 (M+H)⁺.

### RIP1-159

6.4 mg' yield 25%. ¹H NMR (400 MHz, DMSO) δ 8.64 (s, 1H), 8.46 (s, 1H), 8.19 (s, 1H), 7.54 (s, 1H), 7.43 (dd, *J* = 14.3, 7.6 Hz, 1H), 7.23 - 7.09 (m, 3H), 4.97 (dt, *J* = 11.4, 7.7 Hz, 1H), 4.76 (t, *J* = 10.7 Hz, 1H), 4.56 (dd, *J* = 9.5, 7.8 Hz, 1H), 4.22 (s, 2H), 3.57 (s, 3H), 3.47 (s, 3H). ¹³C NMR (100 MHz, DMSO) δ 168.75, 163.85, 161.43, 160.42, 154.61, 149.49, 147.72, 136.71, 131.01, 130.92, 130.12, 125.28, 125.25, 120.68, 120.43, 119.65, 116.08, 115.86, 114.13, 113.93, 99.99, 75.91, 49.06, 35.86, 34.13. ESI-MS *m*/*z* 478.1 (M+H)⁺.

### RIP1-160

8.0 mg' yield 21%. ¹H NMR (400 MHz, DMSO) δ 8.56 (s, 1H), 8.40 (s, 1H), 8.13 (s, 1H), 7.48 (s, 1H), 7.38 - 7.27 (m, 2H), 7.24 - 7.13 (m, 2H), 4.90 (dt, *J* = 11.5, 7.7 Hz, 1H), 4.70 (t, *J* = 10.7 Hz, 1H), 4.49 (dd, *J* = 9.7, 7.6 Hz, 1H), 4.15 (s, 2H), 3.51 (s, 3H), 3.40 (s, 3H). ¹³C NMR (100 MHz, DMSO) δ 168.74, 161.97, 160.42, 159.54, 154.60, 149.48, 147.71, 136.71, 131.75, 131.71, 129.58, 129.50, 125.06, 125.02, 120.67, 120.43, 119.65, 115.92, 115.70, 75.90, 49.05, 35.85, 34.13. ESI-MS *m*/*z* 478.0 (M+H)⁺.

### RIP1-161

¹H NMR (400 MHz, CDCl₃) δ 9.29 (s, 1H), 8.59 (d, *J* = 6.0 Hz, 1H), 8.08 (d, *J* = 5.8 Hz, 1H), 7.98 (d, *J* = 8.7 Hz, 1H), 7.60 (d, *J* = 6.0 Hz, 1H), 7.46 (d, *J* = 8.8 Hz, 1H), 7.22 (td, *J* = 8.3, 4.1 Hz, 1H), 6.95-6.82 (m, 2H), 5.13-5.01 (m, 1H), 4.78 (dd, *J* = 9.8, 7.5 Hz, 1H), 4.48-4.37 (m, 1H), 4.21 (s, 2H), 3.46 (s, 3H); ESI-MS: m/z 465.0 (M+H)⁺.

### RIP1-162

¹H NMR (400 MHz, CDCl₃) δ 9.31 (s, 1H), 8.61 (d, *J* = 6.0 Hz, 1H), 7.99 (d, *J* = 8.8 Hz, 1H), 7.60 (d, *J* = 6.0 Hz, 1H), 7.47 (d, *J* = 8.8 Hz, 1H), 6.73-6.64 (m, 2H), 5.06 (dd, *J* = 10.9, 7.5 Hz, 1H), 4.78 (dd, *J* = 9.9, 7.5 Hz, 1H), 4.46-4.37 (m, 1H), 4.15 (s, 2H), 3.47 (s, 3H); ESI-MS: m/z 483.0 (M+H)⁺.

### RIP1-163

¹H NMR (400 MHz, CDCl₃) δ 9.29 (s, 1H), 8.59 (d, *J* = 6.0 Hz, 1H), 8.10 (s, 1H), 7.99 (d, *J* = 8.8 Hz, 1H), 7.60 (d, *J* = 6.0 Hz, 1H), 7.47 (d, *J* = 8.7 Hz, 1H), 7.29-7.18 (m, 2H), 7.06 (t, *J* = 7.3 Hz, 1H), 7.00 (t, *J* = 9.1 Hz, 1H), 5.14-5.02 (m, 1H), 4.79 (dd, *J* = 9.9, 7.5 Hz, 1H), 4.46-4.37 (m, 1H), 4.19 (s, 2H), 3.47 (s, 3H); ESI-MS: m/z 447.0 (M+H)⁺.

### RIP1-164

¹H NMR (400 MHz, CDCl₃) δ 9.30 (s, 1H), 8.59 (d, *J* = 6.0 Hz, 1H), 8.11 (d, *J* = 7.3 Hz, 1H), 7.99 (d, *J* = 8.7 Hz, 1H), 7.60 (d, *J* = 6.0 Hz, 1H), 7.46 (d, *J* = 8.8 Hz, 1H), 7.22 (dd, *J* = 8.3, 5.4 Hz, 2H), 6.94 (t, *J* = 8.6 Hz, 2H), 5.06 (dt, *J* = 11.0, 7.4 Hz, 1H), 4.77 (dd, *J* = 9.9, 7.5 Hz, 1H), 4.45-4.38 (m, 1H), 4.11 (s, 2H), 3.46 (s, 3H); ESI-MS: m/z 447.0 (M+H)⁺.

### RIP1-165

¹H NMR (400 MHz, CDCl₃) δ 9.30 (s, 1H), 8.60 (d, *J* = 6.0 Hz, 1H), 8.12 (s, 1H), 7.99 (d, *J* = 8.8 Hz, 1H), 7.60 (d, *J* = 6.0 Hz, 1H), 7.47 (d, *J* = 8.7 Hz, 1H), 7.25-7.21 (m, 1H), 7.05 (d, *J* = 7.5 Hz, 1H), 7.01-6.89 (m, 2H), 5.13-5.01 (m, 1H), 4.79 (dd, *J* = 9.8, 7.6 Hz, 1H), 4.43 (t, *J* = 10.5 Hz, 1H), 4.15 (s, 2H), 3.48 (s, 3H); ESI-MS: m/z 447.0 (M+H)⁺.

### RIP1-166

¹H NMR (400 MHz, DMSO) δ 8.53 (s, 1H), 8.10 (s, 1H), 7.75 (s, 1H), 7.46-7.32 (m, 1H), 7.19-7.02 (m, 2H), 4.85 (dt, *J* = 11.1, 7.6 Hz, 1H), 4.65-4.57 (m, 1H), 4.41 (dd, *J* = 9.8, 7.8 Hz, 1H), 4.14 (s, 2H), 3.42 (s, 3H); ESI-MS: m/z 455.0 (M+H)⁺.

### RIP1-167

¹H NMR (400 MHz, DMSO) δ 8.53 (d, *J* = 6.4 Hz, 1H), 8.10 (d, *J* = 13.8 Hz, 1H), 7.74 (s, 1H), 7.35-7.27 (dd, *J* = 8.6, 5.6 Hz, 3H), 7.21-7.10 (m, 3H), 4.86 (dd, *J* = 10.8, 6.8 Hz, 1H), 4.64-4.54 (m, 1H), 4.42 (dd, *J* = 9.8, 7.8 Hz, 1H), 4.30 (dd, *J* = 14.2, 7.1 Hz, 1H), 4.12 (s, 2H), 3.42 (s, 3H); ESI-MS: m/z 437.0 (M+H)⁺.

### RIP1-168

¹H NMR (400 MHz, CDCl₃) δ 8.99-8.91 (m, 1H), 8.28-7.99 (m, 3H), 7.57 (d, *J* = 9.0 Hz, 1H), 7.50 (dd, *J* = 8.6, 4.2 Hz, 1H), 7.21 (td, *J* = 8.2, 4.1 Hz, 1H), 6.88 (t, *J* = 7.7 Hz, 2H), 5.15-5.03 (m, 1H), 4.76 (dd, *J* = 9.8, 7.8 Hz, 1H), 4.40 (t, *J* = 10.4 Hz, 1H), 4.20 (s, 2H), 3.45 (s, 3H); ESI-MS: m/z 465.0 (M+H)⁺.

### RIP1-169

¹H NMR (400 MHz, CDCl₃) δ 8.95 (dd, *J* = 4.2, 1.5 Hz, 1H), 8.22-8.02 (m, 3H), 7.57 (d, *J* = 9.0 Hz, 1H), 7.50 (dd, *J* = 8.5, 4.2 Hz, 1H), 6.65 (t, *J* = 8.0 Hz, 2H), 5.07 (dd, *J* = 10.8, 7.7 Hz, 1H), 4.76 (dd, *J* = 9.9, 7.6 Hz, 1H), 4.39 (t, *J* = 10.4 Hz, 1H), 4.13 (s, 2H), 3.46 (s, 3H); ESI-MS: m/z 483.0 (M+H)⁺.

### RIP1-170

¹H NMR (400 MHz, CDCl₃) δ 8.93 (dd, *J* = 4.2, 1.5 Hz, 1H), 8.26-8.00 (m, 3H), 7.57 (d, *J* = 9.1 Hz, 1H), 7.49 (dd, *J* = 8.6, 4.2 Hz, 1H), 7.25-7.12 (m, 2H), 7.04-6.98 (m, 1H), 6.97-6.91 (m 1H), 5.17-5.04 (m, 1H), 4.76-4.70 (m, 1H), 4.39 (t, *J* = 10.5 Hz, 1H), 4.15 (s, 2H), 3.45 (s, 3H); ESI-MS: m/z 447.0 (M+H)⁺.

### RIP1-171

¹H NMR (400 MHz, CDCl₃) δ 8.95 (dd, *J* = 4.2, 1.6 Hz, 1H), 8.13 (t, *J* = 9.2 Hz, 3H), 7.58 (d, *J* = 9.0 Hz, 1H), 7.50 (dd, *J* = 8.5, 4.3 Hz, 1H), 7.24-7.18 (m, 2H), 6.98-6.91 (m, 2H), 5.09 (dd, *J* = 10.6, 7.7 Hz, 1H), 4.77 (dd, *J* = 9.9, 7.6 Hz, 1H), 4.43-4.36 (m, 1H), 4.11 (s, 2H), 3.46 (s, 3H); ESI-MS: m/z 447.0 (M+H)⁺.

### RIP1-172

¹H NMR (400 MHz, CDCl₃) δ 8.95 (dd, *J* = 4.2, 1.6 Hz, 1H), 8.13 (t, *J* = 9.7 Hz, 3H), 7.58 (d, *J* = 9.0 Hz, 1H), 7.50 (dd, *J* = 8.5, 4.2 Hz, 1H), 7.22 (td, *J* = 7.9, 6.1 Hz, 1H), 7.04-7.00 (m, 1H), 6.98-6.87 (m, 2H), 5.09 (dd, *J* = 10.8, 7.7 Hz, 1H), 4.77 (dd, *J* = 9.9, 7.6 Hz, 1H), 4.43-4.36 (m, 1H), 4.13 (s, 2H), 3.46 (s, 3H); ESI-MS: m/z 447.0 (M+H)⁺.

### RIP1-173

¹H NMR (400 MHz, CDCl₃) δ 8.07 (s, 1H), 7.69 (d, *J* = 2.2 Hz, 1H), 7.44 (s, 1H), 7.38 (d, *J* = 9.0 Hz, 1H), 6.77 (dd, *J* = 2.2, 0.8 Hz, 1H), 6.74 - 6.65 (m, 2H), 5.07 - 4.97 (m, 1H), 4.70 (dd, *J* = 9.8, 7.5 Hz, 1H), 4.27 (dd, *J* = 11.0, 9.9 Hz, 1H), 4.14 (s, 2H), 3.49 (s, 3H); ESI-MS: m/z 470.1 (M-H)⁻.

### RIP1-174

¹H NMR (400 MHz, CDCl₃) δ 8.11 (s, 1H), 7.69 (d, *J* = 2.2 Hz, 1H), 7.44 (s, 1H), 7.36 (s, 1H), 7.22 (dd, *J* = 8.4, 5.4 Hz, 2H), 6.94 (t, *J* = 8.7 Hz, 2H), 6.80-6.75 (m, 1H), 5.04 (dd, *J* = 10.7, 7.8 Hz, 1H), 4.68 (dd, *J* = 9.7, 7.6 Hz, 1H), 4.31-4.22 (m, 1H), 4.10 (s, 2H), 3.48 (s, 3H); ESI-MS: m/z 436.0 (M+H)⁺.

### RIP1-175

15.1 mg' yield 40%. ¹H NMR (400 MHz, CDCl₃) δ 8.83 (d, *J* = 6.8 Hz, 1H), 8.37 (d, *J* = 5.4 Hz, 1H), 8.08 (s, 1H), 7.99 (s, 1H), 7.53 (s, 1H), 7.43 (s, 1H), 7.34 (t, *J* = 7.5 Hz, 2H), 7.17 (d, *J* = 7.1 Hz, 1H), 7.00 (d, *J* = 7.7 Hz, 2H), 6.88 (d, *J* = 3.1 Hz, 1H), 5.05 - 4.91 (m, 1H), 4.73 - 4.65 (m, 1H), 4.30 (t, *J* = 10.6 Hz, 1H), 3.55 (s, 3H), 3.46 (s, 3H). ¹³C NMR (100 MHz, CDCl₃) δ 168.69, 166.13, 163.72, 160.66, 154.87, 153.72, 151.23, 150.15, 147.76, 147.53, 136.56, 130.35, 125.76, 120.95, 120.79, 119.62, 114.53, 110.66, 76.79, 49.21, 36.05, 34.24. ESI-MS *m*/*z* 472.0(M+H)⁺.

### RIP1-176

ESI-MS: m/z 496.0 (M+H)⁺.

### RIP1-177

¹H NMR (400 MHz, CDCl₃) δ 8.84 (d, *J* = 7.2 Hz, 1H), 8.80 - 8.70 (m, 2H), 8.38 (d, *J* = 5.6 Hz, 1H), 7.87 (s, 1H), 7.83 (s, 1H), 7.52 (d, *J* = 2.4 Hz, 1H), 7.34 (t, *J* = 7.9 Hz, 2H), 7.16 (d, *J* = 7.4 Hz, 1H), 6.99 (d, *J* = 7.8 Hz, 2H), 6.88 (dd, *J* = 5.6, 2.5 Hz, 1H), 5.06 (dt, *J* = 11.4, 7.3 Hz, 1H), 4.71 (dd, *J* = 9.7, 7.4 Hz, 1H), 4.37 - 4.25 (m, 1H), 3.54 (s, 3H). ¹³C NMR (100 MHz, CDCl₃) δ 167.68, 165.10, 162.70, 152.68, 150.50, 150.17, 149.11, 144.33, 144.08, 140.90, 139.98, 139.17, 129.31, 124.72, 121.43, 120.34, 119.74, 113.51, 109.64, 75.37, 48.31, 35.13. ESI-MS *m*/*z* 442.1 (M+H)⁺.

### RIP1-178

¹H NMR (400 MHz, CDCl₃) δ 9.22 (s, 1H), 8.84 (d, *J* = 7.3 Hz, 1H), 8.52 (d, *J* = 6.0 Hz, 1H), 8.38 (d, *J* = 5.6 Hz, 1H), 7.91 (d, *J* = 8.7 Hz, 1H), 7.53 (d, *J* = 6.0 Hz, 1H), 7.50 (d, *J* = 2.5 Hz, 1H), 7.40 (d, *J* = 8.7 Hz, 1H), 7.33 (t, *J* = 7.9 Hz, 2H), 7.16 (t, *J* = 7.4 Hz, 1H), 6.98 (d, *J* = 7.7 Hz, 2H), 6.88 (dd, *J* = 5.6, 2.5 Hz, 1H), 5.01 (dt, *J* = 11.0, 7.4 Hz, 1H), 4.73 (dd, *J* = 9.8, 7.5 Hz, 1H), 4.40 - 4.32 (m, 1H), 3.41 (s, 3H). ¹³C NMR (100 MHz, CDCl₃) δ 170.73, 166.11, 163.76, 153.70, 153.01, 152.34, 151.23, 150.16, 144.12, 132.01, 130.34, 129.38, 129.28, 127.21, 125.76, 123.52, 120.77, 115.73, 114.50, 110.64, 78.13, 49.03, 36.98. ESI-MS *m*/*z* 441.0 (M+H)⁺.

### RIP1-179

¹H NMR (400 MHz, CDCl₃) δ 8.87 (d, *J* = 2.9 Hz, 1H), 8.82 (d, *J* = 7.4 Hz, 1H), 8.38 (d, *J* = 5.6 Hz, 1H), 8.06 (dd, *J* = 17.3, 8.8 Hz, 2H), 7.51 (d, *J* = 8.8 Hz, 2H), 7.42 (dd, *J* = 8.5, 4.2 Hz, 1H), 7.33 (t, *J* = 7.8 Hz, 2H), 7.16 (t, *J* = 7.4 Hz, 1H), 6.98 (d, *J* = 7.8 Hz, 2H), 6.88 (dd, *J* = 5.5, 2.4 Hz, 1H), 5.03 (dt, *J* = 10.8, 7.6 Hz, 1H), 4.72 (dd, *J* = 9.7, 7.7 Hz, 1H), 4.34 (t, *J* = 10.4 Hz, 1H), 3.40 (s, 3H). ¹³C NMR (100 MHz, CDCl₃) δ 170.03, 165.07, 162.70, 152.66, 150.24, 149.10, 148.99, 148.16, 145.72, 130.09, 129.96, 129.30, 128.75, 124.71, 124.16, 123.05, 120.65, 119.73, 113.46, 109.59, 77.11, 48.13, 36.30. ESI-MS *m*/*z* 441.1(M+H)⁺.

### RIP1-180

¹H NMR (400 MHz, CDCl₃) δ 8.89 (d, *J* = 7.4 Hz, 1H), 8.45 (d, *J* = 5.6 Hz, 1H), 7.68 (d, *J* = 2.2 Hz, 1H), 7.60 (d, *J* = 2.5 Hz, 1H), 7.46-7.35 (m, 4H), 7.25-7.21 (m, 1H), 7.10-7.04 (m, 2H), 6.94 (dd, *J* = 5.6, 2.5 Hz, 1H), 6.77 (dd, *J* = 2.2, 0.8 Hz, 1H), 5.05 (dt, *J* = 11.1, 7.4 Hz, 1H), 4.72 (dd, *J* = 9.7, 7.5 Hz, 1H), 4.28 (dd, *J* = 11.1, 9.8 Hz, 1H), 3.50 (s, 3H); ESI-MS: m/z 430.0 (M+H)⁺.

### RIP1-181

¹H NMR (400 MHz, DMSO) δ 8.96-8.76 (m, 1H), 8.52 (d, *J* = 5.4 Hz, 1H), 7.99 (s, 1H), 7.66 (s, 1H), 7.44 (t, *J* = 7.7 Hz, 2H), 7.30-7.20 (m, 2H), 7.15 (d, *J* = 7.9 Hz, 2H), 4.87-4.69 (m, 1H), 4.54-4.24 (m, 2H); ESI-MS: m/z 431.0 (M+H)⁺.

### RIP1-182

¹H NMR (400 MHz, CDCl₃) δ 8.08 (s, 1H), 8.05 (brs, *J* = 7.4 Hz, 1H), 7.51 (s, 1H), 7.38 (s, 1H), 7.16 - 7.01 (m, 5H), 4.97 (dd, *J* = 17.4, 7.3 Hz, 1H), 4.62 - 4.52 (m, 1H), 4.24 - 4.15 (m, 1H), 4.01 (s, 2H), 3.39 (s, 3H). ESI-MS *m*/*z* 419.1 (M+H)⁺.

### RIP1-183

¹H NMR (400 MHz, CDCl₃) δ 8.16 (s, 1H), 8.08 (brs, *J* = 6.7 Hz, 1H), 7.62 (s, 1H), 7.49 (s, 1H), 7.22 (dd, *J* = 14.7, 7.4 Hz, 1H), 6.89 (t, *J* = 7.6 Hz, 2H), 5.05 (dd, *J* = 18.0, 7.5 Hz, 1H), 4.73 - 4.65 (m, 1H), 4.33 - 4.25 (m, 1H), 4.20 (s, 2H), 3.49 (s, 3H). ESI-MS *m*/*z* 455.1 (M+H)⁺.

### RIP1-184

¹H NMR (400 MHz, CDCl₃) δ 8.17 (s, 1H), 8.11 (brs, *J* = 7.2 Hz, 1H), 7.61 (s, 1H), 7.49 (s, 1H), 6.63 (t, *J* = 7.8 Hz, 2H), 5.10 - 4.97 (m, 1H), 4.71 - 4.60 (m, 1H), 4.36 - 4.25 (m, 1H), 4.11 (s, 2H), 3.48 (s, 3H). ESI-MS *m*/*z* 473.1 (M+H)⁺.

### RIP1-185

¹H NMR (400 MHz, CDCl₃) δ 8.09 (s, 1H), 8.06 (brs, *J* = 6.1 Hz, 1H), 7.53 (s, 1H), 7.41 (s, 1H), 7.13 - 7.00 (m, 2H), 6.86 - 6.73 (m, 2H), 5.02 - 4.88 (m, 1H), 4.65 - 4.50 (m, 1H), 4.28 - 4.14 (m, 1H), 3.99 (s, 2H), 3.40 (s, 3H). ESI-MS *m*/*z* 437.1 (M+H)⁺.

### RIP1-186

¹H NMR (400 MHz, CDCl₃) δ 8.87 (d, *J* = 7.4 Hz, 1H), 8.44 (d, *J* = 5.6 Hz, 1H), 8.16 (s, 1H), 7.63 (s, 1H), 7.60 (d, *J* = 2.4 Hz, 1H), 7.49 (s, 1H), 7.41 (t, *J* = 7.9 Hz, 2H), 7.25 (t, *J* = 7.5 Hz, 1H), 7.07 (d, *J* = 7.8 Hz, 2H), 6.95 (dd, *J* = 5.6, 2.5 Hz, 1H), 5.06 (dt, *J* = 11.2, 7.5 Hz, 1H), 4.73 (dd, *J* = 9.7, 7.6 Hz, 1H), 4.36 - 4.25 (m, 1H), 3.51 (s, 3H). ¹³C NMR (100 MHz, CDCl₃) δ 169.10, 166.13, 163.72, 154.11, 153.71, 151.29, 150.13, 147.81, 147.17, 138.69, 135.07, 130.35, 125.76, 120.79, 114.50, 114.15, 110.66, 105.77, 77.46, 49.32, 36.15. ESI-MS *m*/*z* 431.1 (M+H)⁺.

### RIP1-187

ESI-MS *m*/*z* 446.1 (M+H)⁺.

### RIP1-188

ESI-MS *m*/*z* 458.1 (M+H)⁺.

### RIP1-189

¹H NMR (500 MHz, CDCl₃) δ 8.92 (d, *J* = 7.2 Hz, 1H), 8.44 (d, *J* = 5.6 Hz, 1H), 8.32 (s, 1H), 7.59 (d, *J* = 2.5 Hz, 1H), 7.46 (s, 1H), 7.40 (t, *J* = 7.9 Hz, 2H), 7.26-7.23 (m, 2H), 7.22 (s, 1H), 7.06 (d, *J* = 7.6 Hz, 2H), 6.94 (dd, *J* = 5.6, 2.5 Hz, 1H), 6.54 (s, 1H), 5.09-5.03 (m, 1H), 4.71 (dd, *J* = 9.6, 7.5 Hz, 1H), 4.24 (dd, *J* = 11.0, 9.8 Hz, 1H), 3.51 (s, 3H); ESI-MS: 429.45 [M+H]⁺.

### RIP1-190

¹H NMR (500 MHz, CDCl₃) δ 8.92 (d, *J* = 7.2 Hz, 1H), 8.45 (d, *J* = 5.6 Hz, 1H), 7.60 (s, 2H), 7.41 (t, *J* = 7.9 Hz, 2H), 7.31 (s, 1H), 7.26-7.22 (m, 1H), 7.06 (d, *J* = 7.9 Hz, 2H), 6.95 (dd, *J* = 5.3, 2.2 Hz, 1H), 5.10-5.03 (m, 1H), 4.69 (dd, *J* = 9.6, 7.5 Hz, 1H), 4.32-4.26 (m, 1H), 3.52 (s, 3H) ; ESI-MS: 430.40 [M+H]⁺.

### RIP1-193

¹H NMR (400 MHz, CDCl₃) δ 8.95 (dd, *J* = 4.2, 1.5 Hz, 1H), 8.72 (d, *J* = 1.3 Hz, 1H), 8.48 (d, *J* = 2.6 Hz, 1H), 8.13 (dd, *J* = 13.3, 8.7 Hz, 2H), 7.65-7.62 (m, 1H), 7.59 (d, *J* = 9.0 Hz, 1H), 7.53-7.44 (m, 2H), 7.40-7.33 (m, 2H), 7.17 (t, *J* = 7.4 Hz, 1H), 7.01 (d, *J* = 7.7 Hz, 2H), 5.11 (dt, *J* = 10.8, 7.3 Hz, 1H), 4.82 (dd, *J* = 9.9, 7.6 Hz, 1H), 4.43-4.35 (m, 1H), 3.47 (s, 3H); ESI-MS: m/z 441.1 (M+H)⁺.

### RIP1-194

¹H NMR (400 MHz, CDCl₃) δ 8.94 (d, *J* = 2.9 Hz, 1H), 8.37 (d, *J* = 7.5 Hz, 1H), 8.16-8.05 (m, 2H), 7.83-7.73 (m, 2H), 7.61-7.45 (m, 4H), 7.32 (t, *J* = 7.4 Hz, 1H), 7.26-7.22 (m, 2H), 7.03 (dd, *J* = 7.7, 1.3 Hz, 1H), 5.05 (dt, *J* = 11.0, 7.6 Hz, 1H), 4.72 (dd, *J* = 9.8, 7.6 Hz, 1H), 4.29 (dd, *J* = 10.9, 10.0 Hz, 1H), 3.44 (s, 3H); ESI-MS: m/z 441.0 (M+H)⁺.

### RIP1-195

¹H NMR (400 MHz, CDCl₃) δ 9.00-8.94 (m, 1H), 8.28 (d, *J* = 5.2 Hz, 1H), 8.15 (t, *J* = 8.8 Hz, 2H), 7.61 (d, *J* = 9.0 Hz, 1H), 7.52 (dd, *J* = 8.6, 4.2 Hz, 1H), 7.46-7.34 (m, 3H), 7.28 (dd, *J* = 5.2, 1.3 Hz, 1H), 7.26-7.20 (t, *J* = 7.4 Hz, 2H), 7.13 (d, *J* = 7.6 Hz, 2H), 5.10 (dt, *J* = 10.8, 7.2 Hz, 1H), 4.88 (dd, *J* = 9.9, 7.6 Hz, 1H), 4.42-4.33 (m, 1H), 3.50 (s, 3H); ¹³C NMR (100 MHz, CDCl₃) δ 171.0, 164.6, 164.5, 153.6, 150.2, 149.2, 148.7, 146.8, 144.2, 131.3, 131.0, 129.8, 129.4, 125.2, 124.0, 121.8, 121.3, 115.6, 109.6, 78.1, 49.6, 37.5; ESI-MS: m/z 441.0 (M+H)⁺.

### RIP1-196

¹H NMR (400 MHz, CDCl₃) δ 8.95 (dd, *J* = 4.1, 1.5 Hz, 1H), 8.74 (d, *J* = 7.5 Hz, 1H), 8.35 (d, *J* = 2.5 Hz, 1H), 8.19-8.08 (m, 2H), 8.00 (d, *J* = 8.6 Hz, 1H), 7.59 (d, *J* = 9.1 Hz, 1H), 7.50 (dd, *J* = 8.6, 4.2 Hz, 1H), 7.44-7.37 (m, 2H), 7.28 (dd, *J* = 8.6, 2.8 Hz, 1H), 7.25-7.19 (m, 1H), 7.09-7.03 (m, 2H), 5.13 (dt, *J* = 11.0, 7.6 Hz, 1H), 4.80 (dd, *J* = 9.9, 7.6 Hz, 1H), 4.41 (dd, *J* = 10.9, 10.0 Hz, 1H), 3.48 (s, 3H); ESI-MS: m/z 441.0 (M+H)⁺.

### RIP1-197

¹H NMR (400 MHz, CDCl₃) δ 8.96 (d, *J* = 3.0 Hz, 1H), 8.89 (d, *J* = 7.4 Hz, 1H), 8.47 (d, *J* = 5.6 Hz, 1H), 8.14 (dd, *J* = 15.2, 8.8 Hz, 2H), 7.59 (d, *J* = 9.1 Hz, 1H), 7.54 (d, *J* = 2.5 Hz, 1H), 7.50 (dd, *J* = 8.6, 4.2 Hz, 1H), 7.14-7.07 (m, 2H), 7.07-7.00 (m, 2H), 6.94 (dd, *J* = 5.6, 2.5 Hz, 1H), 5.10 (dt, *J* = 10.9, 7.5 Hz, 1H), 4.79 (dd, *J* = 9.8, 7.6 Hz, 1H), 4.47-4.37 (m, 1H), 3.48 (s, 3H); ¹³C NMR (100 MHz, CDCl₃) δ 171.1, 166.2, 163.7, 158.9, 151.4, 150.2, 150.1, 149.5, 149.2, 146.8, 131.1, 131.0, 129.8, 125.2, 124.1, 122.4, 122.3, 121.7, 117.2, 116.9, 114.3, 110.3, 78.2, 49.2, 37.4; ESI-MS: m/z 459.1 (M+H)⁺.

### RIP1-198

¹H NMR (400 MHz, CDCl₃) δ 8.96 (d, *J* = 2.8 Hz, 1H), 8.90 (d, *J* = 7.5 Hz, 1H), 8.51 (d, *J* = 5.6 Hz, 1H), 8.14 (dd, *J* = 17.1, 8.8 Hz, 2H), 7.59 (d, *J* = 9.1 Hz, 1H), 7.54 (d, *J* = 2.5 Hz, 1H), 7.50 (dd, *J* = 8.6, 4.2 Hz, 1H), 7.24-7.18 (ddd, *J* = 14.4, 7.0, 4.4 Hz, 1H), 7.07-6.99 (m, 3H), 5.10 (dt, *J* = 11.0, 7.5 Hz, 1H), 4.79 (dd, *J* = 9.9, 7.6 Hz, 1H), 4.41 (dd, *J* = 10.8, 10.0 Hz, 1H), 3.48 (s, 3H); ESI-MS: m/z 477.0 (M+H)⁺.

### RIP1-199

¹H NMR (400 MHz, CDCl₃) δ 8.96 (dd, *J* = 4.1, 1.4 Hz, 1H), 8.72 (d, *J* = 4.8 Hz, 1H), 8.60 (d, *J* = 7.4 Hz, 1H), 8.18-8.09 (m, 2H), 7.69 (d, *J* = 4.8 Hz, 1H), 7.59 (d, *J* = 9.0 Hz, 1H), 7.54 - 7.48 (m, 3H), 7.37-7.32 (m, 1H), 7.28-7.24 (m, 2H), 5.08 (dt, *J* = 10.9, 7.5 Hz, 1H), 4.77 (dd, *J* = 9.9, 7.6 Hz, 1H), 4.37 (dd, *J* = 10.8, 10.0 Hz, 1H), 3.47 (s, 3H); ESI-MS: m/z 442.0 (M+H)⁺.

### RIP1-200

¹H NMR (400 MHz, CDCl₃) δ 8.96 (d, *J* = 2.9 Hz, 1H), 8.91 (d, *J* = 7.5 Hz, 1H), 8.27 (d, *J* = 5.6 Hz, 1H), 8.14 (dd, *J* = 19.8, 8.8 Hz, 2H), 7.59 (d, *J* = 9.1 Hz, 1H), 7.54 (d, *J* = 2.0 Hz, 1H), 7.50 (dd, *J* = 8.6, 4.2 Hz, 1H), 7.35 (t, *J* = 7.8 Hz, 2H), 7.21-7.11 (m, 3H), 6.92 (dd, *J* = 5.6, 2.3 Hz, 1H), 6.24 (s, 1H), 5.12 (dt, *J* = 11.0, 7.6 Hz, 1H), 4.82-4.75 (m, 1H), 4.46-4.38 (m, 1H), 3.48 (s, 3H); ESI-MS: m/z 440.0 (M+H)⁺.

### RIP1-201

¹H NMR (400 MHz, CDCl₃) δ 8.96 (dd, *J* = 4.1, 1.4 Hz, 1H), 8.79 (d, *J* = 7.4 Hz, 1H), 8.68 (t, *J* = 1.4 Hz, 1H), 8.20-8.09 (m, 2H), 7.95 (d, *J* = 1.5 Hz, 2H), 7.60 (d, *J* = 9.0 Hz, 1H), 7.51 (dd, *J* = 8.6, 4.2 Hz, 1H), 7.42-7.30 (m, 2H), 7.18-7.10 (m, 3H), 5.12 (dt, *J* = 10.9, 7.5 Hz, 1H), 4.87-4.76 (m, 1H), 4.41 (dd, *J* = 10.8, 10.0 Hz, 1H), 3.49 (s, 3H); ESI-MS: m/z 440.0 (M+H)⁺.

### RIP1-202

¹H NMR (400 MHz, CDCl₃) δ 8.97 (dd, *J* = 4.2, 1.5 Hz, 1H), 8.81 (d, *J* = 7.1 Hz, 1H), 8.61 (d, *J* = 4.8 Hz, 1H), 8.19 - 8.10 (m, 2H), 7.68 (d, *J* = 7.7 Hz, 2H), 7.61 (d, *J* = 9.0 Hz, 1H), 7.51 (dd, *J* = 8.6, 4.2 Hz, 1H), 7.46 (t, *J* = 7.9 Hz, 2H), 7.37 (d, *J* = 4.8 Hz, 1H), 7.34 (s, 1H), 7.14 (t, *J* = 7.4 Hz, 1H), 5.08 (dt, *J* = 10.9, 7.4 Hz, 1H), 4.84 (dd, *J* = 9.9, 7.6 Hz, 1H), 4.45-4.34 (m, 1H), 3.51 (s, 3H); ESI-MS: m/z 441.0 (M+H)⁺.

### RIP1-203

¹H NMR (400 MHz, CDCl₃) δ 8.95 (dd, *J* = 4.2, 1.6 Hz, 1H), 8.90 (d, *J* = 7.5 Hz, 1H), 8.48 (d, *J* = 5.6 Hz, 1H), 8.19-8.14 (m, 1H), 8.12 (d, *J* = 9.1 Hz, 1H), 7.59 (d, *J* = 9.1 Hz, 1H), 7.55 (d, *J* = 2.5 Hz, 1H), 7.50 (dd, *J* = 8.6, 4.2 Hz, 1H), 7.26-7.13 (m, 4H), 6.97 (dd, *J* = 5.6, 2.5 Hz, 1H), 5.10 (dt, *J* = 11.0, 7.5 Hz, 1H), 4.79 (dd, *J* = 9.9, 7.6 Hz, 1H), 4.41 (dd, *J* = 10.9, 10.0 Hz, 1H), 3.48 (s, 3H); ¹³C NMR (100 MHz, CDCl₃) δ 171.1, 165.6, 163.6, 151.4, 150.2, 150.1, 149.2, 146.8, 131.1, 131.0, 129.8, 127.3, 127.2, 125.3, 125.27, 124.1, 123.5, 121.7, 117.7, 117.5, 113.8, 109.7, 78.2, 49.2, 37.4; ESI-MS: m/z 459.0 (M+H)⁺.

### RIP1-204

¹H NMR (400 MHz, CDCl₃) δ 8.96 (dd, *J* = 4.2, 1.6 Hz, 1H), 8.90 (d, *J* = 7.5 Hz, 1H), 8.50 (d, *J* = 5.6 Hz, 1H), 8.18-8.14 (m, 1H), 8.12 (d, *J* = 9.1 Hz, 1H), 7.60 (dd, *J* = 5.7, 3.3 Hz, 2H), 7.50 (dd, *J* = 8.6, 4.2 Hz, 1H), 7.37 (td, *J* = 8.3, 6.6 Hz, 1H), 7.01-6.93 (ddd, *J* = 10.0, 6.9, 2.1 Hz, 2H), 6.86 (dd, *J* = 8.2, 2.1 Hz, 1H), 6.80 (dt, *J* = 9.4, 2.3 Hz, 1H), 5.11 (dt, *J* = 11.0, 7.6 Hz, 1H), 4.80 (dd, *J* = 9.9, 7.6 Hz, 1H), 4.42 (dd, *J* = 10.8, 10.0 Hz, 1H), 3.48 (s, 3H); ESI-MS: m/z 459.0 (M+H)⁺.

### RIP1-205

¹H NMR (400 MHz, CDCl₃) δ 8.96 (dd, *J* = 4.2, 1.6 Hz, 1H), 8.91 (d, *J* = 7.5 Hz, 1H), 8.54 (d, *J* = 5.6 Hz, 1H), 8.19 - 8.15 (m, 1H), 8.12 (d, *J* = 9.1 Hz, 1H), 7.61 (dd, *J* = 9.0, 5.8 Hz, 2H), 7.50 (dd, *J* = 8.6, 4.2 Hz, 1H), 7.04 (dd, *J* = 5.6, 2.5 Hz, 1H), 6.71 (tt, *J* = 8.8, 2.3 Hz, 1H), 6.65 - 6.57 (m, 2H), 5.11 (dt, *J* = 10.9, 7.5 Hz, 1H), 4.81 (dd, *J* = 9.9, 7.6 Hz, 1H), 4.42 (dd, *J* = 10.8, 10.0 Hz, 1H), 3.49 (s, 3H); ESI-MS: m/z 477.0 (M+H)⁺.

### RIP1-206

¹H NMR (400 MHz, CDCl₃) δ 8.16 (s, 1H), 8.07 (s, 1H), 7.63 (s, 1H), 7.49 (s, 1H), 7.32-7.21 (m, 2H), 7.12-7.01 (m, 2H), 5.1 -5.02 (m, 1H), 4.71 (dd, *J* = 9.8, 7.7 Hz, 1H), 4.33-4.25 (m, 1H), 4.19 (s, 2H), 3.50 (s, 3H); ESI-MS: m/z 437.0 (M+H)⁺.

### RIP1-207

¹H NMR (400 MHz, CDCl₃) δ 8.78 (d, *J* = 5.5 Hz, 2H), 8.04 (s, 1H), 7.88 (s, 1H), 7.83 (s, 1H), 7.35-7.22 (m, 1H), 7.15 (s, 1H), 6.94-6.84 (m, 2H), 5.10-4.97 (m, 1H), 4.75-4.64 (m, 1H), 4.33-4.26 (m, 1H), 4.04 (s, 2H), 3.52 (s, 3H); ESI-MS: m/z 448.0 (M+H)⁺.

### RIP1-208

¹H NMR (400 MHz, CDCl₃) δ 8.81 - 8.72 (m, 2H), 7.88 (s, 1H), 7.82 (s, 1H), 7.17 - 7.06 (m, 2H), 6.92 (dt, *J* = 18.1, 8.1 Hz, 2H), 5.09 - 4.97 (m, 1H), 4.72 - 4.58 (m, 1H), 4.33 - 4.22 (m, 1H), 4.09 (s, 2H), 3.51 (s, 3H). ESI-MS *m*/*z* 448.1 (M+H)⁺.

### RIP1-209

¹H NMR (400 MHz, CDCl₃) δ 8.91 - 8.78 (m, 2H), 7.95 (s, 1H), 7.89 (s, 1H), 7.19 (dd, *J* = 13.9, 7.5 Hz, 1H), 7.06 - 6.79 (m, 3H), 5.17 - 5.01 (m, 1H), 4.78 - 4.67 (m, 1H), 4.43 - 4.30 (m, 1H), 4.12 (s, 2H), 3.59 (s, 3H). ESI-MS *m*/*z* 448.1 (M+H)⁺.

### RIP1-210

¹H NMR (400 MHz, CDCl₃) δ 8.96 (dd, *J* = 4.2, 1.5 Hz, 1H), 8.91 (d, *J* = 7.5 Hz, 1H), 8.52 (t, *J* = 3.9 Hz, 2H), 8.46 (d, *J* = 2.4 Hz, 1H), 8.20-8.07 (m, 2H), 7.64-7.56 (m, 2H), 7.54-7.47 (m, 1H), 7.46-7.34 (m, 2H), 7.00 (dd, *J* = 5.6, 2.6 Hz, 1H), 5.11 (dt, *J* = 10.9, 7.5 Hz, 1H), 4.80 (dd, *J* = 9.9, 7.6 Hz, 1H), 4.42 (dd, *J* = 10.8, 10.1 Hz, 1H), 3.48 (s, 3H); ¹³C NMR (100 MHz, CDCl₃) δ 171.0, 165.4, 163.4, 151.6, 150.5, 150.5, 150.1, 149.2, 146.9, 146.8, 143.1, 131.1, 131.0, 129.7, 128.1, 125.2, 124.6, 124.1, 121.7, 114.6, 110.5, 78.1, 49.2, 37.4; ESI-MS: m/z 442.0 (M+H)⁺.

### RIP1-211

¹H NMR (400 MHz, CDCl₃) δ 8.11 (s, 1H), 7.90 (d, *J* = 8.0 Hz, 2H), 7.82 (brs, *J* = 6.9 Hz, 1H), 7.60 (s, 1H), 7.44 (s, 1H), 7.37 (t, *J* = 7.9 Hz, 2H), 7.22 (d, *J* = 7.5 Hz, 1H), 4.99 - 4.88 (m, 1H), 4.74 - 4.65 (m, 1H), 4.29 - 4.18 (m, 1H), 3.46 (s, 3H). ESI-MS *m*/*z* 421.0 (M+H)⁺.

### RIP1-212

¹H NMR (400 MHz, CDCl₃) δ 8.88 (brs, *J* = 7.4 Hz, 1H), 8.65 - 8.47 (m, 3H), 8.17 (s, 1H), 7.64 (s, 1H), 7.62 (d, *J* = 2.4 Hz, 1H), 7.61 - 7.52 (m, 2H), 7.50 (s, 1H), 7.04 (dd, *J* = 5.5, 2.5 Hz, 1H), 5.06 (dt, *J* = 11.1, 7.5 Hz, 1H), 4.74 (dd, *J* = 9.8, 7.6 Hz, 1H), 4.36 - 4.27 (m, 1H), 3.52 (s, 3H). ESI-MS *m*/*z* 432.0 (M+H)⁺.

### RIP1-213

¹H NMR (400 MHz, CDCl₃) δ 8.77 (d, *J* = 7.3 Hz, 1H), 8.74 (s, 1H), 8.10 (s, 1H), 7.58 (s, 1H), 7.49 (s, 1H), 7.43 (s, 1H), 7.38 (t, *J* = 7.9 Hz, 2H), 7.23 (t, *J* = 7.5 Hz, 1H), 7.07 (d, *J* = 7.9 Hz, 2H), 4.99 (dt, *J* = 11.1, 7.5 Hz, 1H), 4.69 (dd, *J* = 9.7, 7.7 Hz, 1H), 4.29 - 4.18 (m, 1H), 3.45 (s, 3H). ESI-MS *m*/*z* 432.0 (M+H)⁺.

### RIP1-214

¹H NMR (400 MHz, CDCl₃) δ 8.91 (d, *J* = 7.2 Hz, 1H), 8.88 - 8.82 (m, 2H), 8.57 - 8.43 (m, 3H), 7.95 (s, 1H), 7.91 (s, 1H), 7.61 (d, *J* = 2.4 Hz, 1H), 7.48 - 7.33 (m, 2H), 7.01 (dd, *J* = 5.5, 2.5 Hz, 1H), 5.13 (dt, *J* = 11.4, 7.3 Hz, 1H), 4.79 (dd, *J* = 9.7, 7.4 Hz, 1H), 4.39 (dd, *J* = 11.2, 10.0 Hz, 1H), 3.62 (s, 3H). ESI-MS *m*/*z* 443.0(M+H)⁺.

### RIP1-215

¹H NMR (400 MHz, CDCl₃) δ 8.81 (s, 1H), 8.81 - 8.76 (m, 2H), 8.75 (s, 1H), 7.89 (s, 1H), 7.86 (s, 1H), 7.49 (s, 1H), 7.38 (t, *J* = 7.8 Hz, 2H), 7.23 (t, *J* = 7.5 Hz, 1H), 7.07 (d, *J* = 7.9 Hz, 2H), 5.06 (dt, *J* = 11.4, 7.2 Hz, 1H), 4.74 (dd, *J* = 9.6, 7.4 Hz, 1H), 4.39 - 4.23 (m, 1H), 3.55 (s, 3H). ESI-MS *m*/*z* 443.0(M+H)⁺.

### RIP1-217

¹H NMR (400 MHz, CDCl₃) δ 8.16 (s, 1H), 8.14 (s, 1H), 7.66 (s, 1H), 7.43 (s, 1H), 7.18 (dd, *J* = 14.0, 7.6 Hz, 1H), 7.03 - 6.83 (m, 3H), 5.02 (dd, *J* = 18.1, 7.4 Hz, 1H), 4.74 - 4.62 (m, 1H), 4.36 - 4.24 (m, 1H), 4.12 (s, 2H), 3.49 (s, 3H). ESI-MS *m*/*z* 437. 1(M+H)⁺.

### RIP1-218

¹H NMR (400 MHz, CDCl₃) δ 8.09 (s, 1H), 8.04 (d, *J* = 6.9 Hz, 1H), 7.59 (s, 1H), 7.38 (s, 1H), 7.28 - 7.19 (m, 5H), 5.03 - 4.92 (m, 1H), 4.70 - 4.60 (m, 1H), 4.26 - 4.18 (m, 1H), 4.09 (s, 2H), 3.42 (s, 3H). ESI-MS *m*/*z* 419.0(M+H)⁺.

### RIP1-219

¹H NMR (400 MHz, CDCl₃) δ 8.09 (s, 1H), 8.04 (d, *J* = 7.1 Hz, 1H), 7.59 (s, 1H), 7.37 (s, 1H), 7.18 - 7.10 (m, 2H), 7.03 - 6.86 (m, 2H), 4.97 (dt, *J* = 11.0, 7.5 Hz, 1H), 4.70 - 4.57 (m, 1H), 4.28 - 4.17 (m, 1H), 4.10 (s, 2H), 3.42 (s, 3H). ESI-MS *m*/*z* 437.0(M+H)⁺.

### RIP1-220

¹H NMR (400 MHz, CDCl₃) δ 8.12 - 8.04 (m, 2H), 7.56 (s, 1H), 7.42 (s, 1H), 7.29 - 7.13 (m, 5H), 4.92 (dt, *J* = 11.0, 7.4 Hz, 1H), 4.65 - 4.55 (m, 1H), 4.28 - 4.19 (m, 1H), 4.03 (s, 3H), 3.96 (s, 2H), 3.43 (s, 3H). ESI-MS *m*/*z* 433.0(M+H)⁺.

### RIP1-221

¹H NMR (400 MHz, CDCl₃) δ 8.80 (d, *J* = 7.5 Hz, 1H), 8.41 (d, *J* = 5.0 Hz, 1H), 8.08 (s, 1H), 7.86 (s, 1H), 7.57 (s, 1H), 7.42 (s, 1H), 7.25 - 7.20 (m, 2H), 7.15 (dd, *J* = 8.2, 4.4 Hz, 2H), 7.10 - 7.03 (m, 2H), 5.02 (dt, *J* = 11.2, 7.6 Hz, 1H), 4.67 (dd, *J* = 9.7, 7.6 Hz, 1H), 4.29 - 4.18 (m, 1H), 3.94 (s, 2H), 3.42 (s, 3H). ESI-MS *m*/*z* 429.1(M+H)⁺.

### RIP1-222

¹H NMR (400 MHz, CDCl₃) δ 8.09 (s, 1H), 8.06 (d, *J* = 7.3 Hz, 1H), 7.54 (s, 1H), 7.41 (s, 1H), 7.13 (dd, *J* = 14.1, 7.5 Hz, 1H), 6.97 - 6.77 (m, 3H), 4.97 (dd, *J* = 18.3, 7.7 Hz, 1H), 4.68 - 4.52 (m, 1H), 4.23 (t, *J* = 10.5 Hz, 1H), 4.05 (s, 2H), 3.41 (s, 3H). ESI-MS *m*/*z* 437.1(M+H)⁺.

### Example 2: Synthesis of Compound RIP1-018

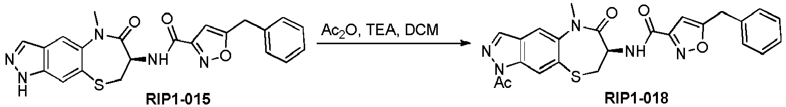

Compound **RIP1-015** (13 mg, 0.03 mmol) was placed into a 25 mL single-neck flask, and then DCM (5 mL), one drop of acetic anhydride, and two drops of TEA were added and reacted at room temperature for 30 min, until the reaction was completed as indicated by TLC. The solvent was removed under reduced pressure, and the residue was extracted with EA, washed with deionized water and saturated NaCl, and dried over Na₂SO₄. The solvent was removed under reduced pressure, and the residue was separated by reversed-phase column chromatography, and freeze dried to obtain **RIP1-018:** white solid 10 mg (70.7%).

**RIP1-018:** ¹H NMR (400 MHz, CDCl₃) δ 8.87 (s, 1H), 8.17 (s, 1H), 7.86 (d, *J* = 7.5 Hz, 1H), 7.70 (s, 1H), 7.38-7.29 (m, 3H), 7.27-7.23 (m, 2H), 6.32 (s, 1H), 4.82-4.72 (m, 1H), 4.12 (s, 2H), 3.86 (dd, *J* = 11.1, 6.8 Hz, 1H), 3.51 (s, 3H), 3.01-2.94 (m, 1H), 2.85 (s, 3H); ¹³C NMR (100 MHz, CDCl₃) δ 174.1, 171.0, 169.7, 158.2, 158.1, 142.3, 139.1, 137.4, 135.3, 129.8, 129.0, 128.9, 127.5, 127.5, 122.5, 116.7, 101.7, 49.5, 37.7, 37.4, 33.3, 23.0. ESI-MS: m/z 476.1 (M+H)⁺.

### Example 3: Synthesis of Compound II-1

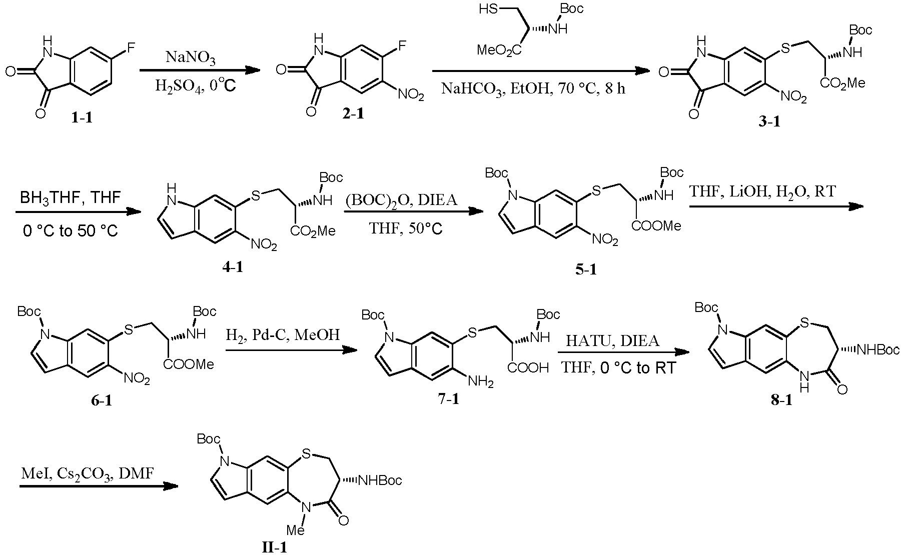

NaNO₃ (11.33 g) and H₂SO₄ (100 mL) were added to a 500 mL reaction flask, stirred, and cooled to 0°C in an ice bath. Compound **1-1** (20.00 g) was dissolved in H₂SO₄ (120 mL), and the solution of **1-1** in H₂SO₄ was slowly added dropwise to the reaction flask. After the completion of addition, the reaction was continued at 0°C for 1 h, and then the reaction solution was poured into ice water (1 L), stirred for 0.5 h, and filtered. The filter cake was washed with water to neutral. The filter cake was slurried and washed with EtOH/H₂O (7:3) (80 mL) for 0.5 h, and filtered. The filter cake was dried to obtain a crude product of **2-1** (18.83 g, yield 74%).

**2-1** (9.10 g), N-Boc-L-cysteine (13.24 g), and NaHCO₃ (5.67 g) were sequentially added to a reaction flask, and then EtOH (200 mL) was added, and reacted at 70°C for 8 h. After the reaction was completed, the reaction solution was cooled with stirring, and a large amount of solid was precipitated. H₂O (100 mL) was added, and stirred for another 0.5 h to fully precipitate the product out. After filtration, the filter cake was slurried and washed with H₂O (500 mL) for 0.5 h, and filtered. The filter cake was dried to obtain a crude product of **3-1** (17.5 g, yield 95%). ¹H NMR (500 MHz, DMSO-*d6*) δ = 11.60 (s, 1H), 8.27 (s, 1H), 7.56 (d, *J* = 8.4, 1H), 6.96 (s, 1H), 4.34-4.28 (m, 1H), 3.70 (s, 3H), 3.58 (dd, *J* = 13.0, 3.8, 1H), 3.31 (dd, *J*=12.8, 10.8, 1H), 1.37 (s, 9H); ¹³C NMR (125 MHz, DMSO-*d6*) δ 181.47, 170.82, 160.37, 155.35, 153.15, 149.16, 139.90, 122.60, 114.61, 107.90, 78.81, 52.43, 51.51, 33.36, 28.04; ESI-MS *m*/*z* 424.1 (M-H)⁻.

**3-1** (10.00 g) was placed into a 250 mL reaction flask, and then was added THF (80 mL) and stirred to dissolve. Then the solution was cooled to -10°C, and a solution of BH₃ in THF (1 mol/L, 40 mL) was slowly added to the reaction solution. After the completion of addition, the reaction solution was warmed to room temperature and reacted for 2 h, and then transferred to 50°C and reacted for 15 min. After cooling to 0°C, the reaction was quenched by slowly adding H₂O (10 mL), stirred for 0.5 h and subjected to rotary evaporation to remove most of the THF. The residue was dissolved in ethyl acetate, and extracted with water. The ethyl acetate layer was dried and rotary evaporated to dryness to obtain a crude product, which was separated by column chromatography on silica gel (eluent: ethyl acetate:petroleum ether=1:5), to obtain **4-1** (6.6 g, yield 71%). ¹H NMR (500 MHz, CD₃OD) δ = 8.40 (s, 1H), 7.59 (s, 1H), 7.43 (d, *J* = 3.2, 1H), 6.63 (d, *J* = 3.1, 1H), 4.39 (dd, *J* = 8.3, 5.0, 1H), 3.68 (s, 3H), 3.49 (dd, *J* = 13.8, 5.0, 1H), 3.26 (dd, *J* = 13.8, 8.5, 1H), 1.39 (s, 9H); ¹³C NMR (125 MHz, CD₃OD) δ 172.95, 157.70, 143.95, 140.05, 129.85, 127.13, 125.92, 120.12, 113.10, 104.46, 80.86, 61.53, 52.89, 28.61, 14.46; ESI-MS *m*/*z* 394.1, 433.4 (M-H)⁻.

**4-1** (3.00 g) was placed into a 100 mL reaction flask, and was added THF (30 mL) to dissolve. DIEA (1.27 g) and Boc anhydride (2.07 g) were sequentially added with stirring. The reaction solution was heated to 50°C and reacted overnight. After completion of reaction, the reaction solution was cooled to room temperature, rotary evaporated to dryness, and extracted with ethyl acetate and water. The ethyl acetate phase was collected, dried, and rotary evaporated to dryness. The residue was separated by column chromatography on silica gel (eluent: ethyl acetate: petroleum ether=1:8) to obtain Compound **5-1** (3.57 g, yield 95%). ESI-MS *m*/*z* 496.3 (M+H)⁺.

**5-1** (3.50 g) was placed into a 100 mL reaction flask, and was added THF (20 mL) to dissolve. Lithium hydroxide monohydrate (0.89 g) and H₂O (10 mL) were added, and stirred at room temperature for 24 h. After completion of reaction, the reaction solution was adjusted to pH 4-5 with 1 mol/L HCl in an ice bath, rotary evaporated to remove most of the THF, and extracted with ethyl acetate and water. The organic phase was collected, dried, and rotary evaporated to dryness to obtain a crude product of **6-1.**

The crude product of **6-1** was added methanol (30 mL) to dissolve, and purged three times with argon. Pd-C (0.75 g) was added under the argon atmosphere, and then hydrogen was added with stirring, and reacted at 25°C for 24 h. After the reaction was complete, the reaction solution was filtered through diatomaceous earth to remove Pd-C, and the filtrate was rotary evaporated to dryness to obtain a crude product of **7-1.**

The crude product of **7-1** was added anhydrous THF (200 mL) to dissolve. DIEA (2.01 g) was added, and cooled to 0°C with stirring. HATU (5.37 g) was added slowly, and after the completion of addition, the reaction solution was warmed to room temperature and reacted overnight. H₂O (10 mL) was added and stirred for 0.5 h, and most of the THF was removed by rotary evaporation. The remainder was extracted with ethyl acetate and water. The ethyl acetate layer was collected, dried, and rotary evaporated to dryness. The residue was separated by column chromatography on silica gel (eluent: petroleum ether:ethyl acetate=6:1), to obtain **8-1** (1.85 g, total yield of three steps 60%).

**8-1** (1.80 g) was placed into a 100 mL reaction flask, and was added DMF (15 mL) to dissolve. Cesium carbonate (1.76 g) was added, and cooled to -5°C with stirring. Then iodomethane (0.68 g) was slowly added. After the completion of addition, the reaction solution was warmed to room temperature, and reacted for 3 h at room temperature. After the reaction was complete, H₂O (60 mL) was slowly added to the reaction solution, during which a large amount of solid was precipitated out. After the completion of addition, the reaction solution was stirred for 0.5 h, and filtered. The filter cake was collected and dried to obtain **1.63 g** (1.63 g, yield 88%). ¹H NMR (500 MHz, CDCl₃) δ = 8.42 (s, 1H), 8.31 (s, 1H), 7.64 (d, *J* = 3.5, 1H), 7.32 (s, 1H), 6.51 (d, *J* = 3.6, 1H), 5.65 (d, *J* = 7.7, 1H), 4.47-4.41 (m, 1H), 3.81 (dd, *J* = 11.0, 6.7, 1H), 2.87 (t, *J* = 11.4, 1H), 1.69 (s, 9H), 1.38 (s, 9H); ¹³CNMR(125 MHz, CDCl₃) δ 172.51, 154.70, 149.32, 134.71, 133.57, 132.01, 128.14, 122.58, 122.02, 116.02, 106.92, 80.16, 50.40, 39.06, 38.74, 28.39, 28.26; ESI-MS *m*/*z* 448.2 (M+H)⁺.

### Example 4: Synthesis of Compound II-2

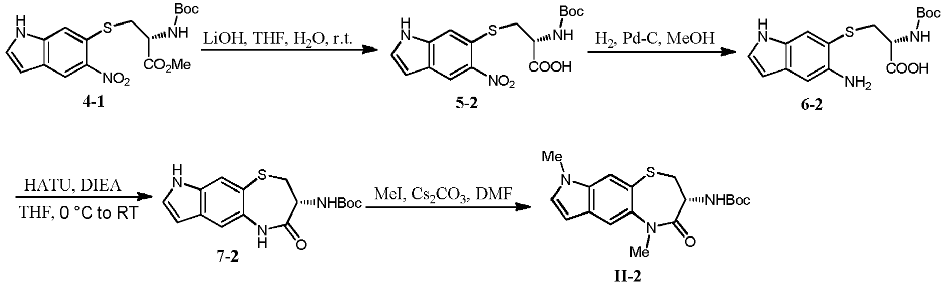

**4-1** (3 g) was placed into a 100 mL reaction flask, and was added THF (20 mL) to dissolve. Lithium hydroxide monohydrate (0.96 g) and H₂O (10 mL) were added, and stirred at room temperature for 24 h. After the reaction was complete, the reaction solution was adjusted to pH 4-5 with 1 mol/L HCl in an ice bath, rotary evaporated to remove most of the THF, and extracted with ethyl acetate and water. The organic phase was collected, dried, and rotary evaporated to dryness to obtain a crude product of **5-2.** MS-ESI: Calcd. for C₁₆H₁₉N₃O₆S (M): 381.1, found ESI-MS *m*/*z* 380.0433.4 (M-H)⁻.

The crude product of **5-2** was added methanol (30 mL) to dissolve, and purged three times with argon. Pd-C (0.81 g) was added under the argon atmosphere, then hydrogen was added with stirring, and reacted at 25°C for 24 h. After the reaction was complete, the reaction solution was filtered through diatomaceous earth to remove Pd-C, and the filtrate was rotary evaporated to dryness to obtain a crude product of **6-2.** ESI-MS *m*/*z* 352.1 (M+H)⁺.

The crude product of **6-2** was added anhydrous THF (200 mL) to dissolve. DIEA (2.15 g) was added, and cooled to 0°C with stirring. HATU (5.77 g) was added slowly, warmed to room temperature and reacted overnight. H₂O (10 mL) was added and stirred for 0.5 h, and most of the THF was removed by rotary evaporation. The remainder was extracted with ethyl acetate and water. The ethyl acetate layer was collected, dried, and rotary evaporated to dryness. The residue was separated by column chromatography on silica gel (eluent: petroleum ether:ethyl acetate=6:1), to obtain **7-2** (1.43 g, total yield of three steps 57%). ESI-MS *m*/*z* 334.1 (M+H)⁺.

**7-2** (1.40 g) was placed into a 100 mL reaction flask, and was added DMF (15 mL) to dissolve. Cesium carbonate (1.78 g) was added, and then cooled to -5°C with stirring. Next, iodomethane (0.69 g) was slowly added, warmed to room temperature, and reacted for 3 h at room temperature. After the reaction was complete, H₂O (60 mL) was slowly added to the reaction solution, during which a large amount of solid was precipitated out. After the completion of addition, the reaction solution was stirred for 0.5 h, and filtered. The filter cake was collected and dried to obtain **II-2** (1.25 g, yield 86%). ¹H NMR (500 MHz, CDCl₃) δ = 7.59 (s, 1H), 7.51 (s, 1H), 7.12 (d, *J* = 2.9, 1H), 6.46 (d, *J* = 2.6, 1H), 5.61 (d, *J* = 7.6, 1H), 4.41-4.32 (m, 1H), 3.79 (s, 3H), 3.68 (dd, *J* = 11.0, 6.7, 1H), 3.45 (s, 3H), 2.75 (t, *J* = 11.2, 1H), 1.36 (s, 9H); ¹³C NMR (125 MHz, CDCl₃) δ 171.13, 154.65, 137.94, 135.56, 131.37, 129.84, 119.70, 117.06, 116.16, 101.52, 79.92, 50.78, 39.01, 37.11, 33.22, 28.40; ESI-MS *m*/*z* 362.1 (M+H)⁺.

### Example 5: Synthesis of Compound II-3

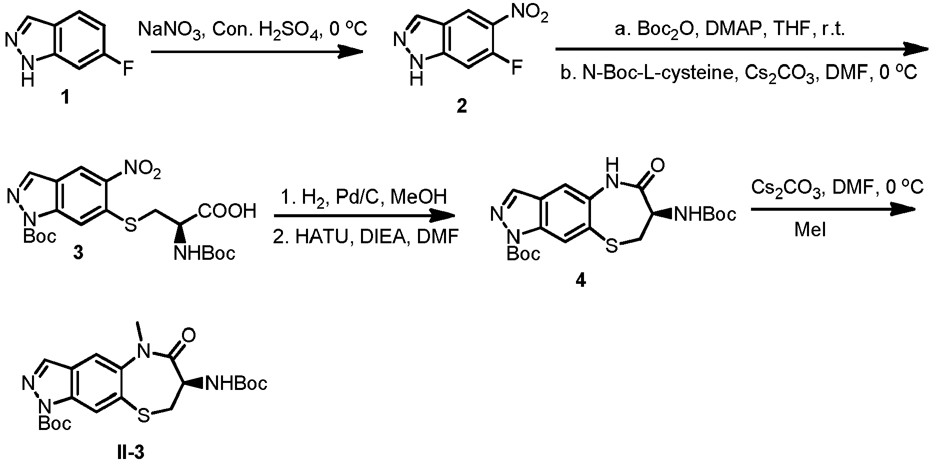

NaNO₃ (5100 mg, 60 mmol) was placed into a 500 mL reaction flask, and then concentrated sulfuric acid (60 mL) was added dropwise at 0°C. 6-fluoroindazole **1** (8167 mg, 60 mmol) was dissolved in concentrated sulfuric acid (60 mL), and then the resulting solution was slowly added dropwise to the solution of NaNO₃ in concentrated sulfuric acid, and reacted at 0 °C for 2.5 h until the reaction was completed as indicated by TLC. The reaction solution was poured into an ice-water mixture (500 mL), and filtered through filter paper under suction. The filtrate was extracted with EA. The organic phase was combined with the solid phase obtained by suction filtration, washed with saturated NaHCO₃ and saturated NaCl, dried over Na₂SO₄, concentrated, and the residue was separated by column chromatography (DCM) to obtain **2:** pale yellow solid (3300 mg, 30.3%).

Compound **2** (1449 mg, 8 mmol) was placed into a 250 mL reaction flask, and then Boc₂O (2095 mg, 9.6 mmol), DMAP (195mg, 1.6 mmol), and THF (100 mL) were added and reacted for 2 h room temperature until the reaction was completed as indicated by TLC. The reaction solution was extracted with EA, washed with deionized water and saturated NaCl, and dried over Na₂SO₄. After the solvent was removed under reduced pressure, a Boc-protected intermediate was obtained. The intermediate was directly used in a next step without purification. N-Boc-L-cysteine (2655 mg, 12 mmol) and Cs₂CO₃ (7819 mg, 24 mmol) were placed into a 250 mL reaction flask, added with DMF (80 mL) at 0°C and reacted for 15 min. Subsequently, a solution of the Boc-protected intermediate obtained in Reaction a in DMF (20 mL) was slowly added dropwise, and reacted overnight at 0°C, until the reaction was completed as indicated by TLC. The reaction solution was acidified with 0.2 M HCl, extracted with EA, washed with deionized water and saturated NaCl, and dried over Na₂SO₄. The solvent was removed under reduced pressure, and the residue was separated by column chromatography (DCM : MeOH = 20:1) to obtain **3:** pale yellow solid 2400 mg (62.2%).

Compound **3** (2310 mg, 4.8 mmol) was placed into a 250 mL three-neck flask, and purged three times with argon. Pd/C (4000 mg, 10 wt%) and MeOH (150 mL) were added under the argon atmosphere, purged three times with hydrogen, and reacted for 8 h under the hydrogen atmosphere at room temperature, until the reaction was completed as indicated by TLC. The reaction solution was filtered through diatomaceous earth under suction. The solvent was removed under reduced pressure, and the residue was re-dissolved in DMF (40 mL). HATU (1825 mg, 4.8 mmol) and DIEA (1240 mg, 9.6 mmol) were added to the solution, and reacted overnight at room temperature, until the reaction was completed as indicated by TLC. The reaction solution was extracted with EA, washed with deionized water and saturated NaCl, and dried over Na₂SO₄. The solvent was removed under reduced pressure, and the residue was separated by flash column chromatography (EA : PE = 1:2) to obtain **4:** earthy yellow solid (800 mg, 38%).

**4** (434.5 mg, 1 mmol) was placed into a reaction flask, and then Cs₂CO₃ (407.3 mg, 1.25 mmol) and DMF (40 mL) were added and reacted at 0°C for 10 min. Iodomethane (177.4 mg, 1.25 mmol) was slowly added dropwise to the solution, and reacted overnight at 0°C, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:2) to obtain **II-3:** pale yellow solid (417 mg, 93%).

**II-3:** ¹H NMR (400 MHz, CDCl₃) δ 8.51 (s, 1H), 8.15 (s, 1H), 7.64 (s, 1H), 5.57 (d, *J* = 7.9 Hz, 1H), 4.34 (dt, *J* = 11.5, 7.3 Hz, 1H), 3.69 (dd, *J* = 11.0, 6.6 Hz, 1H), 3.45 (s, 3H), 2.82 (t, *J* = 11.3 Hz, 1H), 1.73 (s, 9H), 1.35 (s, 9H); ¹³C NMR (100 MHz, CDCl₃) δ 170.7, 154.6, 148.7, 141.8, 139.0, 138.0, 129.4, 126.9, 121.6, 116.8, 85.9, 80.2, 50.6, 38.4, 37.2, 28.4, 28.2. ESI-MS: m/z 471.1 (M+H)⁺.

With Compound **8** as the starting material, Compound **II-5** could be obtained following the same synthesis method from **2** to **II-3** in this example. The synthesis of **8** was as described in Example 7.

### II-5

¹H NMR (400 MHz, CDCl₃) δ 8.49 (s, 1H), 7.58 (s, 1H), 5.57 (d, *J* = 7.9 Hz, 1H), 4.33 (dt, *J* = 11.6, 7.3 Hz, 1H), 3.68 (dt, *J* = 14.1, 7.1 Hz, 1H), 3.47 (s, 3H), 2.84 (t, *J* = 11.3 Hz, 1H), 1.72 (s, 9H), 1.36 (s, 9H); ¹³C NMR (100 MHz, CDCl₃) δ 170.6, 154.6, 148.0, 142.5, 140.5, 138.8, 131.1, 124.6, 122.0, 115.6, 80.3, 50.5, 38.3, 37.2, 28.4, 28.2. ESI-MS: m/z 505.1 (M+Na)⁺.

### Example 6: Synthesis of Compound II-4

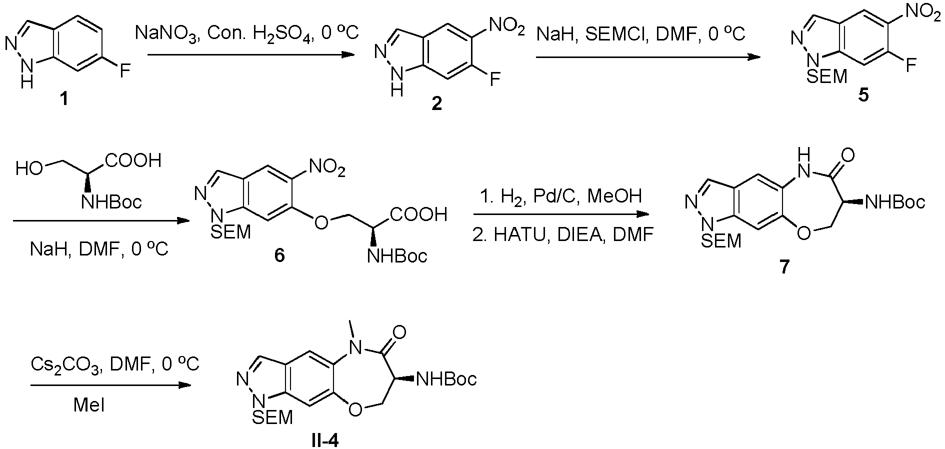

NaNO₃ (5100 mg, 60 mmol) was placed into a 500 mL reaction flask, and then concentrated sulfuric acid (60 mL) was added dropwise at 0°C. 6-fluoroindazole **1** (8167 mg, 60 mmol) was dissolved in concentrated sulfuric acid (60 mL), and then the resulting solution was slowly added dropwise to the solution of NaNO₃ in concentrated sulfuric acid, and reacted at 0°C for 2.5 h until the reaction was completed as indicated by TLC. The reaction solution was poured into an ice-water mixture (500 mL), and filtered through filter paper under suction. The filtrate was extracted with EA. The organic phase was combined with the solid phase obtained by suction filtration, washed with saturated NaHCO₃ and saturated NaCl, dried over Na₂SO₄, concentrated, and the residue was separated by column chromatography (DCM) to obtain **2:** pale yellow solid (3300 mg, 30.3%).

Compound **2** (1086.8 mg, 6 mmol) was placed into a 250 mL reaction flask, Cs₂CO₃ (2443 mg, 7.5 mmol) was added, and then DMF (40 mL) was added at 0°C and reacted for 15 min. Subsequently, SEMCl (1.33 mL, 7.5 mmol) was slowly added dropwise, and then reacted for 4 h to room temperature, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:8) to obtain **5:** pale yellow solid (900 mg, 48.2%). ¹H NMR (400 MHz, CDCl₃) δ 8.59 (d, *J* = 6.9 Hz, 1H), 8.18 (s, 1H), 7.42 (d, *J* = 10.7 Hz, 1H), 5.72 (s, 2H), 3.62-3.51 (m, 2H), 0.93-0.86 (m, 2H), -0.06 (s, 9H); ¹³C NMR (100 MHz, CDCl₃) δ 154.8 (d, *J* = 262.1 Hz), 140.8 (d, *J* = 11.5 Hz), 134.0 (d, *J* = 10.9 Hz), 136.3, 121.0, 120.0, 98.3 (d, *J* = 26.1 Hz), 78.5, 67.2, 17.8, -1.4.

N-Boc-L-serine (1026.1 mg, 5 mmol) and NaH (400 mg, 10 mmol, 60 wt%) were placed into a 250 mL reaction flask, and purged three times with argon. Under the argon atmosphere, DMF (50 mL) was added at 0°C and reacted for 15 min. Next, a solution of 5 (778.5 mg, 2.5 mmol) in DMF (10 mL) was slowly added dropwise, and reacted overnight at 0°C, until the reaction was completed as indicated by TLC. The reaction solution was acidified with 0.2 M HCl, then extracted with EA, washed with deionized water and saturated NaCl, and dried over Na₂SO₄. The solvent was removed under reduced pressure, and the residue was separated by column chromatography (DCM : MeOH = 20:1) to obtain 6: pale yellow solid (473 mg, 38.1%). ¹H NMR (400 MHz, CDCl₃) δ 7.95 (s, 1H), 7.53 (s, 1H), 7.33 (s, 1H), 5.73-5.60 (m, 2H), 5.58-5.47 (m, 1H), 4.70-4.59 (m, 1H), 4.58-4.49 (m, 1H), 4.17-4.09 (m, 1H), 3.60-3.48 (m, 2H), 3.45 (s, 3H), 0.96-0.85 (m, 2H), -0.08 (s, 9H); ¹³C NMR (100 MHz, CDCl₃) δ 169.4, 155.1, 150.0, 138.2, 134.0, 132.2, 121.9, 115.3, 103.1, 80.3, 78.0, 66.6, 49.9, 36.4, 28.3, 17.7, -1.4.

Compound **6** (450 mg, 0.9062 mmol) was placed into a 100 mL three-neck flask, and purged three times with argon. Pd/C (600 mg, 10 wt%) and MeOH (30 mL) were added under the argon atmosphere, purged three times with hydrogen, and reacted for 4 h under the hydrogen atmosphere at room temperature, until the reaction was completed as indicated by TLC. The reaction solution was filtered through diatomaceous earth under suction. The solvent was removed under reduced pressure, and the residue was re-dissolved in DMF (20 mL). HATU (380.2 mg, 1 mmol) and DIEA (234 mg, 1.81 mmol) were added to the solution, and reacted overnight at room temperature, until the reaction was completed as indicated by TLC. The reaction solution was extracted with EA, washed with deionized water and saturated NaCl, and dried over Na₂SO₄. The solvent was removed under reduced pressure, and the residue was separated by flash column chromatography (EA : PE = 1:2) to obtain a brown solid 7 (260 mg, 64%).

Cs₂CO₃ (226.6 mg, 0.6955 mmol) and DMF (20 mL) were added to 7 and reacted at 0°C for 10 min. Iodomethane (98.7 mg, 0.6955 mmol) was slowly added dropwise to the solution, and reacted overnight at 0°C, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:2) to obtain **II-4:** brown solid (202 mg, 75.3%).

**II-4:** ¹H NMR (400 MHz, CDCl₃) δ 7.95 (s, 1H), 7.53 (s, 1H), 7.33 (s, 1H), 5.72-5.62 (m, 2H), 5.54 (d, *J=* 7.2 Hz, 1H), 4.65 (dt, *J* = 11.2, 7.3 Hz, 1H), 4.53 (dd, *J=* 9.5, 7.4 Hz, 1H), 4.18-4.08 (m, 1H), 3.57-3.49 (m, 2H), 3.45 (s, 3H), 1.36 (s, 9H), 0.95-0.86 (m, 2H), -0.06 (s, 9H); ¹³C NMR (101 MHz, CDCl₃) δ 169.4, 155.1, 150.0, 138.2, 133.96, 132.2, 121.9, 115.3, 103.1, 80.3, 78.0, 66.6, 49.9, 36.4, 28.3, 17.7, -1.4.

With Compound **8** as the starting material, Compound **II-6** could be obtained following the same synthesis method from **2** to **II-4** in this example. The synthesis of 8 was as described in Example 7.

### II-6

¹H NMR (400 MHz, CDCl₃) δ 7.48 (s, 1H), 7.32 (s, 1H), 5.65-5.58 (m, 2H), 5.50 (d, *J* = 7.1 Hz, 1H), 4.65 (dt, *J* = 11.3, 7.2 Hz, 1H), 4.55 (dd, *J* = 9.5, 7.3 Hz, 1H), 4.21-4.12 (m, 1H), 3.62-3.54 (m, 2H), 3.49 (s, 3H), 1.38 (s, 9H), 0.97-0.88 (m, 2H), -0.02 (s, 9H); ¹³C NMR (100 MHz, CDCl₃) δ 169.2, 155.1, 151.0, 139.3, 134.1, 133.1, 119.1, 114.0, 103.6, 80.2, 78.1, 77.3, 66.9, 49.8, 36.3, 28.2, 17.7, -1.4.

### Example 7: Synthesis of Compound 8

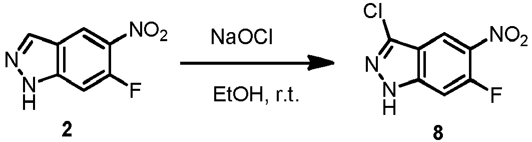

Compound **2** (2000 mg, 11 mmol) was placed into a 250 mL reaction flask, and then EtOH (50 mL) and NaOCl (20 mL, 55 mmol) were added, and reacted overnight at room temperature, until the reaction was completed as indicated by TLC. The reaction was acidified with 1 N HCl, extracted with EA, washed with deionized water and saturated NaCl, and dried over Na₂SO₄. The solvent was removed under reduced pressure, and the residue was separated by column chromatography (EA:PE = 1:4) to obtain **8:** pale yellow solid (2000 mg, 84.3%).

**8:** ¹H NMR (400 MHz, CD₃OD_SPE) δ 8.41 (d, *J* = 6.9 Hz, 1H), 7.42 (d, *J* = 11.1 Hz, 1H); ¹³C NMR (101 MHz, CD₃OD_SPE) δ 156.2 (d, *J* = 260.0 Hz), 143.4 (d, *J* = 12.2 Hz), 137.7, 135.2 (d, *J* = 11.6 Hz), 120.2, 116.8, 99.8 (d, *J* = 26.4 Hz).

### Example 8: Synthesis of Compound II-7

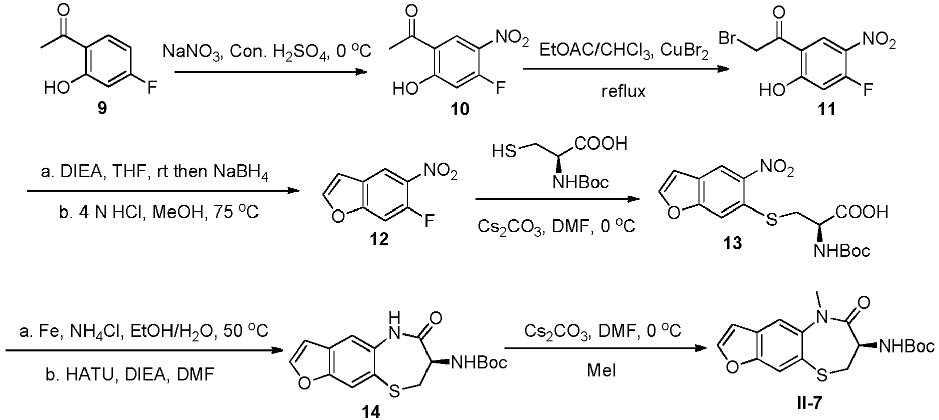

NaNO₃ (5100 mg, 60 mmol) was placed into a 500 mL reaction flask, and then concentrated sulfuric acid (60 mL) was added dropwise at 0°C. 4-fluoro-2-hydroxyacetophenone **9** (9248 mg, 60 mmol) was dissolved in concentrated sulfuric acid (60 mL), and then the resulting solution was slowly added dropwise to the solution of NaNO₃ in concentrated sulfuric acid, and reacted at 0 °C for 2.5 h until the reaction was completed as indicated by TLC. The reaction solution was poured into an ice-water mixture (500 mL), and filtered through filter paper under suction. The filtrate was extracted with EA. The organic phase was combined with the solid phase obtained by suction filtration, washed with saturated NaHCO₃ and saturated NaCl, dried over Na₂SO₄, concentrated, and the residue was separated by column chromatography (EA:PE= 1:20) to obtain **10:** pale yellow solid (4570 mg, 38.2%). ¹H NMR (400 MHz, CDCl₃) δ 13.02 (s, 1H), 8.65 (d, *J* = 8.3 Hz, 1H), 6.83 (d, *J* = 12.2 Hz, 1H), 2.72 (s, 3H); ¹³C NMR (100 MHz, CDCl₃) δ 203.2, 168.5 (d, *J* = 14.5 Hz), 161.9, 159.2, 130.6, 116.0 (d, *J* = 2.5 Hz), 107.6 (d, *J* = 23.0 Hz), 26.9.

CuBr₂ (1898 mg, 8.5 mmol) was placed into a 250 mL reaction flask, and then ethyl acetate (50 mL) was added, and heated to reflux. A solution of **10** (995.7 mg, 5 mmol) in chloroform (50 mL) was added to the reaction solution, and reacted under reflux for 24 h. The green color of the reaction liquid faded, and a lime-colored solid was formed. **10** was mostly converted as indicated by TLC. The solid was filtered off by diatomaceous earth under suction. The solvent was removed under reduced pressure, and the residue was separated by column chromatography (EA:PE = 1:20 to 1:10) to obtain **11:** brown solid (833 mg, 60%). ¹H NMR (400 MHz, CDCl₃) δ 8.37 (d, *J* = 7.3 Hz, 1H), 7.77 (d, *J* = 2.3 Hz, 1H), 7.41 (d, *J* = 10.6 Hz, 1H), 6.90 (dd, *J* = 2.2, 0.7 Hz, 1H); ¹³C NMR (100 MHz, CDCl₃) δ 156.8 (d, *J* = 12.4 Hz), 155.3, 152.7, 148.6 (d, *J* = 3.7 Hz), 123.6 (d, *J* = 2.1 Hz), 119.2 (d, *J* = 1.8 Hz), 107.3 (d, *J* = 1.2 Hz), 101.5 (d, *J* = 25.8 Hz).

Compound **11** (5004 mg, 18 mmol) was placed into a 500 mL reaction flask, THF (180 mL) was added, and then DIEA (3 mL, 18 mmol) was slowly added and reacted for 45 min at room temperature, until the reaction was completed as indicated by TLC. NaBH₄ (2723 mg, 72 mmol) was added to the solution, and reacted for 8 h at room temperature, until the reaction was completed as indicated by TLC. The reaction was quenched by adding methanol slowly. The solvent was removed under reduced pressure, and the remainder was re-dissolved in MeOH (50 mL). Then 4 N HCl (25 mL) was added and heated to 75 °C for 3 h until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:10) to obtain **12:** yellow solid: (1200 mg, 36.8%).

N-Boc-L-cysteine (2660 mg, 12 mmol) and Cs₂CO₃ (7819 mg, 24 mmol) were placed into a 250 mL reaction flask, added with DMF (40 mL) at 0°C and reacted for 15 min. Subsequently, a solution of **12** (1086 mg, 6 mmol) in DMF (10 mL) was slowly added dropwise, and reacted at 0°C for 24 h, until the reaction was completed as indicated by TLC. The reaction solution was acidified with 0.2 M HCl, extracted with EA, washed with deionized water and saturated NaCl, and dried over Na₂SO₄. The solvent was removed under reduced pressure, and the residue was separated by column chromatography (DCM : MeOH = 20:1) to obtain **13:** pale yellow solid 2780 mg (qunt.). ¹H NMR (400 MHz, CDCl₃) δ 8.30 (s, 1H), 7.64 (d, *J* = 2.0 Hz, 1H), 7.54 (s, 1H), 6.75 (d, *J* = 1.9 Hz, 1H), 5.70 (s, 1H), 4.50 (s, 1H), 3.60-3.49 (m, 1H), 3.35 (s, 1H), 1.35 (s, 9H); ¹³C NMR (100 MHz, CDCl₃) δ 175.1, 157.2, 155.9, 147.8, 143.5, 132.4, 124.9, 119.7, 110.4, 107.1, 80.5, 53.5, 36.1, 28.4. ESI-MS: m/z 383.1 (M+H)⁺.

Compound **13** (956 mg, 2.5 mmol) was placed into a 100 mL reaction flask, and Fe (698 mg, 12.5 mmol) and NH₄Cl (267 mg, 5 mmol) were added. Then EtOH (24 mL) and H₂O (6 mL) were added, and reacted at 50°C for 4 h, until the reaction was completed as indicated by TLC. The reaction solution was filtered through diatomaceous earth. The solvent was removed under reduced pressure, and the residue was re-dissolved in DMF (60 mL). HATU (950 mg, 2.5 mmol) and DIE (0.83 mL, 5 mmol) were added to the solution, and reacted overnight at room temperature, until the reaction was completed as indicated by TLC. The reaction solution was extracted with EA, washed with deionized water and saturated NaCl, and dried over Na₂SO₄. The solvent was removed under reduced pressure, and the residue was separated by flash column chromatography (EA : PE = 1:3) to obtain a yellowish-brown solid **14** (500 mg, 59.8%).

**14** (1500 mg, 4.5 mmol) was placed into a reaction flask, and then Cs₂CO₃ (1759 mg, 5.4 mmol) and DMF (60 mL) were added and reacted at 0°C for 10 min. Iodomethane (766.5 mg, 5.4 mmol) was slowly added dropwise to the solution, and reacted overnight at 0°C, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:4) to obtain **II-7:** white solid 1320 mg (84.2%).

**II-7**:¹H NMR (400 MHz, CDCl₃) δ 7.78 (s, 1H), 7.69 (d, *J* = 2.1 Hz, 1H), 7.52 (s, 1H), 6.77 (d, *J* = 1.3 Hz, 1H), 5.58 (d, *J* = 7.8 Hz, 1H), 4.37 (dt, *J* = 11.3, 7.5 Hz, 1H), 3.70 (dd, *J* = 11.1, 6.7 Hz, 1H), 3.44 (s, 3H), 2.79 (t, *J* = 11.2 Hz, 1H), 1.37 (s, 9H); ¹³C NMR (101 MHz, CDCl₃) δ 171.0, 154.6, 153.1, 147.4, 141.4, 129.6, 123.3, 118.1, 117.4, 106.7, 80.0, 50.7, 39.0, 37.0, 28.4.

### Example 9: Synthesis of Compound II-8

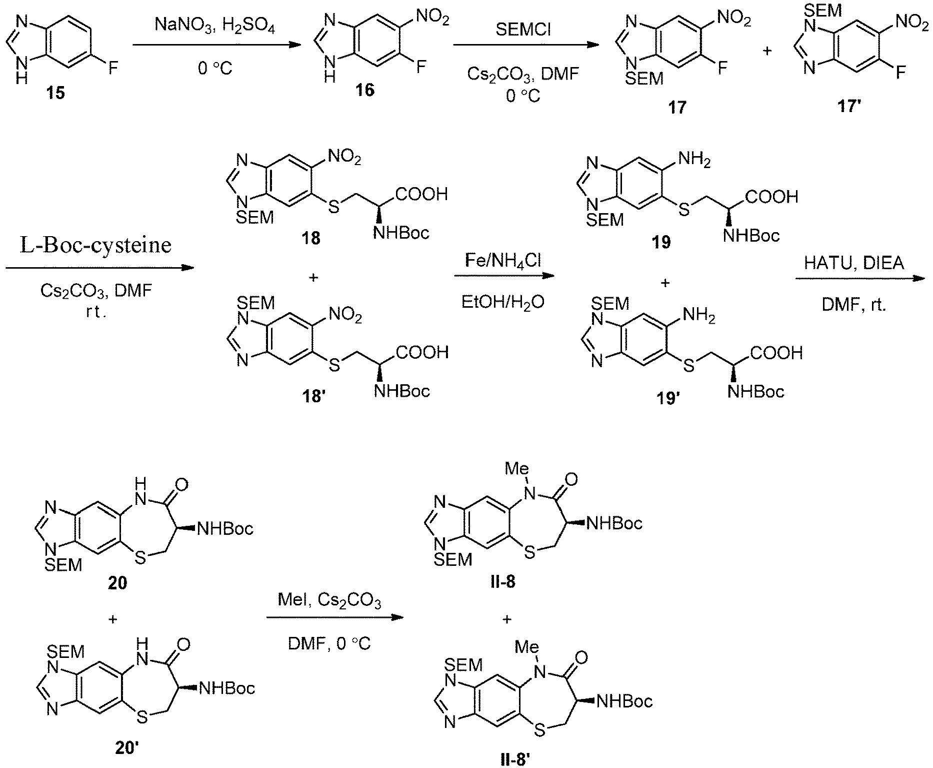

NaNO₃ (2520 mg, 30 mmol) was placed into a 500 mL reaction flask, and then concentrated sulfuric acid (30 mL) was added dropwise at 0°C. 6-fluorobenzimidazole **15** (29 mg, 30 mmol) was dissolved in concentrated sulfuric acid (30 mL), and then the resulting solution was slowly added dropwise to the solution of NaNO₃ in concentrated sulfuric acid, and reacted at 0 °C for 2.5 h until the reaction was completed as indicated by TLC. The reaction solution was poured into an ice-water mixture (500 mL), and adjusted to a basic pH with a saturated NaCO₃ solution. The reaction solution was extracted with EA, and dried over Na₂SO₄. After the solvent was removed under reduced pressure, a brown solid powder was obtained, which was directly used in a next reaction without purification by column chromatography.

Compound **16** (2300 mg, 12.7 mmol) was placed into a 50 mL reaction flask, Cs₂CO₃ (5200 mg, 15.9 mmol) was added, and then DMF (20 mL) was added at 0°C and reacted for 15 min. Subsequently, SEMCl (2.8 mL, 15.9 mmol) was slowly added dropwise, and then reacted for 4 h to room temperature, until the reaction was completed as indicated by TLC. The reaction solution was extracted with EA, washed with saturated NaCl, and dried over Na₂SO₄. The solvent was removed under reduced pressure, and the residue was separated by column chromatography (EA : PE = 1:8) to obtain a pair of isomers **17** and **17'** at a ratio of approximately 1:1 which could not be separated by column chromatography: pale yellow solid (900 mg).

N-Boc-L-cysteine (284 mg, 1.3 mmol) and Cs₂CO₃ (837 mg, 2.6 mmol) were placed into a 50 mL reaction flask, and then DMF (10 mL) was added at room temperature and reacted for 15 min. Subsequently, a solution of mixed **17 and 17'** in DMF (2 mL) was slowly added dropwise, and reacted overnight at room temperature, until the reaction was completed as indicated by TLC. The reaction solution was acidified with 0.2 M HCl, extracted with EA, washed with deionized water and saturated NaCl, and dried over Na₂SO₄. The solvent was removed under reduced pressure, and the residue was separated by column chromatography (DCM : MeOH = 10:1) to obtain a pair of isomers Compounds **18** and **18':** pale yellow solid (140.6 mg, 86%).

Compounds **18** and **18'** (330 mg, 0.64 mmol) were placed into a 250 mL three-neck flask, and purged three times with argon. Under the argon atmosphere, a Fe powder (180 mg, 3.2mmol), NH₄Cl (172.2 mg, 3.2 mmol), ethanol (12.5 mL), and deionized water (10 mL) were added and reacted under reflux for 3 h, until the reaction was completed as indicated by TLC. The reaction solution was filtered through diatomaceous earth under suction, and dried over anhydrous Na₂SO₄. After the solvent was removed under reduced pressure, a crude product of a pair of isomers **19** and **19'** was obtained.

HATU (293 mg, 0.8 mmol) and DIEA (181 mg, 1.4 mmol) were added to a solution of Compounds **19** and **19'** in DMF, and reacted overnight at room temperature, until the reaction was completed as indicated by TLC. The reaction solution was extracted with EA, washed with deionized water and then with saturated NaCl, and dried over Na₂SO₄. The solvent was removed under reduced pressure, and the residue was separated by flash column chromatography (EA : PE = 1:2) to obtain a pair of isomers **20** and **20':** earthy yellow solid (240mg, 38%).

Compounds **20** and **20'** (240 mg, 0.52 mmol) were placed into a reaction flask, and then Cs₂CO₃ (202 mg, 0.62 mmol) and DMF (10 mL) were added and reacted at 0°C for 10 min. Iodomethane (88 mg, 0.62 mmol) was slowly added dropwise to the solution, and reacted overnight at 0°C, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:2) to obtain a pair of isomers **II-8** and **II-8':** pale yellow solid (120 mg, 93%). After the pair of intermediates were subsequently removed of the protecting group, a common intermediate compound was obtained for the final condensation reaction.

Data for **II-8** + **II-8'**: ESI: m/z 479.2 (M+H)⁺.

### Example 10: Synthesis of Compound II-9

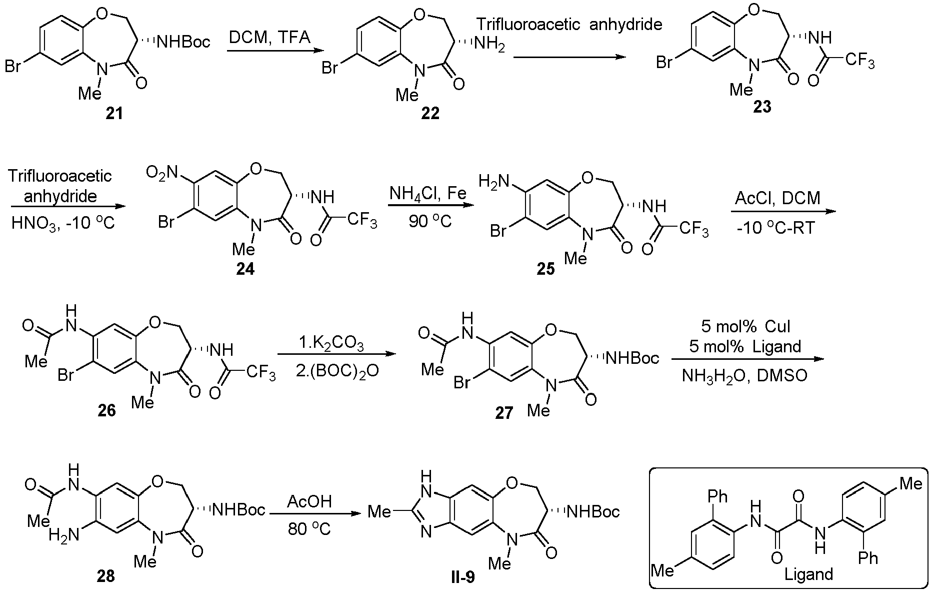

Compound **21** (8 g) was added to a 250 mL single-neck flask equipped with a rotor, and was added dichloromethane (50 mL) to dissolve. Trifluoroacetic acid (12 mL) was added with stirring, and the reaction solution was stirred at room temperature for 1 h, followed by rotary evaporation to remove dichloromethane and trifluoroacetic acid. The residue was extracted with water (80 mL), adjusted to pH 8-9, and then extracted with ethyl acetate. The organic phase was collected, extracted with saturated brine, dried over anhydrous sodium sulfate, filtered under suction, and rotary evaporated to dryness to obtain Compound **22** as a brown solid (5.8 g). ESI-MS *m*/*z* (⁷⁹Br) 271.0 and (⁸¹Br) 273.0 (M+H)⁺.

Compound **22** (5.5 g), N,N-diisopropyl ethylamine (3.93 g), and trifluoroacetic anhydride (5.75 g) were sequentially added to a 250 mL single-neck flask equipped with a rotor, added with dichloromethane (50 mL) to dissolve, and stirred at room temperature for 8 h. After the reaction was complete, the reaction solution was extracted with water (50 mL). The dichloromethane layer was collected, extracted with saturated brine, dried over anhydrous sodium sulfate, filtered to remove anhydrous sodium sulfate, rotary evaporated to dryness, and the residue was separated by column chromatography on silica gel (eluent: petroleum ether:ethyl acetate=7:1), to obtain Compound **23** (7.30 g, yield 98%). ESI-MS *m*/*z* (⁷⁹Br) 367.1 and (⁸¹Br) 369.1 (M+H)⁺.

Trifluoroacetic anhydride (60 mL) was added to a 250 mL two-neck flask equipped with a thermometer and a rotor, and cooled to -30°C. Compound **23** (7 g) was added, and dissolved with stirring. Concentrated nitric acid (1.4 g) was slowly added dropwise, and then the reaction was continued for 4 h while the temperature was maintained at -10°C or below. After the reaction was complete, the reaction solution was poured into ice water (300 mL) and stirred well. A large amount of solid was precipitated and filtered under suction. The filter cake was washed with water until neutral, collected, and dried, to obtain Compound **24** as a yellow solid (7.70 g, yield 96%). EI-MS *m*/*z* (⁷⁹Br) 411 and (⁸¹Br) 413 (M)⁺.

Compound **24** (7 g), iron powder (7.6 g), a saturated aqueous ammonium chloride solution (100 mL), and ethanol (100 mL) were added in sequence to a 250 mL single-neck flask equipped with a rotor, heated to 90°C, and reacted for 5 h. After the reaction was complete, the reaction solution was filtered through diatomaceous earth, and the filter cake was washed three times with dichloromethane (150 mL). The filtrate was collected, and rotary evaporated to remove most of the ethanol and dichloromethan. The remaining liquid was adjusted to pH 8-9 with saturated sodium carbonate, and extracted three times with dichloromethane (300 mL). The organic phase was collected, washed once respectively with saturated sodium bicarbonate and saturated saline, dried over anhydrous sodium sulfate, filtered, rotary evaporated to dryness, and the residue was separated by column chromatography on silica gel (eluent: petroleum ether:ethyl acetate=8:1), to obtain Compound **25** (4.02 g) as a white solid. Yield 62%; ESI-MS *m*/*z* (⁷⁹Br) 382.1 and (⁸¹Br) 384.0 (M+H)⁺.

Compound **25** (4 g) and N,N-diisopropyl ethylamine (1.83 g) were sequentially added to a 250 mL two-neck flask equipped with a thermometer and a rotor, and were added dichloromethane (40 mL) to dissolve with stirring. Then the reaction solution was cooled to -10°C. Acetyl chloride (1.06 g) was dissolved in dichloromethane (10 mL). The solution of acetyl chloride in dichloromethane was slowly added dropwise to the above reaction solution such that the temperature of the reaction solution was maintained at -5°C or below during the addition. Then, the reaction solution was slowly warmed to room temperature, and reacted overnight. After the reaction was complete, the reaction solution was quenched with water (50 mL), and extracted. The organic phase was collected, washed with saturated brine, dried over anhydrous sodium sulfate, filtered, rotary evaporated to dryness, and separated and purified by column chromatography on silica gel (eluent: petroleum ether:ethyl acetate=4:1) to obtain Compound 26 as a white solid (3.19 g). Yield 72%. ¹H NMR (500 MHz, CDCl₃) δ = 8.34 (s, 1H), 7.62 (s, 1H), 7.55 (d, *J* = 6.3, 1H), 7.39 (s, 1H), 4.88-4.82 (m, 1H), 4.70 (dd, *J* = 9.8, 7.4, 1H), 4.22 (dd, *J* = 10.9, 10.1, 1H), 3.39 (s, 3H), 2.26 (s, 3H); ¹³C NMR (125 MHz, CDCl₃) δ 168.38, 167.73, 156.74 (q, *J* = 38.4), 149.71, 135.40, 131.81, 126.45, 115.78, 115.57 (q, *J* = 287.6), 108.53, 77.41, 77.16, 76.91, 76.35, 49.75, 35.76, 25.04; ESI-MS *m*/*z* 424.1 (⁷⁹Br) and 426.1 (⁸¹Br) (M + H)⁺.

Compound **26** (3 g) and anhydrous potassium carbonate (3 g) were added sequentially to a 100 mL single-neck flask equipped with a stir bar, and were added with tetrahydrofuran (15 mL) and water (15 mL) to dissolve. The reaction solution was stirred overnight at room temperature. After the reaction was complete, Boc anhydride (2.31 g) was added to the reaction solution, and stirred at room temperature for 5 h. The reaction solution was extracted with water (30 mL) and dichloromethane (30 mL). The organic phase was collected, washed with saturated saline, dried over anhydrous sodium sulfate, filtered, and rotary evaporated to dryness, to obtain Compound **27** as a white solid (3 g, yield 99%). ESI-MS *m*/*z* (⁷⁹Br) 428.0 and (⁸¹Br) 429.9 (M+H)⁺.

CuI (19.0 mg, 0.1 mmol), N¹,N²-bis(5-methyl-[1,1'-biphenyl]-2-yl)oxamide (42.0 mg, 0.1 mmol), Compound **27** (0.856 g, 2 mmol) and K₃PO₄ (0.424 g, 2 mmol) were sequentially added to a 10 mL Schlenk tube equipped with a rotor. Subsequently, 25% aqueous ammonia (0.28 g) and DMSO (2 mL) were sequentially added via a syringe to the Schlenk tube under an argon atmosphere, and the Schlenk tube was sealed. The reaction solution was heated to 60°C and reacted for 24 h. After the reaction was complete, the reaction solution was cooled to room temperature, diluted with ethyl acetate (30 mL), and filtered through diatomaceous earth. The filter cake was washed with ethyl acetate, and the filtrate was distilled under reduced pressure. The remaining residue was purified and separated by column chromatography on silica gel (developing agent: dichloromethane:methanol=30:1) to obtain Compound **28** as a brown solid (0.59 g, yield 81%). ESI-MS: Calcd. for C₁₇H₂₄N₄O₅ (M):364.1, found ESI-MS *m*/*z* 365.1 (M+H)⁺.

Compound **28** (1 g) was added to a 50 mL single-neck flask equipped with a stirring bar, and then acetic acid (15 mL) was added, heated to 80°C, and reacted for 1 h. After the reaction was complete, the reaction solution was to room temperature, added with water (50 mL), and extracted with ethyl acetate. The organic phase was collected, washed with saturated sodium bicarbonate solution and saturated saline, dried over anhydrous sodium sulfate, filtered, and rotary evaporated to dryness. The residue was separated and purified by column chromatography on silica gel (developing agent: dichloromethane:methanol=25:1) to obtain Intermediate **II-9** as a pale yellow solid (0.74 g, yield 78%). ¹H NMR (400 MHz, CDCl₃) δ = 7.29 (s, 1H), 7.21 (s, 1H), 5.62 (d, *J* = 7.3, 1H), 4.65-4.56 (m, 1H), 4.51 (dd, *J* = 9.4, 7.5, 1H), 4.14 (t, *J* = 10.4, 1H), 3.38 (s, 3H), 2.55 (s, 3H), 1.38 (s, 9H); ¹³C NMR (100 MHz, CDCl₃) δ 169.49, 155.56, 153.52, 149.54, 146.13, 136.19, 131.83, 109.50, 107.07, 80.67, 77.84, 50.25, 36.20, 28.34, 14.94; ESI-MS *m*/*z* 347.0 (M + H)⁺; ESI-HRMS Calcd. for C₁₇H₂₃O₄N₄ (M + H)⁺: 347.1714, found: 347.1717.

### Example 11: Synthesis of Compound II-10

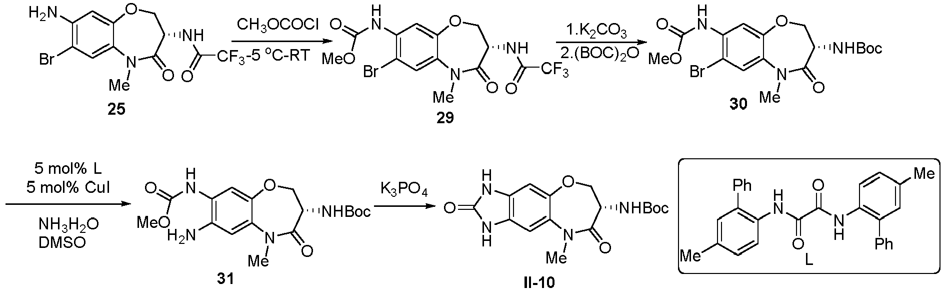

Compound **25** (4 g) and N,N-diisopropyl ethylamine (1.83 g) were sequentially added to a 250 mL two-neck flask equipped with a thermometer and a rotor, and then were added dichloromethane (40 mL) to dissolve with stirring. The reaction solution was cooled to -5°C. Acetyl chloride (1.29 g) was dissolved in dichloromethane (10 mL). The solution of acetyl chloride in dichloromethane was slowly added dropwise to the above reaction solution such that the temperature of the reaction solution was maintained at 0°C or below during the addition. Then, the reaction solution was slowly warmed to room temperature, and reacted overnight. After the reaction was complete, the reaction solution was quenched with water (50 mL), and extracted. The organic phase was collected, washed with saturated brine, dried over anhydrous sodium sulfate, filtered, rotary evaporated to dryness, and separated and purified by column chromatography on silica gel (eluent: petroleum ether:ethyl acetate=4:1) to obtain Compound **29** as a white solid (3.19g, yield 67%), ESI-MS *m*/*z* 440.2 (⁷⁹Br) and 442.2 (⁸¹Br) (M + H)⁺.

Compound **29** (3 g) and anhydrous potassium carbonate (3 g) were added sequentially to a 100 mL single-neck flask equipped with a stir bar, and were added tetrahydrofuran (15 mL) and water (15 mL) to dissolve. The reaction solution was stirred overnight at room temperature. After the reaction was complete, Boc anhydride (2.30 g) was added to the reaction solution, and stirred at room temperature for 5 h. The reaction solution was extracted with water (30 mL) and dichloromethane (30 mL). The organic phase was collected, washed with saturated saline, dried over anhydrous sodium sulfate, filtered, and rotary evaporated to dryness, to obtain Compound **30** as a white solid (3 g, yield 99%). ESI-MS *m*/*z* 465.8 (⁷⁹Br) and 467.8 (⁸¹Br) (M + Na)⁺.

CuI (19.0 mg, 0.1 mmol), N¹,N²-bis(5-methyl-[1,1'-biphenyl]-2-yl)oxamide (42.0 mg, 0.1 mmol), Compound **30** (0.888 g, 2 mmol) and K₃PO₄ (0.424 g, 2 mmol) were sequentially added to a 10 mL Schlenk tube equipped with a rotor. Subsequently, 25% aqueous ammonia (0.28 g) and DMSO (2 mL) were sequentially added via a syringe to the Schlenk tube under an argon atmosphere, and the Schlenk tube was sealed. The reaction solution was heated to 70°C and reacted for 24 h. After the reaction was complete, the reaction solution was cooled to room temperature, diluted with ethyl acetate (30 mL), and filtered through diatomaceous earth. The filter cake was washed with ethyl acetate, and the filtrate was distilled under reduced pressure. The residue was purified and separated by column chromatography on silica gel (developing agent: dichloromethane:methanol=30:1) to obtain Compound **31** as a brown solid (0.40 g, yield 53%). ESI-MS *m*/*z* 381.1 (M + H)⁺.

Compound **31** (1 g) was added to a 25 mL single-neck flask equipped with a stir bar, and then DMSO (10 mL) and potassium phosphate (1.67 g) were added, heated to 100°C, and reacted for 5 h. After the reaction was complete, the reaction solution was cooled to room temperature, added with water (50 mL), and extracted with ethyl acetate. The organic phase was collected, washed with saturated saline, dried over anhydrous sodium sulfate, filtered, and rotary evaporated to dryness. The residue was separated and purified by column chromatography on silica gel (developing agent: dichloromethane:methanol=20:1) to obtain Compound **II-10** as a pale yellow solid (0.69 g, yield 78%). ¹H NMR (500 MHz, CDCl₃) δ = 10.58 (s, 1H), 10.46 (s, 1H), 6.88 (s, 1H), 6.62 (s, 1H), 5.72 (d, *J* = 7.1, 1H), 4.64-4.59 (m, 1H), 4.58-4.50 (m, 1H), 4.21-4.09 (m, 1H), 3.32 (s, 3H), 1.44 (s, 9H); ESI-MS *m*/*z* 371.1.

### Example 12: Synthesis of Compound II-11

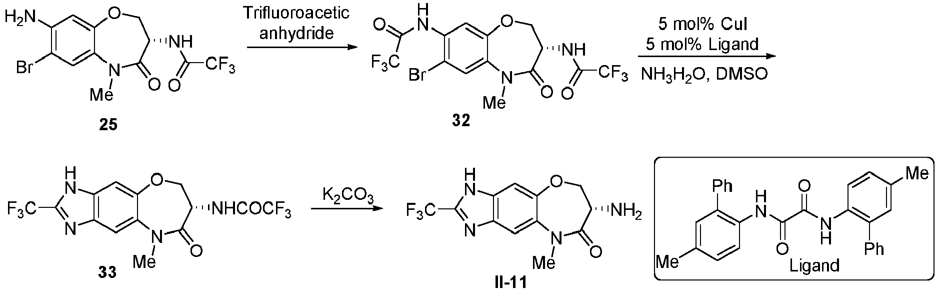

Compound **25** (4 g) and N,N-diisopropyl ethylamine (1.83 g) were sequentially added to a 250 mL two-neck flask equipped with a thermometer and a rotor, and were added dichloromethane (40 mL) to dissolve with stirring. Then the reaction solution was cooled to -20°C. Trifluoroacetic anhydride (2.9 g) was dissolved in dichloromethane (10 mL). The solution of trifluoroacetic anhydride in dichloromethane was slowly added dropwise to the above reaction solution such that the temperature of the reaction solution was maintained at -10°C or below during the addition. Then, the reaction solution was slowly warmed to room temperature, and reacted overnight. After the reaction was complete, the reaction solution was quenched with water (50 mL), and extracted. The organic phase was collected, washed with saturated brine, dried over anhydrous sodium sulfate, filtered, rotary evaporated to dryness, and separated and purified by column chromatography on silica gel (eluent: petroleum ether:ethyl acetate=4:1) to obtain Compound **32** as a white solid (4.20 g). Yield 84%. ESI-MS *m*/*z* 475.8 (⁷⁹Br) and 477.8 (⁸¹Br) (M - H)⁻.

CuI (19.0 mg, 0.1 mmol), N¹,N²-bis(5-methyl-[1,1'-biphenyl]-2-yl)oxamide (42.0 mg, 0.1 mmol), Compound **32** (0.956 g, 2 mmol) and K3PO4 (0.424 g, 2 mmol) were sequentially added to a 10 mL Schlenk tube equipped with a rotor. Subsequently, 25% aqueous ammonia (0.28 g) and DMSO (2 mL) were sequentially added via a syringe to the Schlenk tube under an argon atmosphere, and the Schlenk tube was sealed. The reaction was continued at room temperature for 24 h. After the reaction was complete, acetic acid (5 mL) was added, and stirred for another 8 h. The reaction solution was diluted with ethyl acetate (40 mL), and filtered through diatomaceous earth. The filter cake was washed with ethyl acetate, and the filtrate was distilled under reduced pressure. The residue was purified and separated by column chromatography on silica gel (developing agent: dichloromethane:methanol=30: 1) to obtain Compound **33** as a brown solid (0.40 g, yield 51%). ¹H NMR (500 MHz, cdcl₃) δ = 10.39 (s, 1H), 7.75 (s, 1H), 7.59 (s, 1H), 7.39 (s, 1H), 4.87-4.79 (m, 1H), 4.79-4.72 (m, 1H), 4.24 (t, *J=* 9.0, 1H), 3.53 (s, 3H); ESI-MS *m*/*z* 397.0 (M + H)⁺.

Compound **33** (0.4 g) was added to a single-neck flask equipped with a rotor, and was added tetrahydrofuran (4 mL) to dissolve with stirring. Then, anhydrous K₂CO₃ (0.42 g) and H₂O (4 mL) were added, and stirred overnight at room temperature. After the reaction was complete, the reaction solution was extracted with ethyl acetate (15 mL) and H₂O (15 mL). The organic phase was collected, washed with saturated saline, dried over anhydrous sodium sulfate, and rotary evaporated to dryness, to obtain Compound **II-11** (0.3 g, yield 99%). ¹H NMR (400 MHz, DMSO-*d6*) δ = 7.74 (s, 1H), 7.45 (s, 1H), 4.28 (dd, *J* = 9.9, 7.6, 1H), 3.99 (dd, *J* = 11.4, 10.1, 1H), 3.68 (dd, *J* = 11.5, 7.6, 1H), 3.37 (s, 3H); ESI-MS *m*/*z* 301.3 (M + H)⁺.

### Example 13: Synthesis of Compound 11-14

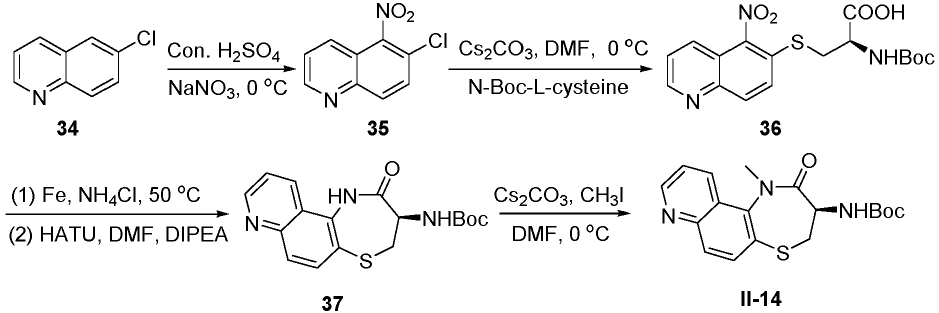

NaNO₃ (5.2 g, 61.1 mmol) was placed into a 500 mL reaction flask, and then concentrated sulfuric acid (60 mL) was added dropwise at 0°C. 6-chloroquinoline (10.0 g, 61.1 mmol) was dissolved in concentrated sulfuric acid (60 mL), and then the resulting solution was slowly added dropwise to the solution of NaNO₃ in concentrated sulfuric acid, and reacted at 0 °C for 2 h until the reaction was completed as indicated by TLC. The reaction solution was poured into an ice-water mixture (500 mL), and filtered through filter paper under suction. The filtrate was extracted with EA. The organic phase was combined with the solid phase obtained by suction filtration, washed with saturated NaHCO₃ and saturated NaCl, dried over Na₂SO₄, concentrated, and the residue was separated by column chromatography (EA: PE= 1: 8) to obtain **35:** pale yellow solid (11.2 g, 88%). ¹H NMR (400 MHz, CDCl₃) δ 9.01 (dd, *J* = 4.2, 1.5 Hz, 1H), 8.19 (d, *J* = 9.1 Hz, 1H), 8.04 (d, *J* = 8.6 Hz, 1H), 7.75 (d, *J* = 9.1 Hz, 1H), 7.59 (dd, *J* = 8.7, 4.2 Hz, 1H).

N-Boc-L-cysteine (3.5 g, 15.8 mmol) and Cs₂CO₃ (10.3 g, 31.6 mmol) were placed into a 250 mL reaction flask, added with DMF (40 mL) and reacted for 15 min at 0°C. Subsequently, a solution of Compound **35** (2.2 g, 10.5 mmol) in DMF (20 mL) was slowly added dropwise, and reacted at 0°C for 24 h, until the reaction was completed as indicated by TLC. The reaction solution was acidified with 0.2 M HCl, extracted with EA, washed with deionized water and saturated NaCl, and dried over Na₂SO₄. The solvent was removed under reduced pressure, and the residue was separated by column chromatography (DCM : MeOH = 20:1) to obtain **36:** pale yellow solid (4.2 g, qunt.). ¹H NMR (400 MHz, CDCl₃) δ 8.83 - 8.67 (m, 1H), 8.05 (d, *J* = 9.1 Hz, 1H), 7.88 (d, *J* = 8.5 Hz, 1H), 7.74 (d, *J* = 9.1 Hz, 1H), 7.34 (dd, *J* = 8.7, 4.5 Hz, 1H), 5.74 (d, *J* = 6.8 Hz, 1H), 4.74 (dd, *J* = 6.3, 3.1 Hz, 1H), 3.99 (dd, *J* = 15.0, 2.5 Hz, 1H), 3.60 (dd, *J* = 15.0, 3.6 Hz, 1H), 1.48 (s, 9H).

Compound **36** (2.0 g, 5.09 mmol) was placed into a 100 mL reaction flask, and Fe (1.42 g, 25.4 mmol) and NH₄Cl (0.43 g, 8.14 mmol) were added. Then EtOH (40 mL) and H₂O (10 mL) were added, and reacted at 50°C for 2 h, until the reaction was completed as indicated by TLC. The reaction solution was filtered through diatomaceous earth. The solvent was removed under reduced pressure, and the residue was re-dissolved in DMF (80 mL). HATU (2.3 g, 6.11 mmol) and DIEA (1.77 mL, 10.2 mmol) were added to the solution, and reacted at room temperature for 30 min, until the reaction was completed as indicated by TLC. The reaction solution was extracted with EA, washed with deionized water and saturated NaCl, and dried over Na₂SO₄. The solvent was removed under reduced pressure, and the residue was separated by fast column chromatography (EA : PE = 1:3) to obtain **37** as a pale yellow solid (1.2 g, yield of 2 steps: 68%).

Compound **37** (1.0 g, 2.9 mmol) was placed into a reaction flask, and then Cs₂CO₃ (1.1 g, 3.48 mmol) and DMF (60 mL) were added and reacted at 0°C for 10 min. Iodomethane (494 mg, 3.48 mmol) was slowly added dropwise to the solution, and reacted at 0°C for 3 h, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:4) to obtain Compound **II-14:** white solid (0.95 g, 91%). ¹H NMR (400 MHz, CDCl₃) δ 8.98 (dd, *J* = 4.2, 1.6 Hz, 1H), 8.18 (d, *J* = 8.5 Hz, 1H), 8.05 (d, *J* = 8.7 Hz, 1H), 7.88 (d, *J* = 8.7 Hz, 1H), 7.52 (dd, *J* = 8.6, 4.2 Hz, 1H), 5.59 (d, *J* = 7.8 Hz, 1H), 4.38 (dt, *J* = 10.6, 7.6 Hz, 1H), 3.82 (dd, *J* = 11.2, 7.0 Hz, 1H), 3.39 (s, 3H), 2.91 (dd, *J* = 19.8, 8.7 Hz, 1H), 1.36 (s, 9H). ¹³C NMR (100 MHz, CDCl₃) δ 172.66, 154.45, 151.37, 149.42, 141.92, 134.89, 131.82, 130.13, 127.13, 124.21, 122.23, 80.18, 50.78, 39.98, 36.76, 28.26.

### Example 14: Synthesis of Compound II-15

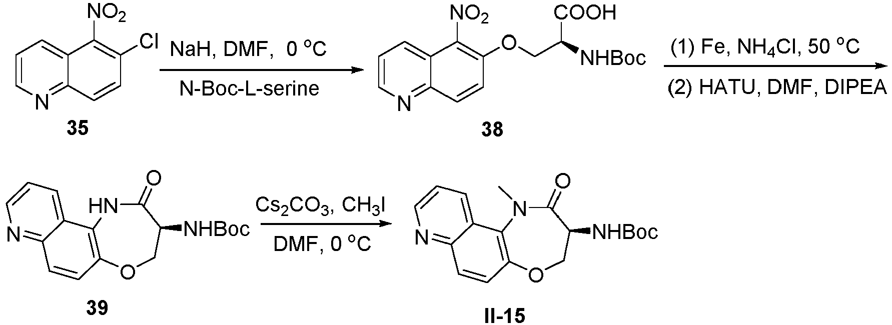

N-Boc-L-serine (3.25 g, 15.8 mmol) and NaH (0.76 g, 31.65 mmol, 60 wt%) were placed into a 250 mL reaction flask, and purged three times with argon. Under the argon atmosphere, DMF (50 mL) was added at 0°C and reacted for 30 min. Then a solution of **35** (2.2 g, 10.55 mmol) in DMF (10 mL) was slowly added dropwise, and reacted overnight at 0°C, until the reaction was completed as indicated by TLC. The reaction solution was acidified with 0.2 M HCl, extracted with EA, washed with deionized water and saturated NaCl, and dried over Na₂SO₄. The solvent was removed under reduced pressure, and the residue was separated by column chromatography (DCM : MeOH = 20:1) to obtain **38:** pale yellow solid 2.1 g (53%). ¹H NMR (400 MHz, MeOD) δ 8.89 (m, 1H), 8.26 (d, *J* = 9.1 Hz, 1H), 8.17 (d, *J* = 8.1 Hz, 1H), 7.89 (d, *J* = 9.0 Hz, 1H), 7.69 (m, 1H), 4.68 (s, 2H), 4.60 (s, 1H), 1.46 (s, 9H).

Compound **38** (1.92 g, 5.09 mmol) was placed into a 100 mL reaction flask, and Fe (1.42 g, 25.4 mmol) and NH₄Cl (0.43 g, 8.14 mmol) were added. Then EtOH (40 mL) and H₂O (10 mL) were added, and reacted at 50°C for 2 h, until the reaction was completed as indicated by TLC. The reaction solution was filtered through diatomaceous earth. The solvent was removed under reduced pressure, and the residue was re-dissolved in DMF (80 mL). HATU (2.3 g, 6.11 mmol) and DIEA (1.77 mL, 10.2 mmol) were added to the solution, and reacted at room temperature for 30 min, until the reaction was completed as indicated by TLC. The reaction solution was extracted with EA, washed with deionized water and saturated NaCl, and dried over Na₂SO₄. The solvent was removed under reduced pressure, and the residue was separated by fast column chromatography (EA : PE = 1:3) to obtain **37** as a pale yellow solid (1.1 g, yield of 2 steps: 66%).

Compound **39** (1.0 g, 3.04 mmol) was placed into a reaction flask, and then Cs₂CO₃ (1.48 g, 4.56 mmol) and DMF (60 mL) were added and reacted at 0°C for 10 min. Iodomethane (518 mg, 3.65 mmol) was slowly added dropwise to the solution, and reacted at 0°C for 3 h, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:4) to obtain Compound **II-15:** white solid (0.96 g, 92%). ¹H NMR (400 MHz, CDCl₃) δ 8.90 (dd, *J* = 4.1, 1.4 Hz, 1H), 8.12 (d, *J* = 8.4 Hz, 1H), 8.04 (d, *J* = 9.1 Hz, 1H), 7.51 (d, *J* = 9.1 Hz, 1H), 7.46 (dd, *J* = 8.6, 4.2 Hz, 1H), 5.54 (d, *J* = 6.7 Hz, 1H), 4.73 - 4.59 (m, 2H), 4.31 - 4.20 (m, 1H), 3.41 (s, 3H), 1.35 (s, 9H). ¹³C NMR (100 MHz, CDCl₃) δ 171.59, 154.95, 149.94, 149.15, 146.67, 131.16, 130.89, 129.73, 125.11, 124.08, 121.63, 80.30, 78.72, 49.94, 37.26, 28.21.

### Example 15: Synthesis of Compound 11-16

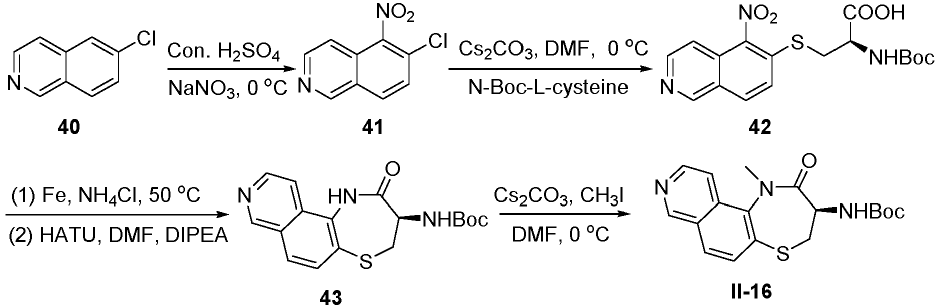

NaNO₃ (5.2 g, 61.1 mmol) was placed into a 500 mL reaction flask, and then concentrated sulfuric acid (60 mL) was added dropwise at 0°C. 6-chloroisoquinoline **40** (10.0 g, 61.1 mmol) was dissolved in concentrated sulfuric acid (60 mL), and the resulting solution was slowly added dropwise to the solution of NaNO₃ in concentrated sulfuric acid, and reacted at 0 °C for 2 h until the reaction was completed as indicated by TLC. The reaction solution was poured into an ice-water mixture (500 mL), and filtered through filter paper under suction. The filtrate was extracted with EA. The organic phase was combined with the solid phase obtained by suction filtration, washed with saturated NaHCO₃ and saturated NaCl, dried over Na₂SO₄, concentrated, and the residue was separated by column chromatography (EA: PE= 1: 8) to obtain **41:** pale yellow solid (11.2 g, 88%). ¹H NMR (400 MHz, CDCl₃) δ 9.36 (s, 1H), 8.73 (d, *J* = 6.0 Hz, 1H), 8.11 (d, *J* = 8.8 Hz, 1H), 7.70 (d, *J* = 8.8 Hz, 1H), 7.56 (d, *J* = 6.0 Hz, 1H).

N-Boc-L-cysteine (3.5 g, 15.8 mmol) and Cs₂CO₃ (10.3 g, 31.6 mmol) were placed into a 250 mL reaction flask, added with DMF (40 mL) and reacted for 15 min at 0°C. Subsequently, a solution of Compound **41** (2.2 g, 10.5 mmol) in DMF (20 mL) was slowly added dropwise, and reacted at 0°C for 24 h, until the reaction was completed as indicated by TLC. The reaction solution was acidified with 0.2 M HCl, extracted with EA, washed with deionized water and saturated NaCl, and dried over Na₂SO₄. The solvent was removed under reduced pressure, and the residue was separated by column chromatography (DCM : MeOH = 20:1) to obtain **42:** pale yellow solid (4.2 g, qunt.). ¹H NMR (400 MHz, CDCl₃) δ 8.52 (s, 1H), 8.32 (d, *J* = 6.3 Hz, 1H), 7.94 (d, *J* = 8.8 Hz, 1H), 7.77 (d, *J* = 8.8 Hz, 1H), 7.44 (d, *J* = 6.3 Hz, 1H), 5.69 (d, *J* = 6.9 Hz, 1H), 4.68 (m, 1H), 3.97 (d, *J* = 14.7 Hz, 1H), 3.55 (dd, *J* = 15.0, 4.4 Hz, 1H), 1.47 (s, 9H).

Compound **42** (2.0 g, 5.09 mmol) was placed into a 100 mL reaction flask, and Fe (1.42 g, 25.4 mmol) and NH₄Cl (0.43 g, 8.14 mmol) were added. Then EtOH (40 mL) and H₂O (10 mL) were added, and reacted at 50°C for 2 h, until the reaction was completed as indicated by TLC. The reaction solution was filtered through diatomaceous earth. The solvent was removed under reduced pressure, and the residue was re-dissolved in DMF (80 mL). HATU (2.3 g, 6.11 mmol) and DIEA (1.77 mL, 10.2 mmol) were added to the solution, and reacted at room temperature for 30 min, until the reaction was completed as indicated by TLC. The reaction solution was extracted with EA, washed with deionized water and saturated NaCl, and dried over Na₂SO₄. The solvent was removed under reduced pressure, and the residue was separated by flash column chromatography (EA : PE = 1:3) to obtain **43** as a pale yellow solid (1.2 g, yield of 2 steps: 68%).

Compound **43** (1.0 g, 2.9 mmol) was placed into a reaction flask, and then Cs₂CO₃ (1.1 g, 3.48 mmol) and DMF (60 mL) were added and reacted at 0°C for 10 min. Iodomethane (494 mg, 3.48 mmol) was slowly added dropwise to the solution, and reacted at 0°C for 3 h, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:4) to obtain Compound **II-16:** white solid (0.95 g, 91%). ¹H NMR (400 MHz, CDCl₃) δ 9.31 (s, 1H), 8.64 (d, *J* = 6.0 Hz, 1H), 7.91 (d, *J* = 8.4 Hz, 1H), 7.78 (d, *J* = 8.4 Hz, 1H), 7.62 (d, *J* = 6.0 Hz, 1H), 5.63 (d, *J* = 7.7 Hz, 1H), 4.37 (m, 1H), 3.82 (dd, *J* = 11.1, 7.0 Hz, 1H), 3.39 (s, 3H), 2.95 (dd, *J* = 12.5, 9.7 Hz, 1H), 1.35 (s, 9H). ¹³C NMR (100 MHz, CDCl₃) δ 172.29, 154.43, 153.10, 144.60, 141.08, 133.02, 131.67, 131.49, 129.73, 128.01, 116.00, 80.25, 50.62, 39.84, 36.41, 28.26.

### Example 16: Synthesis of Compound 11-17

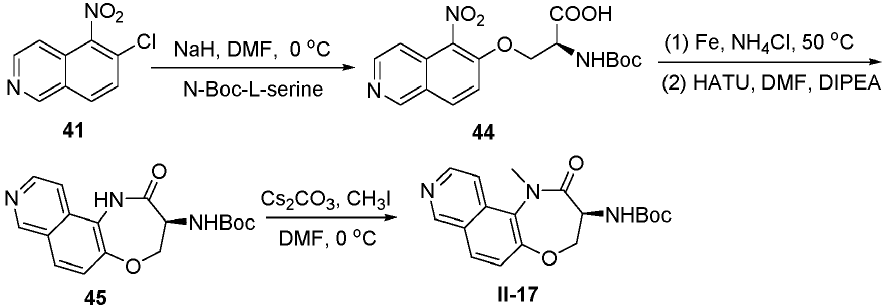

N-Boc-L-serine (3.25 g, 15.8 mmol) and NaH (0.76 g, 31.65 mmol, 60 wt%) were placed into a 250 mL reaction flask, and purged three times with argon. Under the argon atmosphere, DMF (50 mL) was added at 0°C and reacted for 30 min. Then, a solution of **41** (2.2 g, 10.55 mmol) in DMF(10 mL) was slowly added dropwise, and reacted overnight at 0°C, until the reaction was completed as indicated by TLC. The reaction solution was acidified with 0.2 M HCl, then extracted with EA, washed with deionized water and saturated NaCl, and dried over Na₂SO₄. The solvent was removed under reduced pressure, and the residue was separated by column chromatography (DCM : MeOH = 20:1) to obtain **44:** pale yellow solid (2.1 g, 53%).

Compound **44** (1.92 g, 5.09 mmol) was placed into a 100 mL reaction flask, and Fe (1.42 g, 25.4 mmol) and NH₄Cl (0.43 g, 8.14 mmol) were added. Then EtOH (40 mL) and H₂O (10 mL) were added, and reacted at 50°C for 2 h, until the reaction was completed as indicated by TLC. The reaction solution was filtered through diatomaceous earth. The solvent was removed under reduced pressure, and the residue was re-dissolved in DMF (80 mL). HATU (2.3 g, 6.11 mmol) and DIEA (1.77 mL, 10.2 mmol) were added to the solution, and reacted at room temperature for 30 min, until the reaction was completed as indicated by TLC. The reaction solution was extracted with EA, washed with deionized water and saturated NaCl, and dried over Na₂SO₄. The solvent was removed under reduced pressure, and the residue was separated by fast column chromatography (EA : PE = 1:3) to obtain **45** as a pale yellow solid (1.1 g, yield of 2 steps: 66%).

Compound **45** (1.0 g, 3.04 mmol) was placed into a reaction flask, and then Cs₂CO₃ (1.48 g, 4.56 mmol) and DMF (60 mL) were added and reacted at 0°C for 10 min. Iodomethane (518 mg, 3.65 mmol) was slowly added dropwise to the solution, and reacted for 3 h at 0°C, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:4) to obtain Compound **II-17:** white solid (0.96 g, 92%). ¹H NMR (400 MHz, CDCl₃) δ 9.27 (s, 1H), 8.60 (d, *J* = 6.0 Hz, 1H), 7.94 (d, *J* = 8.8 Hz, 1H), 7.60 (d, *J* = 6.0 Hz, 1H), 7.42 (d, *J* = 8.7 Hz, 1H), 5.53 (d, *J* = 6.2 Hz, 1H), 4.76 - 4.60 (m, 2H), 4.36 - 4.25 (m, 1H), 3.44 (s, 3H), 1.38 (s, 9H). ¹³C NMR (100 MHz, CDCl₃) δ 171.24, 154.94, 152.95, 152.31, 144.12, 132.01, 129.26, 129.21, 127.13, 123.44, 115.74, 80.40, 78.75, 49.79, 36.92, 28.23.

### Example 17: Synthesis of Compound II-18

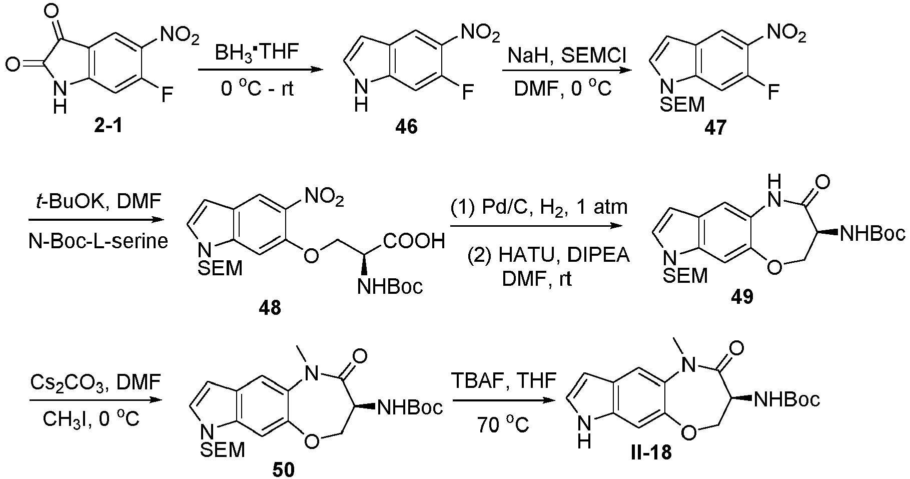

Compound **2-1** (9.48 g, 45.12 mmol) was dissolved in THF (90 mL), and BH₃(135.6 mL, 1 M in THF) was added at 0°C under an argon atmosphere. The reaction solution was warmed to room temperature, and reacted for 2 h until the reaction was completed as indicated by TLC. 1 M HCl was added to quench the reaction, and then the reaction solution was extracted with ethyl acetate, washed with saturated saline, dried over anhydrous sodium sulfate, and the residue was separated by column chromatography (EA/PE = 1:3), to obtain Compound **46** (6.0 g, yield 74%).

Compound **46** (6.0 g, 33.3 mmol) and NaH (1.68 g, 41.64 mmol) were mixed, dissolved in DMF (120 mL) at 0°C, and the temperature was maintained to react for 30 min. Subsequently, SEMC1 (8.82 mL, 49.98 mmol) was added and reacted overnight, until the reaction was completed as indicated by TLC. 1 M HCl was added to quench the reaction, and then the reaction solution was extracted with ethyl acetate, washed with saturated saline, dried over anhydrous sodium sulfate, and the residue was separated by column chromatography (EA/PE = 1:8), to obtain Compound **47** (8.22 g, yield 80%). ¹H NMR (400 MHz, CDCl₃) δ 8.46 (d, *J* = 7.2 Hz, 1H), 7.48 - 7.20 (m, 2H), 6.71 (dd, *J* = 3.4, 0.7 Hz, 1H), 5.50 (s, 2H), 3.52 (dd, *J* = 8.6, 7.7 Hz, 2H), 0.97 - 0.92 (m, 2H), -0.00 (s, 9H). ¹³C NMR (100 MHz, CDCl₃) δ 155.46, 152.91, 140.01, 139.90, 132.89, 132.86, 125.77, 121.15, 121.14, 105.86, 100.31, 100.05, 77.66, 67.93, 19.17, 0.00.

N-Boc-L-serine (4.96 g, 24.15 mmol) and *t*-BuOK (7.23 g, 64.5 mmol) were placed into a 250 mL reaction flask, and purged three times with nitrogen. Under the argon atmosphere, DMF (50 mL) was added at 0°C and reacted for 30 min. Then, a solution of **47** (5.0 g, 16.1 mmol) in DMF (30 mL) was slowly added dropwise, and reacted overnight at 0°C, until the reaction was completed as indicated by TLC. The reaction solution was acidified with 0.2 M HCl, then extracted with EA, washed with deionized water and saturated NaCl, and dried over Na₂SO₄. The solvent was removed under reduced pressure, and the residue was separated by column chromatography (DCM : MeOH = 20:1) to obtain **48:** pale yellow solid (1.67 g, 21%).

Compound **48** (1.67 g) was dissolved in methanol (40 mL), and then Pd/C (167 mg) was added. The reaction was continued for 3 h at room temperature under a hydrogen carrying atmosphere, until the reaction was completed as indicated by TLC. Pd/C was removed by filtering through diatomaceous earth, and the solvent was removed by distillation under reduced pressure. The obtained crude product was dissolved in DMF (40 mL), and then DIPEA and HATU were added and reacted at room temperature for 1 h, until the reaction was completed as indicated by TLC. Then water was added, and the system was extracted with ethyl acetate, concentrated, purified by flash column chromatography (EA/PE = 1:3), to obtain Compound **49** (1.0 g, yield of two steps: 66%).

Compound **49** (1.0 g, 2.23 mmol) was placed into a reaction flask, and then Cs₂CO₃ (1.09 g, 3.36 mmol) and DMF (20 mL) were added and reacted at 0°C for 10 min. Iodomethane (380 mg, 2.68 mmol) was slowly added dropwise to the solution, and reacted at 0°C for 3 h, until the reaction was completed as indicated by TLC. The reaction solution was extracted with EA, washed with deionized water and saturated NaCl, and dried over Na₂SO₄. The solvent was removed under reduced pressure, and the residue was separated by column chromatography (EA : PE = 1:8) to obtain Compound **50:** white solid (0.96 g, 93%). ¹H NMR (400 MHz, CDCl₃) δ 7.44 (s, 1H), 7.29 (s, 1H), 7.21 (d, *J* = 3.2 Hz, 1H), 6.50 (d, *J*= 3.1 Hz, 1H), 5.57 (d, *J* = 6.8 Hz, 1H), 5.50 - 5.39 (m, 2H), 4.70 (dt, *J* = 11.0, 7.3 Hz, 1H), 4.59 (dd, *J* = 9.5, 7.5 Hz, 1H), 4.15 (dd, *J* = 10.9, 9.8 Hz, 1H), 3.54 - 3.47 (m, 5H), 1.41 (s, 9H), 1.00 - 0.91 (m, 2H), 0.00 (s, 9H). ¹³C NMR (100 Hz, CDCl₃) δ 171.03, 156.49, 147.69, 136.09, 131.71, 130.81, 127.63, 116.59, 105.00, 103.74, 81.39, 79.21, 77.27, 67.38, 51.47, 37.56, 29.65, 19.06, 0.00.

Under an argon atmosphere, Compound **50** (0.9 g, 1.95 mmol) was dissolved in anhydrous THF (15 mL), and then a TBAF solution (13.65 mL, 1 M in THF) was added, and reacted at 70 °C for 4 h. After the reaction was completed, THF was removed, and water was added. The reaction solution was extracted 3 times with ethyl acetate. The organic phases were combined, dried over anhydrous sodium sulfate, concentrated, and the residue was separated by flash column chromatography (EA/PE = 1:3) to obtain Compound **II-18** (210 mg, yield 33%). ESI-MS *m*/*z* 330.1 (M-H)⁻.

### Example 18: Synthesis of Compound II-23

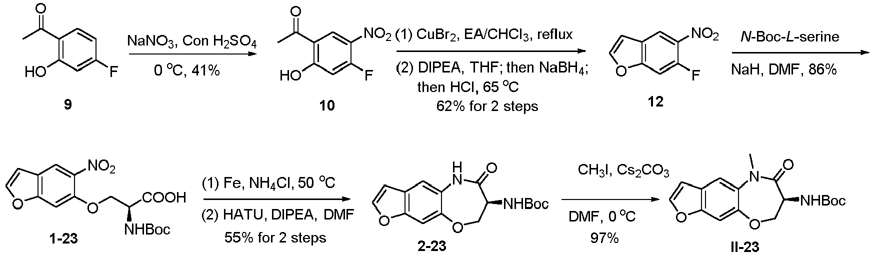

NaNO₃ (5.1 g, 60 mmol) was placed into a 500 mL reaction flask, and then concentrated sulfuric acid (60 mL) was added dropwise at 0°C. 4-fluoro-2-hydroxyacetophenone **9** (9.25 g, 60 mmol) was dissolved in concentrated sulfuric acid (60 mL), and then the resulting solution was slowly added dropwise to the solution of NaNO₃ in concentrated sulfuric acid, and reacted at 0 °C for 2.5 h until the reaction was completed as indicated by TLC. The reaction solution was poured into an ice-water mixture (500 mL), and filtered through filter paper under suction. The filtrate was extracted with ethyl acetate. The organic phase was mixed with the solid phase obtained by suction filtration, washed with saturated sodium bicarbonate and saturated saline sequentially, dried over anhydrous sodium sulfate, concentrated, and the residue was separated by column chromatography (EA/ PE= 1:20 AM) to obtain **10:** pale yellow solid (4.9 g, 41%). Compound **10:** ¹H NMR (400 MHz, CDCl₃) δ 13.02 (s, 1H), 8.65 (d, *J* = 8.3 Hz, 1H), 6.83 (d, *J* = 12.2 Hz, 1H), 2.72 (s, 3H); ¹³C NMR (100 MHz, CDCl₃) δ 203.1, 168.4, 168.3, 161.8, 159.1, 130.5, 115.9, 115.9, 107.6, 107.4, 26.8; ESI-MS: m/z 200.0 (M+H)⁺.

CuBr₂ (1.9 g, 8.5 mmol) was placed into a 250 mL reaction flask, and then ethyl acetate (50 mL) was added, and heated to reflux. A solution of **10** (1.0 g, 5 mmol) in chloroform (50 mL) was added to the reaction solution, and reacted under reflux for 24 h. The green color of the reaction liquid faded, and a lime-colored solid was formed. After **10** was mostly converted as indicated by TLC, the reaction solution was filtered through diatomaceous earth under suction. The solvent was removed under reduced pressure, and the obtained crude product was dissolved in THF (180 mL). DIPEA (3.0 mL, 18 mmol) was slowly added, and reacted at room temperature for 45 min until the reaction was completed as indicated by TLC. NaBH₄ was added to the system, and reacted at room temperature for 2 h. After the reaction was completed, the reaction was quenched by slowly adding methanol, and then 4 N HCl was added and reacted overnight at 65°C. The reaction solution was extracted with ethyl acetate, washed with saturated saline, and dried over anhydrous sodium sulfate. Separation by column chromatography (EA/PE = 1:10) afforded **12:** yellow solid (0.56 g, yield of two steps 62%). Compound **12:** ¹H NMR (400 MHz, CDCl₃) δ 8.37 (d, *J* = 7.3 Hz, 1H), 7.78 (d, *J* = 2.3 Hz, 1H), 7.42 (d, *J* = 10.6 Hz, 1H), 6.91 (dd, *J* = 2.2, 0.7 Hz, 1H); ¹³C NMR (100 MHz, CDCl₃) δ 156.8, 156.7, 155.2, 152.6, 148.5, 148.4, 123.5, 123.5, 119.1, 119.1, 107.2, 107.2, 101.6, 101.3; ESI-MS: m/z 182.0 (M+H)⁺.

N-Boc-L-serine (453 mg, 2.2 mmol) and NaH (105.6 mg, 4.4 mmol) were placed into a 50 mL reaction flask, added with DMF (8 mL) at 0°C and reacted for 15 min. Subsequently, a solution of **12** (200 mg, 1.1 mmol) in DMF (2 mL) was slowly added dropwise, and reacted for 4.5 h at 0°C, until the reaction was completed as indicated by TLC. The reaction solution was acidified with 0.2 M HCl, extracted with ethyl acetate, washed with saturated saline, and dried over anhydrous sodium sulfate. The solvent was removed under reduced pressure, and the residue was separated by column chromatography (DCM : MeOH = 20:1) to obtain **1-23:** 350 mg, yield 86%.

Compound **1-23** (800 mg, 2.18 mmol) was placed into a 100 mL reaction flask, and a Fe powder (610 mg, 10.9 mmol) and NH₄Cl (233 mg, 4.36 mmol) were added. Then EtOH (24 mL) and H₂O (6 mL) were added, and reacted at 50°C for 4 h, until the reaction was completed as indicated by TLC. The reaction solution was filtered through diatomaceous earth. The solvent was removed under reduced pressure, and the residue was re-dissolved in DMF (30 mL). HATU (995 mg, 2.62 mmol) and DIPEA (1.52 mL, 8.72 mmol) were added to the solution, and reacted overnight at room temperature, until the reaction was completed as indicated by TLC. The reaction solution was extracted with ethyl acetate, washed with saturated saline, and dried over anhydrous sodium sulfate. The solvent was removed under reduced pressure, and the residue was separated by flash column chromatography (EA : PE = 1:3) to obtain Compound **2-23** (380 mg, yield of 2 steps: 55%).

Compound **2-23** (380 mg, 1.2 mmol) was dissolved in DMF (10 mL), and Cs₂CO₃ (584 mg, 1.8 mmol) was added. Iodomethane (203 mg, 1.43 mmol) was slowly added dropwise to the solution and reacted at room temperature for 2 h, until the reaction was completed as indicated by TLC. The reaction solution was extracted with ethyl acetate, washed with saturated saline, and dried over anhydrous sodium sulfate. The solvent was removed under reduced pressure, and the residue was separated by flash column chromatography (EA/PE = 1:4) to obtain **II-23:** white solid 385 mg, yield 97%. ¹H NMR (400 MHz, CDCl₃) δ 7.66 (d, *J* = 2.2 Hz, 1H), 7.41 (s, 1H), 7.31 (s, 1H), 6.74 (d, *J* = 1.4 Hz, 1H), 5.51 (d, *J* = 7.0 Hz, 1H), 4.65 (dt, *J* = 11.0, 7.3 Hz, 1H), 4.57 (dd, *J* = 9.5, 7.4 Hz, 1H), 4.15 (dd, *J* = 10.9, 9.7 Hz, 1H), 3.46 (s, 3H), 1.39 (s, 9H); ¹³C NMR (100 MHz, CDCl₃) δ 169.5, 155.1, 152.9, 147.7, 146.6, 132.7, 124.7, 115.2, 106.4, 105.7, 80.2, 77.9, 50.0, 36.1, 28.2; ESI-MS: m/z 333.0 (M+H)⁺.

### Example 19: Synthesis of Compound II-24

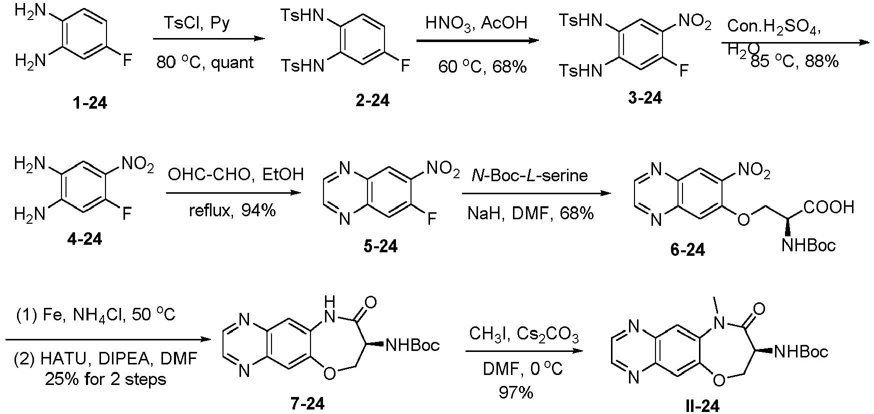

Compound **1-24** (5.04 g, 40 mmol) was dissolved in pyridine (10 mL), and then TsCl (15.25 g, 80 mmol) dissolved in pyridine (25 mL) was slowly added dropwise, heated to 80°C and reacted overnight, until the reaction was completed as indicated by TLC. The reaction solution was cooled to room temperature, and 3 N hydrochloric acid (150 mL) was added with an ice bath. A large amount of solid was precipitated out, which was stirred for 0.5 h and then filtered. The filter cake was an earthy solid. Product **2-24** was obtained (17.4 g, yield: quantitative).

Compound **2-24** (20 g, 46.08 mmol) was dissolved in acetic acid (80 mL), and fuming nitric acid (4.8 mL) was added, heated to 60 °C and reacted for 2 h. After the reaction was completed, the reaction solution was filtered, and then washed with ethanol (40 mL). Compound **3-24** as a white solid was obtained (15 g, yield 68%).

Compound **3-24** (13.4 g, 28 mmol) was added to a mixed system of concentrated sulfuric acid (30 mL) and water (3 mL), heated to 85°C and reacted for 1 h. After cooling, the system was slowly poured into ice water (300 mL), and a solid was precipitated out. The solid was dissolved by slowly heating, adjusted to pH 9 with aqueous ammonia, and filtered. The filter cake was washed with water, and Compound **4-24** as a solid was obtained (4.2 g, yield 88%).

Compound **4-24** (4.2 g, 24.5 mmol) was dissolved in ethanol (120 mL), and oxaldehyde (40% in water, 5.67 mL) was added. The system was heated to 90°C and reacted for 1 h, until the reaction was completed as indicated by TLC. The reaction solution was distilled under reduced pressure to remove ethanol, extracted with ethyl acetate, dried over anhydrous sodium sulfate, and the residue was separated by flash column chromatography (EA/PE = 1:2) to obtain Compound **5-24** as a white solid (4.46 g, yield 94%). ¹H NMR (400 MHz, CDCl₃) δ 9.02-8.97 (m, 2H), 8.86 (d, *J* = 7.5 Hz, 1H), 7.99 (d, *J* = 10.8 Hz, 1H); ¹³C NMR (100 MHz, CDCl₃) δ 155.5, 152.9, 148.4, 146.7, 146.6, 145.3, 145.2, 138.71, 138.69, 128.4, 116.9, 116.7; ESI-MS: m/z 194.0 (M+H)⁺.

N-Boc-L-serine (3.18 g, 15.54 mmol) and NaH (0.93 g, 23.31 mmol) were placed into a 250 mL reaction flask, added with DMF (40 mL) at 0°C and reacted for 15 min. Subsequently, a solution of Compound **5-24** (1.5 g, 7.77 mmol) in DMF (20 mL) was slowly added dropwise, and reacted for 3 h at 0°C, until the reaction was completed as indicated by TLC. The reaction solution was acidified with 0.2 M HCl, extracted with ethyl acetate, washed with saturated saline, and dried over anhydrous sodium sulfate. The solvent was removed under reduced pressure, and the residue was separated by column chromatography (DCM : MeOH = 20:1) to obtain **6-24** (2.0 g, yield 68%).

Compound **6-24** (2.43 g, 6.43 mmol) was placed into a 250 mL reaction flask, and a Fe powder (1.79 g, 32.14 mmol) and NH₄Cl (0.69 g, 12.86 mmol) were added. Then EtOH (80 mL) and H₂O (20 mL) were added, and reacted at 50°C for 4 h, until the reaction was completed as indicated by TLC. The reaction solution was filtered through diatomaceous earth. The solvent was removed under reduced pressure, and the residue was re-dissolved in DMF (30 mL). HATU (2.93 g, 7.72 mmol) and DIPEA (3.32 g, 25.75 mmol) were added to the solution, and reacted overnight at room temperature, until the reaction was completed as indicated by TLC. The reaction solution was extracted with ethyl acetate, washed with saturated saline, and dried over anhydrous sodium sulfate. The solvent was removed under reduced pressure, and the residue was separated by flash column chromatography (EA : PE = 1:3) to obtain Compound **7-24** (540 mg, yield of 2 steps: 25%).

Compound **7-24** (340 mg, 1.03 mmol) was dissolved in DMF (10 mL), and Cs₂CO₃ (503 mg, 1.55 mmol) was added. Iodomethane (175 mg, 1.24 mmol) was slowly added dropwise to the solution and reacted at room temperature for 2 h, until the reaction was completed as indicated by TLC. The reaction solution was extracted with ethyl acetate, washed with saturated saline, and dried over anhydrous sodium sulfate. The solvent was removed under reduced pressure, and the residue was separated by flash column chromatography (EA/PE = 1:4) to obtain **II-24:** white solid 345 mg, yield 97%. ¹H NMR (400 MHz, CDCl₃) δ 8.84 (d, *J* = 1.8 Hz, 1H), 8.82 (d, *J* = 1.8 Hz, 1H), 7.92 (s, 1H), 7.85 (s, 1H), 4.73 (dt, *J* = 11.3, 7.2 Hz, 1H), 4.65 (dd, *J* = 9.5, 7.2 Hz, 1H), 4.26 (dd, *J* = 11.2, 9.7 Hz, 1H), 3.58 (s, 3H); ¹³C NMR (100 MHz, CDCl₃) δ 169.3, 155.0, 151.5, 145.3, 145.1, 141.9, 140.9, 140.2, 122.5, 121.2, 80.5, 50.1, 36.1, 28.2; ESI-MS: m/z 345.0 (M+H)⁺.

### Example 20: Synthesis of Compound II-25

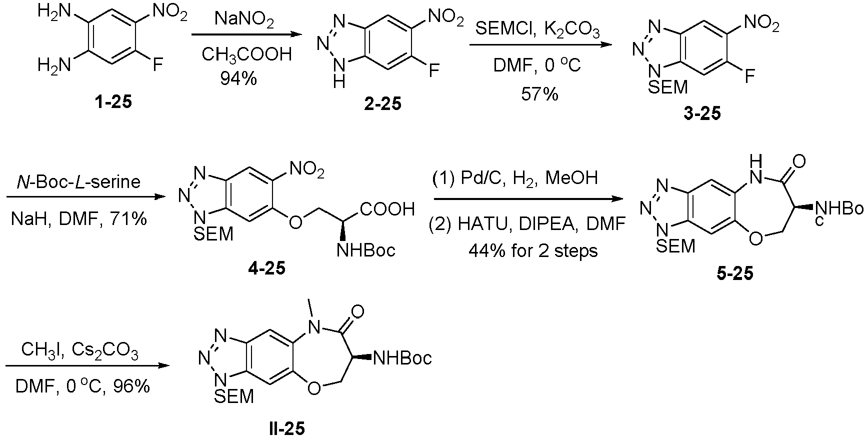

Intermediate Compound **1-25** (4.4 g, 25.71 mmol) was dissolved in acetic acid (100 mL), and then sodium nitrite (3.85 g, 55.79 mmol) was added at 5°C. The temperature was maintained to react for 10 min and the conversion was completed. The reaction solution was rotary evaporated to remove acetic acid, extracted with ethyl acetate, and concentrated, to obtain Compound **2-25** as a solid (4.37 g, yield 94%).

Compound **2-25** (4.0 g, 21.98 mmol) was dissolved in acetic acid (80 mL), and then K₂CO₃ (6.07g, 44 mmol) and SEMCl (5.5 g, 33 mmol) were added at 0°C. The reaction was kept at 0°C for 2 h and the conversion was completed. The reaction solution was extracted with ethyl acetate, washed with saturated saline, dried over anhydrous sodium sulfate, concentrated, and purified by column chromatography (EA/PE = 1:6), to obtain Compound **3-25** (3.9 g, yield 57%).

N-Boc-L-serine (5.11 g, 25 mmol) and NaH (1.5 g, 37.5 mmol) were placed into a 250 mL reaction flask, added with DMF (60 mL) at 0°C and reacted for 15 min. Subsequently, a solution of Compound **3-25** (3.9 g, 12.5 mmol) in DMF (20 mL) was slowly added dropwise, and reacted for 2 h at 0°C until the reaction was completed as indicated by TLC. The reaction solution was acidified with 0.2 M HCl, extracted with ethyl acetate, washed with saturated saline, and dried over anhydrous sodium sulfate. The solvent was removed under reduced pressure, and the residue was separated by column chromatography (DCM : MeOH = 20:1) to obtain Compound **4-25** as a yellow solid powder (4.4 g, yield 71%).

Compound **4-25** (4.3 g, 8.65 mmol) was dissolved in methanol (80 mL), and then 10% Pd/C (2.15 g) was added. Under a hydrogen atmosphere, the reaction was continued for 4 h at normal temperature under normal pressure, until the raw material was completely converted. The reaction solution was filtered through diatomaceous earth to remove Pd/C, and concentrated to remove methanol. The obtained crude product was dissolved in DMF (30 mL), and then DIPEA (2.24 g, 17.3 mmol) and HATU (3.95 g, 10.38 mmol) were added, and reacted for 1 h with stirring at room temperature, until the reaction was completed. The reaction solution was extracted with ethyl acetate, washed with saturated saline, and dried over anhydrous sodium sulfate. The solvent was removed under reduced pressure, and the residue was separated by column chromatography (EA/PE = 1:3), to obtain Compound **5-25** (1.7 g, yield of 2 steps: 44%).

Compound **5-25** (1.7 g, 3.79 mmol) was dissolved in DMF (40 mL), and Cs₂CO₃ (1.85 g, 5.68 mmol) was added. Iodomethane (0.59 g, 4.17 mmol) was slowly added dropwise to the solution and reacted at room temperature for 2 h, until the reaction was completed as indicated by TLC. The reaction solution was extracted with ethyl acetate, washed with saturated saline, and dried over anhydrous sodium sulfate. The solvent was removed under reduced pressure, and separated by fast column chromatography (EA/PE = 1:4) to obtain **II-25:** white solid 1.69 g, yield 96%. ¹H NMR (400 MHz, CDCl₃) δ 7.93 (s, 1H), 7.49 (s, 1H), 5.98 (s, 2H), 5.60 (d, *J* = 7.0 Hz, 1H), 4.69 (dt, *J* = 11.2, 7.2 Hz, 1H), 4.60 (ddd, *J* = 11.2, 7.4, 3.8 Hz, 1H), 4.22 (dd, *J* = 11.1, 9.7 Hz, 1H), 3.66-3.57 (m, 2H), 3.54 (s, 3H), 1.41 (s, 9H), 1.02-0.94 (m, 2H), 0.00 (s, 9H); ¹³C NMR (100 MHz, CDCl₃) δ 170.6, 156.5, 152.2, 144.8, 136.6, 132.7, 115.5, 104.8, 81.8, 68.8, 51.3, 37.9, 29.7, 19.1, 0.0; ESI-MS: m/z 464.1 (M+H)⁺.

### Example 21: Synthesis of Compound II-19

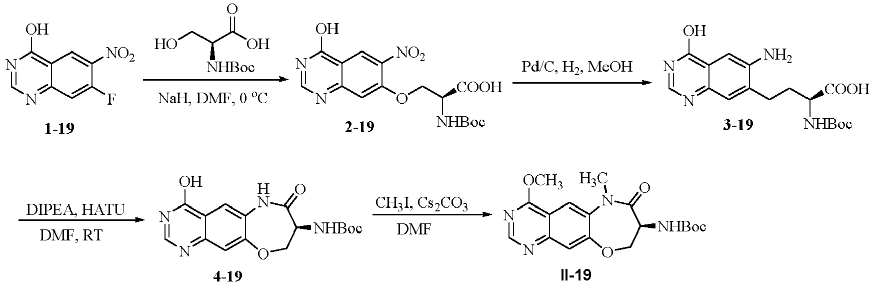

N-Boc-L-Serine (9.80 g, 47.80 mmol) and NaH (3.80 g, 95.60 mmol) were added to a 500 mL reaction flask, and then DMF (120 mL) was added and reacted at 0°C for 30 min. **1-19** (5.00 g, 23.9 mmol) was dissolved in DMF (100 mL), then slowly added dropwise to the reaction solution, and reacted at 0°C for 3.5 h. After the reaction was complete, the reaction solution was washed with saturated NaCl solution, and extracted with ethyl acetate. The organic phase was washed with saturated NaCl solution, dried over anhydrous sodium sulfate, rotary evaporated to dryness, and the residue was separated by column chromatography on silica gel (eluent: dichloromethane:methanol=10:1), to obtain **2-19** (4.99 g, yield 53%). ESI-MS *m*/*z* 393.0 (M-H)⁻.

**2-19** (900 mg, 2.28 mmol) and Pd/C (900 mg) were added to a 100 mL reaction flask, and then were added methanol (20 mL) to dissolve. The reaction was continued for 5 h at room temperature under a hydrogen atmosphere. After the reaction was complete, the reaction solution was filtered through diatomaceous earth to remove Pd/C, and rotary evaporated to dryness to remove the solvent, so as to obtain crude **3-19.** ESI-MS *m*/*z* 363.1 (M-H)⁻.

Crude **3-19,** HATU (1.04 g, 2.74 mmol), and DIPEA (755 µL,4.57 mmol) were added to a 100 mL reaction flask, and then DMF (20 mL) was added to dissolve them. The reaction was continued overnight at 75°C. After the reaction was complete, the reaction solution was washed with saturated NaCl solution, and extracted with ethyl acetate. The organic phase was washed with saturated NaCl solution, dried over anhydrous sodium sulfate, rotary evaporated to dryness, and separated by column chromatography on silica gel (eluent: dichloromethane:methanol=10:1), to obtain **4-19** (155 mg, yield 19.6%). ESI-MS *m*/*z* 347.0 (M+H)⁺.

**4-19** (400 mg, 1.16 mmol) and cesium carbonate (791 mg, 2.43 mmol) were added to a 100 mL reaction flask, and then DMF (15 mL) was added. After reaction at 0°C for 10min, iodomethane (152 µL, 2.43 mmol) was slowly added dropwise and reacted at 0°C for 1 h. After the reaction was complete, the reaction solution was washed with saturated NaCl solution, and extracted with ethyl acetate. The organic phase was washed with saturated NaCl solution, dried over anhydrous sodium sulfate, rotary evaporated to dryness, and separated by column chromatography on silica gel (eluent: dichloromethane:methanol=40:1), to obtain the final product **II-19** (275 mg, yield 64%). ESI-MS *m*/*z* 375.1 (M+H)⁺.

### Example 22: Synthesis of Compound II-26

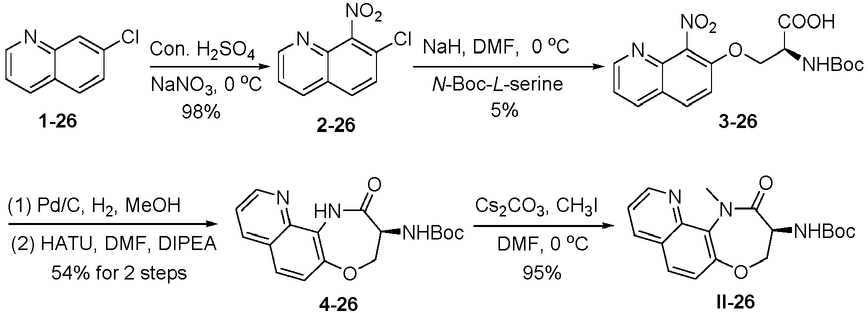

NaNO₃ (2.73 g, 32.1 mmol) was placed into a 100 mL reaction flask, and then concentrated sulfuric acid (15 mL) was added dropwise at 0°C. Compound **1-26** (5.0 g, 30.6 mmol) was dissolved in concentrated sulfuric acid (15 mL), and then the resulting solution was slowly added dropwise to the solution of NaNO₃ in concentrated sulfuric acid, and reacted at 0 °C for 3 h until the reaction was completed as indicated by TLC. The reaction solution was poured into ice water (150 mL), and a large amount of a white solid was precipitated out. The reaction solution was filtered through filter paper, and the filter cake was washed 3 times with ice water, and dried, to obtain Compound **2-36** as a white solid powder (6.25 g, yield 98%). Compound **2-26:** ¹H NMR (400 MHz, CDCl₃) δ 9.01 (dd, *J* = 4.3, 1.6 Hz, 1H), 8.25 (dd, *J* = 8.4, 1.6 Hz, 1H), 7.92 (d, *J* = 8.9 Hz, 1H), 7.61 (d, *J* = 8.9 Hz, 1H), 7.56 (dd, *J* = 8.4, 4.3 Hz, 1H); ¹³C NMR (100 MHz, CDCl₃) δ 153.1, 140.2, 135.9, 130.2, 127.4, 127.2, 125.8, 123.0; ESI-MS: m/z 209.0 (M+H)⁺.

N-Boc-L-serine (5.9 g, 28.8 mmol) and NaH (1.73 g, 43.1 mmol) were placed into a 250 mL reaction flask, added with DMF (40 mL) at 0°C and reacted for 15 min. Subsequently, a solution of Compound **2-26** (3.0 g, 14.4 mmol) in DMF (30 mL) was slowly added dropwise, and reacted overnight at 0°C. The reaction solution was acidified with 0.2 M HCl, extracted with ethyl acetate, washed with saturated saline, and dried over anhydrous sodium sulfate. The solvent was removed under reduced pressure, and the residue was separated by column chromatography (DCM : MeOH = 20:1) to obtain **3-26** as a yellow solid powder: 0.27g, yield 5%.

Compound **3-26** (200 mg, 0.53 mmol) was dissolved in methanol (2 mL), and then 10% Pd/C (100 mg) was added. Under a hydrogen atmosphere, the reaction was continued for 2 h at normal temperature under normal pressure, until the raw material was completely converted. The reaction solution was filtered through diatomaceous earth to remove Pd/C, and concentrated to remove methanol. The obtained crude product was dissolved in DMF (3 mL), and then DIPEA (274.2 mg, 2.12 mmol) and HATU (3.95 g, 0.64 mmol) were added, and reacted for 1 h with stirring at room temperature, until the reaction was completed. The reaction solution was extracted with ethyl acetate, washed with saturated saline, and dried over anhydrous sodium sulfate. The solvent was removed under reduced pressure, and the residue was separated by column chromatography (EA/PE = 1:3), to obtain Compound **4-26** (95 mg, yield of 2 steps: 54%). ESI-MS: m/z 330.0 (M+H)⁺.

Compound **4-26** (40 mg, 0.12 mmol) was dissolved in DMF (5 mL), and Cs₂CO₃ (76 mg, 0.23 mmol) was added. Iodomethane (24.8 mg, 0.18 mmol) was slowly added dropwise to the solution and reacted at room temperature for 2 h, until the reaction was completed as indicated by TLC. The reaction solution was extracted with ethyl acetate, washed with saturated saline, and dried over anhydrous sodium sulfate. The solvent was removed under reduced pressure, and separated by flash column chromatography (EA/PE = 1:4) to obtain **II-26:** white solid 40 mg, yield 95%. ESI-MS: m/z 344.0 (M+H)⁺.

### Example 23: Synthesis of Compound II-27

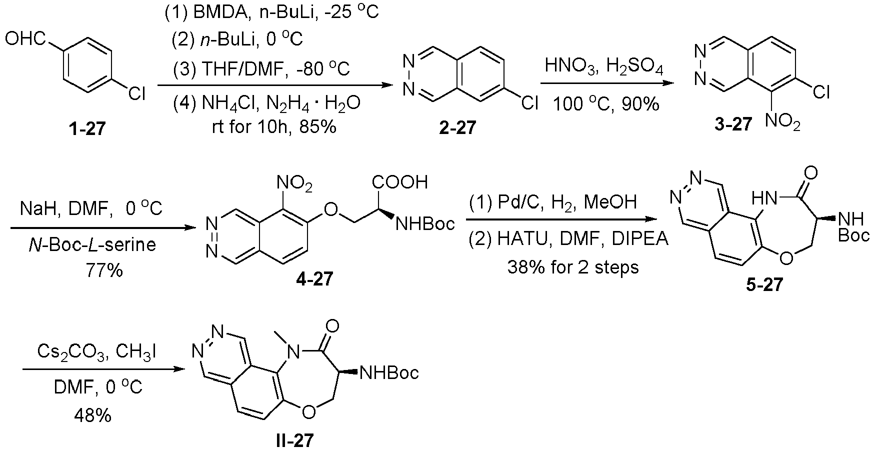

BMDA (5.27 g, 39.6 mmol) was dissolved in a mixed solvent of THF (15 mL) and n-hexane (60 mL), and then *n*-BuLi (24 mL, 1 M in hexane) was added at -25°C. The temperature was maintained to react for 30 min. Compound **1-27** (5.06 g, 36.0 mmol) was added and reacted at -25 °C for 30 min. *n*-BuLi (33.6 mL, 1 M in hexane) was added, and reacted at 0°C for 2 h. Then the system was cooled to -80°C, and then DMF (8.4 mL, 118.8 mmol) was added, and reacted at -80°C for 10 min and then at 0°C for 2 h. NH₄Cl (5.76 g, 118.8 mmol) and N₂H₄·H₂O (4.2 mL, 68.36 mmol) were added, and reacted overnight at room temperature, until the reaction was completed as indicated by TLC. The reaction solution was extracted with ethyl acetate, washed with saturated saline, and dried over anhydrous sodium sulfate. The solvent was removed under reduced pressure, and the residue was separated by column chromatography (EA/PE = 2:1) to obtain Compound **2-27** (5.0 g, yield 85%).

Compound **2-27** (2 g, 12.15 mmol) was dissolved in concentrated sulfuric acid (40 mL), and then fuming nitric acid (3.83 g, 60.75 mmol) was added, heated to 90°C and reacted for 2 h, until the raw materials were completely converted. The reaction solution was cooled to room temperature, poured into ice water (150 mL), and adjusted to a neutral pH with aqueous ammonia. The reaction solution was extracted with ethyl acetate, concentrated, and the residue was separated by flash column chromatography (EA/PE = 2:1) to obtain Compound **3-27** (2.28 g). ¹H NMR (400 MHz, CDCl₃) δ 9.66 (d, *J* = 1.3 Hz, 1H), 9.55 (d, *J* = 1.3 Hz, 1H), 8.15 (d, *J* = 8.7 Hz, 1H), 8.05 (d, *J* = 8.7 Hz, 1H); ESI-MS: m/z 209.0 (M+H)⁺.

N-Boc-L-serine (2.94 g, 14.35 mmol) and NaH (1.15 g, 28.71 mmol) were placed into a 250 mL reaction flask, added with DMF (30 mL) at 0°C and reacted for 15 min. Subsequently, a solution of Compound **3-27** (2.0 g, 9.57 mmol) in DMF (20 mL) was slowly added dropwise, and reacted for 5 h at 0°C. The reaction solution was acidified with 0.2 M HCl, extracted with ethyl acetate, washed with saturated saline, and dried over anhydrous sodium sulfate. The solvent was removed under reduced pressure, and the residue was separated by column chromatography (DCM : MeOH = 10:1) to obtain Compound **4-27** as a yellow solid powder (2.8 g, yield 77%).

Compound **4-27** (1.1 g, 3.18 mmol) was dissolved in methanol (20 mL), and then 10% Pd/C (1.1 g) was added. Under a hydrogen atmosphere, the reaction was continued for 2 h at normal temperature under normal pressure, until the raw material was completely converted. The reaction solution was filtered through diatomaceous earth to remove Pd/C, and concentrated to remove methanol. The obtained crude product was dissolved in DMF (20 mL), and then DIPEA (1.23 g, 9.52 mmol) and HATU (1.45 g, 3.82 mmol) were added, and reacted for 1 h with stirring at room temperature, until the reaction was completed. The reaction solution was extracted with ethyl acetate, washed with saturated saline, and dried over anhydrous sodium sulfate. The solvent was removed under reduced pressure, and the residue was separated by column chromatography (DCM/MeOH = 20:1), to obtain Compound **5-27** (390 mg, yield of 2 steps: 38%). ESI-MS: m/z 331.0 (M+H)⁺.

Compound **5-27** (240 mg, 0.73 mmol) was dissolved in DMF (8 mL), and Cs₂CO₃ (355 mg, 1.09 mmol) was added. Iodomethane (123 mg, 0.87 mmol) was slowly added dropwise to the solution and reacted at room temperature for 2 h, until the reaction was completed as indicated by TLC. The reaction solution was extracted with ethyl acetate, washed with saturated saline, and dried over anhydrous sodium sulfate. The solvent was removed under reduced pressure, and the residue was separated by flash column chromatography (DCM/MeOH = 1:20) to obtain **II-27:** white solid 120 mg, yield 48%. ESI-MS: m/z 345.0 (M+H)⁺.

### Example 24: Synthesis of Compound II-21

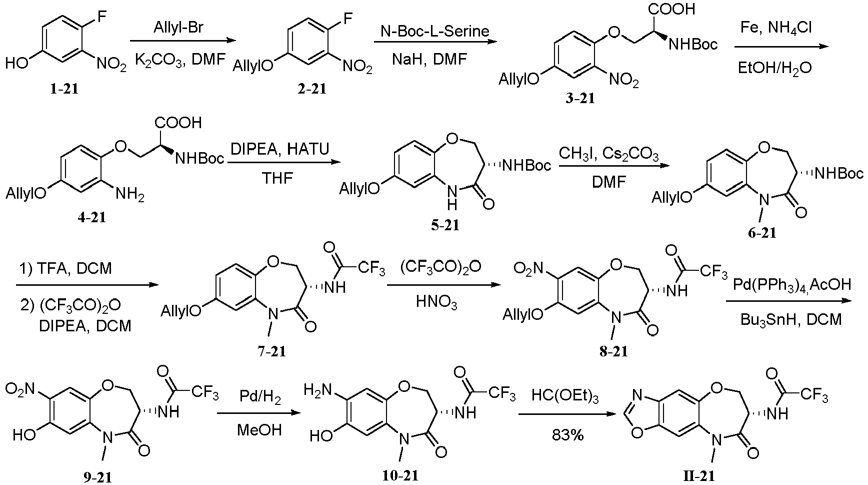

**1-21** (10 g, 63.65 mmol) and potassium carbonate (26.39 g, 190.96 mmol) were added to a 500 mL reaction flask, and then DMF (300 mL) was added. Allyl bromide (17 mL, 190.96 mmol) was added dropwise and reacted at room temperature for 3 h. After the reaction was complete, the reaction solution was washed with saturated NaCl solution, and extracted with ethyl acetate. The organic phase was washed with saturated NaCl solution, dried over anhydrous sodium sulfate, rotary evaporated to dryness, and the residue was separated by column chromatography on silica gel (eluent: petroleum ether: ethyl acetate=5:1), to obtain **2-21** (12.41 g, yield 99%). ESI-MS *m*/*z* 198.0 (M+H)⁺.

N-Boc-L-Serine (7.80 g, 38.06 mmol) and NaH (3.6 g, 88.81 mmol) were added to a 250 mL reaction flask, and then DMF (100 mL) was added as a solvent. The reaction was continued at 0°C for 30 min. **2-21** (5.00 g, 25.37 mmol) was dissolved in DMF (50 mL), then slowly added dropwise to the reaction solution, and reacted at 0°C for 1 h. After the reaction was complete, the reaction solution was washed with saturated NaCl solution, and extracted with ethyl acetate. The organic phase was washed with saturated NaCl solution, dried over anhydrous sodium sulfate, rotary evaporated to dryness, to obtain crude **3-21** (4.99 g, yield 53%). ESI-MS *m*/*z* 381.1 (M-H)⁻.

All the crude product **3-21,** reduced iron powder (7.1 g, 126.87 mmol), and ammonium chloride (2.2 g, 40.6 mmol) were added to a 250 mL reaction flask, and then ethanol/water (120mL/30mL) was added. The reaction was carried out under reflux at 70°C for 1 h. After the reaction was complete, the reaction solution was filtered through diatomaceous earth to remove the iron powder, and the filtrate was rotary evaporated to dryness, so as to obtain crude **4-21.** ESI-MS *m*/*z* 351.1 (M+H)⁺.

All the crude **4-21,** HATU (11.6 g, 30.44 mmol), and DIPEA (17 mL, 101.48 mmol) were added to a 250 mL reaction flask, and then THF (200 mL) was added as a solvent. The reaction was continued at room temperature for 1 h. After the reaction was complete, the reaction solution was rotary evaporated to remove THF, washed with saturated NaCl solution, and extracted with ethyl acetate. The organic phase was washed with saturated NaCl solution, dried over anhydrous sodium sulfate, rotary evaporated to dryness, and the residue was separated by column chromatography on silica gel (eluent: petroleum ether: ethyl acetate=8:1), to obtain **5-21** (4.00 g, yield 23.6%). ESI-MS *m*/*z* 333.1 (M-H)⁻.

**5-21** (2.85 g, 8.50 mmol) and cesium carbonate (4.14 g, 12.7 mmol) were added to a 250 mL reaction flask, and then DMF (85 mL) was added. The reaction was carried out at 0°C for 30 min, and then iodomethane (635 µL, 10.2 mmol) was slowly added dropwise and reacted at 0°C for 1 h. After the reaction was complete, the reaction solution was washed with saturated NaCl solution, and extracted with ethyl acetate. The organic phase was washed with saturated NaCl solution, dried over anhydrous sodium sulfate, and rotary evaporated to dryness. The residue was purified by column chromatography on silica gel (eluent: petroleum ether : ethyl acetate = 10:1) to obtain Compound **6-21** (2.90 g, yield 98%). ESI-MS *m*/*z* 347.2 (M-H)⁻.

**6-21** (2.90 g, 8.30 mmol) was added to a 250 mL reaction flask, and then dichloromethane (64 mL) and TFA (16 mL) were added. The reaction was continued at room temperature for 1 h. After the reaction was complete, the reaction solution was rotary evaporated to remove the solvent, added with water (100 mL), adjusted to pH 8-9 with saturated sodium bicarbonate, and extracted with ethyl acetate. The organic phase was rotary evaporated to dryness, trifluoroacetic anhydride (1.70 mL, 11.6 mmol) and DIPEA (2.1 mL, 12.4 mmol) were added, and DCM (85 mL) was added as a solvent. The reaction was continued overnight at room temperature. After the reaction was complete, the reaction solution was rotary evaporated to remove the solvent, washed with saturated NaCl solution, and extracted with ethyl acetate. The organic phase was washed with saturated NaCl solution, dried over anhydrous sodium sulfate, and rotary evaporated to dryness. The residue was purified by column chromatography on silica gel (eluent: petroleum ether : ethyl acetate = 10:1) to obtain **7-21** (2.65 g, yield 92.7%). ESI-MS *m*/*z* 345.0 (M+H)⁺.

**7-21** (2.65 g, 7.70 mmol) and trifluoroacetic anhydride (80 mL) were added to a 250 mL reaction flask, and cooled to -30°C. Concentrated nitric acid (560 µL, 8.4 mmol) was slowly added dropwise followed by reaction for 1 h at a temperature controlled at -10°C or less. After the reaction was complete, the reaction solution was washed with water (100 mL), extracted with ethyl acetate, and rotary evaporated to dryness. The residue was purified by column chromatography on silica gel (eluent: petroleum ether : ethyl acetate = 6:1), to obtain Compound **8-21** (2.70 g, yield 90.2%). ESI-MS *m*/*z* 390.0 (M+H)⁺.

**8-21** (2.70 g, 6.90 mmol), palladium tetrakis(triphenylphosphine) (3.1 g, 2.7 mmol), tri-n-butyltin hydride (5.6 mL, 20.8 mmol), and acetic acid (2.5 mL, 41.6 mmol) were added to a 250 mL reaction flask, and then DCM (70 mL) was added as a solvent. The reaction was continued for 1 h at room temperature. After the reaction was complete, the reaction solution was rotary evaporated to remove the solvent, washed with saturated NaCl solution, and extracted with ethyl acetate. The organic phase was washed with saturated NaCl solution, dried over anhydrous sodium sulfate, and rotary evaporated to dryness. The residue was purified by column chromatography on silica gel (eluent: petroleum ether : ethyl acetate = 3:1) to obtain Compound **9-21** (1.10 g, yield 45.7%) and by-product **10-21** (849 mg, yield 38.6%). ESI-MS *m*/*z* 350.0 (M+H)⁺.

**9-21** (1.10 g, 3.10 mmol) and Pd/C (1.1 g) were added to a 100 mL reaction flask, and then methanol (35 mL) was added. The reaction was continued for 1 h at room temperature under a hydrogen atmosphere. After the reaction was complete, the reaction solution was filtered through diatomaceous earth to remove Pd/C. The filtrate was rotary evaporated to dryness to obtain **10-21** (860 mg, yield 87%). ESI-MS *m*/*z* 320.1 (M+H)⁺.

**10-21** (1.71g, 5.36 mmol) and triethyl orthoformate (100 mL) were added to a 250 mL reaction flask, and reacted for 3 h under reflux at 130°C. After the reaction was complete, the reaction solution was added with water (100 mL), and extracted with ethyl acetate. The organic phase was washed with saturated NaCl solution, dried over anhydrous sodium sulfate, and rotary evaporated to dryness. The residue was purified by column chromatography on silica gel (eluent: petroleum ether : ethyl acetate = 1:1) to obtain **II-21** (1.70 g, yield 98%). ESI-MS *m*/*z* 330.1 (M+H)⁺.

### Example 25: Synthesis of Compound II-29

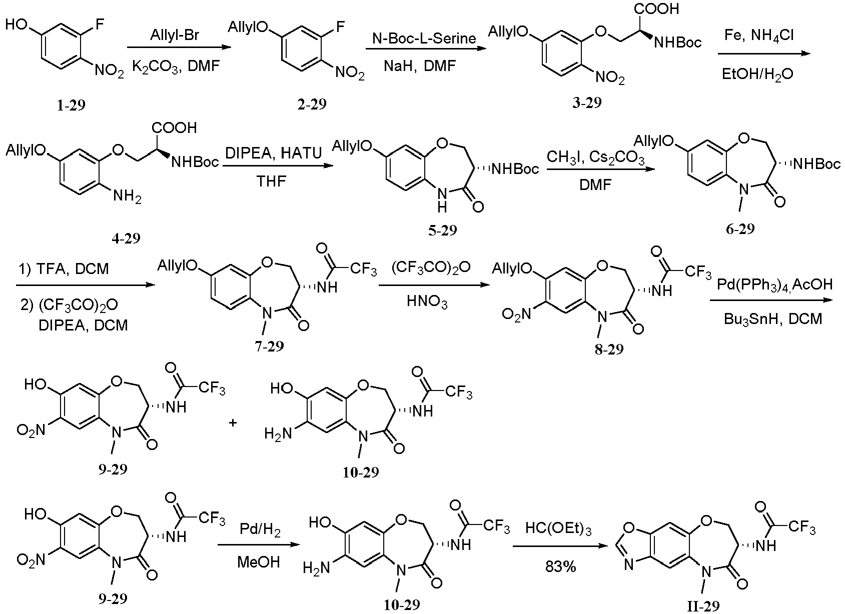

**1-29** (10 g, 63.65 mmol) and potassium carbonate (26.39 g, 190.96 mmol) were added to a 500 mL reaction flask, and then DMF (300 mL) was added. Allyl bromde (17 mL, 190.96 mmol) was added dropwise and reacted at room temperature for 3 h. After the reaction was complete, the reaction solution was washed with saturated NaCl solution, and extracted with ethyl acetate. The organic phase was washed with saturated NaCl solution, dried over anhydrous sodium sulfate, rotary evaporated to dryness, and the residue was separated by column chromatography on silica gel (eluent: petroleum ether : ethyl acetate=5:1), to obtain **2-29** (12.30 g, yield 98%). ESI-MS *m*/*z* 198.0 (M+H)⁺.

N-Boc-L-Serine (7.80 g, 38.06 mmol) and NaH (3.6 g, 88.81 mmol) were added to a 250 mL reaction flask, and then DMF (100 mL) was added as a solvent. The reaction was continued at 0°C for 30 min. **2-29** (5.00 g, 25.37 mmol) was dissolved in DMF (50 mL), then slowly added dropwise to the reaction solution, and reacted at 0°C for 1 h. After the reaction was complete, the reaction solution was washed with saturated NaCl solution, and extracted with ethyl acetate. The organic phase was washed with saturated NaCl solution, dried over anhydrous sodium sulfate, rotary evaporated to dryness, to obtain crude **3-29** (4.99 g, yield 53%). ESI-MS *m*/*z* 381.1 (M-H)⁻.

All the crude product **3-29,** reduced iron powder (7.1 g, 126.87 mmol), and ammonium chloride (2.2 g, 40.6 mmol) were added to a 250 mL reaction flask, and then ethanol/water (120mL/30mL) was added. The reaction was carried out under reflux at 70°C for 1 h. After the reaction was complete, the reaction solution was filtered through diatomaceous earth to remove the iron powder, and the filtrate was rotary evaporated to dryness, so as to obtain crude **4-29.** ESI-MS *m*/*z* 353.1 (M+H)⁺.

All the crude **4-29,** HATU (11.6 g, 30.44 mmol), and DIPEA (17 mL, 101.48 mmol) were added to a 250 mL reaction flask, and then THF (200 mL) was added as a solvent. The reaction was continued at room temperature for 1 h. After the reaction was complete, the reaction solution was rotary evaporated to remove THF, washed with saturated NaCl solution, and extracted with ethyl acetate. The organic phase was washed with saturated NaCl solution, dried over anhydrous sodium sulfate, rotary evaporated to dryness, and the residue was separated by column chromatography on silica gel (eluent: petroleum ether: ethyl acetate=8:1), to obtain **5-29** (1.50 g, yield 17.7%). ESI-MS *m*/*z* 333.2 (M-H)⁻.

**5-29** (1.97 g, 5.84 mmol) and cesium carbonate (2.85 g, 8.75mmol) were added to a 250 mL reaction flask, and then DMF (50 mL) was added. After reaction at 0°C for 30 min, iodomethane (438 µL, 7.03 mmol) was slowly added dropwise and reacted at 0°C for 1 h. After the reaction was complete, the reaction solution was washed with saturated NaCl solution, and extracted with ethyl acetate. The organic phase was washed with saturated NaCl solution, dried over anhydrous sodium sulfate, and rotary evaporated to dryness. The residue was purified by column chromatography on silica gel (eluent: petroleum ether : ethyl acetate = 10:1) to obtain Compound **6-29** (1.65 g, yield 81%). ESI-MS *m*/*z* 293.1 (M+H)⁺.

**6-29** (1.65 g, 4.70 mmol) was added to a 250 mL reaction flask, and then dichloromethane (40 mL) and TFA (10 mL) were added. The reaction was continued at room temperature for 1 h. After the reaction was complete, the reaction solution was rotary evaporated to remove the solvent, added with water (100 mL), adjusted to pH 8-9 with saturated sodium bicarbonate, and extracted with ethyl acetate. The organic phase was rotary evaporated to dryness, trifluoroacetic anhydride (905 µL, 6.5 mmol) and DIPEA (1.17 mL, 7.10 mmol) were added, and DCM (50 mL) was added as a solvent. The reaction was continued overnight at room temperature. After the reaction was complete, the reaction solution was rotary evaporated to remove the solvent, washed with saturated NaCl solution, and extracted with ethyl acetate. The organic phase was washed with saturated NaCl solution, dried over anhydrous sodium sulfate, and rotary evaporated to dryness. The residue was purified by column chromatography on silica gel (eluent: petroleum ether: ethyl acetate = 10: 1) to obtain **7-29** (1.60 g, yield 98.8%). ESI-MS *m*/*z* 345.0 (M+H)⁺.

**7-29** (1.50 g, 4.30 mmol) and trifluoroacetic anhydride (45 mL) were added to a 250 mL reaction flask, and cooled to -30°C. Concentrated nitric acid (320 µL, 4.70 mmol) was slowly added dropwise and reacted for 1 h at a temperature controlled at -10°C or less. After the reaction was complete, the reaction solution was washed with water (100 mL), extracted with ethyl acetate, and rotary evaporated to dryness. The residue was purified by column chromatography on silica gel (eluent: petroleum ether : ethyl acetate = 6:1), to obtain Compound **8-29** (1.43 g, yield 85.6%). ESI-MS *m*/*z* 390.0 (M+H)⁺.

**8-29** (1.33 g, 3.4 mmol), palladium tetrakis(triphenylphosphine) (1.16 g, 1.0 mmol), tri-n-butyltin hydride (2.74 mL, 10.2 mmol), and acetic acid (1.22 mL, 20.5 mmol) were added to a 250 mL reaction flask, and then DCM (35 mL) was added as a solvent. The reaction was continued for 1 h at room temperature. After the reaction was complete, the reaction solution was rotary evaporated to remove the solvent, washed with saturated NaCl solution, and extracted with ethyl acetate. The organic phase was washed with saturated NaCl solution, dried over anhydrous sodium sulfate, and rotary evaporated to dryness. The residue was purified by column chromatography on silica gel (eluent: petroleum ether : ethyl acetate = 3:1) to obtain **9-29** (800 mg, yield 67.4%) and product **10-29** (312 mg, 27.0%). ESI-MS m/z 349.9 (M+H)⁺.

**9-29** (0.80 g, 2.20 mmol) and Pd/C (0.80 g) were added to a 100 mL reaction flask, and then methanol (25 mL) was added. The reaction was continued for 1 h at room temperature under a hydrogen atmosphere. After the reaction was complete, the reaction solution was filtered through diatomaceous earth to remove Pd/C. The filtrate was rotary evaporated to dryness to obtain **10-29** (636 mg, yield 90.6%). ESI-MS *m*/*z* 320.0 (M+H)⁺.

**10-29** (0.948 g, 2.97 mmol) and triethyl orthoformate (50 mL) were added to a 250 mL reaction flask, and reacted for 3 h under reflux at 130°C. After the reaction was complete, the reaction solution was added with water (100 mL), and extracted with ethyl acetate. The organic phase was washed with saturated NaCl solution, dried over anhydrous sodium sulfate, and rotary evaporated to dryness. The residue was purified by column chromatography on silica gel (eluent: petroleum ether : ethyl acetate = 1:1) to obtain **II-29** (0.900 g, yield 92.1%). ESI-MS *m*/*z* 330.0 (M+H)⁺.

### Example 26: Test of inhibition of TNF-induced programmed Jurkat (FADD^{-/-}) cell necrosis (IC₅₀)

RPMI-1640 medium (containing serum, 50 µL per well) was added to a white 96-well cell culture plate, and then 0.5µL of a corresponding concentration of a drug solution comprising the compound of the present application or the control compound 7-Cl-O-Nec-1 or GSK2982772 (7-Cl-O-Nec-1 and GSK2982772 are known RIP1 kinase inhibitors) or 0.5 µL of 100% DMSO was added to each well. Two replicate wells were set for each concentration of each compound.

Jurkat (FADD^{-/-}) cells (human-derived peripheral blood leukemia T cell line, with FADD gene knocked out) were cultured in vitro. After growing to logarithmic growth phase, the cells were harvested, and centrifuged at 1000 rpm for 5 min. The supernatant was discarded, and the cells were resuspended in fresh medium and adjusted to a cell density of 5 × 10⁵/mL. The cell suspension was added to the culture plate containing the drug (40 µL per well). In the treatment groups, 10 µL of TNF (tumor necrosis factor, final concentration 10 ng/mL) diluted with the cell culture medium was added to each well, and incubated for 20 h in a cell incubator (37°C, 5% CO₂). Subsequently, 50 µL of Cell Titer-Glo solution was added to each well and incubated at room temperature for 10 min. Then, the luminescence was detected on BioTek plate reader to measure the intracellular ATP level. The effect of the test compounds in inhibiting TNF-induced cell necrosis was calculated from the full-activity well and background well (with the untreated DMSO control well as 100% cell viability), and the half inhibitory concentration (IC₅₀) of the compound was calculated by Graphpad Prism statistical software. The result was shown in Table 1 below.

### Example 27: Test of inhibition of ADP-Glo™ RIP1 kinase activity (Ki)

The human RIP1 gene was transduced into Sf-9 insect cells, and the RIP 1 protein was purified by Ni+ affinity chromatography and then by S-200 molecular sieve to remove the protein multimers. The obtained RIP1 protein was stored at -70°C, and the activity of the RIP1 protein in catalyzing autophosphorylation was detected by the ADP-Glo™ Kinase Activity Assay Kit (Promega) and the inhibitory activity of the drug against the RIP1 kinase was determined.

The drug (the compounds of the present application and the control compounds 7-Cl-O-Nec-1 and GSK2982772) was dissolved in 100% DMSO and 3-fold serially diluted, to afford a total of 11 concentration gradients, and a 100x solution was prepared. 100 nL of the compound was transferred to a 384-well assay plate. Two replicate wells were set for each concentration of each drug.

The RIP1 enzyme solution and ATP solution were formulated in an assay buffer (containing 50 mM HEPES pH 7.5, 30 mM MgCl₂, 50 mM NaCl, 1 mM DTT, 0.02% CHAPS, and 0.5 mg/ml BSA). Then, 5 µL of the RIP1 enzyme solution (final concentration 50 nM) was added to the assay plate and incubated at room temperature for 1 h. 5 µL of the ATP solution (final concentration 10 µM) was added to the assay plate, and incubate at room temperature for 4 h. Then 5 µL of ADP-Glo reagent was added to the assay plate, and incubated at room temperature for 60 min. 5 µL of a kinase assay solution was added to the assay plate, and incubated at room temperature for 30 min. After readout on Envision, the inhibition rate in each well was calculated from the full-activity well and the background well, and the Ki of the compound was calculated by GraphPad Prism 7 software (using the Morrision equation to fit the curve and calculate the Ki). The results are shown in Table 1.

**Table 1**

| Compound No. | Cellular IC₅₀ (M) Jurkat (FADD^{-/-}) | RIP1K Ki (M) |
|---|---|---|
| 7-Cl-O-Nec-1 | - | 1.50E-07 |
| GSK2982772 | 9.70E-10 | 3.20E-08 |
| RIP1-001 | 1.38E-09 | |
| RIP 1-003 | 6.91E-10 | |
| RIP1-004 | 5.63E-08 | |
| RIP1-005 | >2E-6 | |
| RIP1-006 | 1.25E-09 | |
| RIP 1-007 | 3.40E-07 | |
| RIP1-008 | 2.40E-10 | |
| RIP1-009 | 1.08E-09 | |
| RIP1-010 | 9.60E-08 | |
| RIP1-011 | 3.24E-07 | |
| RIP1-012 | 1.22E-09 | |
| RIP1-013 | 5.30E-10 | |
| RIP1-014 | 2.60E-08 | |
| RIP1-016 | 2.30E-10 | |
| RIP1-017 | >2E-6 | |
| RIP1-018 | 2.70E-10 | |
| RIP1-019 | 6.80E-10 | |
| RIP1-020 | 2.30E-10 | |
| RIP1-021 | 5.80E-10 | 1.03E-08 |
| RIP1-022 | 5.66E-10 | |
| RIP 1-023 | 2.54E-09 | |
| RIP1-024 | 2.03E-09 | |
| RIP1-025 | 2.86E-10 | |
| RIP1-026 | 2.80E-10 | |
| RIP1-028 | 7.00E-11 | |
| RIP1-029 | 1.20E-10 | |
| RIP1-031 | 2.90E-10 | 6.60E-10 |
| RIP1-032 | 2.40E-10 | 2.03E-08 |
| RIP1-033 | 6.80E-10 | 1.08E-08 |
| RIP1-034 | 8.90E-10 | 6.35E-09 |
| RIP1-035 | 1.94E-09 | 7.80E-09 |
| RIP1-036 | 1.80E-10 | |
| RIP1-037 | 1.29E-10 | |
| RIP1-038 | 1.29E-10 | |
| RIP1-039 | 4.50E-10 | |
| RIP1-040 | 3.60E-10 | |
| RIP1-041 | 3.79E-10 | |
| RIP1-042 | 1.93E-09 | |
| RIP1-043 | 1.31E-10 | |
| RIP1-044 | 1.91E-08 | |
| RIP1-045 | >2E-6 | |
| RIP1-046 | 1.46E-07 | |
| RIP1-047 | 2.15E-08 | |
| RIP1-048 | 5.10E-09 | |
| RIP1-049 | >2E-6 | |
| RIP1-050 | 4.92E-07 | |
| RIP1-051 | >2E-6 | |
| RIP1-052 | >2E-6 | |
| RIP1-054 | >2E-6 | |
| RIP1-055 | 2.29E-09 | |
| RIP1-056 | 4.15E-08 | |
| RIP1-057 | >2E-6 | |
| RIP1-058 | 2.53E-10 | |
| RIP1-061 | 5.86E-09 | 4.40E-08 |
| RIP1-062 | >2E-6 | |
| RIP 1-063 | 4.57E-08 | |
| RIP1-064 | >2E-6 | |
| RIP1-065 | >2E-6 | |
| RIP1-066 | 6.70E-07 | |
| RIP1-067 | 6.72E-09 | 2.86E-08 |
| RIP1-068 | 7.62E-09 | 4.28E-08 |
| RIP1-069 | 4.52E-09 | 3.13E-08 |
| RIP1-070 | 2.15E-06 | |
| RIP1-071 | 1.71E-08 | |
| RIP 1-072 | 9.65E-08 | |
| RIP 1-073 | >2E-6 | |
| RIP1-074 | >2E-6 | |
| RIP1-075 | 4.73E-10 | |
| RIP1-076 | >2E-6 | |
| RIP 1-077 | 1.68E-08 | |
| RIP 1-078 | 3.11E-08 | |
| RIP 1-079 | >2E-6 | |
| RIP1-080 | >2E-6 | |
| RIP1-081 | >2E-6 | |
| RIP1-082 | >2E-6 | |
| RIP1-083 | >2E-6 | |
| RIP1-084 | >2E-6 | |
| RIP1-085 | >2E-6 | |
| RIP1-086 | >2E-6 | |
| RIP1-087 | >2E-6 | |
| RIP1-088 | >2E-6 | |
| RIP1-092 | 1.05E-09 | |
| RIP 1-093 | 1.15E-09 | |
| RIP1-094 | 2.00E-09 | |
| RIP1-095 | 9.85E-09 | |
| RIP1-096 | 1.38E-09 | |
| RIP 1-097 | 1.20E-09 | |
| RIP 1-097 | 2.28E-09 | 2.32E-08 |
| RIP1-098 | 7.25E-10 | 2.09E-08 |
| RIP1-099 | 6.92E-09 | 3.77E-08 |
| RIP1-100 | 3.27E-08 | 2.04E-08 |
| RIP1-101 | 5.50E-09 | 1.72E-08 |
| RIP1-102 | 9.00E-10 | |
| RIP1-103 | 6.75E-09 | |
| RIP1-104 | 7.40E-10 | |
| RIP1-105 | 3.22E-09 | |
| RIP1-106 | 7.47E-10 | |
| RIP1-107 | 1.07E-09 | 8.90E-09 |
| RIP1-108 | 1.64E-10 | 1.26E-08 |
| RIP1-109 | 7.22E-10 | 6.85E-09 |
| RIP1-110 | 4.44E-09 | 1.46E-08 |
| RIP1-111 | 1.16E-09 | 1.85E-08 |
| RIP1-112 | 1.22E-09 | |
| RIP1-113 | 3.07E-10 | |
| RIP1-114 | 8.46E-10 | |
| RIP1-115 | 3.48E-09 | |
| RIP1-116 | 5.16E-09 | |
| RIP1-119 | 5.81E-10 | 1.91E-08 |
| RIP1-120 | 2.56E-09 | 2.07E-08 |
| RIP1-121 | 1.37E-08 | 3.27E-08 |
| RIP1-122 | 2.26E-10 | 1.56E-08 |
| RIP1-123 | 1.33E-09 | 2.32E-08 |
| RIP1-124 | 2.09E-08 | |
| RIP1-125 | 8.43E-10 | 1.96E-08 |
| RIP1-126 | 2.61E-09 | 2.56E-08 |
| RIP1-127 | 3.28E-09 | 1.62E-08 |
| RIP1-128 | 4.83E-07 | |
| RIP1-129 | 7.94E-07 | |
| RIP1-130 | 5.43E-08 | |
| RIP1-131 | 1.29E-09 | 2.86E-08 |
| RIP1-132 | 4.57E-09 | 1.93E-08 |
| RIP1-133 | 5.30E-08 | 2.31E-08 |
| RIP1-134 | 9.03E-09 | 2.19E-08 |
| RIP1-135 | 4.25E-09 | 2.15E-08 |
| RIP1-136 | 9.34E-09 | 1.78E-08 |
| RIP1-137 | 7.31E-09 | 1.33E-08 |
| RIP1-138 | 1.68E-08 | 1.98E-08 |
| RIP1-139 | 7.81E-10 | 2.03E-08 |
| RIP1-140 | 1.37E-09 | 5.06E-08 |
| RIP1-141 | 2.19E-08 | |
| RIP1-142 | 1.86E-09 | 1.91E-08 |
| RIP1-143 | 2.06E-07 | |
| RIP1-145 | 4.61E-10 | 1.05E-08 |
| RIP1-146 | 1.85E-09 | 1.32E-08 |
| RIP1-147 | 1.69E-09 | 1.65E-08 |
| RIP1-148 | 1.07E-08 | |
| RIP1-149 | >2E-6 | |
| RIP1-150 | NA | |
| RIP1-153 | 1.52E-09 | 1.28E-08 |
| RIP1-156 | 2.80E-08 | |
| RIP1-157 | 2.49E-07 | |
| RIP1-158 | 8.00E-09 | |
| RIP1-159 | 8.70E-09 | |
| RIP1-160 | 2.20E-09 | |
| RIP1-161 | 8.60E-09 | |
| RIP1-162 | 5.40E-08 | |
| RIP1-163 | 1.30E-09 | |
| RIP1-164 | 3.30E-09 | |
| RIP1-165 | 2.50E-09 | |
| RIP1-166 | 2.98E-07 | |
| RIP1-167 | 1.60E-06 | |
| RIP1-168 | 1.10E-08 | |
| RIP1-169 | 1.13E-07 | |
| RIP1-170 | 1.74E-09 | |
| RIP1-170 | 3.00E-09 | |
| RIP1-171 | 1.40E-08 | |
| RIP1-172 | 5.00E-09 | |
| RIP1-173 | 7.00E-08 | |
| RIP1-174 | 1.30E-08 | |
| RIP1-175 | 1.46E-09 | |
| RIP1-176 | 6.90E-09 | |
| RIP1-177 | 2.03E-09 | |
| RIP1-178 | 3.23E-10 | |
| RIP1-179 | 6.70E-10 | |
| RIP1-180 | 2.66E-10 | |
| RIP1-181 | 5.92E-10 | |
| RIP1-182 | 8.16E-10 | |
| RIP1-183 | 6.15E-09 | |
| RIP1-184 | 3.76E-08 | |
| RIP1-185 | 2.72E-09 | |
| RIP1-186 | 7.52E-10 | |
| RIP1-187 | 2.59E-07 | |
| RIP1-188 | 1.93E-07 | |
| RIP1-189 | 1.93E-10 | |
| RIP1-190 | 1.44E-10 | |
| RIP1-193 | 2.95E-07 | |
| RIP1-194 | 7.28E-06 | |
| RIP1-195 | 5.66E-06 | |
| RIP1-196 | >2E-05 | |
| RIP1-197 | 2.92E-08 | |
| RIP1-198 | 4.57E-09 | |
| RIP1-199 | 4.76E-05 | |
| RIP1-200 | 8.18E-08 | |
| RIP1-201 | >2E-05 | |
| RIP1-202 | >2E-05 | |
| RIP1-203 | 2.33E-09 | |
| RIP1-204 | 3.04E-09 | |
| RIP1-205 | 7.11E-09 | |
| RIP1-206 | 8.18E-10 | |
| RIP1-207 | 4.88E-09 | |
| RIP1-208 | 1.49E-09 | |
| RIP1-209 | 1. 92E-09 | |
| RIP1-210 | 7.8E-09 | |
| RIP1-211 | 2.72E-07 | |
| RIP1-212 | 1.81E-08 | |
| RIP1-213 | 7.68E-08 | |
| RIP1-214 | 2.41E-08 | |
| RIP1-215 | 3.45E-07 | |
| RIP1-217 | 1.38E-09 | |
| RIP1-218 | 1.24E-09 | |
| RIP1-219 | 6.75E-10 | |
| RIP1-220 | >2E-05 | |
| RIP1-221 | 7.31E-09 | |
| RIP1-222 | 5.62E-09 | |

As can be seen from Table 1, the compounds of the present application can effectively inhibit the activity of RIP 1 kinase.

### Example 28: In vivo efficacy in TNF-α induced shock test in mice

C57BL/6 mice were specially sterilized, and the animals are randomly grouped according to their body weight. 0.15 mg/mL TNF-α was prepared. Each mouse was injected with 200 µL TNF-alpha (30 µg per mouse) through the tail vein. 15 min before TNF-α induction, the drug was administered according to the body weight. At 0 h, 1 h, 2 h, 4 h, and 6 h, the body temperature of the mouse was detected by a small animal thermometer and recorded, and the state of the animal was observed during the test. After 6 h, the mice were euthanized with CO₂. The experimental data was plotted by Graph Pad Prism 7, and expressed as mean ± standard error (Mean ± SEM). The area under the clinical scoring curve was analyzed by one-way ANOVA. p <0.05 is considered to have significant differences as compared to the control group.

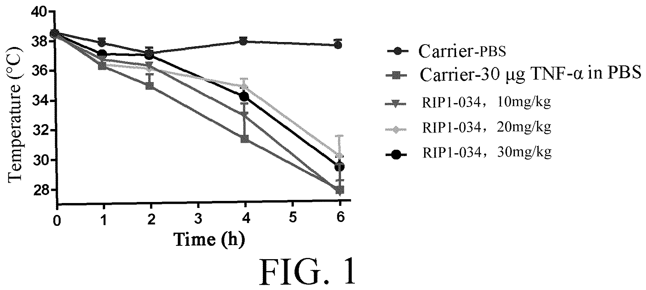

Fig. 1 shows the body temperature of mice as a function of time after the mice were administered Compound RIP1-034 of the present application at different doses (10 mg/kg, 20 mg/kg, and 30 mg/kg). The test shows that the administration of Compound RIP1-034 effectively inhibits the decrease in body temperature due to systemic inflammatory response caused by TNF-α.

### Example 29: Measurement of plasma concentration in mice after a single oral administration

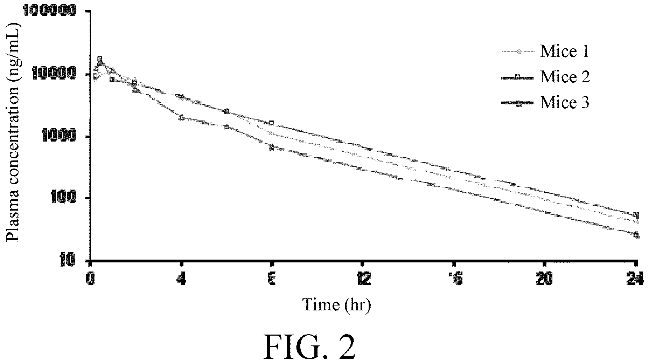

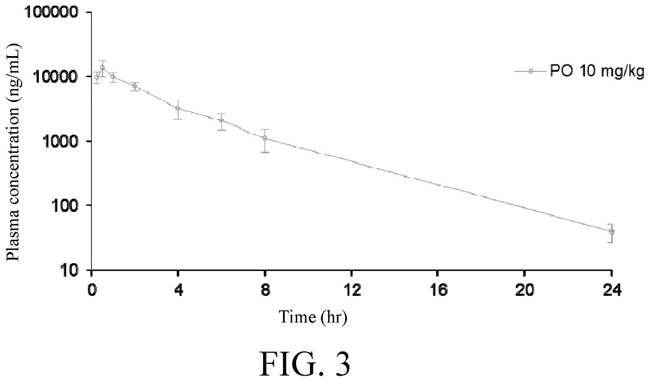

An oral solution containing Compound RIP1-034 of the present application was freshly prepared with an aqueous solution containing 0.5% MC/0.25% Tween 80 on the day of administration. All animals were fasted overnight before administration, and were allowed to have free food intake 4 hours after administration. Each group included three mice, and each mouse was orally administered a single dose of 10 mg/kg on the day of the experiment. About 30 µL of whole blood was collected from the dorsal vein of the foot at 0.25, 0.5, 1, 2, 4, 6, 8, and 24 hours after administration, transferred to an anticoagulation tube containing heparin sodium and mixed thoroughly by inverting for several times. The collected blood sample was centrifuged at 4°C and 4000 g for 5 min to collect plasma, and the plasma sample was dispensed into clean polyethylene microcentrifuge tubes, and then stored in a freezer at -75±15°C until analysis. The actual administration volume was calculated based on the animal's body weight. All samples were analyzed by LC-MS/MS, and the concentration of the drug to be tested in the samples was determined by the standard curve method. The plasma concentration for each mouse at each measurement time point after a single oral administration is shown in Fig. 2. The average plasma concentration in a group of three mice at each measurement time point after a single oral administration is shown in Fig. 3.

This test shows that Compound RIP1-034 can reach a desirable plasma concentration after oral administration.

## Claims

1. A compound of Formula (I): 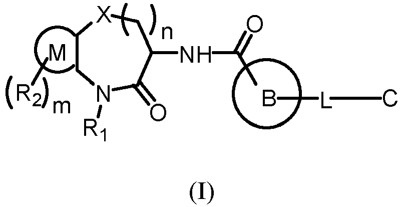 or a pharmaceutically acceptable salt thereof, wherein:
X is O, S or CH₂;
ring M has a structure of 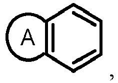 wherein ring A is selected from the group consisting of substituted or unsubstituted 5- to 6-membered heteroaryl and substituted or unsubstituted 5- to 6-membered heterocyclyl;
ring B is selected from the group consisting of substituted or unsubstituted 5- to 12-membered aryl, substituted or unsubstituted 5- to 12-membered heteroaryl, and substituted or unsubstituted 5- to 12-membered heterocyclyl;
C is selected from the group consisting of substituted or unsubstituted (C₃-C₁₂) cycloalkyl, substituted or unsubstituted 5- to 12-membered aryl, substituted or unsubstituted 5- to 12-membered heteroaryl, and substituted or unsubstituted 5- to 12-membered heterocyclyl;
L is selected from the group consisting of O, S, NH, N(CH₃), substituted or unsubstituted C₁-C₆ alkylene-O-, substituted or unsubstituted C₁-C₆ alkylene-NH-, (substituted or unsubstituted C₁-C₆ alkylene)₂-N-, substituted or unsubstituted C₁-C₆ alkylene, substituted or unsubstituted C₂-C₆ alkenylene, and substituted or unsubstituted C₂-C₆ alkenylene-O-;
R₁ is selected from the group consisting of H and substituted or unsubstituted C₁-C₆ alkyl;
R₂ is selected from the group consisting of H, halo, hydroxyl, cyano, oxy, benzyl, substituted or unsubstituted amino, substituted or unsubstituted C₁-C₆ alkyl, substituted or unsubstituted C₁-C₆alkoxy and C₁-C₆acyl; and
m is 0, 1, 2 or 3;
n is 1, 2 or 3;
wherein "substituted" refers to the substitution of one or more hydrogen atoms on the group with a substituent selected from the group consisting of halo, cyano, alkyl, acyl, sulfonyl, hydroxyl, amino, benzyl, oxy, halo alkyl, alkoxy, haloalkoxy, nitro, and alkylC(O)-.

2. The compound or a pharmaceutically acceptable salt thereof according to claim 1, wherein X is O or S.

3. The compound or a pharmaceutically acceptable salt thereof according to claim 1, wherein ring A is substituted or unsubstituted 5-membered heteroaryl, or substituted or unsubstituted 5-membered heterocyclyl.

4. The compound or a pharmaceutically acceptable salt thereof according to claim 1, wherein ring A is substituted or unsubstituted 6-membered heteroaryl, or substituted or unsubstituted 6-membered heterocyclyl.

5. The compound or a pharmaceutically acceptable salt thereof according to claim 1, wherein ring B is substituted or unsubstituted 5- to 12-membered aryl, substituted or unsubstituted 5- to 6-membered heteroaryl, or substituted or unsubstituted 5- to 6-membered heterocyclyl.

6. The compound or a pharmaceutically acceptable salt thereof according to claim 1, wherein C is substituted or unsubstituted 5- to 12-membered aryl, substituted or unsubstituted 5- to 6-membered heteroaryl, or substituted or unsubstituted 5- to 6-membered heterocyclyl.

7. The compound or a pharmaceutically acceptable salt thereof according to claim 1, wherein L is O, NH, or substituted or unsubstituted C₁-C₆ alkylene.

8. The compound or a pharmaceutically acceptable salt thereof according to claim 1, wherein R₁ is H.

9. The compound or a pharmaceutically acceptable salt thereof according to claim 1, wherein R₁ is substituted or unsubstituted C₁-C₆ alkyl.

10. The compound or a pharmaceutically acceptable salt thereof according to claim 1, wherein R₂ is a substituent on ring M selected from the group consisting of H, halo, hydroxyl, oxy, benzyl, substituted or unsubstituted C₁-C₆ alkyl, substituted or unsubstituted C₁-C₆alkoxy, and C₁-C₆ acyl.

11. The compound or a pharmaceutically acceptable salt thereof according to claim 1, wherein n is 1 or 2.

12. The compound or a pharmaceutically acceptable salt thereof according to claim 1, wherein the compound has a Formula (Ia):

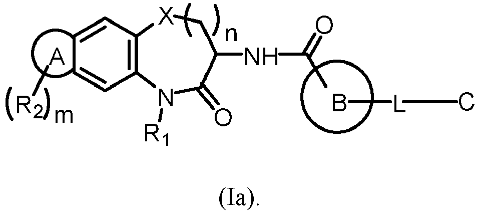

13. The compound or a pharmaceutically acceptable salt thereof according to claim 1, wherein the compound has a Formula (Ib): 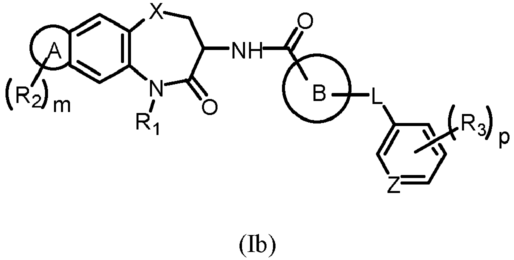 wherein
L is O or CH₂;
Z is N or CH;
R₃ is selected from halo and substituted or unsubstituted C₁-C₆ alkyl; and
p is 0, 1, 2 or 3.

14. The compound or a pharmaceutically acceptable salt thereof according to claim 12 or 13, wherein the structural moiety represented by a formula 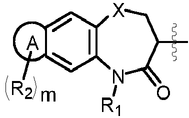 is selected from the group consisting of 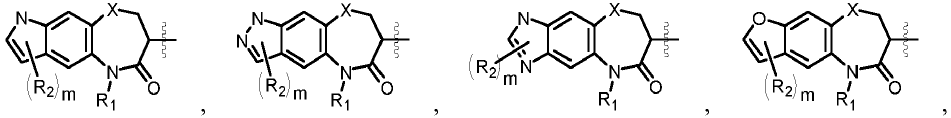 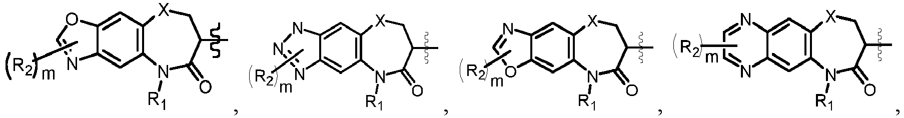 and

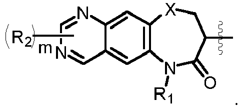

15. The compound or a pharmaceutically acceptable salt thereof according to claim 1, wherein the compound has a Formula (Ic): 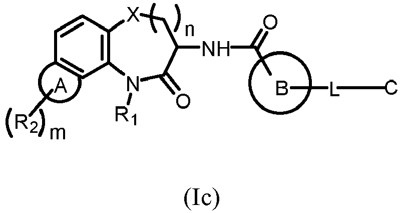 wherein ring A is substituted or unsubstituted 6-membered heteroaryl, or substituted or unsubstituted 6-membered heterocyclyl.

16. The compound or a pharmaceutically acceptable salt thereof according to claim 1, wherein the compound has a Formula (Id): 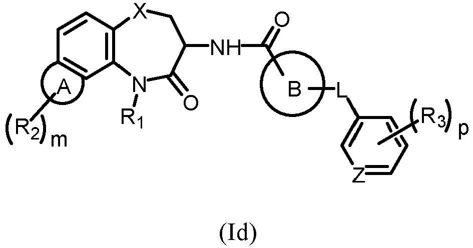 wherein
ring A is substituted or unsubstituted 6-membered heteroaryl, or substituted or unsubstituted 6-membered heterocyclyl;
L is O or CH₂;
Z is N or CH;
R₃ is selected from the group consisting of halo and substituted or unsubstituted C₁-C₆ alkyl; and
p is 0, 1, 2 or 3.

17. The compound or a pharmaceutically acceptable salt thereof according to claim 15 or 16, wherein the structural moiety represented by a formula 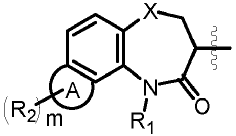 is selected from the group consisting of

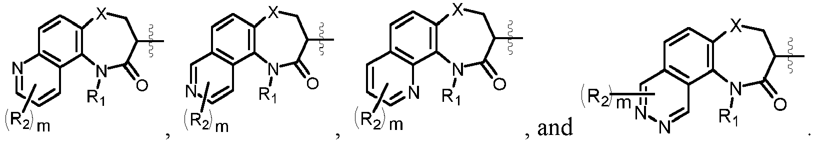

18. The compound or a pharmaceutically acceptable salt thereof according to any one of the preceding claims, wherein ring B is a group selected from the group consisting of

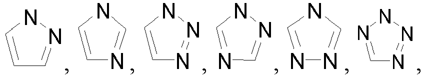

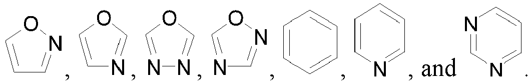

19. The compound or a pharmaceutically acceptable salt thereof according to claim 1, wherein the compound is selected from the group consisting of
| Compound No. | Chemical structure | Compound No. | Chemical structure |
|---|---|---|---|
| RIP1-001 | 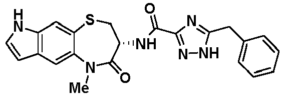 | RIP1-059 | 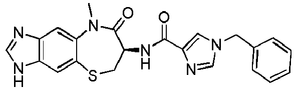 |
| RIP1-002 | 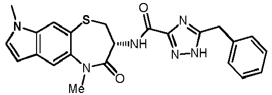 | RIP 1-060 | 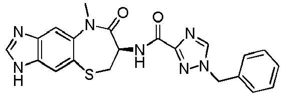 |
| RIP 1-003 | 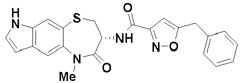 | RIP1-061 | 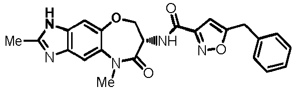 |
| RIP1-004 | 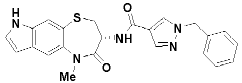 | RIP1-062 | 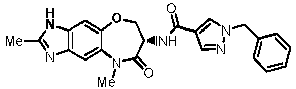 |
| RIP1-005 | 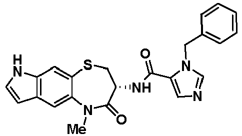 | RIP 1-063 | 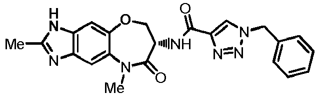 |
| RIP1-006 | 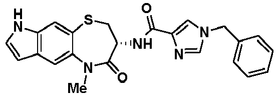 | RIP1-064 | 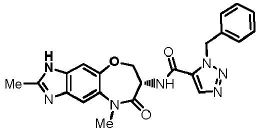 |
| RIP 1-007 | 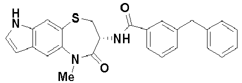 | RIP1-065 | 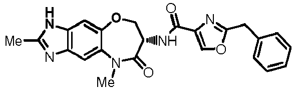 |
| RIP1-008 | 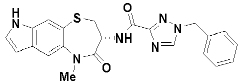 | RIP1-066 | 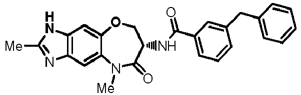 |
| RIP1-009 | 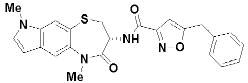 | RIP1-067 | 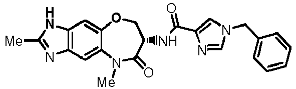 |
| RIP1-010 | 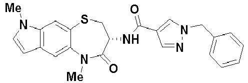 | RIP1-068 | 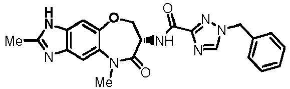 |
| RIP1-011 | 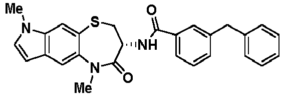 | RIP1-069 | 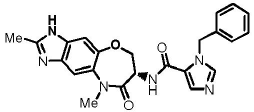 |
| RIP1-012 | 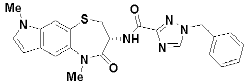 | RIP1-070 | 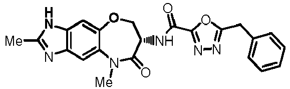 |
| RIP1-013 | 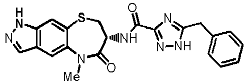 | RIP1-071 | 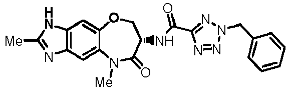 |
| RIP1-014 | 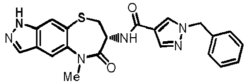 | RIP 1-072 | 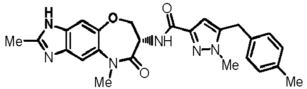 |
| RIP1-015 | 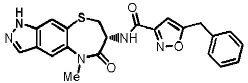 | RIP 1-073 | 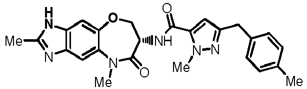 |
| RIP1-016 | 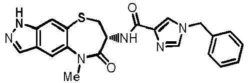 | RIP1-074 | 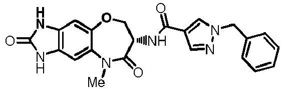 |
| RIP1-017 | 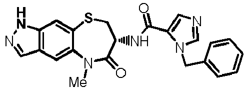 | RIP1-075 | 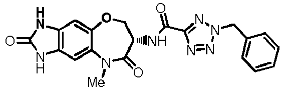 |
| RIP1-018 | 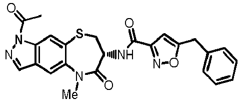 | RIP1-076 | 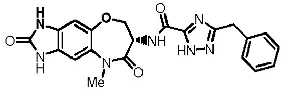 |
| RIP1-019 | 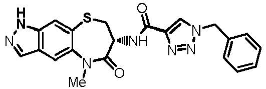 | RIP 1-077 | 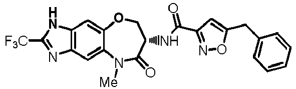 |
| RIP1-020 | 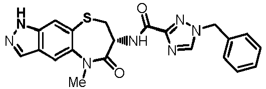 | RIP 1-078 | 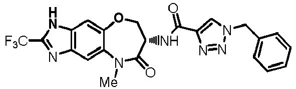 |
| RIP1-021 | 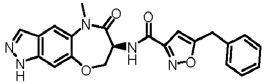 | RIP 1-079 | 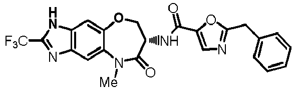 |
| RIP1-022 | 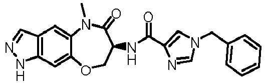 | RIP1-080 | 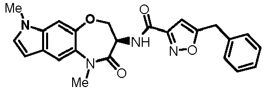 |
| RIP 1-023 | 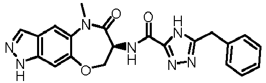 | RIP1-081 | 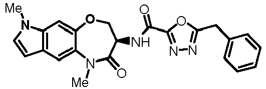 |
| RIP1-024 | 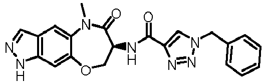 | RIP1-082 | 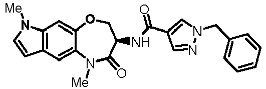 |
| RIP1-025 | 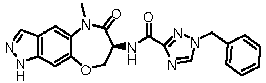 | RIP1-083 | 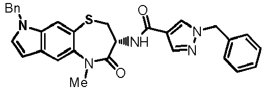 |
| RIP 1-026 | 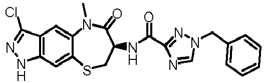 | RIP1-084 | 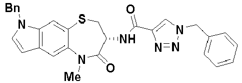 |
| RIP 1-027 | 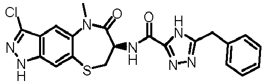 | RIP1-085 | 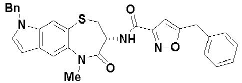 |
| RIP1-028 | 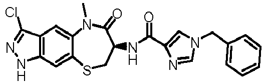 | RIP1-086 | 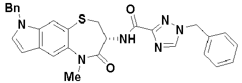 |
| RIP1-029 | 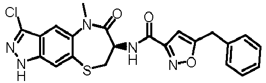 | RIP1-087 | 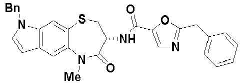 |
| RIP1-030 | 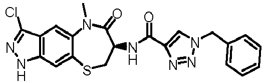 | RIP1-088 | 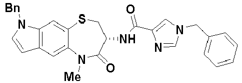 |
| RIP1-031 | 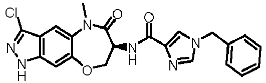 | RIP1-089 | 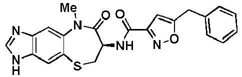 |
| RIP1-032 | 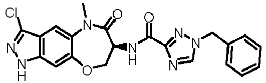 | RIP1-090 | 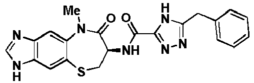 |
| RIP1-033 | 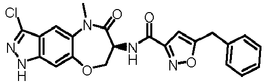 | RIP1-091 | 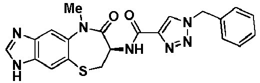 |
| RIP1-034 | 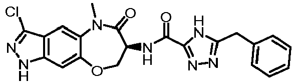 | RIP1-092 | 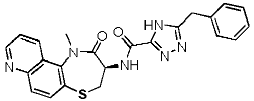 |
| RIP1-035 | 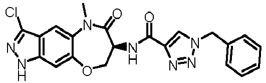 | RIP 1-093 | 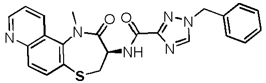 |
| RIP 1-036 | 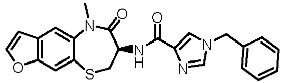 | RIP1-094 | 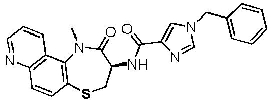 |
| RIP1-037 | 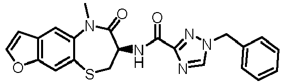 | RIP1-095 | 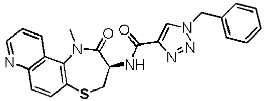 |
| RIP1-038 | 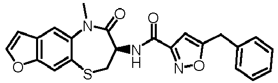 | RIP1-096 | 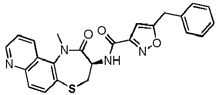 |
| RIP1-039 | 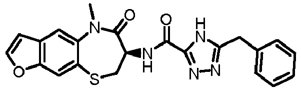 | RIP 1-097 | 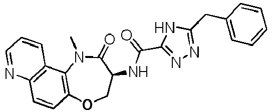 |
| RIP1-040 | 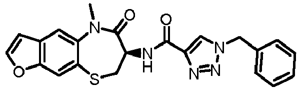 | RIP1-098 | 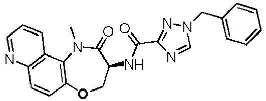 |
| RIP1-041 | 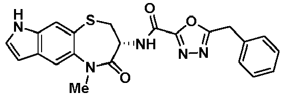 | RIP1-099 | 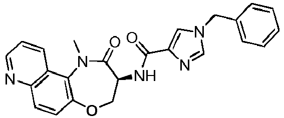 |
| RIP1-042 | 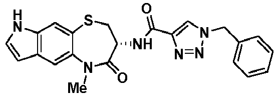 | RIP1-100 | 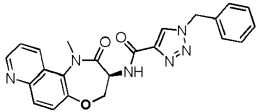 |
| RIP1-043 | 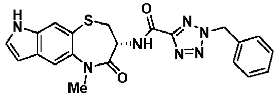 | RIP1-101 | 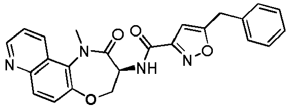 |
| RIP1-044 | 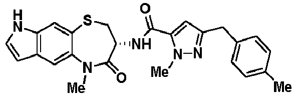 | RIP1-102 | 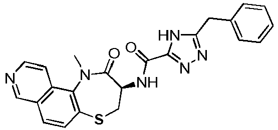 |
| RIP1-045 | 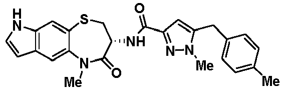 | RIP1-103 | 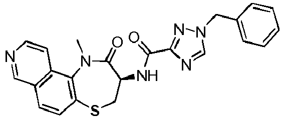 |
| RIP1-046 | 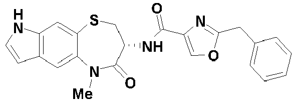 | RIP1-104 | 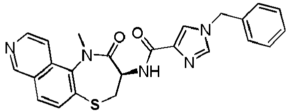 |
| RIP1-047 | 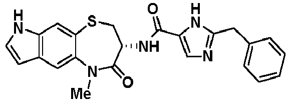 | RIP1-105 | 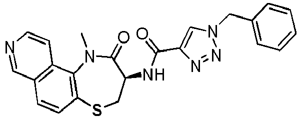 |
| RIP1-048 | 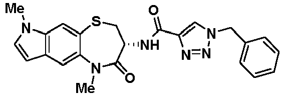 | RIP1-106 | 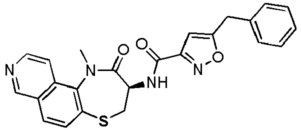 |
| RIP1-049 | 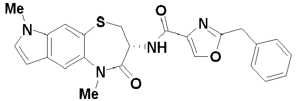 | RIP1-107 | 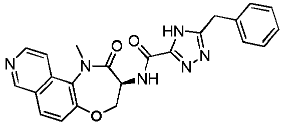 |
| RIP1-050 | 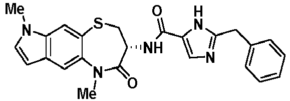 | RIP1-108 | 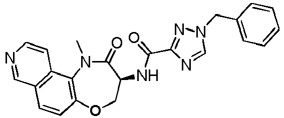 |
| RIP1-051 | 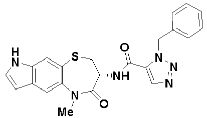 | RIP1-109 | 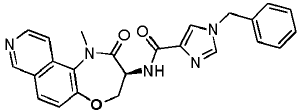 |
| RIP1-052 | 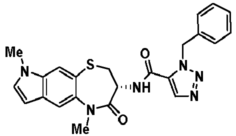 | RIP1-110 | 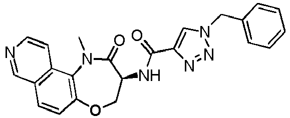 |
| RIP1-054 | 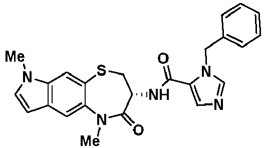 | RIP1-111 | 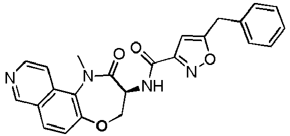 |
| RIP1-055 | 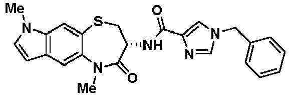 | RIP1-112 | 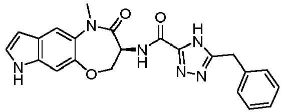 |
| RIP1-056 | 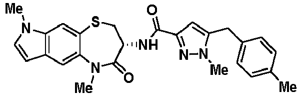 | RIP1-113 | 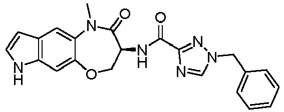 |
| RIP1-057 | 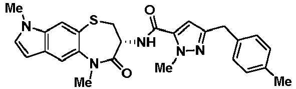 | RIP1-114 | 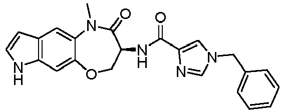 |
| RIP1-058 | 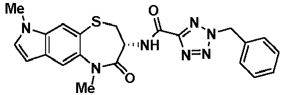 | RIP1-115 | 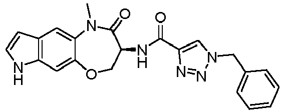 |
| RIP1-119 | 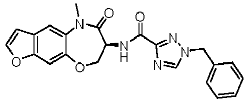 | RIP1-116 | 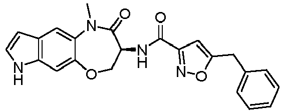 |
| RIP1-120 | 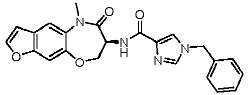 | RIP1-167 | 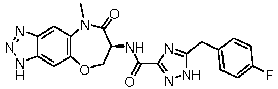 |
| RIP1-121 | 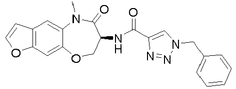 | RIP1-168 | 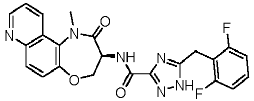 |
| RIP1-122 | 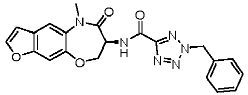 | RIP1-169 | 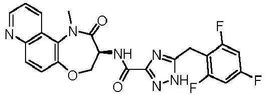 |
| RIP1-123 | 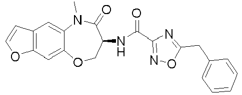 | RIP1-170 | 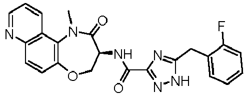 |
| RIP1-124 | 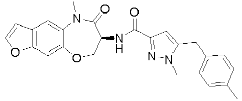 | RIP1-171 | 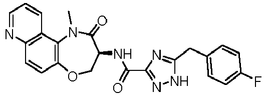 |
| RIP1-125 | 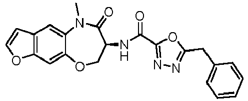 | RIP1-172 | 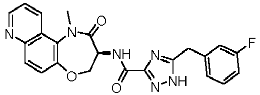 |
| RIP1-126 | 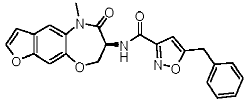 | RIP1-173 | 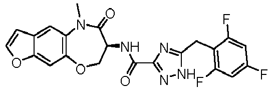 |
| RIP1-127 | 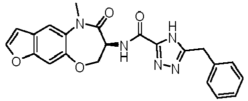 | RIP1-174 | 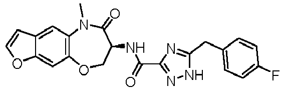 |
| RIP1-128 | 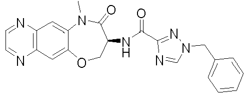 | RIP1-175 | 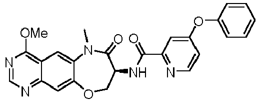 |
| RIP1-129 | 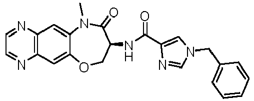 | RIP1-176 | 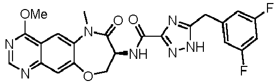 |
| RIP1-130 | 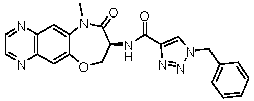 | RIP1-177 | 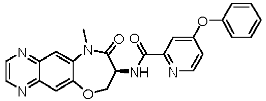 |
| RIP1-131 | 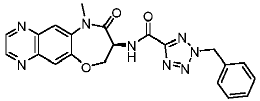 | RIP1-178 | 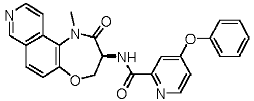 |
| RIP1-132 | 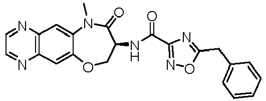 | RIP1-179 | 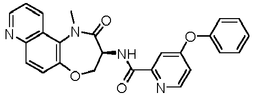 |
| RIP1-133 | 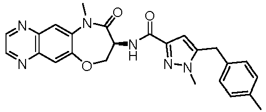 | RIP1-180 | 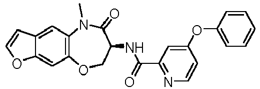 |
| RIP1-134 | 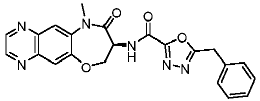 | RIP1-181 | 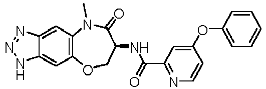 |
| RIP1-135 | 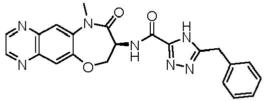 | RIP1-182 | 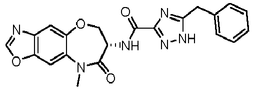 |
| RIP1-136 | 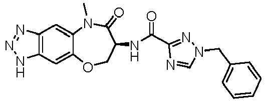 | RIP1-183 | 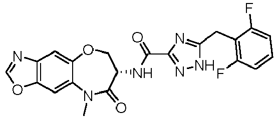 |
| RIP1-137 | 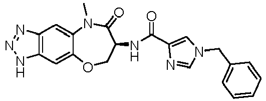 | RIP1-184 | 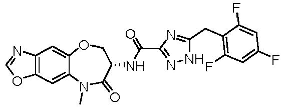 |
| RIP1-138 | 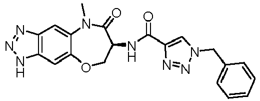 | RIP1-185 | 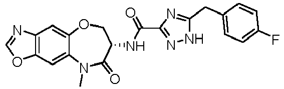 |
| RIP1-139 | 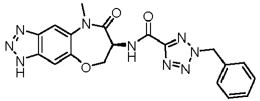 | RIP1-186 | 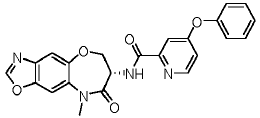 |
| RIP1-140 | 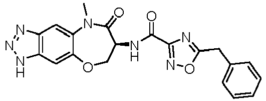 | RIP1-187 | 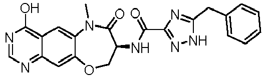 |
| RIP1-141 | 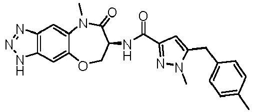 | RIP1-188 | 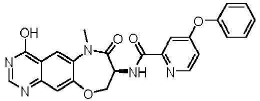 |
| RIP1-142 | 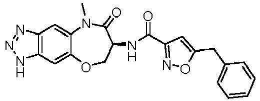 | RIP1-189 | 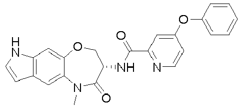 |
| RIP1-143 | 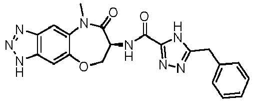 | RIP1-190 | 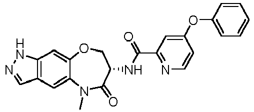 |
| RIP1-145 | 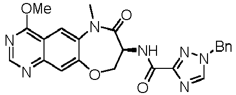 | RIP1-193 | 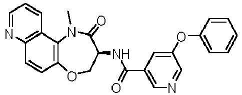 |
| RIP1-146 | 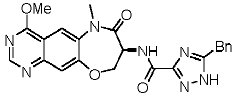 | RIP1-194 | 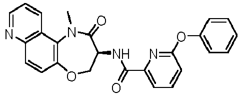 |
| RIP1-147 | 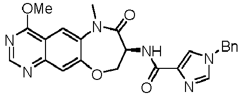 | RIP1-195 | 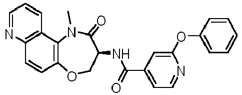 |
| RIP1-148 | 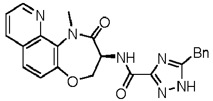 | RIP1-196 | 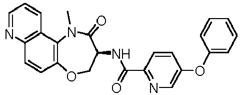 |
| RIP1-149 | 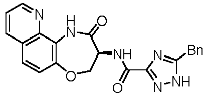 | RIP1-197 | 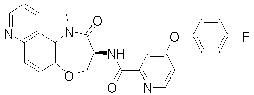 |
| RIP1-150 | 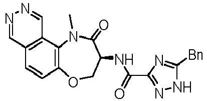 | RIP1-198 | 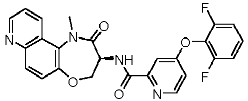 |
| RIP1-153 | 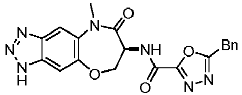 | RIP1-199 | 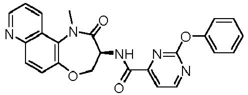 |
| RIP1-156 | 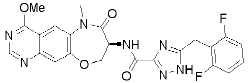 | RIP1-200 | 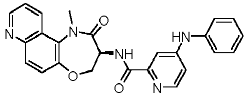 |
| RIP1-157 | 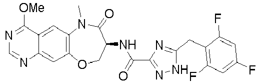 | RIP1-201 | 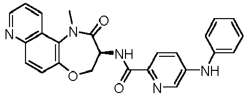 |
| RIP1-158 | 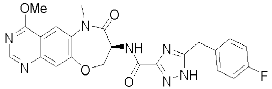 | RIP1-202 | 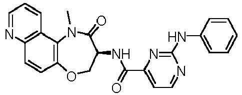 |
| RIP1-159 | 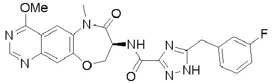 | RIP1-203 | 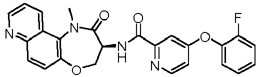 |
| RIP1-160 | 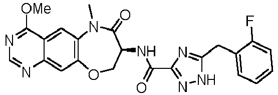 | RIP1-204 | 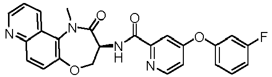 |
| RIP1-161 | 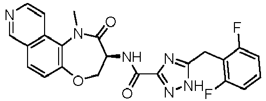 | RIP1-205 | 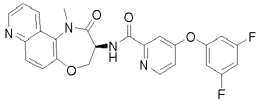 |
| RIP1-162 | 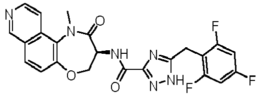 | RIP1-206 | 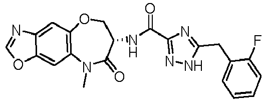 |
| RIP1-163 | 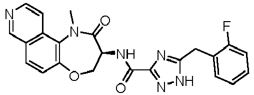 | RIP1-207 | 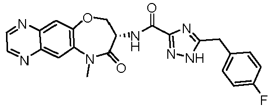 |
| RIP1-164 | 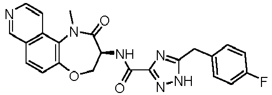 | RIP1-208 | 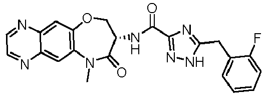 |
| RIP1-165 | 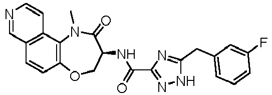 | RIP1-209 | 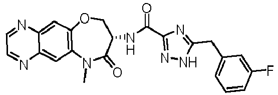 |
| RIP1-166 | 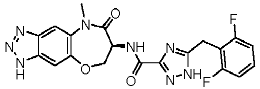 | RIP1-210 | 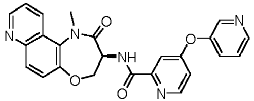 |
| RIP1-211 | 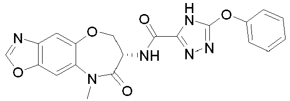 | RIP1-212 | 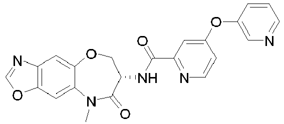 |
| RIP1-213 | 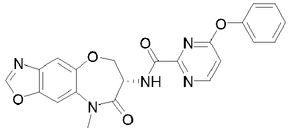 | RIP1-214 | 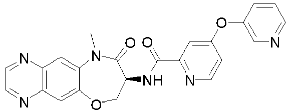 |
| RIP1-215 | 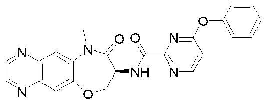 | RIP1-217 | 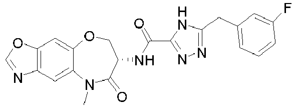 |
| RIP1-218 | 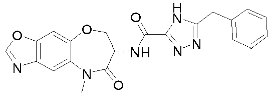 | RIP1-219 | 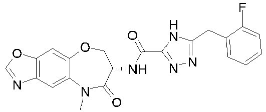 |
| RIP1-220 | 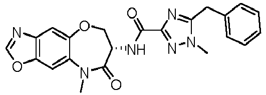 | RIP1-221 | 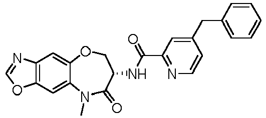 |
| RIP1-222 | 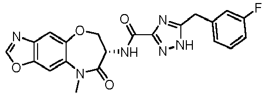 | | |

20. A method for preparing the compound according to claim 1, 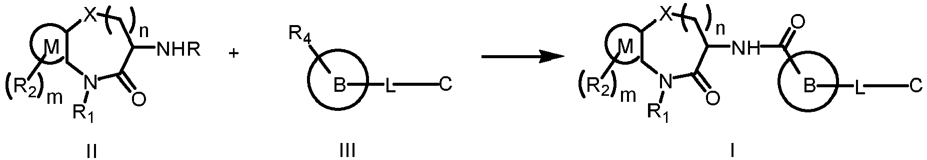 wherein R₄ is -COOH or -COO⁻G⁺, in which G⁺ is an alkali metal ion;
when R is H, the method comprises: reacting a compound of Formula (II) with a compound of Formula (III) in an inert solvent in the presence of a condensation reagent and a base, to obtain the compound of Formula (I) according to claim 1; and
when R is an amino protecting group, the method comprises: removing R from the compound of Formula (II) under an acidic condition, and then reacting the compound of Formula (II) from which R is removed with the compound of Formula (III) in an inert solvent in the presence of a condensation reagent and a base, to obtain the compound of Formula (I) according to claim 1.

21. A pharmaceutical composition, comprising a therapeutically effective amount of the compound of Formula (I) or a pharmaceutically acceptable salt thereof according to claim 1 and a pharmaceutically acceptable carrier.

22. Use of the compound or the pharmaceutically acceptable salt thereof according to claim 1 or the pharmaceutical composition according to claim 21 in the manufacture of a medicament for treating or preventing RIP1 kinase-mediated diseases or disorders or diseases or disorders caused by programmed cell necrosis.

23. The use according to claim 22, wherein the RIP1 kinase-mediated diseases or disorders or diseases or disorders caused by programmed cell necrosis are selected from the group consisting of inflammatory bowel disease, Crohn's disease, ulcerative colitis, psoriasis, retinal degenerative disease, retinal detachment, retinitis pigmentosa, macular degeneration, pancreatitis, atopic dermatitis, rheumatoid arthritis, spondyloarthritis, gout, SoJIA, systemic lupus erythematosus, Sjogren's syndrome, systemic scleroderma, antiphospholipid syndrome, vasculitis, osteoarthritis, non-alcoholic steatohepatitis, alcoholic steatohepatitis, autoimmune hepatitis, hepatitis B, hepatitis C, autoimmune hepatobiliary disease, primary sclerosing cholangitis, acetaminophen poisoning, liver toxicity, nephritis, celiac disease, autoimmune ITP, transplant rejection, ischemia-reperfusion injury of solid organs, sepsis, systemic inflammatory response syndrome, cerebrovascular accident, myocardial infarction, Huntington's disease, Alzheimer's disease, Parkinson's disease, allergic disease, asthma, atopic dermatitis, multiple sclerosis, Diabetes Type I, Wegener's granuloma, pulmonary sarcoidosis, Behcet's disease, Interleukin-1 converting enzyme-related fever syndrome, chronic obstructive pulmonary disease, tumor necrosis factor receptor related periodic syndrome, periodontitis, stroke, burns, burn shock, traumatic brain injury, atherosclerosis, cisplatin-induced kidney injury, acute kidney injury, pancreatitis, chronic kidney disease, acute respiratory distress syndrome, chronic obstructive pulmonary disease, Gaucher disease, Niemann Pick's disease, acute liver failure, cancers (e.g. pancreatic cancer), bacterial infection, smoking-induced injury, cystic fibrosis, NF-κ-B key regulatory gene mutation, heme-oxidized IRP2 ubiquitin ligase-1 deficiency, chain ubiquitin chain assembly complex deficiency syndrome, hematological malignancies, solid organ malignancies, influenza, staphylococcal infections, mycobacterial infections, lysosomal storage diseases, GM2 gangliosidosis, α-mannosidosis, aspartylglucosaminuria, cholesterol ester storage disease, chronic hexosaminidase A deficiency, cystinosis, Danon disease, Fabry disease, Farber disease, fucosidosis, galactosialidosis, GM1 gangliosidosis, mucolipid accumulation, infantile free sialic acid storage disease, juvenile hexosaminidase A deficiency, Krabbe disease, lysosomal acid lipase deficiency, metachromatic leukodystrophy, mucopolysaccharidosis, multiple sulfatase deficiency, neuronal ceroid lipofuscinoses, Pompe disease, pycondysostosis, Sandhoffs Disease, Schindler's Disease, Salla disease, Tay-Sachs disease, Wolman disease, Stevens-Johnson syndrome, and toxic epidermal necrolysis.

24. A method for inhibiting RIP1 kinase in a subject, comprising administering to the subject an effective amount of the compound or a pharmaceutically acceptable salt thereof according to claim 1, or the pharmaceutical composition according to claim 21.

25. A combination of drugs, comprising (a) the compound or a pharmaceutically acceptable salt thereof according to claim 1; and (b) at least one additional active agent.

26. The combination of drugs according to claim 24, wherein the at least one additional active agent is selected from a thrombolytic agent, a tissue-type plasminogen activator, an anticoagulant, a platelet aggregation inhibitor, an antimicrobial agent (antibiotics, broad-spectrum antibiotics, β-lactam, anti-mycobacterial drugs, bactericidal antibiotics, and anti-MRSA therapy), a long-acting beta agonist, combination of an inhalation corticosteroid and a long-acting beta agonist, a short-acting beta agonist, a leukotriene modulator, anti-IgE, a methylxanthine bronchodilator, a mast cell inhibitor, a protein tyrosine kinase inhibitor, a CRTH2/D-type prostaglandin receptor antagonist, an adrenaline inhalation aerosol, a phosphodiesterase inhibitor, combination of a phosphodiesterase-3 inhibitor and a phosphodiesterase-4 inhibitor, a long-acting inhalation anticholinergic drug, a muscarinic antagonist, a long-acting muscarinic antagonist, a low-dose steroids, an inhalation corticosteroid, an oral corticosteroid, a topical corticosteroid, an antithymocyte globulin, thalidomide, chlorambucil, a calcium channel blocker, a topical skin moisturizer, an ACE inhibitor, a serotonin reuptake inhibitor, an endothelin-1 receptor inhibitor, an anti-fibrosis agent, a proton pump inhibitor, cystic fibrosis transmembrane conductance regulator, mucolytics, a pancreatin, a bronchodilator, an intravitreal injection, an anti-vascular endothelial growth factor inhibitor, a ciliary neurotrophic growth factor, a trivalent (IIV3) inactivated influenza vaccine, a quadrivalent (IIV4) inactivated influenza vaccine, a trivalent recombinant influenza vaccine, a tetravalent live attenuated influenza vaccine, an antiviral agent, an inactivated influenza vaccine, a ciliary neurotrophic growth factor, a gene transfer agent, an immunomodulator, a calcineurin inhibitor, interferon γ, antihistamine, a PD-1 inhibitor, a PD-L1 inhibitor, a monoclonal antibody, a polyclonal anti-T cell antibody, an anti-thymocyte gamma globulin horse antibody, an anti-thymocyte globulin rabbit antibody, an anti-CD40 antagonist, a JAK inhibitor and an anti-TCR mouse mAb.

27. An intermediate compound of Formula (II): 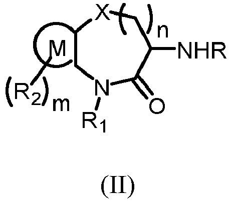 wherein:
R is H or an amino protecting group;
X is O, S or CH₂;
R₁ is selected from the group consisting of H and substituted or unsubstituted C₁-C₆ alkyl;
R₂ is selected from the group consisting of H, halo, hydroxyl, oxy, benzyl, substituted or unsubstituted C₁-C₆ alkyl, substituted or unsubstituted C₁-C₆ alkoxy, and C₁-C₆ acyl;
m is 0, 1, 2 or 3; and
n is 1, 2 or 3;
wherein "substituted" refers to the substitution of one or more hydrogen atoms on the group with a substituent selected from the group consisting of halo, cyano, alkyl, acyl, sulfonyl, hydroxyl, amino, benzyl, oxy, (C₁-C₄) alkyl, halo(C₁-C₄) alkyl, (C₁-C₄)alkoxy, hal0(C₁-C₄)alkoxy, nitro, and (C₁-C₄)alkylC(O)-.

28. The intermediate compound according to claim 27, selected from the group consisting of 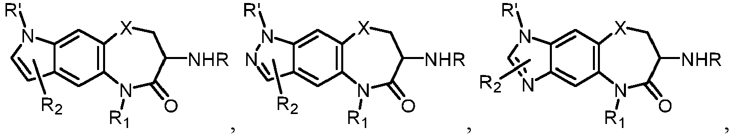 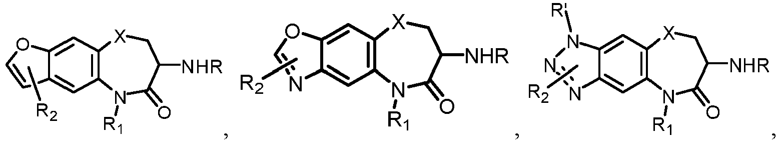 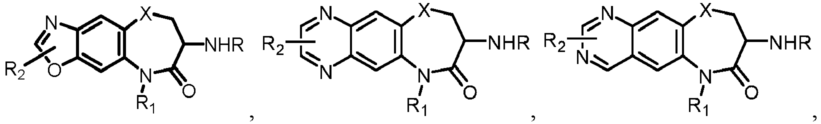 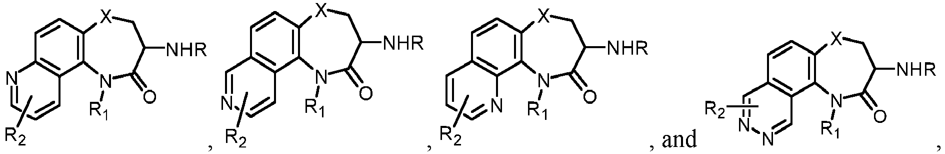 wherein R' is selected from the group consisting of H, Boc, SEM, (C₁-C₄) alkyl and benzyl.
